# EUROPEAN PATENT APPLICATION

(11) **EP 1 652 929 A1**
(43) Date of publication of application: **03.05.2006**
(21) Application number: 04090409.6
(22) Date of filing: 27.10.2004
(51) Int. Cl.: C12N 15/76, C12N 1/20, C12N 15/52, C12N 15/09

(54) **Plasmids for gene transfer by bacterial conjugation**

(71) Applicant: Combinature Biopharm AG, 13125 Berlin (DE); Max-Planck-Gesellschaft zur Förderung der Wissenschaften e.V., 80539 München (DE)
(72) Inventor: Lanka, Erich, D-14195 Berlin (DE); Blaesing, Franca, D-14195 Berlin (DE); Zieglein, Günther, D-14195 Berlin (DE); Mühlenweg, Agnes, Combinature Biopharm AG, D-13125 Berlin (DE); Pelzer, Stefan, Combinature Biopharm AG, D-13125 Berlin (DE)
(74) Representative: Jungblut, Bernhard Jakob

(57) **Abstract**

The invention relates to a novel isolated recombinant plasmid comprising the pSB102 Tra region, or comprising the *mob*/*rep* regions of RSF1010 or pTF-FC2. The invention further relates to a cell comprising such a plasmid, to methods for gene transfer utilizing such cells and to methods for producing derivatives of a compound obtained by biosynthesis or for producing an enzyme.

## Description

### Field of the invention

The invention relates to novel isolated recombinant plasmids and methods of use thereof for gene transfer by bacterial conjugation.

### Background of the invention and state of the art.

Bacterial conjugation is a powerful tool for gene transfer into various bacterial species including the genus *Streptomyces,* the most important industrial producers of antibiotics and other pharmacologically relevant secondary metabolites. The technique allows the identification and functional analysis of biosynthetic pathways for pharmaceutical interesting metabolites. The technique further allows the targeted manipulation of the biosynthesis by pathway engineering of homologous and heterologous genes, a strategy, which is called combinatorial biosynthesis.

In the document Trieu-Cuot et al., FEMS Microbiol. Lett. 48:289-294 (1987) conjugative transfer of a shuttle plasmid between Gram-negative *E. coli* strains and a variety of Gram-positive bacteria is described. A protocol for intergeneric transfer of plasmids from *E. coli* to *Streptomyces spp.* is disclosed in the document Mazodier, P. et al., J. Bacteriol. 171:3583-3585 (1989). This method has been used successfully with a number of different *Streptomyces* strains and other Actinomycetes as *Amycolatopsis, Actinoplanes, Nonomuraea, Saccharopolyspora, Actinomadura, Micromonospora, Nocardia, Rhodococcus,* but also with strains of the amino acid producing genera *Corynebacterium.* Moreover, this system was also adapted for the conjugative transfer of DNA from E. *coli* to the Gram-negative Myxobacteria like *Sorangium cellulosum,* which have gained attention as a valuable source of important natural products including epothilone.

A series of cloning vectors for the conjugative transfer of DNA from E. *coli* to *Streptomyces spp.* have been described in the document Bierman, M., R. et al., Gene 116:43-49 (1992), including non-replicating, autonomously replicating and integrating plasmid or cosmid vectors. Several other vectors which can be transferred from E. *coli* to Actinomycetes have been described (Smokvina et *al.,* Gene 94:53-59.(1990); Motamedi et *al.,* Gene 160:25-31 (1995); Voeykova et *al.,* FEMS Microbiol. Lett. 162:47-52 (1998)). However, all conjugation systems developed so far are based on the IncPα (RP4) transfer system. The system is known to be efficient, mediating effective gene transfer not only between different bacterial species but also into other cell types such as yeast and mammalian cells.

However, the insofar known conjugation systems are not useable for all Actinomycetes strains since some of these strains are not accessible to these techniques. Thus, there is a demand for alternative conjugation systems and novel plasmids for use therein for accessing such heretofore not accessible strains.

### Objects of the invention.

It is an object of the invention to provide novel plasmids for gene transfer by bacterial conjugation. It is a further object of the invention to provide novel plasmids which allow access to gene transfer to cells, like bacterial strains, not accessible to known conjugation systems. It is an even further object of the invention to provide novel cells and methods for gene transfer by bacterial conjugation.

### Summary of the invention and preferred embodiments

These objects are achieved by providing an isolated recombinant plasmid comprising the pSB102 Tra region. Such a plasmid allows for gene transfer to Actinomycetes strains, which are not accessible to the IncPα (RP4) transfer system. Thus, e.g. novel antibiotics may be obtainable by genetically manipulating synthesis pathways of such Actinomycetes strains.

The plasmid of the invention may further comprise one, several, or all of the elements of the group consisting of "lacI^{q} gene of pGZ119EH, promoter Ptac of pGZ119EH, rrnBT1T² terminator of pGZ119EH, ColD origin of replication of pGZ119EH, tetA gene of RP4 with original promoter".

The plasmid may comprise in particular the 31,5 kb XbaI-XbaI fragment of pSB102, wherein the Tra region is located.

The plasmid of the invention is obtainable in that a 31,5 kb XbaI-XbaI fragment of pSB102 containing the Tra region is inserted into the multi-cloning site of pGZ119EH/HE (the term HE/EH expresses that either of the variant EH or HE may be used).

The invention further comprises an isolated recombinant plasmid comprising the mob/rep regions of RSF1010 or pTF-FC2. This may further comprise one, several, or all of the elements of the group consisting of "lacI^{q} gene of pGZ119EH, promoter Ptac of pGZ119EH, rrnBT1T² terminator of pGZ119EH, Tra2 region of RP4, tetA gene with original promoter of RP4". It is obtainable in that A) the RSF1010A1 replicon of pGZ219 is substituted with the mob/rep regions of RSF1010 or pTF-FC2, and B) RP4 Tra2 genes, traF and traG, of pML123 and of pML100 are inserted into the multi-cloning site of the product of step A).

Another aspect of the invention is a cell, in particular an Enterobacterium, preferably E. *coli,* containing at least one plasmid of the invention. Optionally it may contain in addition at least one recombinant integrative or non-integrative oriT plasmid (being co-resident with the first plasmid), wherein the *oriT* plasmid comprises a nucleic acid sequence, which is intended to be transferred to a recipient cell of a different strain. The *oriT* plasmid may be any plasmid being compatible with the aforementioned plasmids of the invention.

The *oriT* plasmid may, in particular, comprise a defined gene coding for a defined peptide or protein, the gene intended for transfer from the cell as donor cell to a recipient cell of a different strain, wherein the gene comprises a defined nucleic acid sequence coding for a modified or unmodified (but enhanced) biosynthesis step in the recipient cell.

The integrative *oriT* plasmid may be any integrative plasmid, which integrates via a site specific integration. It is e.g. selected from the group consisting of "pSET152, pSEToriT, pSET101, pSET201, and pSEToriT102".

The non-integrative *oriT* plasmid may be any plasmid that is replicable in the recipient cell. It is e.g. selected from the group consisting of "RSF1010K, pMS713-1n, pOJ436, and pUWLoriTaph". The defined foreign nucleic acid sequence is inserted into said *oriT* plasmid at an appropriate insertion site.

An other aspect of the invention comprises a method for intergeneric gene transfer, wherein a donor cell of the invention is contacted with a recipient cell, and wherein the integrative or non-integrative *oriT* plasmid is transferred from the donor cell to the recipient cell by conjugation. The donor cell may be any Enterobacterium, e.g. *E. coli*. The recipient cell may be any prokaryotic or eukaryotic cell. Examples are Actinomycetes, in particular Streptomycetes, Myxobacteria, Yeasts, plant and animal cells, like mammal cells (including human cells, but optionally excluding omnipotent human cells). The *oriT* plasmid may comprise a defined nucleic acid sequence or gene. Non-limiting examples for such defined genes are provided in the following embodiments.

The *oriT* plasmid may comprise one heterologeous (with respect to the recipient cell) gene or several heterologeous (with respect to the recipient cell) genes coding for at least one enzyme. The enzyme may then be extracted and isolated from the recipient cell and, optionally, purified. Such isolated enzyme may, e.g. be used for biocatalysis purposes, by adding to a cell culture. The enzyme may also be left in the cell, in particular in case that it is involved in the biosynthesis of metabolites or substances in the recipient cell. Then these metabolites or substances are isolated from the recipient cell culture, optionally after having cultured the recipient cells. The substances or metabolites may be modified or derivatized, if compared to the substances or metabolites biosynthesized by the recipient cells without gene transfer (combinatorial biosynthesis). The heterologeous gene may also be used for biotransformation of the recipient cell.

The oriT plasmid may as well comprise one homologeous (with respect to the recipient cell) gene or several homologeous (with respect to the recipient cell) genes coding for at least one enzyme of the recipient cell. A homologeous gene is a gene, which is identical with a gene naturally present in the recipient cell or differing therefrom but having the same biochemical functionality. This embodiment is useful e.g. for influencing, in particular enhancing, the quantity and/or quality of the biosynthesis of an enzyme or substance in the recipient cell, e.g. by introduction of one or more regulators or for the bypass of bottle-necks of the naturally occuring biosynthesis in the recipient cell.

The *oriT* plasmid may comprise at least one heterologeous or homologeous gene or gene fragment with significant similarities to a gene of the recipient cell or at least one homologeous gene or gene fragment with a mutation (insert, deletion, exchange of at least one nucleotide), wherein the mutation renders the gene disfunctional in the biosynthesis of the recipient cell. This may be utilized for pathway engineering or functional proof, e.g. by way of knock-out experiments.

The oriT plasmid may also comprise one or several mobile elements, like transposons, which are used for a transposon mutagenesis of the recipient cell. The oriT plasmid may as well be used for the curing of plasmids resident in the recipient cell.

A specific aspect of the invention relates to a method for producing a derivative of a defined compound, the compound being obtainable by biosynthesis in a recipient cell comprising the following steps: a) producing a donor cell of the invention, wherein the defined foreign nucleic acid sequence codes for a protein or peptide not naturally occurring in the recipient cell, wherein the protein or peptide effects a modified biosynthesis step in the biosynthesis of the recipient cell being different from the naturally occurring biosynthesis step of the same recipient cell and resulting in a derivative of the defined compound being produced in the modified biosynthesis, b) contacting the donor cell with the recipient cell, wherein the defined foreign nucleic acid is transferred to the recipient cell, c) effecting expression of the defined foreign nucleic acid in the recipient cell, d) culturing the recipient cell, and e) isolating the derivative of the defined compound from a culture supernatant or from an extract of the cultured recipient cell. In this aspect it must be emphasized that the invention is not limited to specific modifications of naturally occuring biosynthesis processes. In contrast, actually any biosynthesis process can be modified to produce a derivative of a compound produced by the wild type recipient cell (or recombinant recipient being already modified in another biosynthesis step of the same biosynthesis), provided that the biosynthesis process, or at least defined steps therein, are known, including the involved peptides, proteins or enzymes, in order to substitute the involved peptides, proteins or enzymes by different ones with the method of the invention, finally yielding the wished derivative.

Thus, the modified biosynthesis step results in the synthesis of a compound or intermediate product different from the naturally synthesized compound or intermediate product in the wild type recipient cell. The recipient cell may be selected from Actinomycetes, in particular Streptomycetes.

In the following the invention is explained by Examples which are intended to describe specific, but non-limiting embodiments.

### Example 1: materials and methods

Bacterial strains and media: *E. coli* strain SCS1 [F⁻, *endA1, hsdR17(*r_{K}^{*-*}*,* m_{K}⁺) , *supE44, thi-1, λ*^{*-*}*, recA1, gyrA96, relA1;* Stratagene] was used for all cloning procedures. *E. coli* strain DH5α[F⁻, φ80d*lac*ZΔM15, Δ*(lacZYA-argF),* U169, *recA1, endA1, hsdR17(*r_{K}^{*-*}*,* m_{K}⁺), *phoA, supE44, λ*^{*-*}*, thi-1, gyrA96, relA1;* Hanahan, D., J. Mol. Biol. 166:557-580 (1983)] was used as donor in intrageneric conjugations and *E. coli* strain GM2163 [F⁻, *ara-14, leuB6, fhuA31, lacY1, tsx78, glnV44, galK2, galT22, mcrA, dcm-6, hisG4, rfbD1, rpsL136* (Sm^{r}) , *dam13::Tn9* (Cm^{r}) *, xylA5, mtl-1, thi-1, mcrB1, hsdR2;* NEB] as recipient in intrageneric conjugations and as donor strain in intergeneric conjugations. E. *coli* strains HB101 [F⁻, Δ*(mcrC-mrr), hsdS20, recA13, ara-14, proA2, lacY1, λ*^{*-*}*, galK2, rpsL20* (Sm^{r}), *xyl-5, mtl-1, supE44* (Boyer, H. W. et al., J. Mol. Biol. 41:459-472 (1969)] and the nalidixic acid-resistant derivative HB101Nx^{r} were additionally applied for intrageneric matings. Recipients in intergeneric conjugations were the following Actinomycetes strains: *Streptomyces aureofaciens* Tü13 *(Streptomyces ambofaciens* DSM 40697); *Streptomyces coelicolor* A3(2)M145 (SCP1-, SCP2-; Kieser *et al.*, Practical *Streptomyces* Genetics. John Innes Foundation, Norwich (2000)); *Amycolatopsis japonicum* MG417-CF17 *(Amycolatopsis japonica.* DSM 44213; Stegmann et *al.,* J. Biotechnol. 92:195-204 (2001)); *Streptomyces lividans* 66 TK24 *(str-6,* SLP2-, SLP3-; Kieser *et al.*, Practical *Streptomyces* Genetics. John Innes Foundation, Norwich (2000)); *Streptomyces diastatochromogenes* Tü6028 (Paululat et *al.,* J. Antibiot. (Tokyo) 52:96-101 (1999)). *E. coli* cells were grown in YT medium (Miller, J.H., Experiments in molecular genetics. Cold Spring Harbor Laboratory, Cold Spring Harbor, N.Y.,(1972)) or LB broth (Sambrook et *al.,* Molecular cloning: a laboratory manual, 2nd ed. Cold Spring Harbor Laboratory, Cold Spring Harbor, N.Y., (1989)) buffered with 25 mM 3-(N-morpholino)propanesulfonic acid (sodium salt, pH 8,0) and supplemented with 25 µg thiamine-hydrochloride per ml and glucose (0,1%). If appropriate, antibiotics were used at the following concentrations: tetracycline hydrochloride (Tc), 10 µg/ml; kanamycin sulfate (Km), 30 µg/ml; chloramphenicol (Cm), 10 µg/ml, apramycin sulfate (Am), 25 µg/ml, nalidixic acid, sodium salt (Nx), 30 µg/ml. The final concentration of HgCl₂ for selective growth of plasmid pSB102 was 20 µg/ml. MS medium (Hobbs et *al.,* Appl. Microbiol. Biotechnol. 31:272-277 (1989)) containing 10 mM MgCl₂ was used for plating of *E. coli*/*Streptomyces spp.* in intergeneric conjugation experiments. Exconjugants were further cultured on selective HA medium (4 g Bacto yeast extract, 10 g malt extract, 4 g glucose-monohydrate per litre; pH 7,3) with antibiotics added in the following concentrations: phosphomycin disodium salt (Pm) 400 µg/ml and Am, 25 µg/ml (selection of pSET vectors) or Km, 50 µg/ml (selection of pUWLoriTaph and RSF1010K, respectively). Actinomycetes cultures for DNA isolation were grown in CRM Medium (10 g Bacto yeast extract, 20 g tryptone soy broth, 103 g sucrose per liter and 20 ml 2,5 M mgcl₂ solution to be added to the sterilized medium) containing 0.5% glycine, 400 µg/ml Pm and the appropiate selective antibiotics (see HA medium).

Plasmids and oligonucleotides: Plasmids used in this study are listed in Table 1. Oligonucleotides used for molecular cloning, PCR analysis or nucleotide sequencing are listed in Table 2.

DNA techniques: Standard molecular cloning techniques were performed as described (Sambrook et *al.,* see above). PCR fragments were generated using the following polymerases under conditions recommended by the manufacturer: Deep Vent_{R} DNA polymerase (New England Biolabs); ProofStart DNA polymerase (Qiagen) or *Taq* DNA polymerase (Qiagen). Constructions were verified by DNA sequence analysis.

Intrageneric conjugation procedure: For quantitative filter matings defined amounts of donor cells (0,5 ml, 1 x 10⁸ cells per ml) and GM2163 recipient cells (5 ml, 3-4 x 10⁸ cells per ml) were mixed and collected onto a cellulose nitrate filter (0,45 µm pore size, 25 mm diameter; Sartorius). The filter was incubated for 1h at 30°C on a YT agar plate without selection. Cells were resuspended, and aliquots of serial 10-fold dilutions were plated on YT agar plates with 10 µg/ml of chloramphenicol for selection of GM2163 plus the appropriate antibiotic for selection of the plasmid to be transferred. The transfer frequency is the number of exconjugants per donor cell.

Intergeneric conjugation procedure: conjugation of mobilizable plasmids from *E. coli* into different Actinomycetes was essentially done as described (Flett *et al.*, FEMS Microbiol. Lett. 155:223-229 (1997); Kieser *et al.*, see above (2000)). *E. coli* donor cells were grown to an A₆₀₀ of 0,5. Cultures were inoculated from single colonies rather than diluted from overnight cultures because of extremely long lag phases of stationary phase donor cells. Cells were harvested by centrifugation, washed twice with LB to remove residual antibiotics and resuspended 10-fold concentrated in LB. The donor cells were counted prior to use. Actinomycetes spore suspensions stored at -80°C were used as recipients. For each mating experiment, 10⁸ Actinomycetes spores were added to 500 µl 2x YT broth, heat shocked for 10 min at 50°C to activate germination and cooled to room temperature. An aliquot of 500 µl of *E. coli* cells was added and the mixture centrifuged for 2 min at 1000 x g. A defined volume of 900 µl of the supernatant was removed and the bacterial pellet was resuspended in the remaining volume of approximately 100 µl.

Serial 10-fold dilutions of the mixture were spread on MS agar containing 10 mM MgCl₂. The plates were incubated for 16-20 h at 29°C and then overlaid with 3 ml soft nutrient agar containing 1 mg Pm for counterselection against *E. coli* and the appropriate antibiotic for exconjugant selection (1 mg Am and 0,75 mg Km, respectively). Incubation was continued for 2-5 days until exconjugants appeared. The transfer frequency is the number of exconjugants per recipient spore. Exconjugants were further cultured on selective HA medium and analyzed by specific PCR or plasmid DNA isolation (see below).

Isolation of DNA from exconjugants: Genomic Actinomycetes DNA was isolated using the Nucleospin Tissue Kit (Macherey & Nagel) according to the product manual for Gram-positive strains with the following modifications: An aliquot of 2 ml of Actinomycetes culture was used. Cells were harvested by centrifugation and washed once with 500 µl of TE/10% sucrose and stored at -20°C for further processing. The incubation step at 56°C after adding of Proteinase K solution was omitted. Samples were gently mixed rather than vortexed. An aliquot of 600 µl of buffer B5 was used instead of 500 µl. DNA was eluted with 200 µl of buffer BE. Plasmid DNA was isolated from Actinomycetes cells by alkaline lysis essentially as described (Birnboim, H. D. et al., Nucleic Acids Res. 7:1513-1523 (1979); Sambrook et *al.,* see above (1989)) with the following modifications: Cells were washed and stored until further processing as described for genomic DNA isolation. The resuspension buffer (50 mM glucose; 25 mM Tris-HC1; 10 mM EDTA; pH 8,0) contained lysozyme (4 mg/ml). The cells were incubated for 30 min at 37°C before adding the alkaline buffer (0.4 N NaOH; 2% SDS). Incubation at room temperature was for 10 min. 3 M potassium acetate, pH 5,2, was used as neutralization buffer. The DNA pellet was resuspended in 50 µl TE buffer (10 mM Tris-HC1; 1 mM EDTA; pH 8,0).

### Example 2: selection of transfer systems and molecular cloning of essential Tra components

pUB307/pSET152 (Flett et *al.,* see above (1997)) served as a reference system for the evaluation of Tra systems used. Plasmid pUB307, a RP4 derivative lacking TnA (Bennett et *al.,* Mol. Gen. Genet. 154:205-211 (1977); Table 1) had to be modified such that it could be used with co-resident Km^{r} plasmids RSF1010K and pUWLoriTaph (see below). A unique HindIII site in the *aphA* gene of pUB307 was used for transformation of E. *coli* with linearized DNA to generate small deletions of the plasmid, yielding the Km^{s} plasmid pUB307ΔK. The plasmid lacks 137 bp, destroying the *aphA* reading frame (nucleotide positions 38.860 through 38.996 of plasmid RP4).

Potential alternatives for the IncPα Tra system to be used for conjugative DNA transfer into *Streptomyces* and related Actinomycetes have to be especially efficient in semisolid surface matings, since conjugative transfer in *Streptomyces* takes place only on solid media under prolonged physical contact of the parental strains. Three systems were selected: The mobilization (Mob) system of IncQ plasmid RSF1010, the Mob system of IncQ-like plasmid pTF-FC2 and the Tra system of pSB102, a plasmid recently isolated from the microbial population of the alfalfa rhizosphere (Schneiker et *al.,* Nucleic Acids Res.
29:5169-5181 (2001)). The selection was done due to the following considerations: RSF1010 and pTF-FC2 are both broad-host-range plasmids. They are known to be highly mobilizable and possess well-characterized Mob functions. In contrast, pSB102 belongs to an as yet undescribed group of broad-host-range plasmids. The putative Mpf (mating pair formation) and Dtr (DNA transfer and replication) genes form a compact cluster, which makes the system appropriate for the cloning procedures required.

Although not self-transmissible, RSF1010 is mobilized at high frequency to a large number of hosts in the presence of conjugative helper plasmids, such as RP4 of the *E. coli* IncPα and R751 of the Incpß group. Transfer of the plasmid is known for a wide range of Gram-negative bacteria and several Gram-positive bacteria including *Streptomyces lividans* and *Mycobacterium smegmatis,* as well as plant and animal cells. Likewise, pTF-FC2 has been mobilized at high frequency between *Escherichia coli* strains by the co-resident IncPα RP4 plasmid, from *E. coli* to *Pseudomonas fluorescens* and from *Agrobacterium tumefaciens* to plant cells.

Conjugative transfer of pSB102 is known for *Sinorhizobium meliloti* L331, *Pseudomonas sp* B131 and *E. coli* HBR101 with *S. meliloti* FP2 as the donor strain.

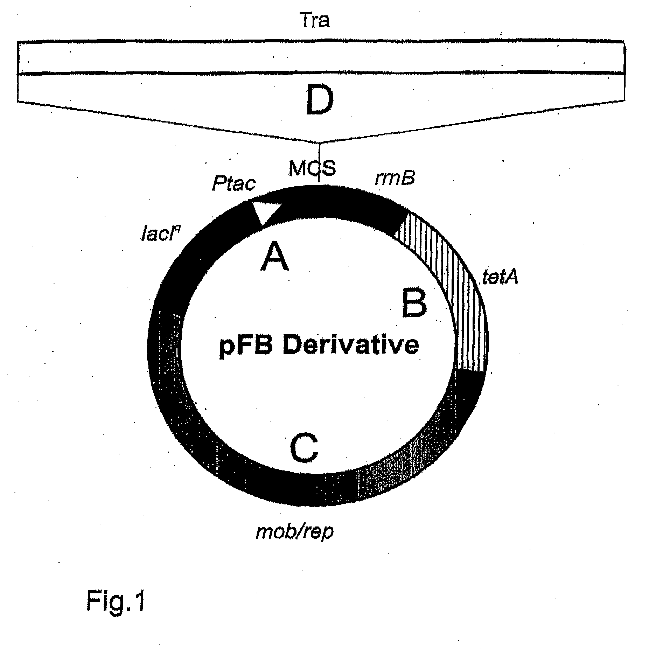

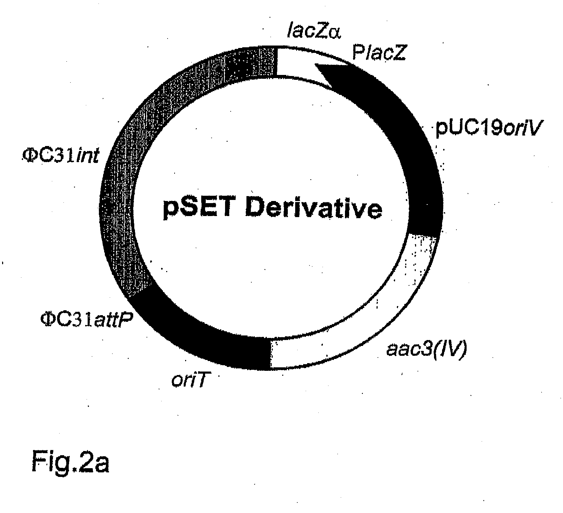

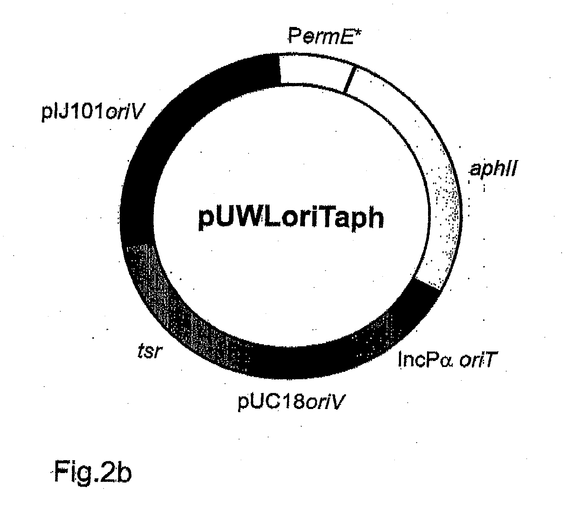

For conjugation experiments, binary vector systems were constructed with the subset of Tra systems. *OriT* sequences as the only cis-acting elements necessary for plasmid transfer were positioned on one plasmid, genes for transfer proteins required for the formation of the relaxosome and subsequent translocation of the DNA to the recipient cell together are provided on co-resident helper plasmids. The physical structure of the conjugative helper plasmids together with an outline of the cloning strategy is given in Figure 1 and the following. Plasmid pMS119EH (Balzer et *al.,* Nucleic Acids Res. 20: 1851-1858 (1992)) was the basis for the construction of conjugative helper plasmids used. The plasmid contains the *lacI*^{q} gene, the tac promoter, a multicloning site and the *rrnBT1*/*T2* terminator (A). The bla gene of pJF119EH was replaced by the tetracycline resistance gene of plasmid RP4 (tetA with its original promoter, RP4 nucleotide positions 13.921 through 15.273; B). For the construction of RSF1010 and pTF-FC2 helper plasmids, the pMB1 replicon was replaced by the corresponding mob/rep regions (Scholz *et al.*, Gene 75:271-288 (1989); Rohrer, J., and D. E. Rawlings, J. Bacteriol. 174:6230-6237 (1992); C). For the construction of the pSB102 helper plasmid, ColD, derived from plasmid pGZ119 (Lessl et *al.,* J. Bacteriol. 174:2493-2500 (1992)), was used as the replicon (C). The RP4 Tra2 region and the genes *traF* and *traG*, required for mobilization of the non-conjugative plasmids RSF1010 and pTF-FC2, were inserted into the multicloning site, yielding plasmids pFB1124 and pFB1224, respectively (D). The putative Tra region of pSB102 located on a 31,5kb XbaI fragment was inserted into the multicloning site, yielding plasmid pFB714 (D). pFB1124 and pFB1224 carry the mobilization and replication functions of plasmids RSF1010 and pTF-FC2, respectively, and the RP4 Tra2 region including the genes *traF* and *traG,* which are required for mobilization of the non-conjugative plasmids RSF1010 and pTF-FC2. pFB714 carries the putative Tra region of plasmid pSB102, which is located on a 31,5kb XbaI fragment. The physical structure of the *oriT* plasmids is given in Figures 2A and 2B and the following. Figure 2A shows *oriT* plasmids integrating into the Actinomycetes host chromosome by site-specific recombination. pSET152 (Bierman et *al.,* see above (1992)) provided the basis for each of the integrative *oriT* plasmids. The plasmids consist of the gene fragment encoding the LacZα peptide, a multi-cloning site, the replicon of plasmid pUC19, the attachment site *attP* and integrase functions int of the temperate phage φC31 for site-specific integration into the recipient Actinomycete chromosome and the apramycin resistance marker *aac3(IV),* allowing for selection in both E. *coli* and Actinomycetes. The 0,8kb IncPα *oriT* fragment of plasmid pSET152 was replaced by the respective *oriT* sequences, which were inserted into the single *SphI* site (pSET152 nucleotide position 3.606). Insertion of the 38 bp RSF1010 *oriT* (Brasch, M. A., and R. J. Meyer, J. Mol. Biol. 198:361-369 (1987)) yielded plasmid pSET101. Insertion of the 143 bp pTF-FC2 *oriT* (Rohrer & Rawlings, see above (1992)) yielded plasmid pSET201. The putative pSB102 *oriT* is located on a 2,36kb HindIII fragment (pSB102 positions 9.863-12.225; EMBL nucleotide sequence database, accession no. AJ304453). Part of the fragment was amplified (pSB102 positions 11.064-11.494) for molecular cloning of the putative *oriT* region, yielding plasmid pSEToriT102. pSET101 was obtained with the RSF1010 sequence in both orientations (pSET101n and u, respectively), while pSET201 contained the pTF-FC2 *oriT* sequence exclusively in the u orientation. Figure 2B shows an *oriT* plasmid autonomously replicating in the Streptomyces host. Plasmid pUWL201 (Doumith et *al.,* Mol. Gen. Genet. 264:477-485 (2000)) was the basis for the construction of an *oriT* plasmid autonomously replicating in *Streptomyces spp.* pUWL201 contributes the replication functions of the *Streptomyces* broad-host-range multicopy plasmid pIJ101 (Kieser et *al.,* Mol. Gen. Genet. 185:223-228 (1982)), the *tsr* gene conferring resistance to thiostreptone in *Streptomyces,* the promoter *PermE** (Bibb et *al.,* Mol. Microbiol. 14:533-545 (1994)) and the pUC18 origin for replication in *E. coli.* Insertion of the 0,8kb PstI fragment of plasmid pOJ436 (Bierman *et al.*, see above (1992)) containing the IncPα *oriT* generated pUWLoriT. Subsequent insertion of the *aphll* gene of plasmid pGM9 (Muth *et al.*, Mol. Gen. Genet. 219:341-348 (1989)) into the *bla* gene of pUWL201 yielded the plasmid pUWLoriTaph (Ap^{s}/Km^{r}). These two plasmids represent two different types. Derivatives of pSET152 do not replicate in *Streptomyces spp.* and related Actinomycetes but allow for site-specific integration in the host chromosome at the bacteriophage φC31 attachment site (Fig. 2A). These plasmids, pSET152 (RP4 *oriT),* pSET101 (RSF1010 *oriT) ,* pSET201 (pTF-FC2 *oriT)* and pSEToriT102 (pSB102 *oriT),* will be referred to as integrative *oriT* plasmids. A derivative of plasmid pUWL201 carrying the IncPα *oriT* of plasmid pOJ436 contains the replication functions of the *Streptomyces* broad-host-range multicopy plasmid pIJ101 allowing for autonomous replication in the host (Fig. 2B). This plasmid, pUWLoriTaph, will be referred to as non-integrative *oriT* plasmid. Detailed descriptions of the cloning procedures of both the helper and the *oriT* plasmids are given in Table 1 and in the following. The new constructs are listed in Table 3.

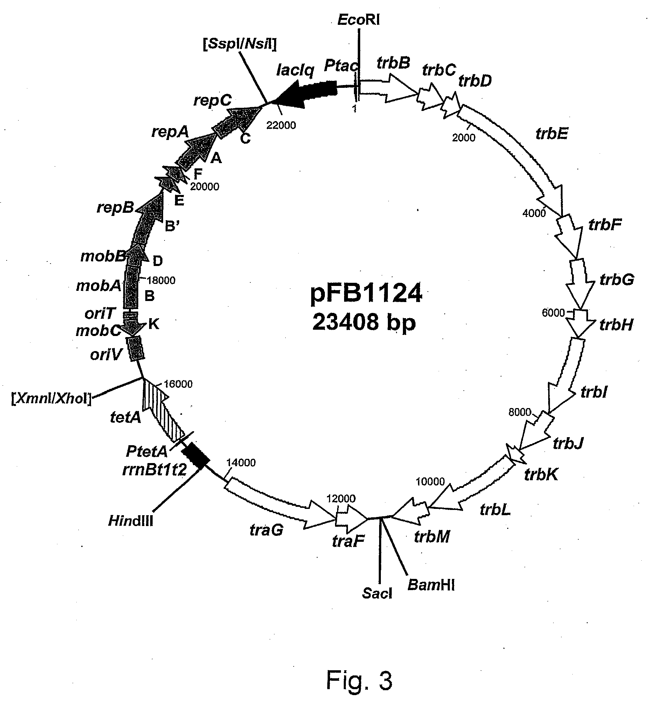

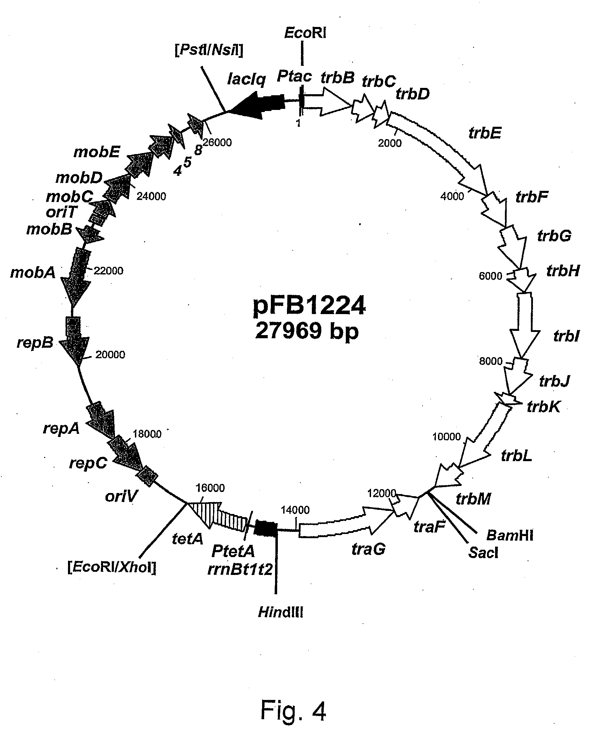

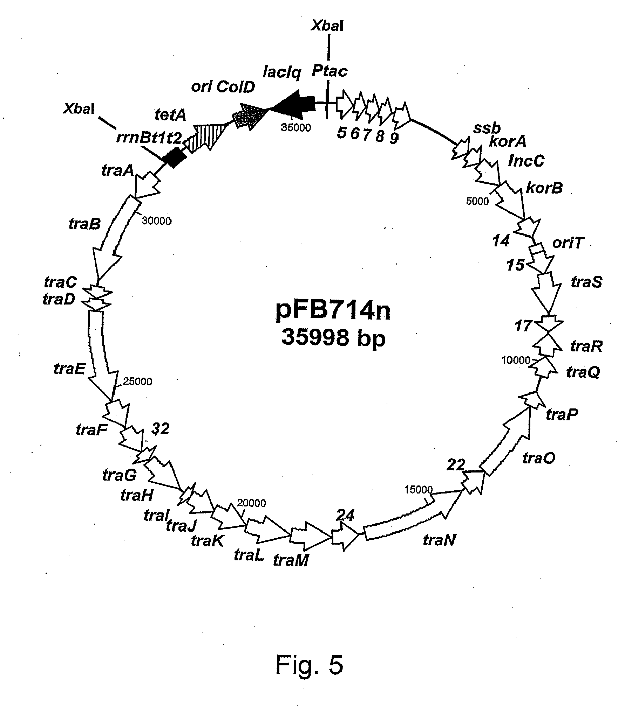

Construction of conjugative helper plasmids: a set of pGZ expression vectors based on pMS119 (Balzer et *al.,* see above (1992)) was used for the construction of conjugative helper plasmids pFB1124, pFB1224, and pFB714 (Figures 3, 4, and 5, respectively). Vector plasmids pGZ219 (Table 1), pGZ119 (Lessl et *al.,* see above (1992)), and pGZ1119 (Table 1) share the 2,06kb *Nru*I/*Bsp*HI fragment *(lacI*^{*q*}/P*tac*/MCS/*rrnBT1T2)* of pMS119EH (Balzer *et al.,* 1992). The vectors differ in replicon and selectable marker genes (Table 1). Expression vector pGZ219 (Table 1) was the basis for the construction of conjugative helper plasmids pFB1124 and pFB1224. The plasmid carries the *tetA* gene of RP4 as selectable marker. The 1,35kb DNA fragment containing the RP4 tetA gene together with its original promoter (Table 1) was fused to the *BspH*I site of the *(lacI*^{*q*}/*Ptac*/*MCS*/*rrnBT1T2)* cassette, previously treated with T4 DNA polymerase to generate a "blunt end". Primers used for the amplification of the RP4 tetA gene are listed in Table 2. The reverse primer introduced a single *Xho*I site to facilitate subsequent molecular cloning steps. A small linker (Table 2), introducing a *Nsi*I site, was fused to the *Nru*I site of the (*lacI*^{q}/P*tac*/MCS/*rrnBT1T2*) cassette. The construction of plasmids pFB1124 and pFB1224 (Figures 3 and 4, respectively) was done in two steps. First, the RSF1010Δ1 replicon of plasmid pGZ219 was substituted with the *mob*/*rep* regions of plasmids RSF1010 and pTF-FC2, yielding mobilizable vector plasmids pFB219 and pFB319, respectively. The RSF1010 mobilization and replication genes are located on a 5,6kb *Xmn*I/*Ssp*I fragment. The pTFFC2 mobilization and replication genes are located on a 10,2kb PstI/*Eco*RI fragment of pTF-FC2 derivative pDER412 (Rawlings et *al.,* J. Bacteriol. 158:737-738 (1984)). The *Nsi*I/*Xho*I *(lacI*^{*q*}/P*tac*/*MCS*/*rrnBTIT2*/*tetA)* cassette of plasmid pGZ219 and the respective *mob*/*rep* regions were treated with T4 DNA polymerase prior to ligation. As the second step, RP4 Tra2 genes, *traF* and *traG,* required for conjugative transfer, were inserted. Plasmids pML123 and pML100 (Less1 et *al.,* J. Bacteriol. 175:6415-6425 (1993)) carry the coding regions for the gene products (TrbB-M) and (TraF/TraG), respectively. To combine the essential *tra* functions on a single plasmid, pML123 was digested with *Bam*HI and *Hin*dIII, and the 3,1kb *Sac*I/*Hin*dIII fragment of pML100, carrying the *traF* and *traG* genes, was inserted via a *Bam*HI/*Sac*I adaptor. The resulting plasmid, pMS124, was digested with *Eco*RI and *Hin*dIII to obtain a 14,4kb fragment carrying the desired *tra* genes for insertion into the multi cloning sites of pFB219 and pFB319, respectively. Plasmid vector pGZ1119 (Table 1) was used for molecular cloning of the pSB102 *tra* functions. The 31,5kb *Xba*I fragment of pSB102 containing the putative Tra region was inserted into the multi-cloning site of pGZ1119, yielding plasmid pFB714 (Figure 5).

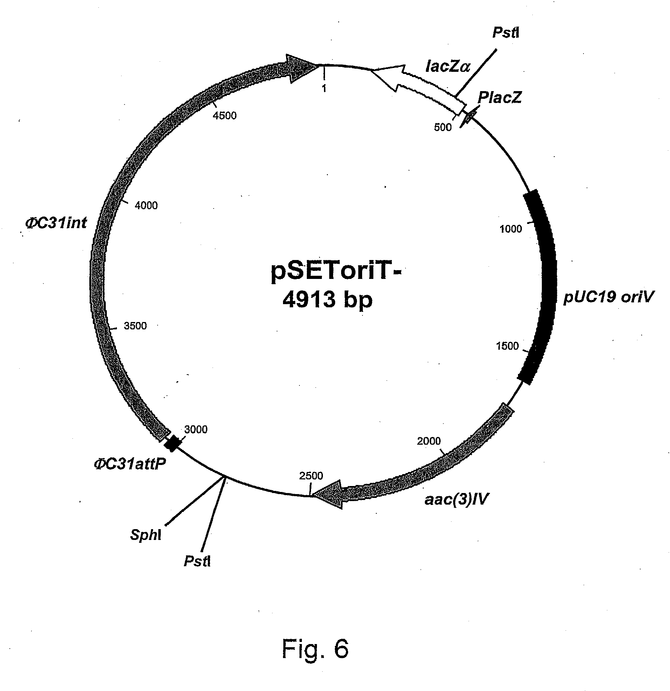

Construction of oriT plasmids suitable for site-specific integration at the ΦC31 attachment site: the integrative *oriT* plasmids used (Table 1) are based on plasmid pSET152 (Bierman et *al.,* see above (1992)). The IncPα *oriT* was removed by digesting pSET152 with *Pst*I*,* yielding three fragments one containing *oriT.* The two remaining fragments were isolated and religated yielding plasmid pSEToriT-(Figure 6). The selected *oriT* sequences were inserted into the single *SphI* site (pSET152 nucleotide position 3.606). A 44bp synthetic oligonucleotide (Table 2) containing the 38 bp minimal *oriT* of RSF1010 (Brasch & Meyer, see above (1987)) together with flanking *Sph*I sequences was used for molecular cloning of RSF1010 *oriT* plasmid pSET101. The 143 bp *oriT* sequence of plasmid pTFFC2 (Rohrer & Rawlings, see above (1992)) was amplified from plasmid pDER412 (Rawlings *et al.*, see above (1984)) with primers introducing *Sph*I restriction sites at both ends (Table 2). The PCR product was digested with *SphI* and inserted into pSEToriT⁻, yielding plasmid pSET201. The putative *oriT* sequence of plasmid pSB102 (Schneiker *et al.*, 2001) is located on a 2,36kb HindIII fragment. For molecular cloning of pSB102 *oriT,* a 0,43kb fragment containing the putative *oriT* sequence, was amplified (for primers used see Table 2) and inserted into the single *Hind*III site of vector pBR329. Following verification of the OriT⁺ phenotype, the resulting plasmid pMS713-1n served as a template for a second amplification of the *oriT* sequence, using primers introducing *Sph*I restriction sites for molecular cloning into *Sph*I-digested pSEToriT- (Table 2).

Construction of an orit plasmid suitable for autonomous replication in Streptomyces spp.: plasmid pUWL201 (Doumith *et al.*, see above (2000)) served as a backbone for the construction of nonintegrative *oriT* plasmid pUWLoriTaph (Table 1), which was achieved in two distinct cloning steps. First, the IncPα *oriT* from plasmid pOJ436 (Bierman *et al.*, see above (1992)), located on a 0,76kb *Pst*I fragment, was inserted into pUWL201. The *oriT* containing fragment was inserted into the PstI site of vector pBlueskriptSK(+). The derivative containing a unique *Sma*I site within the PstI fragment distal to the Bluescript *Sma*I site was used to cut out the *oriT* fragment with *Sma*I*.* The *Sma*I fragment was then inserted into the *Ssp*I site of pUWL201, yielding plasmid pUWLoriT. Second, the kanamycin resistance cassette *aphII*, amplified from plasmid pGM9 (Muth *et al.*, see above (1989)), was inserted as a bluntended fragment into the unique ScaI site of pUWLoriT. Primers for amplification of the *aph*II cassette are listed in Table 2.

Following details of the Figures 3 to 6 are provided.

Figure 3 shows the genetic map of conjugative helper plasmid pFB1124. The map is based on the following sequence data: The *lacI*^{q} gene, promoter Ptac and the *rrnBT1T2* terminator (black arrow and black bar, respectively) are derived from plasmid pMS119EH (Balzer *et al.*, see above (1992)). The mobilization and replication functions (grey arrows and grey boxes) are derived from plasmid RSF1010 (EMBL nucleotide sequence database, accession no. M28829). Genes *trbB-trbM, traF* and *traG* (white arrows) as well as the tetA gene (hatched arrow) with the original promoter are derived from plasmid RP4 (EMBL nucleotide sequence database, accession no. L27758). Restriction sites used for molecular cloning are indicated.

Figure 4 shows the genetic map of conjugative helper plasmid pFB1224. The map is based on the following sequence data: The *lacI*^{*q*} gene, promoter Ptac and the *rrnBT1T2* terminator (black arrow and black bar, respectively) are derived from plasmid pMS119EH (Balzer *et al.*, see above (1992)). The mobilization and replication functions (grey arrows and grey boxes) are derived from plasmid pTF-FC2 (EMBL nucleotide sequence database, accession no. M57717, M64981, M73777 and M26309). Genes *trbB-trbM, traF* and *traG* (white arrows) as well as the tetA gene (hatched arrow) with the original promoter are derived from plasmid RP4 (EMBL nucleotide sequence database, accession no. L27758). Restriction sites used for molecular cloning are indicated.

Figure 5 shows the genetic map of conjugative helper plasmid pFB714. The map is based on the following sequence data: The *lacI*^{*q*} gene, promoter *Ptac* and the *rrnBT1T2* terminator (black arrow and black bar, respectively) and the ColD origin of replication (grey arrow) are derived from plasmid pGZ119EH (Less1 *et al.*, see above (1992)). The tetA gene (hatched arrow) with the original promoter is derived from plasmid RP4 (EMBL nucleotide sequence database, accession no. L27758). Tra functions (white arrows) are derived from plasmid pSB102 (EMBL nucleotide sequence database, accession no. AJ304453). Restriction sites used for molecular cloning of the pSB102 Tra region are indicated.

Figure 6 shows the genetic map of plasmid pSEToriT⁻. The map is based on the sequence data of plasmid pSET152 (EMBL nucleotide sequence database, accession no. AJ414670). pSEToriT⁻ consists of the larger two *Pst*I fragments of pSET152 with the 0,8kb fragment carrying the IncPα *oriT* omitted. Integrative *oriT* plasmids used were constructed by inserting the corresponding *oriT* sequences into the single *SphI* site.

### Example 3: Tra constructs are functional in intrageneric matings between two E. coli strains

Plasmids pFB1124 (Tra system IncQ/IncPα Mpf, Table 3), pFB1224 (Tra system pTFFC2/IncPα Mpf, Table 3) and pFB714 (pSB102 Tra system, Table 3) were constructed as self-transmissible plasmids with the ability to mobilize co-residing plasmids carrying the homologous *oriT* sequence. The presence of functional Tra elements was confirmed by intrageneric matings between E. *coli* strains.

In a semi-quantitative approach, the capacity of conjugative transfer of the helper plasmids themselves was tested first. 10-fold serial dilutions of plasmid-harboring HB101 cells were spotted onto a lawn of nalidixic acid resistant HB101Nx cells and selected for tetracycline/nalidixic acid resistance. TC^{r}/Nx^{r} colonies were obtained with each of the plasmids with dilutions as far as 10⁻⁵, indicating the presence of functional Tra systems in each of the constructs (data not shown). The mating experiments show that the combination of the IncQ or IncQ-like Mob functions with the IncPα Mpf functions, including the genes *traF* and *traG,* on a single plasmid renders the plasmid self-transmissible. With these experiments it is demonstrated that the 31,5kb *XbaI* fragment of pSB102, carrying the predicted Tra functions, is sufficient to confer the ability of conjugative transfer to a recombinant plasmid. This is the first approach to determine the extension of the pSB102 Tra region.

Quantitative filter mating experiments with DH5α as donor and GM2163 as recipient strain were carried out to assess the frequencies of mobilization of the recombinant *oriT* plasmids by their respective conjugative helper plasmids (Table 4). pUB307ΔK/pSET152 served as the reference system. With regard to the intergeneric matings between *E. coli* and Actinomycetes, pUB307ΔK-mediated mobilization of plasmids RSF1010K and pUWLoriTaph was included in the analysis. Employing IncPα plasmid pUB307ΔK as helper, transfer efficiencies approached one per donor cell with each of the *oriT* plasmids. Transfer frequencies obtained with plasmids pFB1124, pFB1224 and pFB714 were about 6- to 25-fold lower compared to those obtained with pUB307ΔK. pFB714 was the most efficient of the three, yielding transfer frequencies with an average of 1,4x10⁻¹. In contrast, employing the original plasmid pSB102, the number of exconjugants obtained was about 150-fold lower compared to that obtained with of pFB714 harboring the isolated pSB102 Tra region (Table 4). This might result from replication deficiencies of the environmental plasmid pSB102 in the E. *coli* host, since retarded growth of the DH5α (pSB102/pSEToriT102) donor cells was observed. Mobilization experiments with both pSB102 and pFB714 confirm the postulated location of the pSB102 *oriT* sequence. Control experiments with pSEToriT- yielded transfer frequencies near the detection limit with the exception of experiments carried out with pSB102 as helper plasmid. The latter exhibited reproducible higher transfer frequencies with an average of 4x10⁻⁶. Recombination events taking place during the long growth period might be the reason for this observation.

### Example 4: selected Tra systems are suitable for intergeneric conjugative transfer from E. coli to Streptomyces spp.

Subsequently, RSF1010, pTF-FC2 and pSB102 Tra systems were compared among one another and with IncPα system pUB307ΔK/pSET152 in intergeneric conjugation experiments between *E. coli* strain GM2163 as donor and five different Actinomycetes recipient strains. Actinomycetes strains selected were the following. *Streptomyces lividans* and *Streptomyces coelicolor* were selected as genetically well characterized strains often used in standard experiments.

Additionally, *Streptomyces diastatochromogenes,* a polyketomycin producing strain, was chosen as a potentially convenient recipient strain for combinatorial biosynthesis, and *Streptomyces aureofaciens,* a producer of congocidin and spiramycin. The fifth recipient strain selected was *Amycolatopsis japonicum,* a nocardioform Actinomycete producing the biodegradable ethylenediaminetetraacetic acid (EDTA) isomer ethylenediaminedisuccinic acid (EDDS). The selected Actinomycetes carry the φC31*attB* site for site-specific integration of the mobilized pSET derivatives, which lack a replicon to be autonomously maintained in these hosts. To assess whether the need of the mobilized plasmids to integrate into the host chromosome proves a limiting factor for the formation of exconjugants, conjugation experiments with two plasmids able to replicate in *Streptomyces spp.,* pUWLoriTaph and RSF1010K were included. pUWLoriTaph contains the replication functions of the *Streptomyces* broad-host-range multicopy plasmid pIJ101 (Kieser et *al.,* see above (1982)). RSF1010 is known to be transferred to and stably maintained in *Streptomyces lividans* and *Mycobacterium smegmatis.* For more convenient selection, the Km^{r} derivative RSF1010K (Table 1) instead of the original RSF1010 plasmid encoding resistances against streptomycin and sulfonamide was used.

Table 5 summarizes the frequencies of exconjugant formation per recipient spore. Exconjugants were obtained with all Tra systems and all Actinomycetes employed. However, conjugation experiments employing *Amycolatopsis japonicum* as recipient strain yielded transfer frequencies near the detection limit and are therefore left out in the following evaluation of the Tra systems. The actual transfer of pSET DNA into *A. japonicum* could be confirmed, though, for the IncPα and pSB102 system (see below and Fig. 3).

The most efficient Tra system for the recipient strains used in this study proved to be the established IncPα system with transfer frequencies between approximately 5x10⁻³ (pUB307ΔK/pSET152 with *S. lividans* as recipient) and 5x10⁻⁷ (pUB307ΔK/pUWLoriTaph with *S. diastatochromogenes* as recipient). The direct comparison between mobilization of an integrative and a non-integrative *oriT* plasmid (pSET152 vs. pUWLoriTaph) could be settled in favor of the integrative *oriT* plasmid with transfer frequencies about 2-fold to 300-fold higher than those obtained with the non-integrative *oriT* plasmid (Table 5). The integration of the plasmid into the host chromosome seems therefore not a limiting factor for the formation of exconjugants. No transfer frequency could be calculated for pUB307ΔK/pSET152 with *S. aureofaciens* as recipient strain. Exconjugant colonies were extremely small and formed a lawn on the agar plate even at high dilutions.

IncP/IncQ or IncQ-like helper plasmids pFB1124 and pFB1224 were about 2 to 2.000 times less effective in mobilizing the corresponding *oriT* plasmids than IncPα plasmid pUB307ΔK (Table 5). Transfer frequencies range from 3x10⁻⁷ (pFB1124/pSET101n with *S. diastatochromogenes)* to 3x10⁻⁵ (pFB1124/pSET101u and pFB1224/pSET201u with *S. aureofaciens).* Using the IncPα system yielded highest transfer frequencies with *S. lividans* as recipient. In contrast, matings with the IncP/IncQ as well as with the IncP/IncQ-like Tra system exhibited the highest frequencies of transfer using *S. aureofaciens* spores as recipients. Additionally, *S. aureofaciens* exconjugants obtained with the IncP/IncQ and the IncP/IncQ-like Tra system were large and well growing in contrast to exconjugants obtained with the pUB307AK/pSET152 system. Plasmid RSF1010K could be mobilized into and replicated in *S. coelicolor* and *A. japonicum,* but no exconjugants were obtained with *S. lividans.*

The pSB102 Tra system proved to be more efficient in intergeneric conjugation experiments with all *Streptomyces* strains than the IncP/IncQ and the IncP/IncQ-like system, regardless whether the original pSB102 plasmid was used as helper plasmid or plasmid pFB714 containing pSB102 Tra region. As in intrageneric conjugation experiments, E. *coli* donor cells carrying pSB102 together with the corresponding *oriT* plasmid were growing rather slowly, but transfer frequencies observed with pSB102/pSEToriT102 are corresponding to those obtained with pFB714/pSEToriT102. Transfer generally occurred at frequencies of 2x10⁻⁶ to 5x10⁻⁴ (Table 5). Transfer frequencies obtained with *S. aureofaciens* leveled those obtained with *S. lividans* or were even higher. *S. aureofaciens* exconjugants displayed good growth as in conjugation experiments using the IncP/IncQ or IncP/pTF-FC2 Tra system.

Taken together, the Tra systems assayed proved to be suitable for intergeneric conjugation between E. *coli* and *Streptomyces spp.* in the following gradation: pUB307dK>pSB102>pTF-FC2RSF1010, with integrative>non-integrative.

### Example 5: confirmation of plasmid transfer

Samples of exconjugants were picked and cultivated on selective HA agar plates to confirm the conjugative transfer of the pSET derivatives and their stable maintenance in the Actinomycetes hosts. Phosphomycin was added to counterselect the sensitive E. *coli* donor.

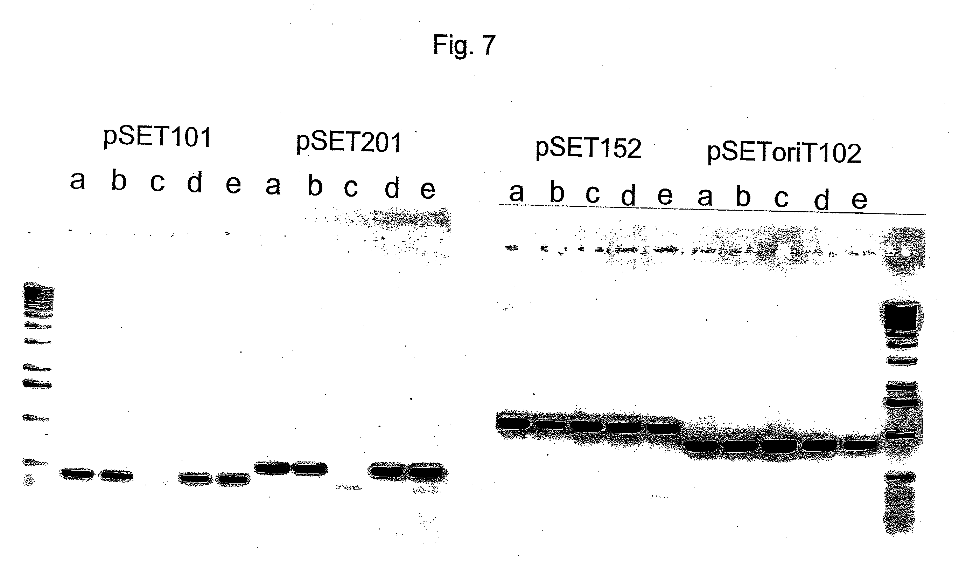

For a further verification of transfer of the integrative *oriT* plasmids into the five different Actinomycetes strains, genomic DNA was isolated and the presence of integrated pSET DNA was confirmed by PCR analysis using primers mapping at the *Sph*I site (see Table 2). PCR products of the size specific for the corresponding *oriT* sequences located at this site were obtained with all samples with the exception of RSF1010 and pTF-FC2 *oriT* (plasmids pSET101 and pSET201, respectively) in *A. japonicum* (Fig. 7). The weak band visible in the latter two samples is not *oriT* specific but a PCR by-product also contained in some of the other samples (Fig. 7). However, true exconjugants were also obtained with *A. japonicum* as shown for the conjugation experiments with pSET152 and pSEToriT102. Three colonies obtained in control experiments with pSEToriT did not yield a PCR product (data not shown).

Figure 7 shows the PCR analysis of exconjugants. PCR reactions were carried out in 100 µl with 2 µl of genomic Actinomycetes DNA, 50 pmol primers each, 200 µM dNTPs and 2U *Taq* polymerase in PCR buffer (10 mM Tris-HCl, pH 8,3; 50 mM KCl; 1,5 mM MgCl₂). The template was denatured for 3 min at 95°C. Amplification was for 30 cycles with 1 min 95°C, 1 min 58°C and 1 min 72°C and additional 5 min for the final synthesis step. 10 µl of each PCR reaction were loaded onto 0,8% agarose gels and subjected to electrophoresis in TAE buffer (40 mM Tris-HCl, pH 7,9; 5 mM sodium acetate; 1 mM EDTA). Primers used map to both sides of the *SphI* site in pSEToriT and yield a PCR product of 390 bp (not shown). *oriT* specific products display bands of 390 bp plus the size of the corresponding *oriT* sequence. Exconjugants: lane a: *Streptomyces aureofaciens;* lane b: *Streptomyces coelicolor;* lane c: *Amycolatopsis japonicum;* lane d: *Streptomyces lividans;* lane e: *Streptomyces diastatochromogenes.* Molecular size standard: 1 kb ladder.

Likewise, the conjugative transfer and stable maintenance of non-integrative plasmids pUWLoriTaph and RSF1010K was confirmed by cultivating the exconjugants on selective HA agar plates with simultaneous counterselection against the *E. coli* donor. The plasmids were then isolated from stable exconjugants and analyzed by agarose gel electrophoresis (data not shown). Plasmid pUWLoriTaph was stably maintained in *S. coelicolor, S. lividans* and *S. diastatochromogenes,* but not in *S. aureofaciens* and *A. japonicum. S. aureofaciens* exconjugants did not grow on HA medium containing kanamycin and phospomycin, indicating a loss of the plasmid. *A. japonicum* exconjugants were stable under the selective conditions applied, but pUWLoriTaph plasmid DNA was not obtained. Likewise, RSF1010K plasmid DNA was isolated from *S. coelicolor,* but not from *A. japonicum.* This might indicate, that the plasmid isolation protocol used is not applicable for this strain, or else, that spontaneous chromosomal mutations led to the formation of Km^{r} *A. japonicum* colonies.

### Example 6: comparision of the transfer system of the invention with the state of the art.

*S. aureofaciens* was subjected to a conjugative transfer with the established system IncPα pUB307/pSET152. Exconjugates were, however, extremely small and transfer frequencies could therefore not be determined even when resuspensions were plated in high dilutions. The conjugative transfer of IncPα pUWLoriTaph could not be confirmed.

In turn, employing pSB102, *S. aureofaciens* exconjugants formed large colonies and reasonable transfer frequencies were obtained. Similarly, *S. aureofaciens* exconjugants obtained with pTF-FC2/IncP Mpf and RSF1010/IncP Mpf formed large colonies.

Taken together, the transfer systems of the invention provide the basis for the development of new conjugative systems apart from the mere modification of the established ones exclusively based on conjugative plasmid RP4. Especially pSB102 might offer the access to a wider range of bacterial species since cell surface and membrane components with possibly different affinities and/or specificities from those of the IncPα system are involved.

In the following a reference list for the SEQ.-ID numbers and the corresponding plasmids of the invention is given. 1: pFB219EH; 2: pFB319EH; 3: pFB714EH; 4: pFB1124; 5: pFB1224; 6: pGZ119EH; 7: pGZ219EH; 8: pGZ1119EH; 9: pJF119EH; 10: pML100; 11: pML123; 12: pMS124; 13: pMS713-1n; 14: pSEToriT-; 15: pUB307ΔK; 16: pSEToriT102; 17: pSET101u; 18: pSET101n; 19: pSET201u. SEQ.-ID numbers 20 to 38 are the primers of Table 2 in the order thereof.

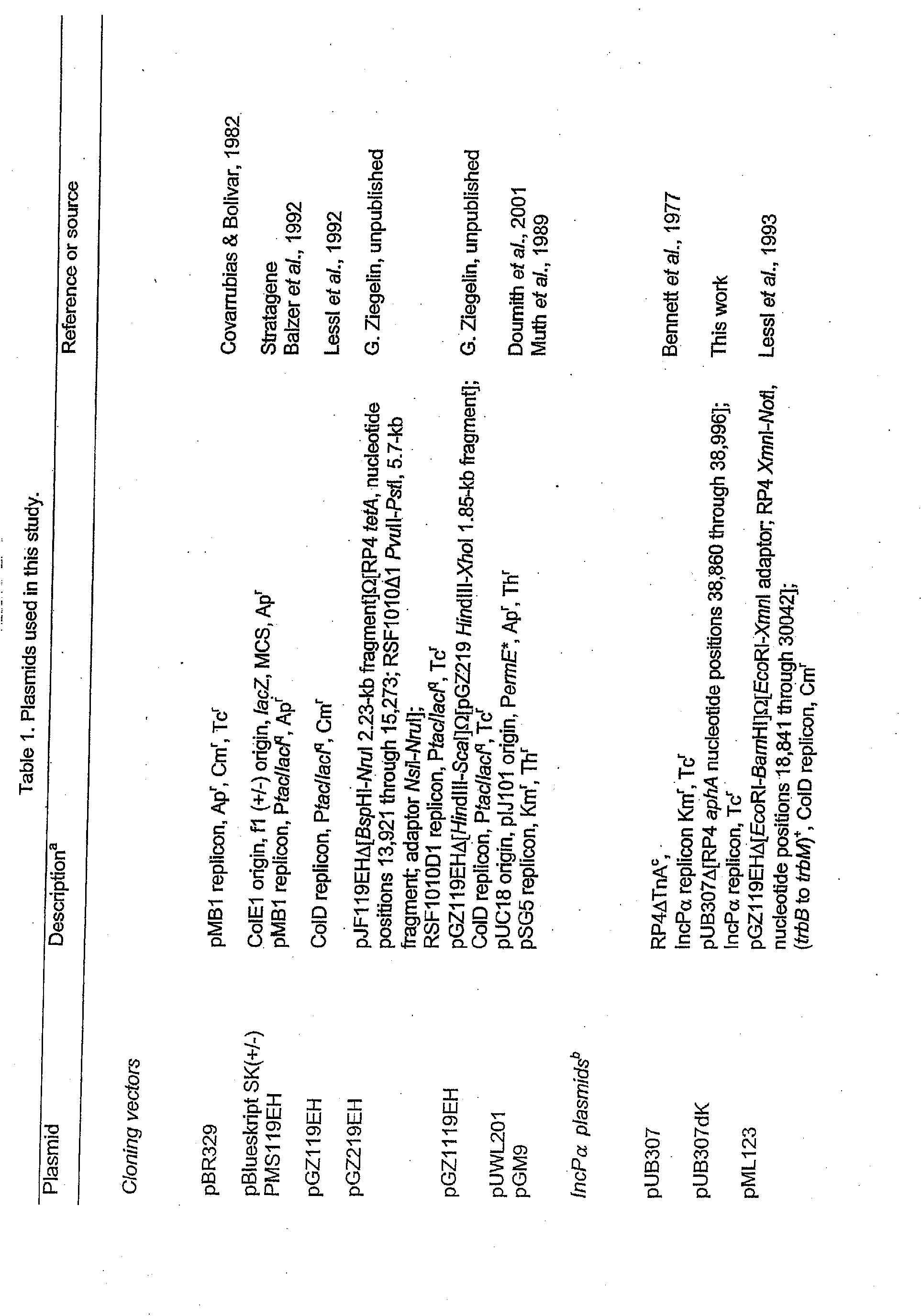

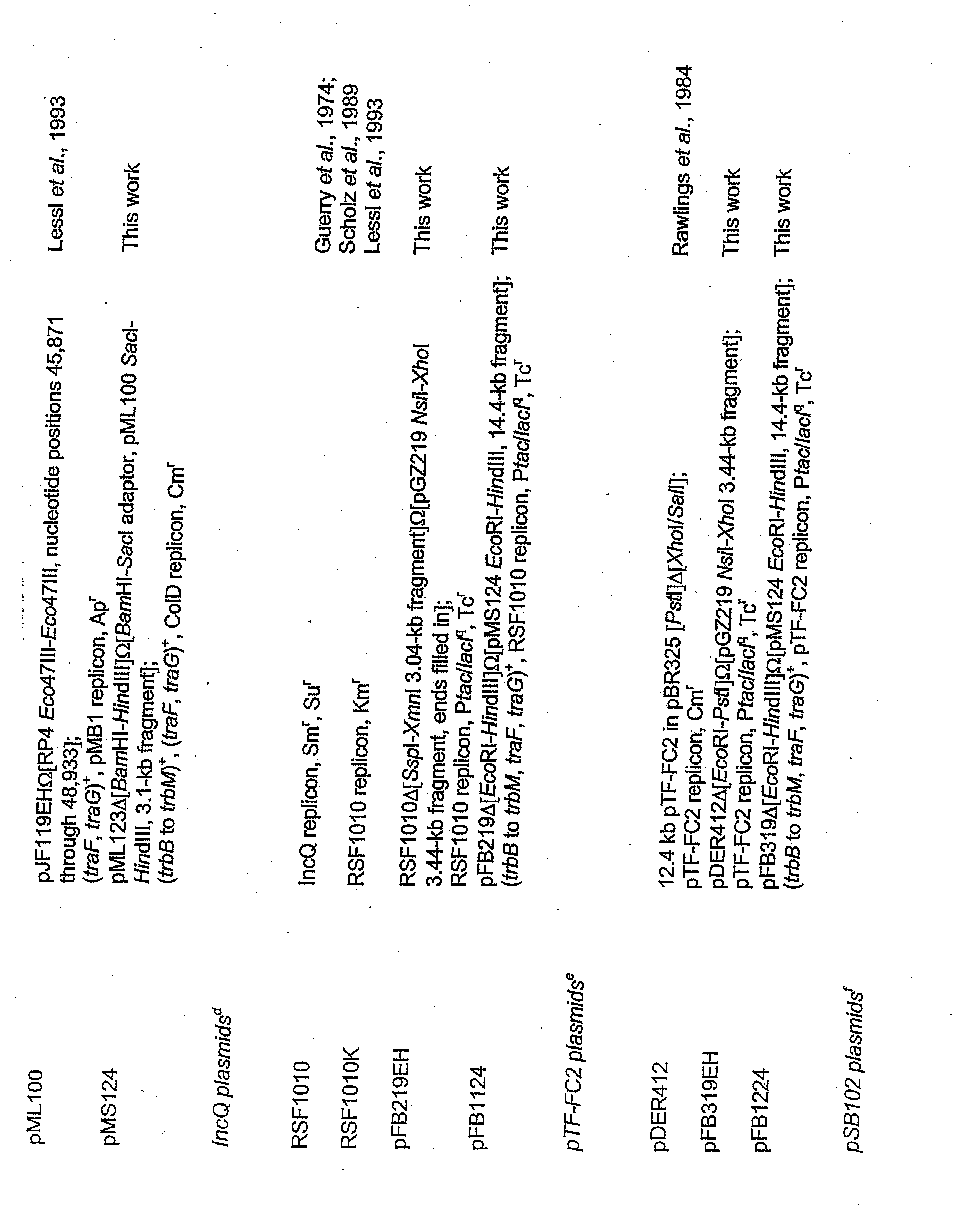

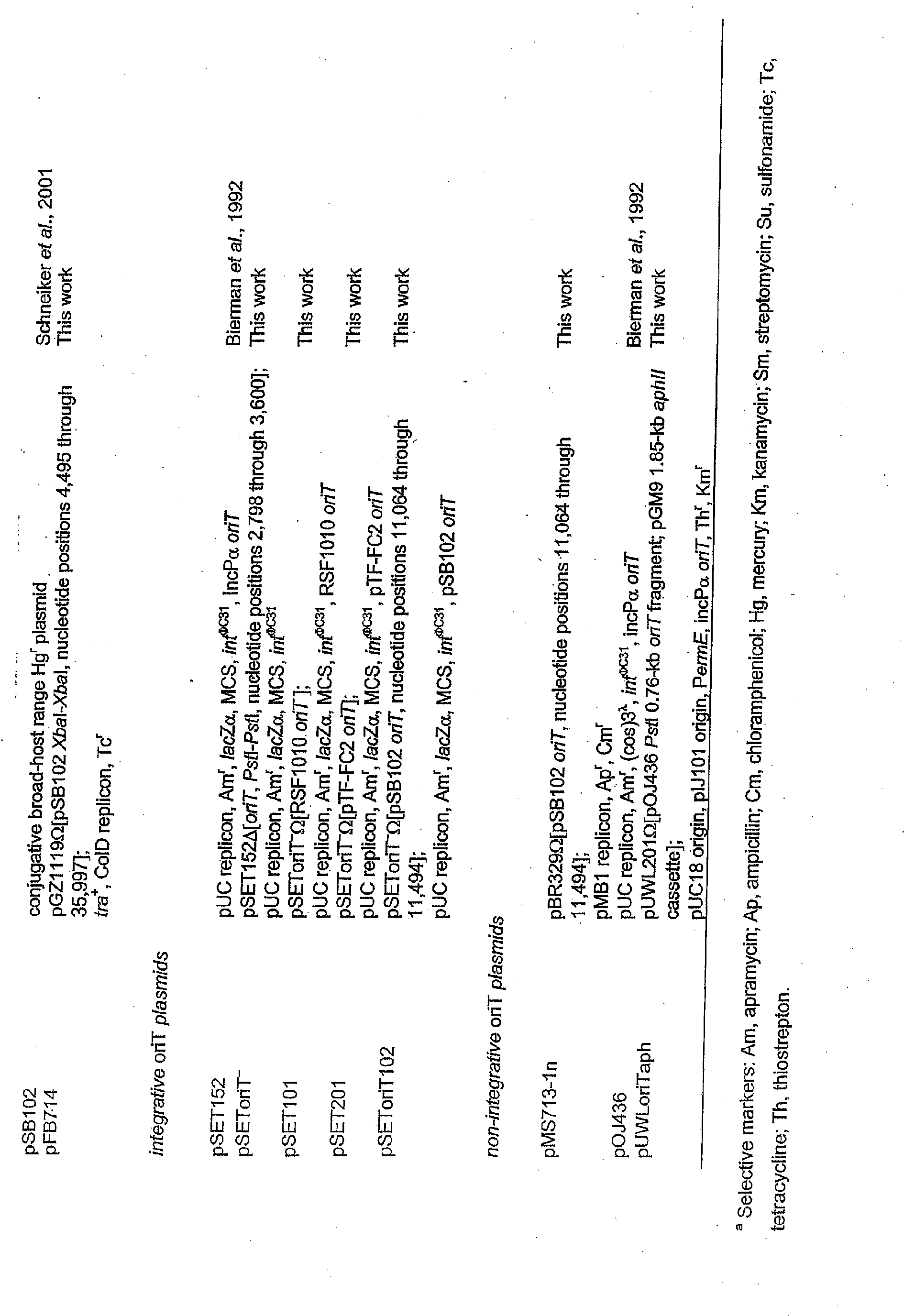

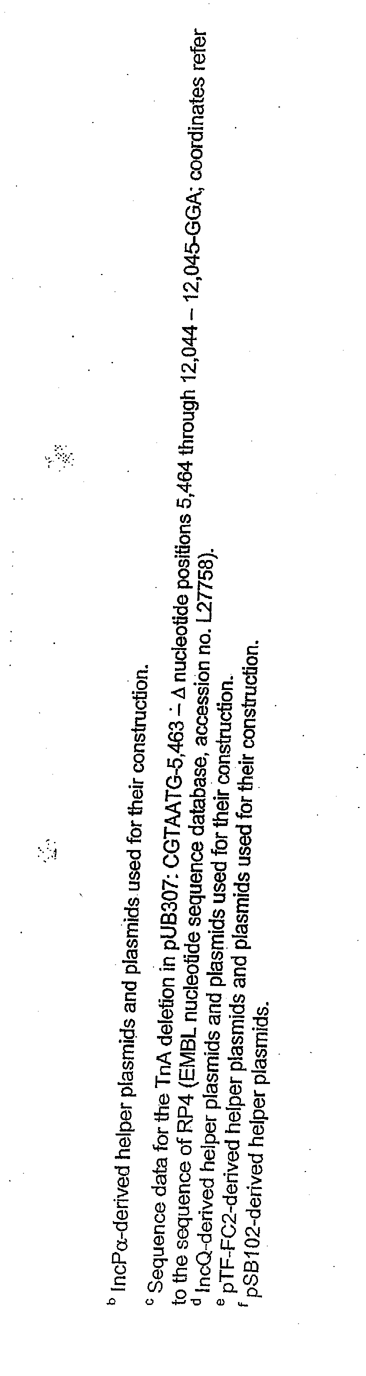

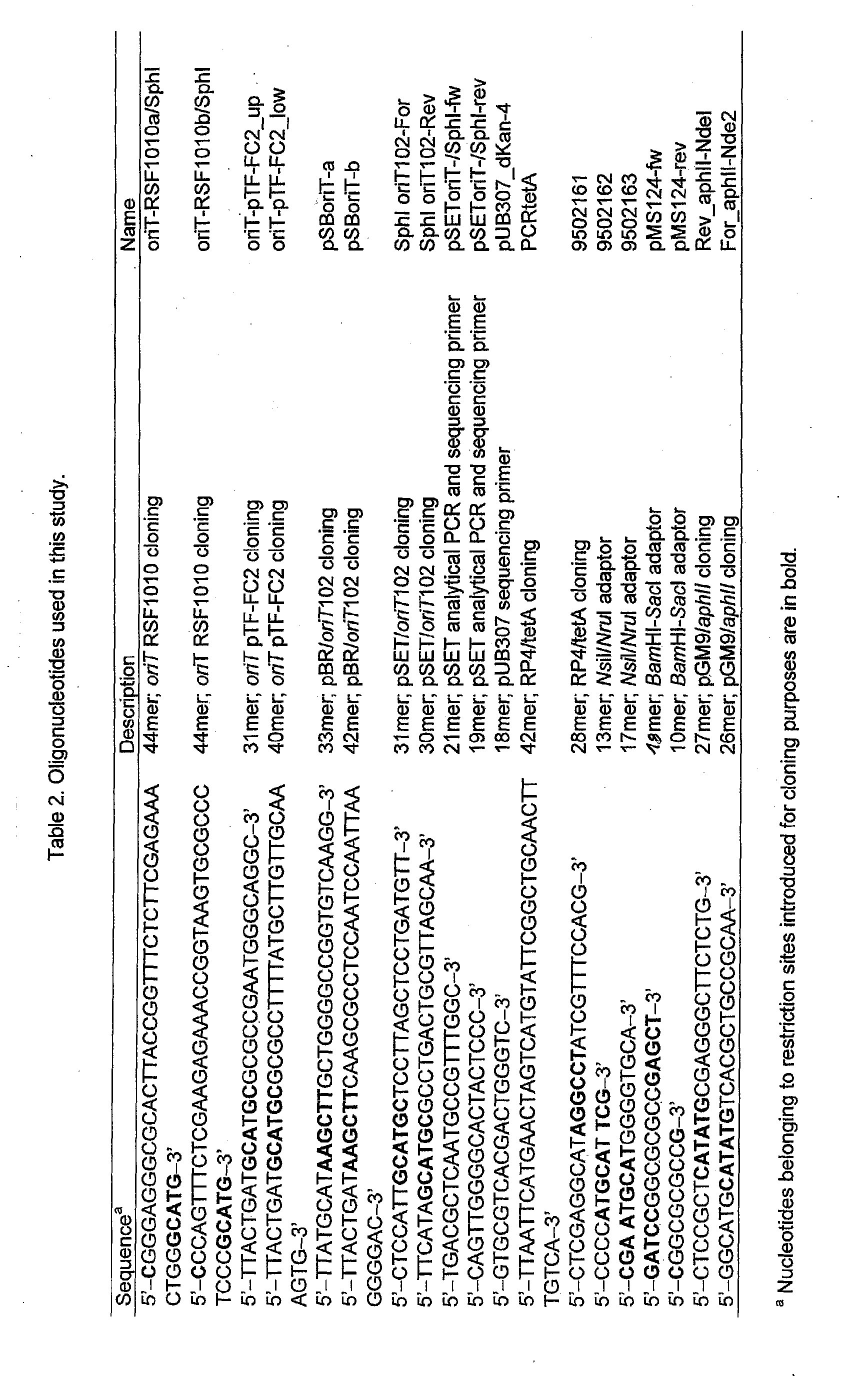

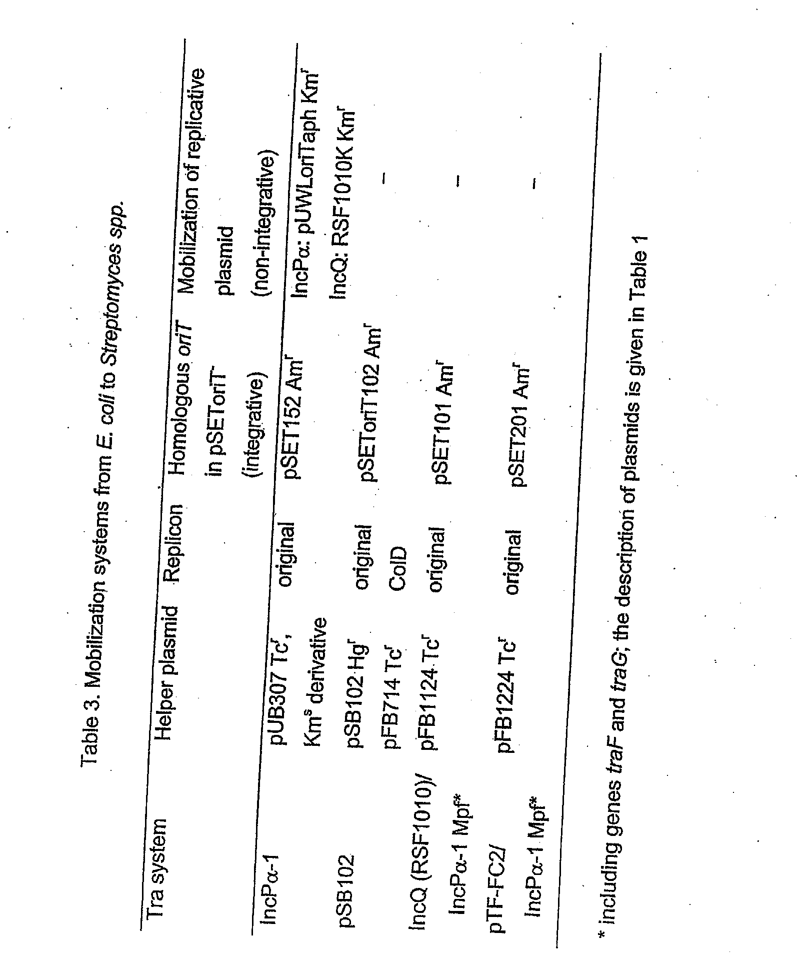

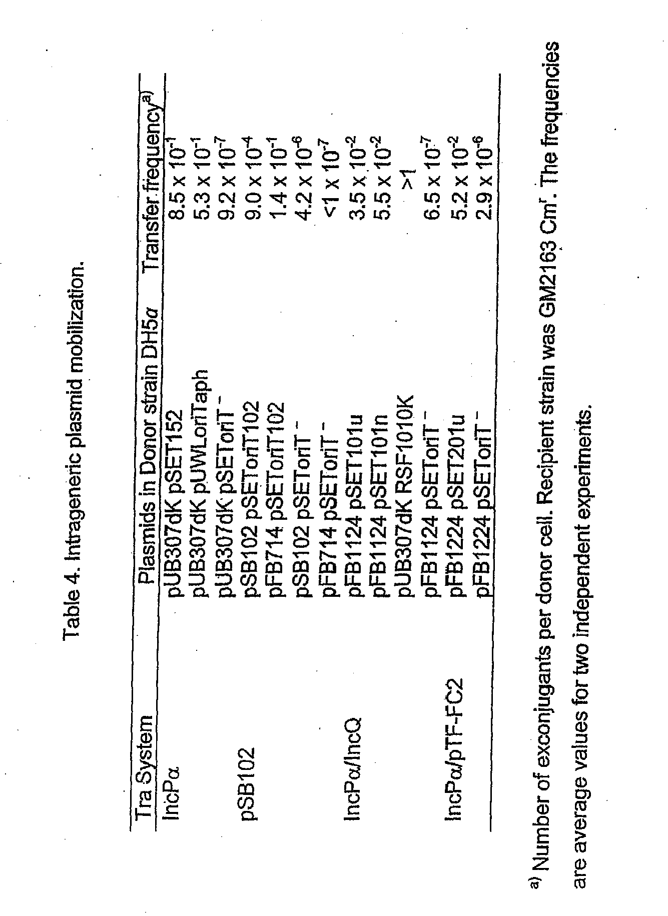

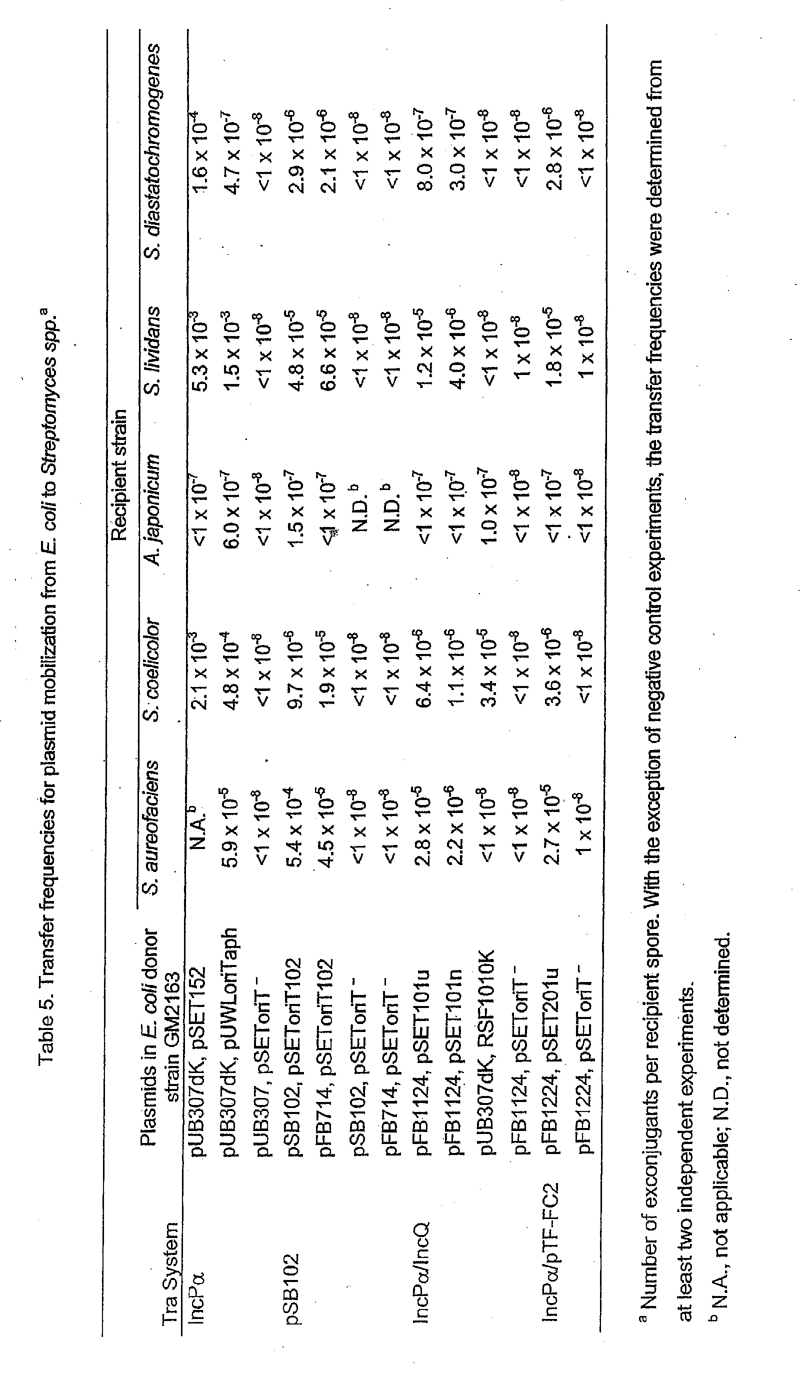

## Claims

1. An isolated recombinant nucleic acid comprising one or several of the sequences SEQ.-ID 1 to 19.

2. An isolated protein or peptide coded by one or several of the nucleic acids according to claim 1.

3. An isolated recombinant plasmid comprising the nucleic acid of claim 1.

4. An isolated recombinant plasmid comprising the pSB102 Tra region, in particular the 31,5 kb XbaI-XbaI fragment of pSB102, wherein the Tra region is located, optionally further comprising one, several, or all of the elements of the group consisting of *"lacI*^{*q*} gene of pGZ119EH, promoter Ptac of pGZ119EH/HE, rrnBT1T2 terminator of pGZ119EH/HE, ColD origin of replication of pGZ119EH/HE, *tetA* Gene with original promotor of RP4", preferably being pFB714EH.

5. An isolated recombinant plasmid comprising the *mob*/*rep* regions of RSF1010, RSF1010K or pTF-FC2, optionally further comprising one, several, or all of the elements of the group consisting of *"lacI*^{*q*} gene of pGZ119EH/HE, promoter Ptac of pGZ119EH/HE, rrnBT1T2 terminator of pGZ119EH/HE, Tra2 region of RP4, *tetA* Gene with original promotor of RP4", preferably being pFB219EH, pFB1124, pFB319EH, or pFB1224.

6. A cell, in particular an Enterobacterium, preferably E. coli, containing at least one plasmid according to one of the claims 3 to 5, optionally containing in addition at least one recombinant integrative or non-integrative *oriT* plasmid, wherein the *oriT* plasmid may comprise a defined gene coding for a defined enzyme, peptide or protein, the gene intended for transfer from the cell as donor cell to a recipient cell of a different strain, wherein the gene in particular comprises a defined nucleic acid sequence coding for a modified or unmodified biosynthesis step in the recipient cell.

7. A cell according to claim 6, wherein the integrative *orit* plasmid is selected from the group consisting of "pSET152, pSET101, pSET201, and pSEToriT102", and wherein the non-integrative *oriT* plasmid is selected from the group consisting of "RSF1010K, pOJ436, and pUWLoriTaph", wherein the defined nucleic acid sequence is inserted into said *oriT* plasmid.

8. A method for intergeneric gene transfer, wherein a donor cell according to claim 6 or 7 is contacted with a recipient cell, wherein the integrative or non-integrative *oriT* plasmid is transferred from the donor cell to the recipient cell by conjugation.

9. The method according to claim 8, wherein the recipient cell is selected from Actinomycetes, in particular Streptomycetes.

10. A method for producing a derivative of a defined compound, the compound being obtainable by biosynthesis in a recipient cell comprising the following steps:
a) producing a donor cell according to claim 6 or 7, wherein the defined nucleic acid sequence codes for a protein or peptide not naturally occurring in the recipient cell, wherein the protein or peptide effects a modified biosynthesis step in the biosynthesis of the recipient cell being different from the naturally occurring biosynthesis step of the same recipient cell and resulting in a derivative of the defined compound being produced in the modified biosynthesis,
b) contacting the donor cell with the recipient cell, wherein the defined foreign nucleic acid is transferred to the recipient cell,
c) effecting expression of the defined foreign nucleic acid in the recipient cell,
d) culturing the recipient cell, and
e) isolating the derivative of the defined compound from a culture supernatant or from an extract of the cultured recipient cell.

11. A method for producing an enzyme, the enzyme being expressed in a recipient cell comprising the following steps:
a) producing a donor cell according to claim 6 or 7, wherein the defined nucleic acid sequence codes for an enzyme expressable in the recipient cell,
b) contacting the donor cell with the recipient cell, wherein the defined nucleic acid is transferred to the recipient cell,
c) effecting expression of the defined nucleic acid in the recipient cell,
d) culturing the recipient cell, and
e) isolating the enzyme from a culture supernatant or from an extract of the cultured recipient cell.


SEQUENCE LISTING

<110>  Combinature Biopharm AG

<120>  Plasmids for Gene Transfer by Bacterial Conjugation

<130>  COM/EP/0408

<160>  38

<170>  PatentIn version 3.2

<210>  1
<211>  9088
<212>  DNA
<213>  Artificial

<220>
<223>  partial sequence

<400>  1
gaattcgagc tcggtacccg gggatcctct agagtcgacc tgcaggcatg caagcttggc      60

tgttttggcg gatgagagaa gattttcagc ctgatacaga ttaaatcaga acgcagaagc     120

ggtctgataa aacagaattt gcctggcggc agtagcgcgg tggtcccacc tgaccccatg     180

ccgaactcag aagtgaaacg ccgtagcgcc gatggtagtg tggggtctcc ccatgcgaga     240

gtagggaact gccaggcatc aaataaaacg aaaggctcag tcgaaagact gggcctttcg     300

ttttatctgt tgtttgtcgg tgaacgctct cctgagtagg acaaatccgc cgggagcgga     360

tttgaacgtt gcgaagcaac ggcccggagg gtggcgggca ggacgcccgc cataaactgc     420

caggcatcaa attaagcaga aggccatcct gacggatggc ctttttgcgt ttctacaaac     480

tctttttgtt tatttttcta aatacattca aatatgtatc cgctcatgtt aattcatgaa     540

ctagtcatgt attcggctgc aactttgtca tgcttgacac tttatcactg ataaacataa     600

tatgtccacc aacttatcag tgataaagaa tccgcgcgtt caatcggacc agcggaggct     660

ggtccggagg ccagacgtga aacccaacat acccctgatc gtaattctga gcactgtcgc     720

gctcgacgct gtcggcatcg gcctgattat gccggtgctg ccgggcctcc tgcgcgatct     780

ggttcactcg aacgacgtca ccgcccacta tggcattctg ctggcgctgt atgcgttggt     840

gcaatttgcc tgcgcacctg tgctgggcgc gctgtcggat cgtttcgggc ggcggccaat     900

cttgctcgtc tcgctggccg gcgccactgt cgactacgcc atcatggcga cagcgccttt     960

cctttgggtt ctctatatcg ggcggatcgt ggccggcatc accggggcga ctggggcggt    1020

agccggcgct tatattgccg atatcactga tggcgatgag cgcgcgcggc acttcggctt    1080

catgagcgcc tgtttcgggt tcgggatggt cgcgggacct gtgctcggtg ggctgatggg    1140

cggtttctcc ccccacgctc cgttcttcgc cgcggcagcc ttgaacggcc tcaatttcct    1200

gacgggctgt ttccttttgc cggagtcgca caaaggcgaa cgccggccgt tacgccggga    1260

ggctctcaac ccgctcgctt cgttccggtg ggcccggggc atgaccgtcg tcgccgccct    1320

gatggcggtc ttcttcatca tgcaacttgt cggacaggtg ccggccgcgc tttgggtcat    1380

tttcggcgag gatcgctttc actgggacgc gaccacgatc ggcatttcgc ttgccgcatt    1440

tggcattctg cattcactcg cccaggcaat gatcaccggc cctgtagccg cccggctcgg    1500

cgaaaggcgg gcactcatgc tcggaatgat tgccgacggc acaggctaca tcctgcttgc    1560

cttcgcgaca cggggatgga tggcgttccc gatcatggtc ctgcttgctt cgggtggcat    1620

cggaatgccg gcgctgcaag caatgttgtc caggcaggtg gatgaggaac gccaggggca    1680

gctgcaaggc tcactggcgg cgctcaccag cctgacctcg atcgtcggac ccctcctctt    1740

cacggcgatc tatgcggctt ctataacaac gtggaacggg tgggcatgga ttgcaggcgc    1800

tgccctctac ttgctctgcc tgccggcgct gcgtcgcggg ctttggagcg gcgcagggca    1860

acgagccgat cgctgatcgt ggaaacgata ggcctatgcc tcgatcttca aattcccgtt    1920

gcacatagcc cggcaattcc tttccctgct ctgccataag cgcagcgaat gccgggtaat    1980

actcgtcaac gatctgatag agaagggttt gctcgggtcg gtggctctgg taacgaccag 2040
tatcccgatc ccggctggcc gtcctggccg ccacatgagg catgttccgc gtccttgcaa 2100
tactgtgttt acatacagtc tatcgcttag cggaaagttc ttttaccctc agccgaaatg 2160
cctgccgttg ctagacattg ccagccagtg cccgtcactc ccgtactaac tgtcacgaac 2220
ccctgcaata actgtcacgc ccccctgcaa taactgtcac gaacccctgc aataactgtc 2280
acgcccccaa acctgcaaac ccagcagggg cgggggctgg cggggtgttg gaaaaatcca 2340
tccatgatta tctaagaata atccactagg cgcggttatc agcgcccttg tggggcgctg 2400
ctgcccttgc ccaatatgcc cggccagagg ccggatagct ggtctattcg ctgcgctagg 2460
ctacacaccg ccccaccgct gcgcggcagg gggaaaggcg ggcaaagccc gctaaacccc 2520
acaccaaacc ccgcagaaat acgctggagc gcttttagcc gctttagcgg cctttccccc 2580
tacccgaagg gtgggggcgc gtgtgcagcc ccgcagggcc tgtctcggtc gatcattcag 2640
cccggctcat ccttctggcg tggcggcaga ccgaacaagg cgcggtcgtg gtcgcgttca 2700
aggtacgcat ccattgccgc catgagccga tcctccggcc actcgctgct gttcaccttg 2760
gccaaaatca tggcccccac cagcaccttg cgccttgttt cgttcttgcg ctcttgctgc 2820
tgttcccttg cccgcacccg ctgaatttcg gcattgattc gcgctcgttg ttcttcgagc 2880
ttggccagcc gatccgccgc cttgttgctc cccttaacca tcttgacacc ccattgttaa 2940
tgtgctgtct cgtaggctat catggaggca cagcggcggc aatcccgacc ctactttgta 3000
ggggagggcg cacttaccgg tttctcttcg agaaactggc ctaacggcca cccttcgggc 3060
ggtgcgctct ccgagggcca ttgcatggag ccgaaaagca aaagcaacag cgaggcagca 3120
tggcgattta tcaccttacg gcgaaaaccg gcagcaggtc gggcggccaa tcggccaggg 3180
ccaaggccga ctacatccag cgcgaaggca agtatgcccg cgacatggat gaagtcttgc 3240
acgccgaatc cgggcacatg ccggagttcg tcgagcggcc cgccgactac tgggatgctg 3300
ccgacctgta tgaacgcgcc aatgggcggc tgttcaagga ggtcgaattt gccctgccgg 3360
tcgagctgac cctcgaccag cagaaggcgc tggcgtccga gttcgcccag cacctgaccg 3420
gtgccgagcg cctgccgtat acgctggcca tccatgccgg tggcggcgag aacccgcact 3480
gccacctgat gatctccgag cggatcaatg acggcatcga gcggcccgcc gctcagtggt 3540
tcaagcggta caacggcaag accccggaga agggcggggc acagaagacc gaagcgctca 3600
agcccaaggc atggcttgag cagacccgcg aggcatgggc cgaccatgcc aaccgggcat 3660
tagagcgggc tggccacgac gcccgcattg accacagaac acttgaggcg cagggcatcg 3720
agcgcctgcc cggtgttcac ctggggccga acgtggtgga gatggaaggc cggggcatcc 3780
gcaccgaccg ggcagacgtg gccctgaaca tcgacaccgc caacgcccag atcatcgact 3840
tacaggaata ccgggaggca atagaccatg aacgcaatcg acagagtgaa gaaatccaga 3900
ggcatcaacg agttagcgga gcagatcgaa ccgctggccc agagcatggc gacactggcc 3960
gacgaagccc ggcaggtcat gagccagacc cagcaggcca gcgaggcgca ggcggcggag 4020
tggctgaaag cccagcgcca gacaggggcg gcatgggtgg agctggccaa agagttgcgg 4080
gaggtagccg ccgaggtgag cagcgccgcg cagagcgccc ggagcgcgtc gcgggggtgg 4140
cactggaagc tatggctaac cgtgatgctg gcttccatga tgcctacggt ggtgctgctg 4200
atcgcatcgt tgctcttgct cgacctgacg ccactgacaa ccgaggacgg ctcgatctgg 4260
ctgcgcttgg tggcccgatg aagaacgaca ggactttgca ggccataggc cgacagctca 4320
aggccatggg ctgtgagcgc ttcgatatcg gcgtcaggga cgccaccacc ggccagatga 4380
tgaaccggga atggtcagcc gccgaagtgc tccagaacac gccatggctc aagcggatga 4440
atgcccaggg caatgacgtg tatatcaggc ccgccgagca ggagcggcat ggtctggtgc 4500
tggtggacga cctcagcgag tttgacctgg atgacatgaa agccgagggc cgggagcctg 4560

ccctggtagt ggaaaccagc ccgaagaact atcaggcatg ggtcaaggtg gccgacgccg 4620
caggcggtga acttcggggg cagattgccc ggacgctggc cagcgagtac gacgccgacc 4680
cggccagcgc cgacagccgc cactatggcc gcttggcggg cttcaccaac cgcaaggaca 4740
agcacaccac ccgcgccggt tatcagccgt gggtgctgct gcgtgaatcc aagggcaaga 4800
ccgccaccgc tggcccggcg ctggtgcagc aggctggcca gcagatcgag caggcccagc 4860
ggcagcagga gaaggcccgc aggctggcca gcctcgaact gcccgagcgg cagcttagcc 4920
gccaccggcg cacggcgctg gacgagtacc gcagcgagat ggccgggctg gtcaagcgct 4980
tcggtgatga cctcagcaag tgcgacttta tcgccgcgca gaagctggcc agccggggcc 5040
gcagtgccga ggaaatcggc aaggccatgg ccgaggccag cccagcgctg gcagagcgca 5100
agcccggcca cgaagcggat tacatcgagc gcaccgtcag caaggtcatg ggtctgccca 5160
gcgtccagct tgcgcgggcc gagctggcac gggcaccggc accccgccag cgaggcatgg 5220
acaggggcgg gccagatttc agcatgtagt gcttgcgttg gtactcacgc ctgttatact 5280
atgagtactc acgcacagaa gggggtttta tggaatacga aaaaagcgct tcagggtcgg 5340
tctacctgat caaaagtgac aagggctatt ggttgcccgg tggctttggt tatacgtcaa 5400
acaaggccga ggctggccgc ttttcagtcg ctgatatggc cagccttaac cttgacggct 5460
gcaccttgtc cttgttccgc gaagacaagc ctttcggccc cggcaagttt ctcggtgact 5520
gatatgaaag accaaaagga caagcagacc ggcgacctgc tggccagccc tgacgctgta 5580
cgccaagcgc gatatgccga gcgcatgaag gccaaaggga tgcgtcagcg caagttctgg 5640
ctgaccgacg acgaatacga ggcgctgcgc gagtgcctgg aagaactcag agcggcgcag 5700
ggcgggggta gtgaccccgc cagcgcctaa ccaccaactg cctgcaaagg aggcaatcaa 5760
tggctaccca taagcctatc aatattctgg aggcgttcgc agcagcgccg ccaccgctgg 5820
actacgtttt gcccaacatg gtggccggta cggtcggggc gctggtgtcg cccggtggtg 5880
ccggtaaatc catgctggcc ctgcaactgg ccgcacagat tgcaggcggg ccggatctgc 5940
tggaggtggg cgaactgccc accggcccgg tgatctacct gcccgccgaa gacccgccca 6000
ccgccattca tcaccgcctg cacgcccttg ggcgcacctt cagcgccgag gaacggcaag 6060
ccgtggctga cggcctgctg atccagccgc tgatcggcag cctgcccaac atcatggccc 6120
cggagtggtt cgacggcctc aagcgcgccg ccgagggccg ccgcctgatg gtgctggaca 6180
cgctgcgccg gttccacatc gaggaagaaa acgccagcgg ccccatggcc caggtcatcg 6240
gtcgcatgga ggccatcgcc gccgataccg ggtgctctat cgtgttcctg caccatgcca 6300
gcaagggcgc ggccatgatg ggcgcaggcg accagcagca ggccagccgg ggcagctcgg 6360
tactggtcga taacatccgc tggcagtcct acctgtcgag catgaccagc gccgaggccg 6420
aggaatgggg tgtggacgac gaccagcgcc ggttcttcgt ccgcttcggt gtgagcaagg 6480
ccaactatgg cgcaccgttc gctgatcggt ggttcaggcg gcatgacggc ggggtgctca 6540
agcccgccgt gctggagagg cagcgcaaga gcaagggggt gccccgtggt gaagcctaag 6600
aacaagcaca gcctcagcca cgtccggcac gacccggcgc actgtctggc ccccggcctg 6660
ttccgtgccc tcaagcgggg cgagcgcaag cgcagcaagc tggacgtgac gtatgactac 6720
ggcgacggca agcggatcga gttcagcggc ccggagccgc tgggcgctga tgatctgcgc 6780
atcctgcaag ggctggtggc catggctggg cctaatggcc tagtgcttgg cccggaaccc 6840
aagaccgaag gcggacggca gctccggctg ttcctggaac ccaagtggga ggccgtcacc 6900
gctgaatgcc atgtggtcaa aggtagctat cgggcgctgg caaaggaaat cggggcagag 6960
gtcgatagtg gtggggcgct caagcacata caggactgca tcgagcgcct ttggaaggta 7020
tccatcatcg cccagaatgg ccgcaagcgg caggggtttc ggctgctgtc ggagtacgcc 7080
agcgacgagg cggacgggcg cctgtacgtg gccctgaacc ccttgatcgc gcaggccgtc 7140

atgggtggcg gccagcatgt gcgcatcagc atggacgagg tgcgggcgct ggacagcgaa 7200

accgcccgcc tgctgcacca gcggctgtgt ggctggatcg accccggcaa aaccggcaag 7260

gcttccatag ataccttgtg cggctatgtc tggccgtcag aggccagtgg ttcgaccatg 7320

cgcaagcgcc gccagcgggt gcgcgaggcg ttgccggagc tggtcgcgct gggctggacg 7380

gtaaccgagt tcgcggcggg caagtacgac atcacccggc ccaaggcggc aggctgaccc 7440

cccccactct attgtaaaca agacattttt atcttttata ttcaatggct tattttcctg 7500

ctaattggta ataccatgaa aaataccatg ctcagaaaag gcttaacaat ttcgcgaacg 7560

ccagcaagac gtagcccagc gcgtcggcca gcttgcaatt cgcgctaact tacattaatt 7620

gcgttgcgct cactgcccgc tttccagtcg ggaaacctgt cgtgccagct gcattaatga 7680

atcggccaac gcgcggggag aggcggtttg cgtattgggc gccagggtgg tttttctttt 7740

caccagtgag acgggcaaca gctgattgcc cttcaccgcc tggccctgag agagttgcag 7800

caagcggtcc acgctggttt gccccagcag gcgaaaatcc tgtttgctgg tggttaacgg 7860

cgggatataa catgagctgt cttcggtatc gtcgtatccc actaccgaga tatccgcacc 7920

aacgcgcagc ccggactcgg taatggcgcg cattgcgccc agcgccatct gatcgttggc 7980

aaccagcatc gcagtgggaa cgatgccctc attcagcatt tgcatggttt gttgaaaacc 8040

ggacatggca ctccagtcgc cttcccgttc cgctatcggc tgaatttgat tgcgagtgag 8100

atatttatgc cagccagcca gacgcagacg cgccgagaca gaacttaatg ggcccgctaa 8160

cagcgcgatt tgctggtgac ccaatgcgac cagatgctcc acgcccagtc gcgtaccgtc 8220

ttcatgggag aaaataatac tgttgatggg tgtctggtca gagacatcaa gaaataacgc 8280

cggaacatta gtgcaggcag cttccacagc aatggcatcc tggtcatcca gcggatagtt 8340

aatgatcagc ccactgacgc gttgcgcgag aagattgtgc accgccgctt tacaggcttc 8400

gacgccgctt cgttctacca tcgacaccac cacgctggca cccagttgat cggcgcgaga 8460

tttaatcgcc gcgacaattt gcgacggcgc gtgcagggcc agactggagg tggcaacgcc 8520

aatcagcaac gactgtttgc ccgccagttg ttgtgccacg cggttgggaa tgtaattcag 8580

ctccgccatc gccgcttcca ctttttcccg cgttttcgca gaaacgtggc tggcctggtt 8640

caccacgcgg gaaacggtct gataagagac accggcatac tctgcgacat cgtataacgt 8700

tactggtttc acattcacca ccctgaattg actctcttcc gggcgctatc atgccatacc 8760

gcgaaaggtt ttgcaccatt cgatggtgtc aacgtaaatg ccgcttcgcc ttcgcgcgcg 8820

aattgcaagc tgatccgggc ttatcgactg cacggtgcac caatgcttct ggcgtcaggc 8880

agccatcgga agctgtggta tggctgtgca ggtcgtaaat cactgcataa ttcgtgtcgc 8940

tcaaggcgca ctcccgttct ggataatgtt ttttgcgccg acatcataac ggttctggca 9000

aatattctga aatgagctgt tgacaattaa tcatcggctc gtataatgtg tggaattgtg 9060

agcggataac aatttcacac aggaaaca 9088

<210> 2
<211> 13649
<212> DNA
<213> Artificial

<220>
<223> partial sequence

<400> 2
gaattcgagc tcggtacccg gggatcctct agagtcgacc tgcaggcatg caagcttggc 60

tgttttggcg gatgagagaa gattttcagc ctgatacaga ttaaatcaga acgcagaagc 120

ggtctgataa aacagaattt gcctggcggc agtagcgcgg tggtcccacc tgaccccatg 180

ccgaactcag aagtgaaacg ccgtagcgcc gatggtagtg tggggtctcc ccatgcgaga 240

gtagggaact gccaggcatc aaataaaacg aaaggctcag tcgaaagact gggcctttcg 300

ttttatctgt tgtttgtcgg tgaacgctct cctgagtagg acaaatccgc cgggagcgga 360
tttgaacgtt gcgaagcaac ggcccggagg gtggcgggca ggacgcccgc cataaactgc 420
caggcatcaa attaagcaga aggccatcct gacggatggc ctttttgcgt ttctacaaac 480
tctttttgtt tatttttcta aatacattca aatatgtatc cgctcatgtt aattcatgaa 540
ctagtcatgt attcggctgc aactttgtca tgcttgacac tttatcactg ataaacataa 600
tatgtccacc aacttatcag tgataaagaa tccgcgcgtt caatcggacc agcggaggct 660
ggtccggagg ccagacgtga aacccaacat acccctgatc gtaattctga gcactgtcgc 720
gctcgacgct gtcggcatcg gcctgattat gccggtgctg ccgggcctcc tgcgcgatct 780
ggttcactcg aacgacgtca ccgcccacta tggcattctg ctggcgctgt atgcgttggt 840
gcaatttgcc tgcgcacctg tgctgggcgc gctgtcggat cgtttcgggc ggcggccaat 900
cttgctcgtc tcgctggccg gcgccactgt cgactacgcc atcatggcga cagcgccttt 960
cctttgggtt ctctatatcg ggcggatcgt ggccggcatc accggggcga ctggggcggt 1020
agccggcgct tatattgccg atatcactga tggcgatgag cgcgcgcggc acttcggctt 1080
catgagcgcc tgtttcgggt tcgggatggt cgcgggacct gtgctcggtg ggctgatggg 1140
cggtttctcc ccccacgctc cgttcttcgc cgcggcagcc ttgaacggcc tcaatttcct 1200
gacgggctgt ttccttttgc cggagtcgca caaaggcgaa cgccggccgt tacgccggga 1260
ggctctcaac ccgctcgctt cgttccggtg ggcccggggc atgaccgtcg tcgccgccct 1320
gatggcggtc ttcttcatca tgcaacttgt cggacaggtg ccggccgcgc tttgggtcat 1380
tttcggcgag gatcgctttc actgggacgc gaccacgatc ggcatttcgc ttgccgcatt 1440
tggcattctg cattcactcg cccaggcaat gatcaccggc cctgtagccg cccggctcgg 1500
cgaaaggcgg gcactcatgc tcggaatgat tgccgacggc acaggctaca tcctgcttgc 1560
cttcgcgaca cggggatgga tggcgttccc gatcatggtc ctgcttgctt cgggtggcat 1620
cggaatgccg gcgctgcaag caatgttgtc caggcaggtg gatgaggaac gccaggggca 1680
gctgcaaggc tcactggcgg cgctcaccag cctgacctcg atcgtcggac ccctcctctt 1740
cacggcgatc tatgcggctt ctataacaac gtggaacggg tgggcatgga ttgcaggcgc 1800
tgccctctac ttgctctgcc tgccggcgct gcgtcgcggg ctttggagcg gcgcagggca 1860
acgagccgat cgctgatcgt ggaaacgata ggcctatgcc cgagcgcgcc ttgattcggg 1920
agcggcacgt gaaggcattg cgctggccaa gcagccgggc gcgtatccgc gccagaacgg 1980
cctggccgtg gcattgcgcg acgtggggcg catcgagcgc accatgttca ttctggactg 2040
gctgcaaagc gtggagttgc gccgccgtgt gaacgctggg ctgaacaaag gcgaagcccg 2100
caacgccttg gcccgtgccg tgttcttcaa ccgcctaggt gaaagccgcg accgcagttt 2160
cgagcagcag cgctatcgcc gcagcggcct caacctggtg acgacggcca tcgtgttatg 2220
gaacacggtc tatctggagc gtgcagcaca tgcgctgcgc ggcagcggtc acggcgtcga 2280
tgaccggctg ttgcagtacc tgtcgccgct gggttggtaa cacatcaacc tgaccggcga 2340
ttacctgtgg cgcagcagcg ccacgatcgg cgcgggcgag ttcaggccgc tacggctgca 2400
accggcttag cgtgctttat ttttccgttt ctgagacgac ccctgcttgt cctggcggtg 2460
gcgtttttcg cctcaacggc acaagcaagc ccaagaccgt cccggcaatg ctcgcagaag 2520
ccccgcaaac agccataggc gttttcttta ccaaccctag ctcaaggcag ccagaacatc 2580
caaaaatccg ctgtaggcga ttctgatggc ttctgttcgg gtatgaggcg atgtgctcca 2640
caagcacgac cgacaagcca gccgaaacgc tcgccaagct ggccgctgcg ctacctgccg 2700
gagaggccaa acgctaccgg gtgatggtgt ccattgtccc ggtggaagaa tgaaggcgag 2760
cggggcgggg cggggtgtgt ctggccgggc ttgtcccggc cgtttctttt ggggtttttg 2820
gtaaggactt ttgacgccct caagggcggc gaaaagtacc gcgcctagtg gatgatcttg 2880

gatagatcat ggagggattt tgcgaatcgc ccgcaaaccc gcgccgttgc taggtttttc 2940
agatttcggc ggggaacgtt acgccggggg ttggggaacg ttacgccggg ggttggggaa 3000
cgttacgccg ggggttgggc ctgctgattt cccacttgcc cttggcgtac tcgttcaccg 3060
tccaaccaac ggcggcaagc tcgacaagcg ccttgcgcgc ggtctggcgg cgctttttca 3120
ttgcttcatc gttggctgcg tctggccata cataaccgca cagcgtgtcg atttcagctt 3180
tgccagactt tccggggtca atccagccac atagccgctg gtgtagcagc cgtgccgggt 3240
ctgtttccag cttgcgaact tctgccatgt tgatgcgggt gtgcgggcgc tctcccataa 3300
ccgcatccgc cagccgggga ttaagcgcaa caaataactt gccttcttgc tcgtcgctcg 3360
cgtactcgga cagaatgcgg aacccttgcc gcttaccacc tctttcgaca atcaccgata 3420
ccgcccaaag ccgttcgatg ctttcccgga tggttttaaa ctgacttcct ccatcttcgg 3480
cgtagccaag ttctcgggcc aactggcgga agctgccctt ggctaccatc gtatctttct 3540
ctatggcgtc ccaccgcata tcaagccata ggcggagttg ctggcctgct tctgattccg 3600
tttcgtgccg tagcacgatg ccgcgcccgt tatctccgga aattgcagcc attgccacca 3660
gcccttgcaa tacgcgcaag tcatcgccgc caagtggttc aggcccccaa aaacggattg 3720
agtcatctcc gtagttgtag gtcacatcga gcttgagcct ctttcgttct ccgcgcttga 3780
gactgcggaa aagccccggc gtgaggcaat gcgcggggtc gtgccgcgca tgggtgaggt 3840
catagctcat tgcgctttcc cccttttttt cctgctccat tgctgattgc ctcctgtagt 3900
tcaacgggca acaacacccc gccgctgtgc cgctgatacc agcggtctag cggggtcgcg 3960
tcgtagttct gcttgctcac gccaaagcgg acaaaaaggc cgcgccgctc gttgccggat 4020
ggcgaacgat caaagccccg gtcactcatg cgctcggctt cctgctccgt cattttggcg 4080
acatagccgc accatctggc gttgtcgatc agggcagacg cgccccgcgc tgcctgctgc 4140
tggtcggtct ggccgtcgcg ggcgctgccc ttgttgacgt ggtgcaggta caggacagcc 4200
gccccggtgg tcgccgcgat gtgttccaac acggaaacaa ggtgggccat gtcgccattg 4260
ctgttctcgt cgaggatgtg aatccggctc agggtgtcca gcacaatcag ccgggcccct 4320
gcgctgtagt cgatgacgcg gcgcaagtgc gcgtcgtcca tcacgtttag ccgcttgccc 4380
atgatcggct caagcatcag gttctcagcg atggcttcgc gggccgactg gttgaggtgc 4440
tggccgatgg cgtggacacg tcgcacaagg gcgggctgtg gatcttcgcc agcgagataa 4500
accacgcgcc cggtgtgcgc cggggttagt cccacaaggt cgccgcctgc cacactgcaa 4560
gcgattgaca tggccgcttc aagagcaaaa aagctcttgc cagttgcgcc aggtgcgaca 4620
agtgcgccca cggttccggc caagaatccg ggccagatga aatcaagttc tggcggctca 4680
ttggtgaagg ccgccataat gtctaaagcc atgcttgtct ccttttcaga cagttgtggt 4740
taggcgctgc cggggccgca atcccctgcc gcgccgctca attttcgccc ctcgatgcca 4800
gccacgccga agcctcggcc aaggcgtcct cataggcgta cgtggccgct tcttctgcgt 4860
tctctgctgt ggcgtactcg gacaacggga acactccgcc gccgacttcc tcgccgcttt 4920
ccaacaggcg caaccgccag ccgccgccaa ggtcggaggg gcgcgggtcg atctggtagc 4980
tgtattcgat cattggcggt aaacctcctt acgattgccg atgcgcataa cgaggatgcg 5040
caaagcaccg tcctcgatgc tggcgatgat tcgccaatcc ccaacgcggt atttccagaa 5100
ggctcccagt ttggagcctt tgagggcttc gccaatgctg cgcgggtcgt cgagctgggc 5160
gacacggcca tgcaaaaaag cgaggatgcg gcgggcggtc tgctggtcga tcttgcctag 5220
ctcgcgctcg gcggctgggt cgagttcaac cttccatgcc atagcgtttc atcacttctt 5280
cgagtggcac ggtttgggtt ttgcctgcgc gaatgtcgat caggcgttgc tctgcaaggt 5340
acaaatcttc gaggtcatcc aagtgctcaa ggatggcttc gcgggcatag aaagtcttgg 5400
tccgccctgt ggcctgcgcc agtgcttcaa ggcgggtttc cacttcggcg ggcagtcgga 5460

ttgcaagcat gtttcggctc ctgttctgaa aacgctatac aagtatatca caaccaccac 5520
gcaacgctag agactcattc ccggcgagtt gtcgcgctcg atcttctgcg cctttgcctg 5580
ctcctgctgt cgtacaggct cgcgcccttc cagcttctcc cactgctgcc ggtacttgcc 5640
aagatcggcg gcttgcggct gtgcggcgct gtaggcatag cgggcggtgc gctgcgcgta 5700
gtcgttccaa tcgcggccct cgtctttctg ccgggtggcc ggtgcgcact ggcggatagc 5760
gccctcgatg gccgcttgat cgtggccggt gacgcgcata cgcacggcaa tcatggaatc 5820
cacgcgggac aagtccacct cgccgccacg ctgccgcttg agcacgtcgc ggtaatgccg 5880
ctggtaggcg tcgatcgcgc tgccgctggc cgctgcaagc tccaaggcgg gcttggcttt 5940
cgtgcgctcg ggctgctgcg ccttcaaggc cgcttgccgc tgatactcgg cgtcgatctg 6000
gctggacaag gccagcgcct tgacgcactc ccggcgctcg gccttgagca agcgcacctc 6060
tggatagctg ccgtcctccc gctggtgctt gggcttgcgg ttctcgtagc cgggtgcgcg 6120
gtgcgggtgg atggctcccg atagcttcgg gtcgccgtat tcacggttca gggcatcgct 6180
caggcggttg cccacgtcct tatcgtgggc cgtccccagc ttcgacacgg tgatgatggc 6240
ctgatagttg ccggggctgg attccagcac ggcggcgggc tggtagccgt ctttgataag 6300
ccgctccagc ttctcccggt tcatgtcgtc gatgaggatg tgatgcttct tgtccgatag 6360
cggcgtgtag tagaggtttt cgccccggcg ctgtaggcgc tgcatctccg gcgtgcgctg 6420
ctcgatctcc tgcggtgtga aaccccgcgt gatgccgtcc ttcttgtcga ggatgaaggt 6480
ttgcttcctt ccgtctgcct gcatcttgat ggacgttacc cggtagcgct ccgcgcctac 6540
cgcttccgcg taccgctcga actgctttag ctgctccgat ttcatcgcct gcgccctctc 6600
ctgctgtatc cgctgccgct cctgctggat gctctcctga agctctgcat ttgtgatctt 6660
gaagccgtgc tctgctgcta acttcccgca catggccttg tattcgtcgt tcccggttac 6720
ggtgaagctg ccccacttct gcgccgatag ctgcaaggcg gcaagcgtgc tgtcccggtt 6780
gcgccagtcg tgaatatcga tgcttttgcc cttgtccacg aaagacacac ccccgccccg 6840
ccccccttct tccttccggc tgtaatgcac ctgctggccc acgatttcag gctgataggc 6900
gcgaatgtcg cgcggcgtcg gcggctcgct gcggtcgccc tcgatgcgct gcggctcgct 6960
ggcccggtgt cgccactgct cggcaagctc cgggcttttc tgcatccgct gccactgctc 7020
caggtcggga tagggtctga actgctgccg gtgctgctcg cgctgcttct ggtgcttctc 7080
cttcaaggcg gctttctctg cggcctgctc ggcggcaatc acgctgcgca tggcgttgag 7140
cacttcgccc ttgcctttcc agttgccgcg catgagttca tcgcggcggg ccttctgctg 7200
ctcggcaagt tgcttgcgct cctgctcctg ccgcttgtcc tgtaccagct tgtccgcgtt 7260
cttctccgag tagtgcgcct tgcgcccggt gatgtagtcc ttccatcccg gcacgtctgg 7320
cttgatcggc tccggctcgc gctgtgctac ctgctgacgc tgcggcggct ggtaagctcc 7380
gagtcgcttt tgcaacttgg ataggctcgc gtcccggtct gcgctgctgg ccttcacgcc 7440
aacatcaccg acaaagagcg tcgccccgct gcctgttttc tcgtagcgca tccccttggc 7500
tgctaactcg cggtgcaact gctcccacgt ctgcgccttt ttgatgatcg gtgcgccgtc 7560
ctcaatggcg atgcgttcgg cggatttctc cccggtgcgg ttttccatgt cgcgcttcgg 7620
ctgggctggc tggcgcggct tgtccttgtc gatgtgctcc cggcctaact caccattctc 7680
caacacctga taacgcccgt tctgctcacg ctgccagcct tgtgcgttct cgattcgtgc 7740
tatcgccttg tgcgctgcct caatgtcgaa gccgttattg atcttcacca ccttgagcgt 7800
ttcaggatgc acgcggttga tggcaaggtg caggtgcaag ttgtccgtgt cggcgtgcaa 7860
accatagatg gcttgatggt ctttaacacc tagctcatcc atgaaaatgc ttaccgcttc 7920
ttcgacctgc tccgggctgg gctgctcgcc ttctcgccaa ctcaggacat agtgattgat 7980
cgtatccttg cttcgcactg cctcttgtga caaggcaatc atttctgccg tctggctctg 8040

tggttcatcg gtgatgaatc cacgcgcccc ggcatggatg cacttctcct gcgagttctc 8100
acgctcaggc tcacggatgt agccagttag gcgggaaacg cgaaccgcct tgcttgctgc 8160
cttctgcggg ttcttaacct tcttgacgat cacggctcag tttcccgata taggctttca 8220
agaccacaag cgccccggct gtgtccttgc tgtacacgcc tccgctctcg ttgtggatgt 8280
gcttaagcaa tccaccgatg cggcgcaact ctttgagcat taccgcgtcg gcgctggcaa 8340
tgatcggcct gccaaagtag cggcggcgga caagctccga catgctcaac ccggcaaggt 8400
cggcatcctc tttcaagcgg gccttctccg ctgcggtcag ccgtacattg ataacggcgt 8460
ccagcggttc cggcccctgc gtttcaaatg gcatcgtgtc tccttctcgc ggggggcggg 8520
cggggtgtgt cttggacagt aagaagcgag agcgccgcac tgtccggggt caaggggata 8580
ccccttgcag gataagccag accgtgagaa gcgaagcgcc gaatgggcag gcgtccgttg 8640
gaaccacgtg gcgaagccat gtgtgttaca acggtcatcc tgtattgctc aaccgctcta 8700
ctatcatatc agcagtttag gcacactaga tgcactttgc aacaagcata aaaggcgcta 8760
tgctgtcaac tccgaaacca gtaagcaaag gagttagcag aatgaaatac acgacagagc 8820
agctagaggc tatcgcatcg aagctgcgtg atatgccaca agtcgaaaaa aagaagcagg 8880
agcacagcaa gcaagaggcc gtgcgcgtgc tatcaaaaga gatcgcggct ttgcaaaagc 8940
gcggatacac gcttgaccag atttcggaaa cgcttcgcgg tgaaggtctg agcatcgcaa 9000
ccccgacact caagagctat ttgcagagag caaagccaac gaaaaaagcc ccggtgcaag 9060
cgccgggaga cacacccccg cccgcccccg ccgtcaagaa gcctgcggat acatcgaagg 9120
cgacatttac cccaaaaccc gacagcgacg acatttaagg agtaagcaac atggcaaaaa 9180
gcatttacct gatcggcggc agcaagggcg gcgttggcaa gtcgcttgtc acgatggcaa 9240
cggttgatta cctgcaagaa cgcggcgaaa gcgtattgct gattgaatcc gacacctcta 9300
acccggacgt gtggaaggca tacaaggaaa gcacggaaac ggagcttatc aatctggatg 9360
aggcggacgg atggattcag ctagtgaact tgtgcgatag caagccggat agcgtcgtgg 9420
taattaacgc ggctgcgagg aataacaagg gagtgagcgc ctacggtgaa acattgaata 9480
gcacacttgc ggaactcaag cgcaagctgg tcacgctgtg ggtgattaac cgccaacggg 9540
atagcctcga actgctcaag gaatacatgg atgcaatccc gaacgcggat gttcatgtgg 9600
tgcgcaatgg acacttcgga gaggaaaaga aattcgagct ttacaacggc tcaaagctgc 9660
gcacggcagt agaggaacgc ggcgggcagt cggtgacatt cccggatatg gccgaccggg 9720
tgagcgatga catttacagc aagcgcatga gcatttcggt agcactcaag gaactgccaa 9780
ttggcaaccg tgcggaactt acccggtggc gcaatgaggc taaaaaggta ttggagggcg 9840
taatccatga gtgaccttga cgacagcttt gcaaagctgc tcggcaggca acccagtgac 9900
gcggagcggc aaagccttta ccgtgtgcgg gatgcactcg ggctgaaaaa caatgatgcg 9960
ctctggctgg tgctcatggc cttgcagcac taccaaggcc agtatgagaa gttcccgcaa 10020
gcgatagcac aagcagccaa agataccttg gtcaatttca aggtaacggc ggatgcaacc 10080
gtgaaagcat cagcggaagc agccaaggcg gatttagcgc aggcggtagc agcagcagcg 10140
caagaggtcg cgcacaacac atctgcaaag cagatgtggc agtgggcggc gggctgcatc 10200
gccgtggcct tcctgtgcgt cggcctgttc ggctggtaca tgcacagcag cggcaaggat 10260
tccggctacc aagccggata cggcgcgggc tacacggaag ccaaggacga gaaagcggcg 10320
gcggcatggg caaacacgcc agaaggtcga acggcctacc gcttcgccca gtccggcgaa 10380
ctgcaacgcc ttgcccggtg cagtggcaag ggctggaagg tcgagaaagg cgcttgctac 10440
ccctacccag tagcgaatga gggcacttac ggatgggcgt tgccatgatc gacgcgctca 10500
acatcgccac gatgccgatt gcagacaagg tgcatcggca catgctggca agctactacg 10560
ccttgcaact ggatgccttg caggccgaag ccaagcggct cggctacttc ttcgcgcaag 10620

ccgacacggc ggcaaccatg ccgccagtgc tggccgattg cttggcgtgg gcggtcgagt 10680
accggcgcag gtgctacctg tacccgaatt gcccggaaag ctgggaacgc cacaccgccg 10740
acagcatgag cgacggttac gcccaagagc attgccgctc gatgctcgcg gcgctggccg 10800
cgctcggcat ccgtttgcag gaaggccagc aagcctacgc cctgctggcg gcggaggaat 10860
gccgccaacg cgagaaagcc agcttgccac ccgagccttc gccactctcg gaagtcgaag 10920
tgtccagctt cgtcgatgag tttttcacca agctggacgc acccaaggag gaagtcccga 10980
catgagcaca accgccaaga tcgactttga aaccggcctg ttcgatgagg gccagtacga 11040
ggaagcgcca gacctgccag ccatccagcc gaaacggcgg acaagtcgcg ccccgttcat 11100
cgtggccggt ggcttgctgc ttggggtcgt ctcagaaaac ggaaattaaa gcacgctaag 11160
gcacgacaag cgtgcgcata tgctgcttca cggactgaag ggctgttttt cctgttcccg 11220
gtgcgggcga tccgggcgtt gcgcgaaacc gcataacccg ctcagttagt acttgacgtt 11280
gaagtaaggg ccaaggtcta tcgtaatgac gcgcggcgct catggcttct ccggccggga 11340
gttctcgcgc acctgaccac acttttttgg agaaatacca tgactaccaa acgcaaagtc 11400
gaagttttca gcgcgggatg cccctcctgc cagaccgcca ttgaactcgt caatcgcctc 11460
gcatgcggtt cgtgcgaggt ctcaatcctc gatatgaacg acatcaatgt ggccaaacgc 11520
gcccgtgatc tcggtgttcg gtcggtgccg gcggtcgcca tcaacggcca gctggcttcg 11580
tgctgctccg gcagcggcat cgaggaacag gcgcttcgct ggcctcggca agcctaggcc 11640
tccccatcgg cacgcgcgtg cctctcgctg gaagctcgcg attaatttct taggcggaac 11700
tcttgaccat ggtgttcact ccaaagttta tattaacact tacaaatgga ggacaccatg 11760
cacaccatcg gcaaggtggc aacgctagcc ggagtcagca ccgatacgct gcgctactat 11820
gaaaaagagc acctgatcga acctgcatct agaaccgctg cgggctaccg gctgtacaac 11880
gatgaagcgg tgcggcgcat ccgttttatc aaaaccgctc agcactgcgg attctcgttg 11940
tcggacattc aggagttgct gacgctgaag cgaactgata gtgcctgttg cgaggatgtg 12000
cgcagcgtgg ccatcgagaa gaggctgcgc atcgcgcgca aactacgggt cttgcaggcc 12060
atgtcggccg ccctcaatga cttgatccag agttgcgaag gcggtgctgc attcgcgaac 12120
gccagcaaga cgtagcccag cgcgtcggcc agcttgcaat tcgcgctaac ttacattaat 12180
tgcgttgcgc tcactgcccg ctttccagtc gggaaacctg tcgtgccagc tgcattaatg 12240
aatcggccaa cgcgcgggga gaggcggttt gcgtattggg cgccagggtg gtttttcttt 12300
tcaccagtga gacgggcaac agctgattgc ccttcaccgc ctggccctga gagagttgca 12360
gcaagcggtc cacgctggtt tgccccagca ggcgaaaatc ctgtttgctg gtggttaacg 12420
gcgggatata acatgagctg tcttcggtat cgtcgtatcc cactaccgag atatccgcac 12480
caacgcgcag cccggactcg gtaatggcgc gcattgcgcc cagcgccatc tgatcgttgg 12540
caaccagcat cgcagtggga acgatgccct cattcagcat ttgcatggtt tgttgaaaac 12600
cggacatggc actccagtcg ccttcccgtt ccgctatcgg ctgaatttga ttgcgagtga 12660
gatatttatg ccagccagcc agacgcagac gcgccgagac agaacttaat gggcccgcta 12720
acagcgcgat ttgctggtga cccaatgcga ccagatgctc cacgcccagt cgcgtaccgt 12780
cttcatggga gaaaataata ctgttgatgg gtgtctggtc agagacatca agaaataacg 12840
ccggaacatt agtgcaggca gcttccacag caatggcatc ctggtcatcc agcggatagt 12900
taatgatcag cccactgacg cgttgcgcga gaagattgtg caccgccgct ttacaggctt 12960
cgacgccgct tcgttctacc atcgacacca ccacgctggc acccagttga tcggcgcgag 13020
atttaatcgc cgcgacaatt tgcgacggcg cgtgcagggc cagactggag gtggcaacgc 13080
caatcagcaa cgactgtttg cccgccagtt gttgtgccac gcggttggga atgtaattca 13140
gctccgccat cgccgcttcc actttttccc gcgttttcgc agaaacgtgg ctggcctggt 13200

tcaccacgcg ggaaacggtc tgataagaga caccggcata ctctgcgaca tcgtataacg 13260
ttactggttt cacattcacc accctgaatt gactctcttc cgggcgctat catgccatac 13320
cgcgaaaggt tttgcaccat tcgatggtgt caacgtaaat gccgcttcgc cttcgcgcgc 13380
gaattgcaag ctgatccggg cttatcgact gcacggtgca ccaatgcttc tggcgtcagg 13440
cagccatcgg aagctgtggt atggctgtgc aggtcgtaaa tcactgcata attcgtgtcg 13500
ctcaaggcgc actcccgttc tggataatgt tttttgcgcc gacatcataa cggttctggc 13560
aaatattctg aaatgagctg ttgacaatta atcatcggct cgtataatgt gtggaattgt 13620
gagcggataa caatttcaca caggaaaca 13649

<210> 3
<211> 35998
<212> DNA
<213> Artificial

<220>
<223> partial sequence

<400> 3
   gaattcgagc tcggtacccg gggatcctct agatgacctg attgtgcgta ttgcgaccgg 60
   aaccctccgg gcgattctcg ccgtgggcac tggccttgtt tacgttcaat tcgtagggct 120
   ggcctttgtg ctggcgatgt ttgggaccgt gacttttgcg gtgattgtgg cggccgtcaa 180
   gaccgcgatg tttggagctt aatgcacatg gcaaccaaga caaagaaggc acagcacacg 240
   gccgaactgg tgaagctctc cgctgacctg ctgccgttaa ttgaagccct ggaagtcgaa 300
   atagagacgg tttcccagcg catggcggcg ctgaaaaagg ccggcatcat ttacgcatcc 360
   gaacattggc gcaaggaaaa gggcgagccg aaatacttct atctgctgta tccgcagcag 420
   cccggcgagc cgcgcaagcg cgaatacatc gggtgcgatc ctgagcgcat cgcagaagct 480
   cgccagggca tcgcccgttc caaggagtac gacgagcttg ccaccatcct agcgggcctc 540
   accagtcgcg tgcatcacgt tacccaggcg atgcacgaag ctcgccgcta ccttaccggc 600
   aagcgctggt aagccccttt cgcccttgcg gagactgcgc acatggactt gtcgcacggc 660
   tttatccccc tagcgctgaa tgcgtcactg tccgcgctcg atgccctcgc cgctggcgac 720
   gggcttcgct ccgatgacct gatcgcaggc gcaatcgcgc tcgatctgct agctagccaa 780
   ggtcattact cacatacgcg cttcaacggt ttgatgacgg ccgtgcttgg cttgcgcatc 840
   ctagcgacgc ggcaaagcat ctctgaaagc caggaaggac gccgagcggc gacgcacttc 900
   gcggccatcg tgcgcgaaat gcaaaaccat cttttcaact gaccaccacc aacagcagga 960
   gaacaaacca tgaaccaaga cgatttgcgc acgcttacgt ccaccccgga gccgatgacg 1020
   gccgcagaac gggccgcgct gcaacggttg ctcgctatcg ccaaaagtga caccgggcag 1080
   tcccggcgca ccgctgattt tctgctggca tggtggaacg ccggtagttg cggcagcttc 1140
   gaccttaccg agctatgggg cctcgacacc gccattgctc aagacatggt gacggtgttc 1200
   gcgcttgtcg cgcgggtcca cgtctacccc gacaccctcg gcttcaaggc cgactttgaa 1260
   gccatcgtgc gtgagtggcg gccggagctg gtgaaatgaa tacgcagacc accccgaaga 1320
   agcgcagcaa ggtcaagctg gccgttttga tcctcctgag cctgggcggc tgggccgccg 1380
   ccattggcac agggatgacc ctcggcgcgg cgaaaaatca agccgttgag gatcaacggc 1440
   aagtgcagtt gtggcgggcc aaggcggcca aggcttacac cgaattgctc aaagccaagt 1500
   ccggtacacc tgggcaaatc acggtcccgg ccggctcact ggtggactgc gcagcaaatg 1560
   gcaccggaca gctagagtgc agcggcttga tattgccgcc cgccgattac tgactggcga 1620
   cagcaaacgg acctggccag ctcggccgcg tcaccaaaca agcgaccgtt aagcaggtaa 1680
   aaccgcatga aaatcatgtt cgcaatcgca cttctgacgc tcaccgcaaa cgccatggca 1740
   gcggagccag ccgtgaacat agcccccggc acgtcggtta agtgcgctcc attcccgact 1800
   atcaaaacgc tcaatgcgag cctttttgag ggcgggaagc tggtcggcgc ggggcaagtg 1860
   gcgtgctacg tcatagacaa ccagccgcta ccaaagggga ccaaactagt cgcggggcaa 1920
   gtggacggcc ccatagccaa ttcgtacacc ctggcgtggt cagctctaca gctacaggat 1980
   ggctatgtag tgccattgga taacaacttg attgtgactc ggccgggggg gccggatcat 2040
   atcgttctca catttcggcg cggcctcgat gcgggtgtga gtgcccagga agcggcggaa 2100
   aagtagtcca tccccacgga aagcaaagcc cggccaagag ccgggttttc tttttggcga 2160
   cggttgagca gatccagggc gggcggtcag tcaccaaagg ccggaagcgg tgtcaagggc 2220
   gggaccggag gccgcagccg tgagcgcagc gaggggaccg ggcgaggatg ccggcgaagc 2280
   gaacccttga tgccggtgga gtgcccccca cccttcgcgc agggatgcag ggagctttac 2340
   cccctgcaac cctgctgccc ggcgagggac ggagagtcca gaggggcagc gcccctttgg 2400
   gtaagcccgc cagggcgcgc aggaaggcca cagcgcggcg cagcaggtct tgccggtgcg 2460
   aacccacgcg ggcgcaggct ggagggccac cacgggccgt taaagccaca aagcgggcct 2520
   gtggataacc ctgttatttc agcgacttat ccacgctgaa agtgtcttgc gcgcagagcg 2580
   ttgatttacc taggcaaaaa gcacttctcc acagaatcgc acgctgaaag tgtcttgcac 2640
   cctcgcgctg aaactgtctt acgcaccgcg aaccaaaacg gccgcaaagc cccgccaatg 2700
   ctggctattc tggccgtcgc cgatggctcc ccctgtagtt ttctttaaag atatacctat 2760
   aaaaaatcct gtaggttatg gcgcggcgtt gtgaacaacc gccgaaaaca aaagcgcctg 2820
   cccttcgtgc ttcgcactcg ccccgccgcc tacggcgacg gcctgtcggt gggcaagccc 2880
   acctcaaaaa tgtgtgccct ttgggcacga ctctttacca aaagccggcg actcgactat 2940
   tgcgtagtgg atggtctaag cggcccggcc gacaagtcgg ccgccctcac ggcaagccgt 3000
   gacccctgcg gggcgctaag acccggacaa atcacgcccg accggctacg ccggtcgccc 3060
   gctcagccgg gttcgtgtgc gacccatcca tgaaccgtcc gcgctggtgg agaggtcgaa 3120
   aaggttttgc agggaaaagg aaggggtcgg ttgaactcgg agtgcccata ggaccgccag 3180
   agagggccgc cagagccgtt ttcagcctga tttgatacct cgggcacccc ctgccctgaa 3240
   agcgcgctgt agggcttcct atcggcccgc gtgattctga gtgaactaga cggcgatggc 3300
   cgcttacgca cattccctct aaccctcggg tttacggcca tatcaggcgc ttctatttgt 3360
   tttcccgttg ttgtcttttg ttttcctgtt gttgtctttt ttgttttccc ggtgttatac 3420
   tggctgtgcg gtcaatgtct ggccgtgcca cctggagaac gtcatgcaga tcaacgtgtt 3480
   tgaaggtcat cttgcgaaaa ccccaacaat cagcgggaca ggcgaccgcg ctgtaacccg 3540
   tttcaccctg atttccaacg aatacgccgg caaggatcgc gaaacaggcg aggcccgcga 3600
   gcggtcggtt tcgatccagt tcacggcctt tcgtcagaag gccgaaacca tcgcaaagaa 3660
   cgcgatgaag ggcgaccagc tcatcattca gtaccgaatc gagaacaacg actacacgaa 3720
   agacggcgaa accgtctacg ggtacaactt cgttgtcgag gacttcaagt acggtgcgcc 3780
   cggcaaggaa aaacgagagc aattcaaggc gagtgtgtga gcaatggcgt cccctacccc 3840
   aaggatggac gccgcgcagt tcgaccgcgt agcgtccaaa tgcagatggt ctgagcggtc 3900
   cttgggcgtt gtgcgcgctg tattggttga cgggctgtcg ctgccggatg cggtggcgac 3960
   ggcaataccg gcaatgacaa ccaagcaagc gcgagttctt cgggacaggt tcaccgcgaa 4020
   agcggagagc ctgcgccttg aggaattcat gcgacgcgaa acaccaaagc tcgctaccac 4080
   agcactagag ccgtatgcca gcgaagtaag cacccttcgg gacaagggtt acacgattga 4140
   gcaaatcgtc gcgttcttca aggcaaacgg tgtgaagaca tcaccgacga ccgttcggaa 4200
   ctttttaagg agcattcggg catgaaaacc actgtacttg cgaaccagaa aggcggcgtc 4260
   ggtaagtccg caatcgcggt tcaattcgca gactacttgg cgcttttgcg caacaagcgc 4320
   gttctcgtta tcgacttcga ccatcagcgc aacacgtcga aagcgatccg cacaggtggc 4380
   cttgccactg tatcggccgt gaccagcagc atgattttga agaacaaaat tacgagcatc 4440
   gagcacgctg attttgtctt gatcgctggt gatgctgact tgatcaaaat ggaaaaacag 4500
   gcggccgagc acaacgcttt cgcgacgaac ctttacacgt tcctggcatc cgtctccgat 4560
   cagttcgatt tctgcatcat cgacaccaac ccgaaccccg acattcggca actcgcttcg 4620
   cttgtcgtga gtgacttcgt gctgtcgcca ttgcagttga accaggaggc aatcgacggc 4680
   atcggcgacc tgttgaatca cgagaacatc ggtattcgca agatacaggc gaccttgaac 4740
   aagaaactgc aactcattgg cattctgccg aacatggtcg aaccgacacc gttccagcgt 4800
   gacaacttcc gcgacttgtc caaggctttc gcaaagctgc tgatccccct tgataacggc 4860
   tttgccgcca tcaagaaaac caccgcgatt ccagaggcgc aggcccaggg cctgcccgtt 4920
   tggaaagtag gcaaaaccag tgcgcgcgaa gcctggacgc agatgcgccc gatcttcgac 4980
   aagatcgctc agaccatggg agttgaacaa gatgcttgac ctttccgcct tggaagaaaa 5040
   gccaaccgcg cccggcgcgg agccgaccgg caaagccatg cgcgccaagc tgaccgatgt 5100
   cgagccggac cccggccagc cgcgcaccga gaagaagcag gaggagattg aagaactcgg 5160
   cacaaacatc aaatcgcgcg gcgtaaagca gcctatctcc gtcaagaagc acccaaccaa 5220
   gccgggcaag tggcttatca atgacggcga gctgcgttac ctcagttcgt tgtgggccgg 5280
   cgtcgaagac attccgattg tcgttgacga ggattttgac gacttcgacc aagtgaacgc 5340
   caatgaaaag cggttcgcat tacgaccgat ggaactcgcc gtattcatca agcgtaagct 5400
   tgatgaaggg gtgaagaaag ctgaaatcgc tcgccgactg gggaagccgg gcagtgctat 5460
   cactgaattg ctggcattgg tggatgcgcc gcaatgcatc caagtcgtct acacctctgg 5520
   tcgctgcacg tcaccaaaga cgctctacga gttgcgggct ctacacgaca agttcggcga 5580
   gaaagtcgag gcatggtgcg aggcggccga ggaaattact cgcaagggtg tttccgaact 5640
   cggcgacatt ttgaaaggca agaagaagcc agcaccgggc agtgacaaca tcgctggtgc 5700
   ggaccaaacc agcggcggcg gatcagaaag tcttagtcat gacgaagact ttccagggcg 5760
   agacggttcg caaaacagcg acagcggcaa tggtggggga cgtgagacgg aagacgatga 5820
   ccaggatacg ggcggcaaaa agtcgagcgc tagcggccag ggcaagggcg agggtgacga 5880
   caatatcgac acgggcgagc tgacgagttg gccgcgcggg aaggcggtat ctgatcccga 5940
   tagtatgaaa aagccgctgc tgatcgttga gcacgacggc cgcgcggcgg cggttctgct 6000
   taaccgtcgt ccttccaccg ctggactgat ccgcatacgt tacgaggatg gcggcggcga 6060
   cgccgaagtc gatgccggtt cactgaaaat caacctgctg accgaggccg aaaaatgaac 6120
   gccaggaagg cggcccgtaa cggtgtcgcc ggcctgatcg tggccgggct agtgctcgcg 6180
   tttgttgtcg gtaggcagca gacgcgggcc gcaacaccga ccgaattttg tacgctgcat 6240
   ttcagcggcg gcgtgatgct tgaaggcgta ccggtagcga agacgccaga gcaagaggcc 6300
   aggggcctca tgcaccgcga cgatgccggc cccggcatgc tgttctcctg ggacaaagcc 6360
   gaaccgcgcg ttttctggat gcggaatacc cgcatgccac tatcaatcgg cttcatgtcc 6420
   gcagacggaa ccttgttcgc catcgaggac atgcagccaa acagcgacga gtaccatttt 6480
   tcgcaagacc ccgcccagga tgccctagaa ctcgcgcaag gcgatttctc acgcaggggc 6540
   ctgggtgtgg gttcccgcct gctacgtcga gagtgccagg accggaacac gtattaggct 6600
   ggggccggtg tcaagggtga aaccggaggc cgcagcccgt agcgaagcgc agggaccggg 6660
   cgaggatttc agcgaagcga acccttgatt ccggtgtaag ccgaaaaccc ttcgggcggg 6720
   gttgcaggag agtttttccc tcctgcaacc ctgctaccgg cgagggcgga gagtccagag 6780
   gggcagcgcc cctttgggca agcccgaagg gcgcgcagtc tgacccatcc cattttttgc 6840
   ttcacaactc actcgacatt tctcgttcgt gttctgcaaa accacgtttc ttttcccaag 6900
   ctcggcattc cttcgtcatg actaaagatt agaggcttgt gaggtctatt gcaggatagg 6960
   ctaacgccgg taaagtgtat tacactacgt tcatacgtat gtccccttaa ttggattgga 7020
   ggcgcttgtg ccgcgaaatt ctgagattgg agccacccga ggtagctatg tctatacctt 7080
   gcggctgccc gacgatctgc gtgatcagtg gacggcctac tgttcacgca acaagaaaaa 7140
   agcggctgcc acgatgcggg cgcttatgcg ttatttgatc caagacgata tgccgcccga 7200
   ggttcgtcgt tggatcgctg cgcaaacaga gggcgagcca gacgacggtc caaaggaacg 7260
   tctcgaagtt cgcctaacac cgacagagtt ccagtcggta aaagttcgtt ccgaggccga 7320
   aggatgttcc ccccagcgct gggtcataaa cagcgttcgt gcaagcctca cccatgaacc 7380
   gcaattcacg atggaaacga cgaaagcgtt atgggagtcc tcttaccagc tccgcgcaat 7440
   cggtcggaac ctaaaccaga tcgccaagcg cttgaatgag gggaagtcgg ccaaagtcac 7500
   gaccgaacag atggaaaagc tagcgagcta catctaccag cacacggaca aggtggcgtc 7560
   cgtccaggac gcgagcctta gccgttgggg cgtacaaatt caggggatcg ccgatggcaa 7620
   agcgagagat tgacggtgtt ctgaaggatt ggggcgagcg tgtcgattac ggccgagtcc 7680
   agggccgtaa aggcaagaac attgccggag gtcgctattc caagccggcc ccggaaaaga 7740
   aaccatcggg ccgcgagaag cttgaagcca cagtgcgtaa agcacccgaa gtcatggtga 7800
   aaatctccgg gggcggcaag gatatgcgca gtatcaaagc tcacatggac tacatatcgc 7860
   gcaatggtgc tgtcgagctg gaggacgagc agggcagggt tcaccagggc aaggaggacg 7920
   ttcgagccgt gcgcgatgac tggcgaggcg gcggcatccc gtacgaggat ggcaccaagc 7980
   gcgaagcgtt taacatcgtt ttgtcgatgc ctcctggcac gaacaggcag gcagtcaaag 8040
   atgcagcgcg ggctttcgcc gccgagctgt tcggtaatca tcaatacgtt tttgcggcgc 8100
   acgatgacga gaaacacccg catgttcact tggcggtcaa ggccgttgac ctagatgggg 8160
   tgcgcctcaa cccgcgcaaa gcagatttgc agaattggcg cgagacgttc gccgaaaagc 8220
   tccgggcgca agggatcgac gcgaacgcga cgcctcgcaa ggcgcggggc attgtcagaa 8280
   aagcggaaaa acaagccgtc gtccacatcg agcagcgggg taaaactccg cgcgtcaagg 8340
   cgagtcggcg cgcagatgcg gaacgcgagg caagcggcgt ggcagtaaag cccaatccag 8400
   cgcaagacag aatcagcgtg actcgaaaag acgttcagcg cgcatacggc acggccgccc 8460
   gtgcgctggc gaagggcgag gccaaggaca agcagttggc cgtcgagatt gttcacttcg 8520
   tccagcagat gccgccgccg atcacgcagc acgaagccat ggtgcggcag cttcgcgaga 8580
   agaaacggga tgatgcgcgg actatcggtc ccgctaagac caaagagact caacaggaca 8640
   aggaccaggg cggccccgac cagtcgcgct aatcttggtg tatatcagcc aaagtgatat 8700
   attttggttt catcgtatag ggtattaagg gggctgtatg tcacagcgca acttggaaag 8760
   ggcacaggtt ttcgcggagc tagagcaaca tctcaccgag gcggaacgcg cgaagttcga 8820
   agaatggaag caagagaacg ccccaggtga aggctcgacc tcgccggagg attggcctgg 8880
   atggtgcgac gttgttatta gacacgccag tcgtgaacgt tcggtgagct tggagacagt 8940
   ttcaaacgtg ttctattacg ctgcctgcgg cgttgccatg ttatgtatgg cattcgtttt 9000
   tgtgtcaggg cttctaaccg tcctgggcgt gatcgatgat gggaacgaaa gcactgccgt 9060
   tatcaatgcg aacctgaatg gcggcgcgca ggtcgttttt cttcttgtca tcgcgcacgt 9120
   ccttatgaaa ctggtgggtc gaagtttcaa gcgccggacg aagttgctgc catgacccta 9180
   ggcggatgac aaaaaaagcg cggcatatgc cgcgcttttg ctttcccatg gggttgacgc 9240
   ttagcggtcg aattgccgcc tttgattctg atactggccg ccgtttgcgt cttcgtccgc 9300
   gatccgcttc tggtactttg ccagagcggc gtcgtagtta gccgggtcta cccaaaaacc 9360
   gccgcgatcc ggcgtgccaa cgtaccgggc cgcaacgcca ttggtttgat cgacactatc 9420
   gttactcgcg taagccgagc aatgcgaagg catcgtgttg cggatagtcc ggtggccgtc 9480
   atcgcgatag ctccagacca tttgttgcga gttcaaagca gcagcatcgc aacggcccgc 9540
   accgttggcg attgcccctt tcaaatcgct catttcacct tgttcatcgg ggcacatgtc 9600
   gaggaagttg cggcgggcct tgatggtgtc gcgcatcttc ttcttcgtga tgttgaagta 9660
   tcgcgatagt gagggttggc attcgccggg ccgctgcccc gtggaaaggc acagaatcgc 9720
   ttcgcaggcc agccgtgtgt cacctgagaa catatcttcg gcctgggcgg gtagagcgcc 9780
   gaacatcgtc gcagcgatgg acgacgctat gagtagacgt ttcatagatt ccctatctcc 9840
   tagcggttga acacttcgat atcaacgcgg cggttctgcg cacgtccggc cgcagaaacg 9900
   ttgtccgcga cgtaatcgcc ggccgaaacg tagttcactg agattttcag cggcgataca 9960
   ccttgcgcga cgagccagtt cttggctgcc agggcgcgat tcagggcgac acgttcgtcg 10020
   gccgcacttg gcctgtcgcc gtcagtgcgg ccacgaaccc aaatttgccg aacgttaccg 10080
   cccaggagcg gaagcagcgt agcgacctgg gtttgcgtcg gtcggaatgc gctgctgttg 10140
   aatgggaaat agacgcttac cgtttgcgag gctggcaatg ccggcttcac aggcgctgcc 10200
   agcaccacgg gctgcgtaga ctgattgcgc agcttgtctt gcgcgtctgc aagctcagca 10260
   cgaagcgaaa gcacctcggc cgtgcgtgcg tcattgaccg ggtgccgatt cgacccgcta 10320
   acggtcggag gtttcggcgg ggatgagcac gcggccaata agaccgcgca cgacaaaata 10380
   gccaatttgt gcatgtgtga accccgtaat aggaaacatt ggtgagccaa tattatatat 10440
   cttggcaagc ggaccgcaat caaagtcgcg ctgaaattgt cttggatggt gacgactgcc 10500
   caacctggcc atcctttcgg cgtcaccagt tgcaacgagg atcggccttg ttggcgacaa 10560
   ccaggcgcaa ccaggtccgg acttttgtcg ccaaaaaaaa gcgctccacg tcggaagcgc 10620
   tgggagtggt gacagatccc gccggcgccg gcggggaata tgtcaccaaa cgagaggggg 10680
   agggggaaag gatggtgaca tgccggcttg atcgagcggg ccggcttgtc accagaggcc 10740
   gaatcatgga atcacgtaag tccttgtccc cgtgggcgaa tcagaaattc caagacaaat 10800
   tcagcgtagt caagactgcg tgaatttacc gttcaagcgc gcgttcctgg ctagcgactt 10860
   gaccttgccc gcgcgtgtac tcgaccttca ccattgcacc gacagccggc agtttgtcct 10920
   tgaataagcc ggcatcgtgc ttgaccagct cgggcttgcc cttcgcattg gtctggagct 10980
   gatacaccga cttcttgtcg atgtgcagca cattgccttc gtaactcttc accggggccg 11040
   tcttgtccgg tttcgttgct ccggtcaggt catagacgcc cttgggcaac gagttgcggg 11100
   agccgacgtg cctcgacacc cattcgccgt tctcctgggt gtcaacaagc cgtgactcgt 11160
   tggaaatgac tagcagttgg tggccctctg ctttcaacgc ccgttgcccc aggaaaccct 11220
   ctttattgac atcacgcata actaactctc cttaacgact gcgttcggaa ttacgttcaa 11280
   caaccgggcg ggtccgctcg gcatcatgcg acttcgacgg cgctgccttg tcgtagatcg 11340
   cgacggccgg caccttgccg gccttggcgc gctcttgaag ccgcgtatcc accgcttggc 11400
   gcaacgcttc acgttgtgcc gggtctttca ccttcgcgtc gatcagagcg gccgcgacgg 11460
   cctcgacaac cttgtgcttg tcggatttct ggacttccca ggtgttgcgg ttcgtctcga 11520
   taggctcgct accgatgacc ttgcctttgt catcgcgctt caaagcgttc accacgacca 11580
   tctgactgcc cttgtattca agtcgaacgt cgtcgcccgg cttcgccttg ctttcaccca 11640
   tggccctttc aagatcgatg ccccaaacgg ttttctcgcc gtccttcgtg gcgagcttga 11700
   cgtagtagtt cggcttttcg tctgggtcgt ggttgtagtt cgccgcgccg tgttcaagca 11760
   ctcgacccgc atagacccgc agcgcggccg tagcggcctt ttctgccccg tcgccacctt 11820
   tgccctggct gcgtgtgttc gtgctctcct gggcgcgttc tgtcgctttc gcggcttgtt 11880
   ccggcgcgga aggggagcgg ccacggccag tagtccgttc ggccttttgc tggtcgcggg 11940
   cagcatcatg ttcaacggtg ttgcgcatcg accgttcgcg gcgttcttcc agggcgcgca 12000
   aatcctgttc ttgaggcttg tagccgcgca cctcgatacc gcgcaagctc gcttccatcc 12060
   agacttcgcg cttgaagtcg ggatgaccat tgacggtgat cgtcttccag cctttcgcat 12120
   cggccatcgt cgccatggac tttgctacac gctcatcgtt ggtggccgtc accatgcgct 12180
   cgcctttgtc cttgaaggcg atcttgccgg gctggtcttt gaagtggaac ttcgcgccgg 12240
   ccactcggta ctgctggtga agctggtcca tcagttccgc ttgacggttc ttttcgcctt 12300
   cggtcacgac ttggctgtcg taatcagtgt caccgccagt tcgtgcggca aggtcgttag 12360
   cgttgtcctg ttcctcggct ttctcggaca ggttctcaac gtcgatacgc ttaccctcgg 12420
   cctggatgcc gaggcgttct tggtcagcgg ccaacagctc tcggtcacgc tggcgagcgc 12480
   gcgcggcacg ctccagggct tcggctacat caacttgttc aagttgcggt tcggtcgcgt 12540
   gctcaaccgt gttcgactcg cccggttcat ccggttcaat ctcaagcgac acttcggcag 12600
   ccttacgggc ttcctttgca gccgttcggc gcgccgattc ttcggggctg attccgtact 12660
   cgttttgcag gtcttcgcct ttgagcgacc ccttaacctc ggggttctcc aagatcgcct 12720
   tggcgttctt gtcgcctact gcgtccgcca gggcgtccgc atcgaggcct ttcagtcgag 12780
   cgacttgttt gttgcccata tgaaacgaca cggtgtaagc ggcttcgccg gctttttcgg 12840
   tgcgctcgaa acgaagcgac gtggccggtt tgtcgtcaat catggtgatg ccgtaggccg 12900
   gaccggacac ttgcttgtct tcgacgccgg gcactgcccg ttctctagct ggggccgcta 12960
   cgccgagcga tttatcgcgt tcggctatcg ccatgttgtt ttgttcatag gtctgctgcg 13020
   catcctcggg gccgaggaat tgcgacgcta tgtattcatc gaaccgttct ttgggcatca 13080
   gcttcaatgc acgttctggc tcacctgcga tgtctcgatt gacgtaattc tcatagtggg 13140
   ccttatcgac ttcctgaggc tcccccatgt agtcccaccc gttttcgggg tagtacagag 13200
   acgacaagac ctgtttatgc gcgtcttggc cgatagcctc gatggccgta tctctgagct 13260
   tcgcggttaa ttcggtttgc tggttgttct gttccattgc cgtagcccct tatcgttcca 13320
   ttccgtgatc ggcttgcttc gcttgcgaag ggccggcttt tgcctgttcc ttcgcgccct 13380
   gttcttggcg ctgctggggc tggaacgctt gctgcgtcac gttgcccagg tgcttcaaga 13440
   aagcctcgcg ctgcttgacg ttcttgatgt tctccatggc gtatttctcg gcctcgctca 13500
   tgacctttgt tgcggtggaa acgttctgat ccgccgcctt ttccgtggac cgggccgcgc 13560
   gttgctgctg ttcttttgca gcatcccacg gataggcttt ggcctgatcg ccccggtagt 13620
   gaacctgaat gttccggttc tccaggtcgt tgttggcatc gcgacctttc agggacgcgc 13680
   ccatcatttc cacgtcagtg cgactatgga cgacggccgt ttgttggttc ttgccgactg 13740
   cctgcacgac aaaatctttg tcggcatgaa tgaccggacc tttgtaacta ccgttctcct 13800
   ttgcggggaa cacttcggcc tcgacaccga gcttgccggc gacgctgttg cgtatttcgg 13860
   gagggatatt gccggcatcc atgcgatacg cgcctacggt cggcgctttg gatttgtcgg 13920
   ctttttgggg agtgttggca gagtcgccaa tggtgcgttc ttgagacatc gaaggttctc 13980
   ctaagtgatg ggcattgccc gcattgcgac ggggccgaca tggccccgtg cgcaaaatat 14040
   gtaaccttgg ttagccaaga ttatctgttt tggctcactg gcttgtcaag aacgctcaaa 14100
   tccagtatgg gcgcgtcgtc ttcaagctcg ggcggcaggt ctggaacgtc ttccatttcg 14160
   tccagggcgg cgagcatgtc cgcttcgtcg ggcaagtgac cgtcgtcagg aaaaacgtca 14220
   tccatcatgg ccgccagttc gtcatcggtc ggctcatcgg cgacaagctc accttcgtca 14280
   tgacgaagat tttcatcagc ctgcacggcg acttcctcag caactgcggc cacggcgacg 14340
   acgggcgcaa cggcaggcgc tgccttcggt tttcgtttcg ccttgggctt tggttccggt 14400
   tttggcgcgg ccggcgcagc ctggggcact tcctcggcgg ccgtgttcgc gttggtttcc 14460
   gcgagcgctt ccgcttccgc ctcggcatcc agggcggcaa gttcgtcgtc acttggcagc 14520
   gcgcccgagc cgtcatcatc gaccggagca tcgtcgtcgt attcgttcgg atcggcagca 14580
   tcgagcgcgt cgaagaagtc gttcacgaaa ccttcaattt cgtcaggttc caggccatcc 14640
   gatccggcga ctacggcaat tttcgaggta tcgacagcaa gcgcccggag gtcgatgcct 14700
   ttgtctacat cggcaaccgt catttcacgc acacgcgatt gcacgacggc ttcgtgcagg 14760
   tccaggttca aatcaggtac gggcgcggcc agctcacccg aaccccatgt gtcctcaagc 14820
   tgctttttgg acggcagctt tttgcccagg gcggccaaag tcggcgaaac cgacttgagc 14880
   cgatccatga aagcatggtc cgagaaatag gaaatcttgt cgcatttgat gggtttcatg 14940
   ttctcaaggc tgatgatttg cttgcgctgg ctcatttcct tgagttcctg cggcatcatc 15000
   agcgctcgtt tttgccctgc gccttcgcct tcggcctcgg agacgctgga cccactcttg 15060
   ccgtggctac ggttgcgcga atgcaccgtc atgtagccga gcatttctga atactcgttg 15120
   gcgtcgcgtt gttcctttgg cgtgtagagg atttgcagcg catggttcgt tacgaacgtg 15180
   cgggcactct cccggccgta gcctttcggc gactccgctt cgagctggcc cggcgactgg 15240
   atgatggtca gcaggcgcag accgtaaccg gccatgtagg agttcgcctt gtcgatgatg 15300
   ccgatccggc caggggccgt gaattcgtca tccagcatca ggcaggtgaa tttcagctcc 15360
   ggtgtcgagt gcagcagttg gtttgtgttg aggttgacca actgcgtgta gaacatgttc 15420
   agcagaagct tggcctcaga gagcttgttc gccgggatgc ccaggtagat cgacatgcgc 15480
   tttttgcgca cgtcgcgcag gtcgaagtcg ttcgcgctcg ttgctgcatc gacaattggg 15540
   cttgcccaaa tggtcaacgg cacgtgtaac gtcgccatga tgctcgacag cgtgttgtcc 15600
   gaggtggacg tgaagcggtt cagagcgtcc acgcattcca tggacagcat gggcaagcct 15660
   tcgccgtccc acaccgtcaa tggaactagg cggcggtcgg attcaaattc cgcgccggct 15720
   ccactcagca gcaattcgaa ttggtcgagt tcggtgttcc tgacgttctc cacgactgcc 15780
   acgggcacgt catcgcacag ggcttcggcc tcggcttcgt cgcagcctcg aattgccatg 15840
   acggcctgca cgacggcttc gcggtcgtcg cccaggtcgt tcaacatcag gtcgccgtac 15900
   tcgcggtagt ttcgttcggt gatgaagcct tgaatgtgct cttttaccgg ccggcccttg 15960
   ccggacgatt gccgcagcaa ttcgcccatg gtgcgcggca aggtcggggt ttcgcacagg 16020
   tacagcgtca ggcccaggaa caggttgcga gctgcatcat cgaaaaacgc atcgcggcct 16080
   tcgcctggat acagcgaata gccaatcgcc aggatgtccg ttacccgcaa gcggggatcg 16140
   tcgctgacat agccaagggg gttgtagcga tgcgtcttgc cgtgtagcgg gttgccgtcc 16200
   gcgtcctcgc tgatcgagaa cgggttgaac aggaagacct cttggcccca cttggcgcga 16260
   tacttcgacg tgatgaggaa gttttctaac ttcacatcga gcacgacgac ggattccgcc 16320
   caattgagca ggttcggaat cacgatcccg acacccttac cgcttcgcgt tggggcggcc 16380
   agcaacacga attgcatgct ggcgtacatc aggtatcgac ggccccactt gccgacgatg 16440
   atgccggttt tggcgaacag gcccgctttc gcaatctcgc cggcagtcgc gaaccgcgca 16500
   tcaccatgga gcgaacgaac ctcccgcata gccgcgatga ttgcgacgag ggcacccccg 16560
   taggcaacga cggccgcgac gatcaaggcg accttgaggc gtttcgcgac gaccggatcg 16620
   gccgcgtaaa actgccagta ggtccaccac gtccagaagt ccgtcttccc catggggttc 16680
   gtcttgctga aagcgaagaa caggaagccg gacagccata ttgccccggc cgtcgcggcg 16740
   atgaacccga caatgccgag taaccatttg agccacttcg ggatttccat gcttaacgct 16800
   ccttggtgac gaccgccgct aaccaggtgc ggctggctgt cgccacgtct atttgttgaa 16860
   actggtgaca ccgcggcttg ctgcagcaag tccaggcgtc accaaatatg gcgacagatt 16920
   cgagcgatcg agctgccttg tttgtcacca ccatggtctt accgtgatgt cgatcacgtc 16980
   gaagacaacg ccaagccaca gggcggcaac agtcgcaaga ccgccgaagc cgagcatcat 17040
   gaagaagaac tgagcggggc cgagactctt gcggttttgc tgggcatcca gctcgaaccc 17100
   cgaaagcgca tcgggttcgg gttcggattc ggtgtaaggc ttgctaaccg cgaattccgc 17160
   gtaggcttgt ttcacatctt tccagcgttc aaccccgcac aggacctcgc cgcgcgtgtc 17220
   gtccagtccg cagtacgcga ggatggcggc gggcagttgc gagcgcgtct tgggagacat 17280
   ttcgctgtaa tgcgtgcaat gaagtgctcg gaacgccgga tagatttctc gctccatcgg 17340
   cgttttatct ttatcaggcc aaagcacagg gcggtcttga gggcctgtaa gagcgctgac 17400
   cgctgcgtag gcgctatcga ggtcacagat ggaaaagaac gtcccggcca agagcttttc 17460
   aaacgtggcc gctaaaacct cagcgcgaaa atccagcagt tcgtcgcttg gcatcgacgg 17520
   atcgatgtgg gcaaacacag catccaaata ggcaatcgat gcgtcatcag gttgatgttt 17580
   gaaatcgtcc ataaaggctt cctcacgcgc ccgctttggc gcgtttcatc gttggttcgt 17640
   accaaagttc ggtaatgtga cgaccgagtt cagggtgata cacgtcgttg tggacgtgaa 17700
   tcacgatgtc cacgacctga tagagcagcc gcttgatgac gttgtagggc agggtacgac 17760
   cctgccggtt ctgcaacatc atcagagcca tgcgctcgaa agccatgtca cacgacccag 17820
   catgcagact ggtgatgctt cccccgtgac cggaggcgca gacgttgacg aaatcgaacg 17880
   tctcgccgcc gcgcagctca gccaggagaa tgcgcgtcgg tttcatccgc aggcagcttt 17940
   tcagcaacga tgcagccgtt accggtgcgt tttcttcttc cttggcttcg ctcgggtaga 18000
   acaggtgaac gtggttcggg tgatccggca agaacaactc gggcacgtct tcaatcgtga 18060
   tgatgcgctg tgaggtcgga atcgcctgca tcaacgcctt catgagcgtg gtcttgcccg 18120
   agccagtttc gccggccacg acaatgactt tttcgagctg gaccgcccga cgcaggaagc 18180
   cgacgtaatc gccggcttgc ttgagggcta gcagttcatg ttcgtccggc gacaactcgc 18240
   cattcattgg ccgaatgtgc ttgaagaagc cttgctcttc atagtcggtc agcgtgcgac 18300
   gggagaagga cggctttcgg atcgtgaccg aaatcgtccc gtgttctgcc gctggtggca 18360
   tcacgatttg cacacgctca ccagcaggca gcacggccga aaggatcggc cgggaatcgg 18420
   tgatgtcttg attggcgaac ttcgcggtcg cggttgccag ggattggcag tgatcgaacg 18480
   tcaaagcgtc aaccttgtgg gtttcccaca cgctttgccg ttcgcaccag atttcacccg 18540
   gccggttcac gcacacttcg gtgatagccg gatcggccat ccatatgttg accacttcca 18600
   gatggaagtt aaccgaagtc gctcgctgta ccgcaccgtt gccagcgaga tattcgttcg 18660
   aattattgag ctgcgacatc gtacacaccc gaaaagtcca gatcgcgggc gacgtagata 18720
   ccgacttgct cgccctgatt cttgtagagg gttggcggaa tgttgagagt gttgcgcaac 18780
   acttccgccg ccatgttctg cgaagcttcg ccggtcgagt tgaaattgaa ctggttgcca 18840
   tcgccgccgc tgctgctttg actggtaacg cctcgcgcca cgtcatcgac cagggacagc 18900
   atgagcgcgc cgccgaaacg ctcccaaaag tgcgtatcga cgtagccagg aaggccagcg 18960
   ccgcccaggg gatcggtgcc cggcgagtcg agattgacca cgacgccgtt cggcgtcttg 19020
   atgcggttcc acagcacaaa gatgcgggcc atgccctggc gcatgttcga tttgtattcg 19080
   cccgtgacgg tcgaacccct ttcgaccagc agcaccttgc cgttgtcgct gtagatgttc 19140
   cgcgtcacga cgcacgcggt catgcctgga acagtcgtgt ccaacttcgt ttgcaggccg 19200
   caatcaatca tggtgccctt ggtcagcagg aaattccggt tgcccaggtt ggtcgccttg 19260
   cgggttgctg tttgggtcga actcagaagc gccgacatac cgccttcgcc gtcctggccg 19320
   gagttgcctt gctgtctcgc gccgccatca gcgccggccg gcgcggtttt tgccgagctg 19380
   ctgccaacca tcagcgacga cccggattta tccaagacag gtgccgattg atgttgagca 19440
   gcttcgccgg gaagcgccgg ggcagtagcc gtttgttgct ggcccgtcac agccggaacg 19500
   acagttgtcg ttggggtctg ttcgggcaat ggcggtgcgg ccttcacagc cggctccgtg 19560
   aaggtgcggg gcggtacggc ggttgtcagt ttcagctttt tttccgcttc ggccgccgcg 19620
   ctggcgtcgg cccggtgcgt ccacttccaa taaccgacgc cgcccgccac ggccagaacg 19680
   gccaggatcg ccagcagagc taaggcccgc tttttcgtgg aagggccgtc ctgattcacg 19740
   ctcggcatgc cgcgctcgcc atcgagcgct tccagttccg agggcgcgtt gttggcttga 19800
   ttgctcattt ctctacctcg cccgatttga tgacacgttg aacgcctgga acggtcgttc 19860
   ccttgttcgg cggcaagccg tcagggtcga aactctcgtt gtagacaccg acgatggagt 19920
   tacccaggcg cagggcgaat tcacgaacga cccgatgaat caccagaacg tccgggttgt 19980
   tcgggtcgat atggctgttc ggaatgctct ctgtaccgtc tgctgctacg aggaaagccg 20040
   ttgggaaatc ccggttgttc gggaagcgca ggtaggtaaa gcgcccatcg tcgtaagcca 20100
   tggttggcgc aatcccttcg gagttgtcgc cgatttccat ggagtaatgc cagttcatag 20160
   gagctggctg cacatcgagc ttggcttgcg cggcccgctt gcgggcttcc tcgcgttcct 20220
   tcgcccgagc ctcgaccgga tacaggtact ggacccggta cgcgacacgc tgattgatgg 20280
   ccggcttgcc gtcgttgccg ccacccggca tgagcttcaa gtcgatgtcg tacatgtact 20340
   tgttggtcgt caccatcaag ttcgtgtccc aatcgccagc cttgggcggc atgacgacat 20400
   cgacaccctc ggcattctgc accttgatgg atttgggctt catgtagacg atgttgcgcc 20460
   ggtcaaggaa ttcccatccc tggctaaagc cggatgcgat atccaaaacg tgttcgtcct 20520
   cgccgaagac aacgcgggca cccaggccgg gcagtgcccg gaccaaaacc acgtcgccgg 20580
   ggttgtagtt cacgtactgg atgcgattgt cataacgcga cgaggtaggc acatcgaccg 20640
   cgtaggcggc cgagctggca aagcccagga ccagcgacag agcgatgacg gcgtgtttca 20700
   ttgtccaccc ccgaccattt cagggtcttg gcgatagctc agcacctgga acccgaacgg 20760
   gttcaccaga cgctgcgatt ccgagataag cgacgggttg cggtattcgt atgcaacggt 20820
   cgcaacccac ttcgtgacgg tgcctggacc gtcatcatcg gtgcgtttgg tggtcttgat 20880
   gaatcgcacg gtgccaacgc cgcgaccatt gggaacgacg ctcacaatct caaccgtggc 20940
   ccgtaccgat ttgccaaact tcttgtcgag ggcttcgggg ccttcgaaca gagcggcgta 21000
   gtccttgcca acgtttggcg aggccagcag ccccacggtg ttgtagtcct tctggagcgt 21060
   gtaccagtcg tatgtttcac gggcgcggac aaattgggcc agccaatact tgtccatcac 21120
   ttcctcatag ccgacgccct tgctgttgag ggcggtcacg atgtcaggaa cgcccgtggt 21180
   gttatccacg cgaatgacgt agggaatggt ttccttgagc ggcatcatca agacgatagc 21240
   gagccaagac agcaccgcta cccctacggc tccataggcc acgtaccacg cccgttgctc 21300
   tgacttttcg atagcttggg tgcgcgaggc ttcccaattg ttttcttgct cgaacgggtc 21360
   tttggcctgg acggcattcg gcggggtcac ttcgtcaccc gccgcctttt tgcgtctgaa 21420
   catacgttac tcccctttga aatcaatgac gggtgaaggc gtcgccgctt tgatcggcgt 21480
   cggagcttgc ttgttgattg gcacgcgcgg tttcccttcc agttggggaa gcacggacgg 21540
   ccgattggtc gcgcatccag cgagagcggt cgcgagcatg agaaggacta aagcgcgttt 21600
   catagtgttg gtcctacagt tagtggtgga cgtgccagat tatacacgcg ccacgaccaa 21660
   attaccctat tttggtaatc gtgagcgcaa aaaaagccgc cctgtccccc gaaggaaaca 21720
   gagcggcgag agagttttat cgcttgactt ggccgccttg cgcgctgccc cagttcttgc 21780
   ccatgttctg catgacgtgt tgccggtagg ccgggttcca catcgtgtta cctgcaacca 21840
   tatggttcga ccggcgcgcc gtggtcatca tgccggactg catgtcgcgg cgggtggaca 21900
   tcgggtcaac ggcattgcgt gcggcgctaa gccccttcat cgcgcctgtg accggcgaaa 21960
   tcaggtgccg aatgcccatg gcggccatcg acaccccgcc ggccagcccg gaggctatgc 22020
   cgcccgcctg gaggatgatc caaatcagca cccccgtaag agcgccgatc tgcaaggccg 22080
   caaacatcgg gttatcgtca ccgctacccg agaagtccgc gccctggatg aaggtatcgt 22140
   atgccttcat ggcaaacgtc atgaccacgg ccatgacgac gatggttagc gtgtagttca 22200
   tcacctgggc gaaccagcta tcgaagaact tcgcagtcat cgggaacatc aggcagagga 22260
   tgaacagcgg ccccatggcg aagacaatcg caagcgagaa tttcgccacg atgaccacag 22320
   cgccgccgag catcgagacg agcacggtgc ccacggcaac aaccaagcca gcaatgaccc 22380
   agccgagcgc cgcgccgatg ctccaaccag cctcatcggc tttctggaag cacgtcccca 22440
   caagctccat gcccttgccc agcgactggt caagaacctg atagatcgag gacgcctgag 22500
   tgccgccaac gttgagcgcg tccgcaaggc cggtttcgag tccgtttaat gatcccatca 22560
   ccgcgtccgt gtacccgtcg gctgtcagag caaaggcggc aatgatgatg atcttcaagc 22620
   actgcttgag gaacgtgtaa aacggagcct caaccgcgcc cgtgctgatc gcgtagccgg 22680
   tcagggtgat gtacagcgtc acccccgtca gggcgagcat ttgaagccca aacatcagct 22740
   tcgaggcggc gggcgacaca aacgctgtgg tcgcgttttg caccgtctcg ccaatgaact 22800
   gaaagaccat cgggtccata cgtcaccgtc ctatttcttt ttgagttcgt cggctgtgag 22860
   cggcgcaaca ggcttgatgc caccgccacg cgcgccccag gttgtggcgc tgtcggcacg 22920
   ggcggcattg acgcagttcg gcgtcgccat gcgctcgccg gggttctcgt tgcacttggc 22980
   gagcatggcc ttacgctcgg ccgcgtgttc cttgtaccaa tccgtggtct gtacgggtgc 23040
   gtcgtcttcc ttgcagccgg acaggacaac cagaacagca gcagcaaggc ccatcgcgag 23100
   ggtgcgtttc ataagtgaat ctcctatcag ttggtgacgc ggccgggttg gccaggcgca 23160
   gcggctgtca ccatgccggt aacgaatacg tcaccttggg ccggtgtgtt gaagcgctcg 23220
   cgcaatctgt ccgccacggc ggccgcaaag tggtttttga aaccgaaacg ggagccgaca 23280
   atgatcatca gccccacgaa aataatggcc gccgtagcaa tcgtggtcat caacaaccgc 23340
   acgaacgctt gcacgtcggc cgtgctggcc gtttgcagtg cgtgcagcgt ctcggtgcag 23400
   ctctccggcg tcacaattgc agcaccaaca aacagccaga acaggaacag aggcaccgcg 23460
   accagaagct tgccggcaaa agtcagggcg acatgcgcag tattcgccag ttgccccagg 23520
   aacggccggg catcttgaaa aacttcaagc tgcacagcgg cgtattccga atcgctcaag 23580
   gtgccaatag gtacgttgtt gagccacact ttccaggtcg tgggcttatt ctcagtaatg 23640
   cgacgcttgt acgtctcagc gtgccgggcg gccaggagct gcacccccag gacaaacagc 23700
   ccgctcaacg cggccgacag aacaagactc agcaccatgg aaagcgcgcc gcgttcgctg 23760
   tagtcggttg tcagaaggtt gaacgtcacc gaggccacgg ccgcgatgcc aaagggtaca 23820
   gcgctctttt tcagtgttgc tttcatcgcc ggttcccccg tcgaatggtg acaagcgacg 23880
   ccggcaggat ctctgccggc gtcaccagtt gcgttactgc ccgtcgaaag tgaccgggcg 23940
   tacctcgata ccgccgcgcg cggaccatgt gcgggcctga atctcacgct gacgttgagc 24000
   ctgcaccttg tcttcggcct gggcgaccat cgaataaagt tgcagcttcg tcgcttcgtt 24060
   ctggattttg gcctgctcga tggcgatccg gccttgcagc tccgcgatgg ccttcgggtc 24120
   ttgcgtcgtg ttgatgcgag ccatcaggcc gtcaatctgc gagagccggc ttttcgccgc 24180
   gtcgtaggca tccagggcaa ccgccttatc ctgggaaggc ttcacggccc gcgcttcgca 24240
   tgcacgctgc gcatcacggg tcttgatgta ggtgcagcta tcgaacacct tgttgtcggc 24300
   ataaacctgc ttgccgcgac cggtaaggcc ggcgtaaccg ccgctcttca cggagtcgta 24360
   gacctgctgc cagtcttgcg gcaggtaatc ccgcagagcc gggttgtcca tgatgttacc 24420
   cataccacgc gatccggtga tcgactggta ttgctgcttc atctggtcaa tttgcgaagt 24480
   catttgctcg acttgctgtg cccaacgcgc cgtattggcc gcgaactcgg ccatggtcat 24540
   gggcgagtcg ctggcctggg ttgtcaccgt tacgggaatt tgcgcattgg cggcccccac 24600
   aaatgccgct gcaactaacg cgccgacgag aatccttttc atagtcacct cacgcttgct 24660
   tggaagacgc cacgcgcgcc ttacggcgtt cgtggaacac agggagccac tgccgggggt 24720
   cgtctccaac ctcggcaatg acagcatcaa gcagttcgtt attgtccgaa ctgccggata 24780
   ggatagccag ttcatcatcg aaaccggcca gatcgagccg cgcaatcatg gattggtggc 24840
   cctgtttcac caaaaacatc cggctttctt cgctcatgct cttgatgatt tcgaactccg 24900
   cgtcggtaac tttaaagccg tccgtgtatt cgtggtaatc ggctttcggg ttcggcaggt 24960
   agatttcagt cgccacctgt tgcaccaggg acgccgcgat tttcgatttc aacaggctgc 25020
   taggcatttg cgtcgcgaag acgcccaagc cattttgctt acggatggtc aattgcttgt 25080
   tgccggcgaa ctcggcaaag gcttcgtcat ccacccactt ccacgcctca tccatccagt 25140
   agatgaagcg ccggccgtca atgaccgatt ccatgcgatg gagcaggtac atcgaaatcg 25200
   gcgtgcgcac gtcgctgttg tcgaggaagt cggttccatc gaacccgtag ttgttgtggg 25260
   tcgtgaagtc gatttgatct tccgggcaat ccagcaccca ccagaatgcg ccgcgtttgc 25320
   cgttgccgtc gtcgtagcac caacgcgaca gccgcttgga cacgctgttt tcgcggtcct 25380
   cgcggtcggt gccctcggtg atgttctgaa tgaccgtaga gagccggcgc agagggcgcg 25440
   gcatgcgcat caccgtccgg acggcgtggc taatgcgcag ttcgtcggtg gttgtgacgc 25500
   gctggtcttc gcccgagacg agcacacgca ccaaccgctc aaggaacaga atattgtcct 25560
   cggtcggctc catctggaac gggttgaagc cggtcggctt accatttttg atcgaaaggt 25620
   acttgccgcc aataacaaga atggtcgctt tcgcgccttc gtccttgtcc atatagacgt 25680
   tgcagaaccc ggacggggaa ttgtgtttgt acttctgcga ttggcaaacc gcgaagttca 25740
   tgaacacagt tttacccgca ccggactgcc cgatgatccg ggtgttgccg agcagcttct 25800
   tgtcgtaatt gtcttcgtcg cccttcgagt agtggaaatt caggtagagc ggctggcccg 25860
   atggggtttt gagcagtgtc acggcctgcc cccaagggtt gccgtcccgc ttgcctgccc 25920
   ggaaattgtg gaagctcgac aggcccgcga aattcttgct ggtcaggccg gccacacggg 25980
   gccgatagga ccagttgcac ggcaactgag cgtaaaacgc cgcatcggtc gcagtcgcca 26040
   ccattgccgc caggaagccg cgatcctgaa tgatcgtcat cgccgaagtg gtgttgcggc 26100
   gcagtttttc cacagattcg ccgaagatca tcagcgaata gtggtactcg cccatggcga 26160
   attcgccctg gatgagctgg tcgatggcga gccgcatttc ttcaatctgc gtggcgctac 26220
   catcttccgc gttcgacagt tgcttcattt ggcgctccaa gaactctttg ccttggcgct 26280
   tggacatgaa gctataggac tgcgtaatga cgtactcgta atcctcgtac atcaagccgt 26340
   tgagaatgcc cggttcggtg tgcgcggtgt agtccttgaa gtcgatgcac tgcgcaaagc 26400
   gggtcgtctt cggcgagcga atttcaatcg tctcggtccc tacgaacacc caggcggtgc 26460
   ccaggtattc attgattggc ccggacggga cgcgcacccg ttgccactcg ccgctgacga 26520
   ggaagttcag gaactccagt tgctgggagt acatcacccc ttcgtcatcg gtgtacgtcg 26580
   tcagttcttc aaggccgcgc ctttcatcga cgccatagcg ccgcatgctg gactcgattt 26640
   gataggcgat ttcatccagc ttgcggatgg cggccttctg gtcttgcttg atttcagcca 26700
   acgaccgacg cgacgacttg accagggcac ggtcgatgcg cgacgggttc ggccggtaca 26760
   cgaccgtcaa atacagctca ttcgccatca ttcgataacc ggcgaaggag tcgtagtatt 26820
   tcttgtccag gtcccggcaa aaatcgttgt cgtacaccga tttgagccgg tcactggtct 26880
   tgcgccgcac gttgtgcgtc cagagggcga cgtggttcgt gccgatggac cgaaacagcg 26940
   tattcagttg ctctttgcgc aacagaattt cgtccgcgtc ggtcgtctcg aaggcgatcc 27000
   ccgccacctt ccagatgcgc aggtaatcgc cgtccttcgt gacgatggtg ttcggcgcta 27060
   catggctgct gtagggcaga aagggcgcta ccgtcctttc tcgattcagt tgttggaaat 27120
   acttcggttg agcgttcatg cgcgcctcac ttccgtttct tgaaagggat cgggctgtaa 27180
   gccgaagcct tccagaaccg tccgttgtgg ttgtagttca ccccccggaa cagcaatttc 27240
   aggcccaaga gcctgaattg ctgatcgtcc gactttgcga taagccgcat cgtcaggacg 27300
   attgggaaca gcgccagggc aaggaaaacg gtcgtccaga ccgctatcag gatgaccacg 27360
   ccagaaacga cggcgagcgg caccaaaggc acgccgaaca ccatcgccgg ccgcgtgcac 27420
   cccttaaaga gcgggtcgcg cgggtcttgc atgtccattg gtgccgacat cgctgctacc 27480
   ccttatccca ggaggaagcg ggcaagctcg gccgcgccac cgatgagcaa gccaccggcc 27540
   aggattttgg cgcactcggc gatgtccgca tgcttgaaca ggaacttgta accggcccac 27600
   atgatggcga tggtcacaac ggtgatagac acgccgcgca ggatcgacag cacgttgtcc 27660
   atgaaggtgt tcaccttgtc caagccacct tgcgcgaacg caggttcagc ggcgaacaga 27720
   gcgacgaaag ccagcagagc cagcagcgga acggcgctct ttgcgccctt gttgatggtc 27780
   agtgattcca taggtagttc gactccaaag tagatagaaa acgcaagtgc gggttgatcg 27840
   gcccgcgccg tgaaaacgat tagtggtcag ttcatccgat gctctcccct gccgctttct 27900
   aaccggatta tagcgcagtg aacaccaaaa tatatcattt tggtgttctt cttttacatc 27960
   ccatggtaat gcgctatcgc ttcccggtgg acttcccggc cggctttttg gccgccttcg 28020
   gaacgggttt cccgttcttg tcatccatca cgtttttgca gccggtcgtg tacccgctgc 28080
   acccccacca gtagcccgtg ccttgcgtgc tcttgcgccg atacatcgct ttgccgcagg 28140
   acgggcaagg tgctgtcttg atgacgacgg gcttgccctt ttcgtcatca aggaaggtct 28200
   tgcattcgtc attggtgcag aaccagccga agccaccctt gggcttgccg gtttccttgt 28260
   ccttcttctg gtagcgccgc aggtcggatt tgcacttggg gcagttatga atcgtcgtga 28320
   gtggcttgcc gtccacgtcg tcacagaagg tcttgcattc atcgctcggg caggcccaca 28380
   ggtagccgcc gctctttcgc ttgaaacggc gcaaggatga atcgcactta gggcatggat 28440
   acgatttccg ctccacgggc ctgcccttcg catcgtccat gaaatgcttg cactcatcgt 28500
   tcttgcaaca ccagaacggc ccacgagtgg cgtggttgaa gcgacgcaag ccggatgcgc 28560
   atttcgggca ggtgaaccga ggtttgaact cgccgcccag ggcaacgtct accagctcct 28620
   tgttcagttg ctcgtatgca ggagccacaa catcaaagaa cgtcgttttg ccctctgcaa 28680
   tctggtcgag ctggccttca aggtcgcggg taaaatccag ttccataaag gcgaaaccgc 28740
   ctttgacaag gttgtctacg atcagctcgc caagttcagt tggctcaagg aagcgtttca 28800
   cctcggccag atacccgcgc gtcatgatat tgctcatgat cgccgggtac gtcgctggcc 28860
   ggccaatccc ttcggcttcc agcttcgcaa tgagcgatgc tttcgtgtag cgcttaggcg 28920
   gcatggtctg cttcttgagc agcttgccag tgtcagcgcg cttatcagat ccgacctcaa 28980
   gcatcggcac cttgccggcg tccttgcctt cggcttcgtc cttgtcgtct aaatcggcgt 29040
   cttcgactgc atcttttgca gtcagcacac gccacccggc atcaatgagc acgcggcctt 29100
   tggccttgaa ctcgaaggct tgcgggccat cggtcgattc cagggtgaca gcgttgacct 29160
   tgtatcgcgc gtcagcgagt tgtgacgcta ccgagcgttg ccatatcagc cgatacagcg 29220
   cccgctgttt gtcgtcttcg ccggcttctt cgacctcgat atgggtcggc cgaatggctt 29280
   cgtgcgcctc ctgggcaccg cccttcgact tgaaccggcg cggtgcgtcc ggcagcttcc 29340
   agcccttgcc ttcggcaaag gcacggattt cgctgatagc gtcgtcgccg aaattcacgc 29400
   tgtcggtgcg gatgtacgtg atcgcgcctt gctcaaagag cttctgcgcg agtttggccg 29460
   taacctcggg atcgagcttc aacgcgacac ttgccgcttg cagcagcaaa gacgtggaga 29520
   acggcgacgg cggcgcttcg cgggcggtgt ccgtagtgga gtcgatgacg cggaactgac 29580
   gcgagttagc ggcacggttc gccagttctt cgtccatcac gtaaggcact tgttccgacg 29640
   tgaaaggctt cgtgttccac tcggccttcc aagcgtcgcc gtcgaaaaac acgaccgcgc 29700
   cgaagtggtt cgtcttcgag aaccgcttaa tgcgacgctc aaggtctacc acgacacgca 29760
   ccgcgacact ttggacgcgg cccgctgaaa gattcatgcc gagcatgtca gacagcagcg 29820
   gcgacaccat gtagccaacc atgcgatcca gggcgcggcg cgcttcctgg gcctgcacaa 29880
   gatcgtcatc gattttgcgc ggcttcgcca gggcagcccg gataaccttg ccggtcactt 29940
   cgtcgaaggt gacacgcaga taatcgtctt cacccaaaga gagcgattct tttaggtgcc 30000
   atgcgatggc ttccccttcg cgatccgggt cggtcgcgag gtagaccatc tctgcgtttc 30060
   gaacttcttt gaggatgcga gtgactcggg cctcaccgcc tggaaacgtt cggtccccga 30120
   ccttggtctc aggcagaaat tcgtaaacca tcgtgaagtt tggcttctcg atacctaaac 30180
   cagttttggg aaggtcgcgg atgtggccga cacttgccat taccttccag ccttccccaa 30240
   gctccgcctc gatctttttg atcttgttcg ggctttccac caccatcaat ttcataaatc 30300
   gtcctcagaa gacgacgacg gagctttttg cttcgccgtc agtcccggcc ttttcgctgg 30360
   tcggggccgg ttcgttttgt ttggtgccgg actgatcccc cgcaggggtt tcggtcggga 30420
   ccacccgttg taggcgcacc gcttcgcgtt ccgacgaggt tcgcagcacg accggcgaat 30480
   ggtcggccgg ctcggccgct acaggtgccg gggtcttcgg cacggatgcg ctatccgcag 30540
   ccttgacggc tttcggacgc accgcgccgc cctcccctgg cttgattgaa ggaacgacag 30600
   ggatcgcctt cggctggacg ttcgcttgcg cgagcacctt ttgcacatag ctcggttcgc 30660
   cggccttgtc aggccggaaa cctcgggtga aattgccgga gtagtagcag gagatagcgg 30720
   cctgcaaagc cgcttgttcg ttggcggtgc gcggggtcgc acgcacgaaa caatcttcaa 30780
   gaatcttcga gcctacgcgg aggttcgcgc aggcgtcgaa ggcttgttcg taggtgagct 30840
   tgtacttggg gaggttgtag cggttgactt gcgcaacccc tacagagaag ttccaaccgt 30900
   ctgcctcaag cgcctttgcg gtcgctatgg cttcgtcacg gttggatggt tgccgggcta 30960
   gccggccacc aacgacgccg atagcgtagg ggttatagct tgattcgaca tgaacaaccg 31020
   ctgccatcgt ctgcggtgcg accgtgggcg cgcattgctc cgcaagggcc aaaaattcca 31080
   gcatggttct gtctccgatt catcagtgga cagataccaa tataccatac tttggtcatt 31140
   gaacattgcc ttttttggtt gcggatgttc gggtctgagc ctttggcttg gggtgtttgg 31200
   ggcgggcttt cgtcttgggt gtaccgggca cgtcttggcc tcttattagt tcgaaacggt 31260
   cagtcggata ggcttccttc aagtggtcgt tgaggcgctt taagtccttg aaaagcttcg 31320
   gcactgtccg gtcgcgtcgt gttgtcaagt accactcttt gccgggttca gagagcattt 31380
   gagcgatgat gtagaaccat tccggtagac ccgtgccctt gtgtttctca agaacgtggg 31440
   atttgtccga aaattcggcg gtaacgtaga agcctatggg cgtctccatg actctgagct 31500
   tctcaattat ttgatgcttc gccgcaagct ctagagtcga cctgcaggca tgcaagcttg 31560
   gctgttttgg cggatgagag aagattttca gcctgataca gattaaatca gaacgcagaa 31620
   gcggtctgat aaaacagaat ttgcctggcg gcagtagcgc ggtggtccca cctgacccca 31680
   tgccgaactc agaagtgaaa cgccgtagcg ccgatggtag tgtggggtct ccccatgcga 31740
   gagtagggaa ctgccaggca tcaaataaaa cgaaaggctc agtcgaaaga ctgggccttt 31800
   cgttttatct gttgtttgtc ggtgaacgct ctcctgagta ggacaaatcc gccgggagcg 31860
   gatttgaacg ttgcgaagca acggcccgga gggtggcggg caggacgccc gccataaact 31920
   gccaggcatc aaattaagca gaaggccatc ctgacggatg gcctttttgc gtttctacaa 31980
   actctttttg tttatttttc taaatacatt caaatatgta tccgctcatg ttaattcatg 32040
   aactagtcat gtattcggct gcaactttgt catgcttgac actttatcac tgataaacat 32100
   aatatgtcca ccaacttatc agtgataaag aatccgcgcg ttcaatcgga ccagcggagg 32160
   ctggtccgga ggccagacgt gaaacccaac atacccctga tcgtaattct gagcactgtc 32220
   gcgctcgacg ctgtcggcat cggcctgatt atgccggtgc tgccgggcct cctgcgcgat 32280
   ctggttcact cgaacgacgt caccgcccac tatggcattc tgctggcgct gtatgcgttg 32340
   gtgcaatttg cctgcgcacc tgtgctgggc gcgctgtcgg atcgtttcgg gcggcggcca 32400
   atcttgctcg tctcgctggc cggcgccact gtcgactacg ccatcatggc gacagcgcct 32460
   ttcctttggg ttctctatat cgggcggatc gtggccggca tcaccggggc gactggggcg 32520
   gtagccggcg cttatattgc cgatatcact gatggcgatg agcgcgcgcg gcacttcggc 32580
   ttcatgagcg cctgtttcgg gttcgggatg gtcgcgggac ctgtgctcgg tgggctgatg 32640
   ggcggtttct ccccccacgc tccgttcttc gccgcggcag ccttgaacgg cctcaatttc 32700
   ctgacgggct gtttcctttt gccggagtcg cacaaaggcg aacgccggcc gttacgccgg 32760
   gaggctctca acccgctcgc ttcgttccgg tgggcccggg gcatgaccgt cgtcgccgcc 32820
   ctgatggcgg tcttcttcat catgcaactt gtcggacagg tgccggccgc gctttgggtc 32880
   attttcggcg aggatcgctt tcactgggac gcgaccacga tcggcatttc gcttgccgca 32940
   tttggcattc tgcattcact cgcccaggca atgatcaccg gccctgtagc cgcccggctc 33000
   ggcgaaaggc gggcactcat gctcggaatg attgccgacg gcacaggcta catcctgctt 33060
   gccttcgcga cacggggatg gatggcgttc ccgatcatgg tcctgcttgc ttcgggtggc 33120
   atcggaatgc cggcgctgca agcaatgttg tccaggcagg tggatgagga acgccagggg 33180
   cagctgcaag gctcactggc ggcgctcacc agcctgacct cgatcgtcgg acccctcctc 33240
   ttcacggcga tctatgcggc ttctataaca acgtggaacg ggtgggcatg gattgcaggc 33300
   gctgccctct acttgctctg cctgccggcg ctgcgtcgcg ggctttggag cggcgcaggg 33360
   caacgagccg atcgctgatc gtggaaacga taggcctatg cctcgaactg cgatgagtgg 33420
   cagggcgggg cgtaattttt ttaaggcagt tattggtgcc cttaaacgcc tggtgctacg 33480
   cctgaataag tgataataag cggatgaatg gcagaaattc gaaactaatc ggggtggcgg 33540
   ttcagtgaaa aatattaaag gatcttcttg agatcctttt tttctgcgcg taatctgctg 33600
   cctgtaaacg aaaaaacccg ccttgtggcg ggtccttccg tccgatcaga agtcttcgac 33660
   ctcttctaat cggcggtggt aactggcttg gaggagcaca gtcaccaaaa ctgtcttttc 33720
   agtttagcct taactggttc ataacttcaa gagcaactcc tctaaatcag ttaccaatgg 33780
   ctgctgccag tggcgttaag tcgtgtcttt tccgggttgg ccccaagaga acagttaccg 33840
   gagaaggtgc agcagtcggg ctgaacgggg ggttcctgca tacagcccgg cccggagcga 33900
   actgcctaag tcgccccgag ataccagaag acgtgagcta tgagaaagcg ccacgtgtcc 33960
   cgcaagggag aaaggcggaa aaggtatccg gcaatcgata tggcgggaac gtgggggcgc 34020
   aggagggagc gattcaccgg aaacggtgat gatctttaag ccacgtcggg tttcgccacc 34080
   gctggcggat ttcatggttg agtcggaaga ctcccacaga tgcaccgaaa aagtgtctgt 34140
   tttgtgaagt ccgtcagggg ggcggagcct atggaaaaag cgccagcggc gcggcctttt 34200
   gtcctggctt tgcctttttg ctcacatgtt ctttccggcc ttatcccctg attctgtgga 34260
   taactgtgtt accgtttttg tgtgagtccg taccgctcgc cgcagccgaa cgaccgaggg 34320
   tagcgagtca gtgagcgagg aagcggaaga gcactggaag ctgcattttc tccttacgca 34380
   tctatgcggc atttcacacc cggcatggtg cacttttcat acaatgcgcc ctgatgccga 34440
   ataagtaagc cagtatcctc gaggcgaacg ccagcaagac gtagcccagc gcgtcggcca 34500
   gcttgcaatt cgcgctaact tacattaatt gcgttgcgct cactgcccgc tttccagtcg 34560
   ggaaacctgt cgtgccagct gcattaatga atcggccaac gcgcggggag aggcggtttg 34620
   cgtattgggc gccagggtgg tttttctttt caccagtgag acgggcaaca gctgattgcc 34680
   cttcaccgcc tggccctgag agagttgcag caagcggtcc acgctggttt gccccagcag 34740
   gcgaaaatcc tgtttgctgg tggttaacgg cgggatataa catgagctgt cttcggtatc 34800
   gtcgtatccc actaccgaga tatccgcacc aacgcgcagc ccggactcgg taatggcgcg 34860
   cattgcgccc agcgccatct gatcgttggc aaccagcatc gcagtgggaa cgatgccctc 34920
   attcagcatt tgcatggttt gttgaaaacc ggacatggca ctccagtcgc cttcccgttc 34980
   cgctatcggc tgaatttgat tgcgagtgag atatttatgc cagccagcca gacgcagacg 35040
   cgccgagaca gaacttaatg ggcccgctaa cagcgcgatt tgctggtgac ccaatgcgac 35100
   cagatgctcc acgcccagtc gcgtaccgtc ttcatgggag aaaataatac tgttgatggg 35160
   tgtctggtca gagacatcaa gaaataacgc cggaacatta gtgcaggcag cttccacagc 35220
   aatggcatcc tggtcatcca gcggatagtt aatgatcagc ccactgacgc gttgcgcgag 35280
   aagattgtgc accgccgctt tacaggcttc gacgccgctt cgttctacca tcgacaccac 35340
   cacgctggca cccagttgat cggcgcgaga tttaatcgcc gcgacaattt gcgacggcgc 35400
   gtgcagggcc agactggagg tggcaacgcc aatcagcaac gactgtttgc ccgccagttg 35460
   ttgtgccacg cggttgggaa tgtaattcag ctccgccatc gccgcttcca ctttttcccg 35520
   cgttttcgca gaaacgtggc tggcctggtt caccacgcgg gaaacggtct gataagagac 35580
   accggcatac tctgcgacat cgtataacgt tactggtttc acattcacca ccctgaattg 35640
   actctcttcc gggcgctatc atgccatacc gcgaaaggtt ttgcaccatt cgatggtgtc 35700
   aacgtaaatg ccgcttcgcc ttcgcgcgcg aattgcaagc tgatccgggc ttatcgactg 35760
   cacggtgcac caatgcttct ggcgtcaggc agccatcgga agctgtggta tggctgtgca 35820
   ggtcgtaaat cactgcataa ttcgtgtcgc tcaaggcgca ctcccgttct ggataatgtt 35880
   ttttgcgccg acatcataac ggttctggca aatattctga aatgagctgt tgacaattaa 35940
   tcatcggctc gtataatgtg tggaattgtg agcggataac aatttcacac aggaaaca 35998

<210> 4
<211> 23408
<212> DNA
<213> Artificial

<220>
<223> partial sequence

<400> 4
gaattcgtag gggttactga aaagtgagcg ggaaagaaga gtttcagacc atcaaggagc 60
gggccaagcg caagctggaa cgcgacatgg gtgcggacct gttggccgcg ctcaacgacc 120
cgaaaaccgt tgaagtcatg ctcaacgcgg acggcaaggt gtggcacgaa cgccttggcg 180
agccgatgcg gtacatctgc gacatgcggc ccagccagtc gcaggcgatt atagaaacgg 240
tggccggatt ccacggcaaa gaggtcacgc ggcattcgcc catcctggaa ggcgagttcc 300
ccttggatgg cagccgcttt gccggccaat tgccgccggt cgtggccgcg ccaacctttg 360
cgatccgcaa gcgcgcggtc gccatcttca cgctggaaca gtacgtcgag gcgggcatca 420
tgacccgcga gcaatacgag gtcattaaaa gcgccgtcgc ggcgcatcga aacatcctcg 480
tcattggcgg tactggctcg ggcaagacca cgctcgtcaa cgcgatcatc aatgaaatgg 540
tcgccttcaa cccgtctgag cgcgtcgtca tcatcgagga caccggcgaa atccagtgcg 600
ccgcagagaa cgccgtccaa taccacacca gcatcgacgt ctcgatgacg ctgctgctca 660
agacaacgct gcgtatgcgc cccgaccgca tcctggtcgg tgaggtacgt ggccccgaag 720
cccttgatct gttgatggcc tggaacaccg ggcatgaagg aggtgccgcc accctgcacg 780
caaacaaccc caaagcgggc ctgagccggc tcgccatgct tatcagcatg cacccggatt 840
caccgaaacc cattgagccg ctgattggcg aggcggttca tgtggtcgtc catatcgcca 900
ggacccctag cggccgtcga gtgcaagaaa ttctcgaagt tcttggttac gagaacggcc 960
agtacatcac caaaaccctg taaggagtat ttccaatgac aacggctgtt ccgttccgtc 1020
tgaccatgaa tcgcggcatt ttgttctacc ttgccgtgtt cttcgttctc gctctcgcgt 1080
tatccgcgca tccggcgatg gcctcggaag gcaccggcgg cagcttgcca tatgagagct 1140
ggctgacgaa cctgcgcaac tccgtaaccg gcccggtggc cttcgcgctg tccatcatcg 1200
gcatcgtcgt cgccggcggc gtgctgatct tcggcggcga actcaacgcc ttcttccgaa 1260
ccctgatctt cctggttctg gtgatggcgc tgctggtcgg cgcgcagaac gtgatgagca 1320
ccttcttcgg tcgtggtgcc gaaatcgcgg ccctcggcaa cggggcgctg caccaggtgc 1380
aagtcgcggc ggcggatgcc gtgcgtgcgg tagcggctgg acggctcgcc taatcatggc 1440
tctgcgcacg atccccatcc gtcgcgcagg caaccgagaa aacctgttca tgggtggtga 1500
tcgtgaactg gtgatgttct cgggcctgat ggcgtttgcg ctgattttca gcgcccaaga 1560
gctgcgggcc accgtggtcg gtctgatcct gtggttcggg gcgctctatg cgttccgaat 1620

catggcgaag gccgatccga agatgcggtt cgtgtacctg cgtcaccgcc ggtacaagcc 1680
gtattacccg gcccgctcga ccccgttccg cgagaacacc aatagccaag ggaagcaata 1740
ccgatgatcc aagcaattgc gattgcaatc gcgggcctcg gcgcgcttct gttgttcatc 1800
ctctttgccc gcatccgcgc ggtcgatgcc gaactgaaac tgaaaaagca tcgttccaag 1860
gacgccggcc tggccgatct gctcaactac gccgctgtcg tcgatgacgg cgtaatcgtg 1920
ggcaagaacg gcagctttat ggctgcctgg ctgtacaagg gcgatgacaa cgcaagcagc 1980
accgaccagc agcgcgaagt agtgtccgcc cgcatcaacc aggccctcgc gggcctggga 2040
agtgggtgga tgatccatgt ggacgccgtg cggcgtcctg ctccgaacta cgcggagcgg 2100
ggcctgtcgg cgttccctga ccgtctgacg gcagcgattg aagaagagcg ccggcggcat 2160
ttcgagagcc tgggaacgat gtacgagggc tatttcgtcc tcaccttgac ctggttcccg 2220
ccgctgctcg cccagcgcaa gttcgtcgag ctgatgtttg acgacgacgc gaccgcaccg 2280
gatcgcaagg cgcgcacgcg gggcctcatc gaccaattca agcgtgacgt gcgcagcatc 2340
gagtcgcgcc tgtcgtcggc cgtgtcgctc actcgcttga aggggcacaa gatcgtcaac 2400
gaggacggca cgaccgtcac gcatgacgac ttcctgcgct ggctgcaatt ctgcgtgacg 2460
ggcctgcacc atccggtgca gctccccagc aacccgatgt acctggacgc cctggtcggc 2520
ggacaggaaa tgtggggcgg ggtagtgccc aaggtcggcc gcaagttcgt ccaggtggtc 2580
gctctcgaag gcttcccctt ggagtcctat cccggcatcc tgacggcgct cggcgagctg 2640
ccctgcgagt atcggtggtc gagccggttc atcttcatgg accagcacga agccgtgaag 2700
cacctcgaca agttccgcaa gaagtggcgg cagaagattc gcggcttctt cgaccaggtg 2760
ttcaacacga acaccggccc ggtcgatcag gacgcgcttt cgatggtggc cgatgctgag 2820
gcggccattg ccgaagtcaa cagcggcatc gtggccgtgg gctactacac cagcgtcgtc 2880
gtgctgatgg atgaggaccg cacgcgcctg gaagctgcgg cccgcgatgt tgaaaaggcc 2940
gtcaaccggt tgggctttgc cgcgcgcatc gagtccatca acaccctgga cgccttcctt 3000
ggtagtttgc cgggccacgg cgtggaaaac gtccgccggc cgctcatcaa cacgatgaac 3060
ctggccgacc tgctgccgac cagcaccatc tggaccggca acgcgaacgc gccatgcccg 3120
atgtacccgc cgctgtcgcc ggcgctcatg cactgcgtca cgcaaggatc aacgccgttc 3180
cggctgaacc tgcacgtgcg cgacctcggc cacaccttta tgttcggccc gaccggcgca 3240
ggtaaatcga cgcacctggc gatcctcgcc gcgcagctcc gtcgctatgc cggcatgtcg 3300
atcttcgcct ttgacaaggg catgtcgatg tacccgctgg ccgccggcat ccgtgcggcc 3360
acgaagggca ccagcggcct gcacttcacc gtggcggccg acgacgaacg cctggcgttc 3420
tgcccgttgc agttcctgag caccaagggc gaccgtgctt gggcgatgga gtggatcgac 3480
accatcctgg cgttgaacgg cgtcgaaacg accccggccc agcgcaacga aatcggcaac 3540
gcgatcatga gcatgcacgc cagcggcgcg cgcacgctct ccgagttcag cgtgacgatt 3600
caggatgagg cgatccgcga ggcgatccgc cagtacaccg tcgatggcgc aatgggccat 3660
ctgctcgacg ccgaagagga cggcttggcg ctgtccgact ttacagtgtt cgagatcgaa 3720
gagctgatga acctcggcga gaaattcgcc ctgcctgtgt tgctctacct gttccgccgt 3780
atcgagcgcg ccctgacggg ccagccggcc gtcatcatcc tggacgaagc ctggttgatg 3840
ctcggccacc cggcattccg cgcgaagatc agggaatggc tcaaggtgct gcgtaaggcc 3900
aactgccttg tgctgatggc aacgcagagc ctgtccgacg ccgccaacag cggcatcctg 3960
gacgtgatcg tggaatcgac cgcgaccaag attttcctgc cgaatattta cgccagggat 4020
gaggacacgg cggccctgta ccgccgcatg ggcctgaacg ctcgccagat cgagattctg 4080
gcccaggccg ttcccaagcg tcagtactac tacgtgtcgg aaaacggccg ccgtctctac 4140
gacctggcac ttggcccgct cgcgctcgcg ttcgtcggcg catccgacaa ggaatccgtc 4200

gccatcatca agaacctgga agccaagttc ggcgaccagt gggtggatga atggctgcgt 4260
ggccggggcc tcgcccttga tgaatacctg gaggcagcat gagttttgca gacacgatca 4320
agggcttgat cttcaagaag aagcccgcaa cggccgcagc agcggcgacg ccggccgcga 4380
ccggcccgca aaccgacaac ccgtacctga cggcgcggcg cacctggaac gaccacgttg 4440
gttccgttgt gtcgcaaaag cagacctggc aggttgtcgg catcctttcg ctgatgatcg 4500
tcctcgcggc ggtcggcggc atcatccaca tcggcagcca gtcgaagttc gtgccctatg 4560
tctacgaggt agacaagctc gggcagacgg ccgccgtggg gccgatgacc agggcgtcga 4620
aagccgatcc gcgtgtcatt cacgcctcgg tggctgagtt cgtcggcgat gctcgcctgg 4680
tgacgccgga cgtagctttg cagcgcaagg ccgtctaccg cctctatgcc aagctcgggc 4740
cgaatgaccc ggccaccgcc aagatgaacg aatggctcaa cggcaccgcc gacgccagcc 4800
cgttcgctcg cgcggccgtc gaaacggtca gcaccgaaat cacttccgta atcccgcaga 4860
cgcccgacac ctggcaggtc gattgggtcg agacgacgcg cgacaggcaa ggcgtggtga 4920
aaggccagcc cgtgcgcatg cgggccttgg tgacggtcta cgtcgtcgag ccgacggcgg 4980
acaccaagga agaacaactg cgaaacaacc cggccgggat ctacgtccgg gacttctcct 5040
ggtcgagact tctgtgaggc actgaattat gaaaaaggaa ctgtttgctt tggtcctggc 5100
cgcgtccgtt agcgtgcctg catttgccgc cgatcccggc gcggacctga ctgacctcta 5160
tttttccggc aagaacccgg agctgaccgc gcaagagcgg gcggccatcg ccatcgccaa 5220
gaagtgggag gcgggtaccg ccggcatgcg gccggtggcc ggccccggtg gttcggtgcg 5280
cttcctgttc ggcgcgcagc agccgagcat cgtatgcgcc gtgctgcaag tgtgcgacgt 5340
ggccctgcaa cccggcgagc aagtcaactc gatcaacctg ggcgacaccg cccgttggac 5400
ggtcgagccg gccattaccg gcagcggcgc gaacgaaacc cagcacctca tcatcaagcc 5460
gatggatgtg ggcctggaaa ccagcctggt cgtgaccacg gaccgccgca gctaccacat 5520
gcgcctgcgc tcgcatcgca cgcagtacat gccgcaggtg tcgttcacct acccggaaga 5580
tgcccttgcg aagtgggacg ccatcaagaa ccgcgaacag cgggatcgcg tcgagaaaac 5640
cattccgcag accggcgagt acctgggcaa cctgagcttc aactactccg tcagcgggtc 5700
cacgtcgtgg aagccggtgc gcgtctacaa cgacggcaag aaaaccatca tccagatgcc 5760
gcactcgatg gaacagaccg aagcgccgac gctcctggtc gttcgcaggg agggcggcct 5820
gttctccgac gatgaaacgg tgatggtcaa ctaccgggtc cagggcgacc gctacatcgt 5880
cgatacgatt ttcgacaagg ccatcctcat cgcgggcgtg ggcagcagcc aggaccgcgt 5940
gaccatttca agggggaact aaaccatgcg taagattctg accgtcatcg cactcgcggc 6000
cacgttggcc ggctgcgcga cctccaagta cggcagcttc gtccaggacg cgccggccgc 6060
ctacaaccag accattgcga ccgacgcggt gaagcagctc gtcaagctct acccgccggc 6120
gcaaaccaag ctggaattgc agcaggctac gcccgatccg ttcggcattg ccctggtcac 6180
tgaccttcgc gcccagggct atgctgtcat ggagtacaag cccgacggca acgcggccgc 6240
agctccggct gctgcgtcct cggccgctgc gaagccggca acgccgcaag cccagggcgg 6300
ctatccgctg cgctacgtgc tggaccaatt cagcgacagc aacctgtatc gcctgaccgt 6360
catggtcggc tctcaatcgc tcacgcgcgc ctacctcgcc caaaacaaca cgatggtccc 6420
ggccggcgca tgggttcgga aggagtaagc caatgagcga agatcaaatg gcaccggacg 6480
catcgccaga tgcggtcaag ccgaaaagcg gggttcgccg cgtcaacaac atgccgatgt 6540
acctcatcgg cggtgtgctc ggcatcttcc tgctggtgat ggccctggtt gctgcggatc 6600
gcgctgcgca gcagaaccag ccgggagctg cgaaggctga gaaggccggc agcaccagca 6660
tgtttgccga cgaaattgcc ggcaaacagc aggacggcat catcaaggcc aagccgctgg 6720
agattccgcc ggaacaaacc gcccagcaac cgacgacgga gctgacgcca gccccggcgc 6780

agggaacgac tatcacggtc gcacggcccg agaacctgga ccagcccccg acgccgccgc 6840
agggtgcgcg caacgaggac ctggaccgca tccgcatggc gaagttgcag atgctggaag 6900
aggcgatcaa ggccaagacg acggtgcgca tcgacgcgcc gcgcagccag ggcagcgccg 6960
gcggcggtgc tccgcagggc cgcgaggaaa cccttgcgcg catccaggag ctgcgtcggc 7020
aggctgagaa cgcccgcgcc accgatccga ccgccgccta tcaggccgcg cttgcgcagg 7080
ctcgcacgat gggcggcgcg gcagggggtg gcggtatggg cggctcgggt gcgccgaccc 7140
tcgtgcagac ctcgaaccgc agtggtggcg gcgctggcta tgggtcgttc gacaaccgca 7200
gcgagggcga ccgttggcgg ctcgactccc agccggaagc acctgcaacg ccctatgtgc 7260
tgcgcgctgg cttcgtcgtt ccggctacgc ttatctcggg catcaactcc gatctgccag 7320
gccaaatcat ggcccaggta tcgcagtcgg tgtacgacac ggcgaccggc aagcacatgc 7380
tcatccccca aggctcgcgc ctggtgggca gctactcgaa cgatgtggcc tacgggcaga 7440
agcgcgttct ggtggcatgg cagcgcatca tcttccccga cggcaaggca atggacattg 7500
gggccatgcc gggcggcgat agcgctgggt atgcaggctt caacgacaag gtcaacaacc 7560
actacttccg caccttcgca tcggcattcc tcatgtcggg cgtcgttgcg ggcatcagct 7620
tgagtcagga ccgtggcaac agcaacagcg gttacggacg acaagacgcg ggttccgcga 7680
tgagtgaagc gttgggtcaa cagctcggcc aagtaacggc gcagatgatc gccaaaaact 7740
tgaatatcgc gccgacgctg gaaatccgtc cgggctatcg cttcaacgtc attgtcacga 7800
aagacatgac gttttctaag ccctaccagg cgtttgacta ttaactccaa ggagtaactt 7860
atgaagaagc tcgctaagaa tgttttagcc gctaaagtag ctctggtgct ggccctctcg 7920
gtcggcacct tggcggtcac gcctgcgcaa gcgggcattc cggtcatcga cggcaccaac 7980
ctgtcacaaa ccactgtcac cgcgattcag caggttgcgc aggtccagaa gcaaatcgag 8040
gaataccgga cgcagttgca gcagtacgaa aacatgctgc aaaacacggt ggccccggcc 8100
gcctacgtgt gggaccaggc gcagtccacc atcaacggcc tgatgagcgc cgttgatacc 8160
ctgaactact acaagaacca ggcgggcagc atcgacgctt acctgggcaa gttcaaggac 8220
gtgtcctact acaaggggtc gccgtgcttc tccctgtcgg gctgctcgga aagcgagcgc 8280
aaggcgatgg aagagaaccg ccgcctggcg tccgaatcgc agaaaaaggc caacgatgcg 8340
ctgttccgtg gcctcgatca gcagcagagc aacctcaagt ccgacgccgc cacgctggag 8400
caattgaagg gcaaggcgac gacggcgcag ggccagttgg aagccctcgg ctacgccaac 8460
cagttcgcca gccagcaggc caaccagctc atgcaaatcc gtggccttct gcttgcgcag 8520
cagaacgcca tcgccacgca gatgcaggcc cagcaggacc ggcaggccca gcaggacgct 8580
gcgggcgcga agctgcgcga gggttcgtac cgcgcaagcc cgtctaagac ctggtgaggg 8640
gaggcgcgat gaagaaatcc aacttcatcg cagttgccgc gctggccgcc gtcatggcgg 8700
ccagcctggc aggctgcgac aacaagcccg acaccgacaa gctgacctgc gccgatctgc 8760
cgaaggtcac ggatgccgct caacgcgcgg agctgttgaa gaagtgcccg cgcggagaac 8820
cgggaggctt caagcccagc gaaaagaaag agtggtgatg acgtatgaaa atccagacta 8880
gagctgccgc gctcgcggtc ctgatgctgg ccttgatgcc ggtagcggca tacgcccaaa 8940
tcgacaattc gggcatcctc gacaacgtat tgcagcgcta ccagaacgcc gcgagcggct 9000
gggccactgt cgtccagaac gccgcaacct ggctgttctg gaccttgacc gtgattagca 9060
tggtctggac cttcggcatg atggcactgc gcaaggccga cattggcgag ttcttcgccg 9120
agttcgtgcg gttcaccatc ttcaccggct tcttctggtg gctgctgacc aacggcccga 9180
atttcgcgtc gtccatctat gcgtccctgc ggcagattgc aggccaggca acggggttgg 9240
ggcaggggct ttcgccgtcc ggcatcgtcg atgttggctt cgagattttc ttcaaggtga 9300
tggacgaaac ctcgtactgg tcgccggtcg atagcttcgt cggtgcctcg ttggcggccg 9360

ccatcctctg catcctggcc ctggtcggcg tgaatatgct tctgctcctg gcgtccggat 9420
ggattcttgc ctacggcggt gtgttcttcc tgggcttcgg cggctcgcgc tggacctcgg 9480
acatggcgat caactactac aagaccgtcc tcggggtcgc cgcgcagctc ttcgcaatgg 9540
tgctgctcgt aggcatcggc aagaccttcc tcgatgacta ctacagccgc atgagcgaag 9600
gcatcaactt caaggaactt ggagtgatgc tgatcgtcgg cctgatcctg ctcgttctgg 9660
tcaacaaggt gccgcagctc atcgccggca tcatcaccgg cgcgagcgtc ggcggtgctg 9720
gtatcggcca gttcggcgct ggcacgctcg tcggtgcggc cgcgacggcc ggcgcggcaa 9780
tcgcaactgg cggcgcatct atcgcggccg gcgctgcggc ggcggccggt ggcgcgcagg 9840
ccatcatggc ggccgcgtcg aaggccagcg ataacgtctc tgccggcact gacattctgt 9900
cgagcatgat gggcggcggc ggtggcggcg gcggtggtag cgccggcacc agcggcggcg 9960
acggcggcgg ctcgggtggc ggcggtggct cgggcggcgg tgaaaccccg atggcctcgg 10020
ccgccggcga caacagcagc ggcgcacgcg gcggcagttc gggcggcggc tcgggtggtg 10080
gccgttcgtc tggcggtatc ggtgccacgg cggccaaggg cggccggatc gcggccgata 10140
ccgtcgccaa cctggcgaaa ggtgccggct cgattgccaa ggccaaggcc ggcgaaatgc 10200
gcgcatcggc ccaggaacgc atcggcgata ccgtaggcgg caagatcgcg caggcaattc 10260
gcggcgcggg tgcggcggcg cagaccgctg caaccgtcgc cgatagcaac agccaggcgc 10320
aggaacaacc tgcaccggca cccgcaccgt cgttcgacga caacagcctt tccgcaagca 10380
acaacaggga agcggccgcc gacgcggatt ccgaagtggc gagcttcgtc aacaagcccg 10440
cccaatcctg aaacgactct taggagctac gaccatgcaa ctgaaaaaag cgttctcgtc 10500
ggccgccctg gtggtggcct tgggcctcgg cgcaactggc tcggccagcg cgcaagacgt 10560
gctgacgggc gatacccgcc tggcctgcga ggccattctg tgcctgtcca cgggcagccg 10620
gcccagcgag tgcagcccgt cgctctcgcg gtacttcggc atccacaagc gcaagctgtc 10680
ggacacgctc aaggcgcggc tgaacttcct caacctctgc ccggtatcga accagacgcc 10740
ggaaatgcag acgctcgttt cctcgatttc gcgcggggcc gggcgctgcg atgcgtcctc 10800
gctgaactcc gtgctgcgtg agtggcggag ctgggacgac cagttctaca tcggcaaccg 10860
cctgccggac tactgcgcgg cctacaccgg ccatgcctat accgacttca acacgaccgc 10920
gccgcgctac gtcggcacgc cggaagaggg cggctattgg atcgaggcgg ccgactacga 10980
ccgcgcgctc aaggagtacg aggcgaagct gaaagagcgg cagcagcagt acggtcgcta 11040
tggcagcgac gcctaccgtc ggttcgagcg gtaaggggag gggatagcga tgccgtttgc 11100
caagctgctg gcacggaacg ctctgccggt ggtcgccctg gtggcggcca ctggcttcgg 11160
tgcggcggat gcgaccgccg cacggctctt ccccgatctg tcggaacaga tggaagagcg 11220
cgttgtgtgc tcggtgtctg cggccgctgg atccggcgcg ccgagctcgg tacccgcgct 11280
cgtcgtagtc ctccgggtct agcaaggcca gcatgtgacc gtagcaccag gtcacgcggt 11340
cggagccgca ttcgtaatag ccgtccttgc ggctgccgcc gcccaggccc tcgacgatgg 11400
cttttgccag ctccggtttt tcagcgatta caaggcgttc aaattgcata tatcccccta 11460
ccctcaccag gtcagaaccg gcctgatgac ggtgatgatt tgcgaacgat tgacaggccc 11520
gaagtagcgg ccgtcgaaag acgtgtcgct tacgtcggac ataagcagaa cctcggcggt 11580
ccccagggtg tagctgtcgg actgataacg aggcagcggc cgtcctgatg gatcggcctt 11640
gatgagcgcg ctgtgaggca gcagcccgcc attcacgcgc acgccggcgt cggtgatggc 11700
aacctcgtcg cctttagcgg ctaaaactcg cttcatcatg tagccgtagt cgccggggca 11760
gaaaccgccg gcgatgtagc cccgctcctt ggcgtccgaa aacacgccga cttgcggcgg 11820
gcagaacatg acgtaagccc ccttctccac cggcgcattc gatttccagt acaggccgac 11880
cggaatgctt ttggtggtgt tgaccttcgc gccggcgaga taggccgcgc cggcgagcaa 11940

caaggccgcg ccgcctccga tggcgacgta cttggtgagg cgctggaagc ggctcatatc 12000
gtgatcccct ccccttcctc gacggtggcc gtctggatca gcttgtcgct gaccttcgga 12060
gccggtacgg ccgcgcgggc ctggaatatc gggtctttga agtagagcgg ctgcttgccg 12120
tagatcgcgg gatagccggc gacgtacaca accatgtcgc ccgcctcttc aatgctgccg 12180
tcggcgctct tcttcggccc cggcatgcgc aggcattcat cgggggtcag caatggccgc 12240
tgcacttcct ggaaggtccg cgagacgttg cccaacagcg ccgacgtgcg gcggccgctc 12300
gtcgtgatct gctccttcac gatggtcgtg gtgcctgtca gttttgacag gtgctcggcc 12360
gtctccacgc ggttcggcgg gtaggcgttc tgcacgtggc agttcgacgt gatgctttcg 12420
tcgtggccgt agccggtttc gcggctcttg agctggttaa tgtcctggca gatgaggtag 12480
cacttgatgc cgtagccggc gacgaaggca agggactctt gcaggatttc gagcttgccc 12540
aggctgggga actcgtcgag catcatcagc agacgatgct tgtagtgcgc gacaggacgg 12600
ccgttctcga agtccatctt gtcggccagc agccggacga tcatgttgac catgacgcgc 12660
accagaggcc gcagacgggc cttgtcgttg ggctgcgtca cgatgaacag gcttaccggg 12720
tcgtcgtggt gcatcagttg cttgatgcgg aagtcggact tgctgacgtt gcgggccaca 12780
accgggtcgc ggtacagggc caggtaggac ttggcggtgg acagcacgga accggattct 12840
tcctccgggc ggtccatcat gtcgcgggcc gcagagccga ccgcagggtg gttctgcccg 12900
tcaacgtggc cgtaggtggt catttccatc caaagctcgc ccacgtcgcg gttcgggtcg 12960
gcaagcatgc cgtccaccga cggcagggtg gccggcgtac cctcgttctt agccttgtag 13020
agcgcgtgca ggatgacgcc gacaagcagc gcctggctgg ttttctgcca gtgcgattcc 13080
aggcccttgc cgtccggatc gacgatcagg gtggcaaggt tctgcacgtc gccaacctcg 13140
tactcggtcc ccaagcggat ttcatcgagc gggttccagc acgcgctacc ctgcgcggat 13200
gccggctcaa agcgcacgac cttgttgcgg gcatgcttct tccgccagcc ggcggtcagc 13260
gcccacaact cgcctttcag gtcggtgatg acggcgctgt gcgcccagga aagcagcgtc 13320
ggaacgacca ggccgacgcc cttgccggag cgcgtcggcg cgtaggtcaa gacgtgctcg 13380
gggccgttgt gccgcaggta gtggaacttg ccgtccttgt cctgccagcc gcccacatag 13440
acgccgctgg aagtgggcgg gtgtttgcct gacaccagct cgacgacggt gcgcggccgg 13500
ggcagcaggc cggcggcctg tatgtccttc ttgtcggccc agcgggccga accgtgcaga 13560
tagtcgttcg ccttgccggt gttcgccttg accatctgcg tgacggccgt gcccagcagg 13620
cccacggtcg aaacgaccat acccatgctg gccgcgcgca tgaaatcgtc gggatattgg 13680
ccgtaccact tgccggccca ttgaaggatc gaccagggcg tgtagacgtg gttgatattc 13740
cagccaagtc cggcctgata ctggaaggaa tgggcgaaat attgcgtcgc ggtctgcaag 13800
cctgccccaa gggacaggcc ggcgaggatg ggaacggtct tgctggcctt cggttttttc 13860
gcccgtatct gtggccccac ggcgttgttt cggttcttca tctactccta cctcgggtag 13920
ttttaaggga gcctcgcggg gtcacggtga cgggatcacc gatggcgagg cgcttcatgc 13980
gttgcaccgt ggccttatcg acgggcagca ccagaatctc gtcgttttct ttcctcaaca 14040
gggccagcgc ctggtcctcg acgttccggg tgcctgcata ggacagcgca ccaacataat 14100
cagtatatcg tgcatgcttc ggtatatcga agccgtttag ccgcttttgc tcgcgctcgg 14160
caacatattt ctcggccgcc gcgatctgtt cgggctttag ccctcttcct ggcccagaaa 14220
ctccccgtcg cagtgcgtga gctggttcgg ctccttgctg ctccacgtga ccaggaacat 14280
cacgcggcaa tagcatttca gctccgccgg cgatgcgaac cacaccgagt tgggacagcg 14340
gggatcctct agagtcgacc tgcaggcatg caagcttggc tgttttggcg gatgagagaa 14400
gattttcagc ctgatacaga ttaaatcaga acgcagaagc ggtctgataa aacagaattt 14460
gcctggcggc agtagcgcgg tggtcccacc tgaccccatg ccgaactcag aagtgaaacg 14520

ccgtagcgcc gatggtagtg tggggtctcc ccatgcgaga gtagggaact gccaggcatc 14580
aaataaaacg aaaggctcag tcgaaagact gggcctttcg ttttatctgt tgtttgtcgg 14640
tgaacgctct cctgagtagg acaaatccgc cgggagcgga tttgaacgtt gcgaagcaac 14700
ggcccggagg gtggcgggca ggacgcccgc cataaactgc caggcatcaa attaagcaga 14760
aggccatcct gacggatggc ctttttgcgt ttctacaaac tctttttgtt tatttttcta 14820
aatacattca aatatgtatc cgctcatgtt aattcatgaa ctagtcatgt attcggctgc 14880
aactttgtca tgcttgacac tttatcactg ataaacataa tatgtccacc aacttatcag 14940
tgataaagaa tccgcgcgtt caatcggacc agcggaggct ggtccggagg ccagacgtga 15000
aacccaacat acccctgatc gtaattctga gcactgtcgc gctcgacgct gtcggcatcg 15060
gcctgattat gccggtgctg ccgggcctcc tgcgcgatct ggttcactcg aacgacgtca 15120
ccgcccacta tggcattctg ctggcgctgt atgcgttggt gcaatttgcc tgcgcacctg 15180
tgctgggcgc gctgtcggat cgtttcgggc ggcggccaat cttgctcgtc tcgctggccg 15240
gcgccactgt cgactacgcc atcatggcga cagcgccttt cctttgggtt ctctatatcg 15300
ggcggatcgt ggccggcatc accggggcga ctggggcggt agccggcgct tatattgccg 15360
atatcactga tggcgatgag cgcgcgcggc acttcggctt catgagcgcc tgtttcgggt 15420
tcgggatggt cgcgggacct gtgctcggtg ggctgatggg cggtttctcc ccccacgctc 15480
cgttcttcgc cgcggcagcc ttgaacggcc tcaatttcct gacgggctgt ttccttttgc 15540
cggagtcgca caaaggcgaa cgccggccgt tacgccggga ggctctcaac ccgctcgctt 15600
cgttccggtg ggcccggggc atgaccgtcg tcgccgccct gatggcggtc ttcttcatca 15660
tgcaacttgt cggacaggtg ccggccgcgc tttgggtcat tttcggcgag gatcgctttc 15720
actgggacgc gaccacgatc ggcatttcgc ttgccgcatt tggcattctg cattcactcg 15780
cccaggcaat gatcaccggc cctgtagccg cccggctcgg cgaaaggcgg gcactcatgc 15840
tcggaatgat tgccgacggc acaggctaca tcctgcttgc cttcgcgaca cggggatgga 15900
tggcgttccc gatcatggtc ctgcttgctt cgggtggcat cggaatgccg gcgctgcaag 15960
caatgttgtc caggcaggtg gatgaggaac gccaggggca gctgcaaggc tcactggcgg 16020
cgctcaccag cctgacctcg atcgtcggac ccctcctctt cacggcgatc tatgcggctt 16080
ctataacaac gtggaacggg tgggcatgga ttgcaggcgc tgccctctac ttgctctgcc 16140
tgccggcgct gcgtcgcggg ctttggagcg gcgcagggca acgagccgat cgctgatcgt 16200
ggaaacgata ggcctatgcc tcgatcttca aattcccgtt gcacatagcc cggcaattcc 16260
tttccctgct ctgccataag cgcagcgaat gccgggtaat actcgtcaac gatctgatag 16320
agaagggttt gctcgggtcg gtggctctgg taacgaccag tatcccgatc ccggctggcc 16380
gtcctggccg ccacatgagg catgttccgc gtccttgcaa tactgtgttt acatacagtc 16440
tatcgcttag cggaaagttc ttttaccctc agccgaaatg cctgccgttg ctagacattg 16500
ccagccagtg cccgtcactc ccgtactaac tgtcacgaac ccctgcaata actgtcacgc 16560
ccccctgcaa taactgtcac gaacccctgc aataactgtc acgcccccaa acctgcaaac 16620
ccagcagggg cgggggctgg cggggtgttg gaaaaatcca tccatgatta tctaagaata 16680
atccactagg cgcggttatc agcgcccttg tggggcgctg ctgcccttgc ccaatatgcc 16740
cggccagagg ccggatagct ggtctattcg ctgcgctagg ctacacaccg ccccaccgct 16800
gcgcggcagg gggaaaggcg ggcaaagccc gctaaacccc acaccaaacc ccgcagaaat 16860
acgctggagc gcttttagcc gctttagcgg cctttccccc tacccgaagg gtgggggcgc 16920
gtgtgcagcc ccgcagggcc tgtctcggtc gatcattcag cccggctcat ccttctggcg 16980
tggcggcaga ccgaacaagg cgcggtcgtg gtcgcgttca aggtacgcat ccattgccgc 17040
catgagccga tcctccggcc actcgctgct gttcaccttg gccaaaatca tggcccccac 17100

cagcaccttg cgccttgttt cgttcttgcg ctcttgctgc tgttcccttg cccgcacccg 17160
ctgaatttcg gcattgattc gcgctcgttg ttcttcgagc ttggccagcc gatccgccgc 17220
cttgttgctc cccttaacca tcttgacacc ccattgttaa tgtgctgtct cgtaggctat 17280
catggaggca cagcggcggc aatcccgacc ctactttgta ggggagggcg cacttaccgg 17340
tttctcttcg agaaactggc ctaacggcca cccttcgggc ggtgcgctct ccgagggcca 17400
ttgcatggag ccgaaaagca aaagcaacag cgaggcagca tggcgattta tcaccttacg 17460
gcgaaaaccg gcagcaggtc gggcggccaa tcggccaggg ccaaggccga ctacatccag 17520
cgcgaaggca agtatgcccg cgacatggat gaagtcttgc acgccgaatc cgggcacatg 17580
ccggagttcg tcgagcggcc cgccgactac tgggatgctg ccgacctgta tgaacgcgcc 17640
aatgggcggc tgttcaagga ggtcgaattt gccctgccgg tcgagctgac cctcgaccag 17700
cagaaggcgc tggcgtccga gttcgcccag cacctgaccg gtgccgagcg cctgccgtat 17760
acgctggcca tccatgccgg tggcggcgag aacccgcact gccacctgat gatctccgag 17820
cggatcaatg acggcatcga gcggcccgcc gctcagtggt tcaagcggta caacggcaag 17880
accccggaga agggcggggc acagaagacc gaagcgctca agcccaaggc atggcttgag 17940
cagacccgcg aggcatgggc cgaccatgcc aaccgggcat tagagcgggc tggccacgac 18000
gcccgcattg accacagaac acttgaggcg cagggcatcg agcgcctgcc cggtgttcac 18060
ctggggccga acgtggtgga gatggaaggc cggggcatcc gcaccgaccg ggcagacgtg 18120
gccctgaaca tcgacaccgc caacgcccag atcatcgact tacaggaata ccgggaggca 18180
atagaccatg aacgcaatcg acagagtgaa gaaatccaga ggcatcaacg agttagcgga 18240
gcagatcgaa ccgctggccc agagcatggc gacactggcc gacgaagccc ggcaggtcat 18300
gagccagacc cagcaggcca gcgaggcgca ggcggcggag tggctgaaag cccagcgcca 18360
gacaggggcg gcatgggtgg agctggccaa agagttgcgg gaggtagccg ccgaggtgag 18420
cagcgccgcg cagagcgccc ggagcgcgtc gcgggggtgg cactggaagc tatggctaac 18480
cgtgatgctg gcttccatga tgcctacggt ggtgctgctg atcgcatcgt tgctcttgct 18540
cgacctgacg ccactgacaa ccgaggacgg ctcgatctgg ctgcgcttgg tggcccgatg 18600
aagaacgaca ggactttgca ggccataggc cgacagctca aggccatggg ctgtgagcgc 18660
ttcgatatcg gcgtcaggga cgccaccacc ggccagatga tgaaccggga atggtcagcc 18720
gccgaagtgc tccagaacac gccatggctc aagcggatga atgcccaggg caatgacgtg 18780
tatatcaggc ccgccgagca ggagcggcat ggtctggtgc tggtggacga cctcagcgag 18840
tttgacctgg atgacatgaa agccgagggc cgggagcctg ccctggtagt ggaaaccagc 18900
ccgaagaact atcaggcatg ggtcaaggtg gccgacgccg caggcggtga acttcggggg 18960
cagattgccc ggacgctggc cagcgagtac gacgccgacc cggccagcgc cgacagccgc 19020
cactatggcc gcttggcggg cttcaccaac cgcaaggaca agcacaccac ccgcgccggt 19080
tatcagccgt gggtgctgct gcgtgaatcc aagggcaaga ccgccaccgc tggcccggcg 19140
ctggtgcagc aggctggcca gcagatcgag caggcccagc ggcagcagga gaaggcccgc 19200
aggctggcca gcctcgaact gcccgagcgg cagcttagcc gccaccggcg cacggcgctg 19260
gacgagtacc gcagcgagat ggccgggctg gtcaagcgct tcggtgatga cctcagcaag 19320
tgcgacttta tcgccgcgca gaagctggcc agccggggcc gcagtgccga ggaaatcggc 19380
aaggccatgg ccgaggccag cccagcgctg gcagagcgca agcccggcca cgaagcggat 19440
tacatcgagc gcaccgtcag caaggtcatg ggtctgccca gcgtccagct tgcgcgggcc 19500
gagctggcac gggcaccggc accccgccag cgaggcatgg acaggggcgg gccagatttc 19560
agcatgtagt gcttgcgttg gtactcacgc ctgttatact atgagtactc acgcacagaa 19620
gggggtttta tggaatacga aaaaagcgct tcagggtcgg tctacctgat caaaagtgac 19680

aagggctatt ggttgcccgg tggctttggt tatacgtcaa acaaggccga ggctggccgc 19740
ttttcagtcg ctgatatggc cagccttaac cttgacggct gcaccttgtc cttgttccgc 19800
gaagacaagc ctttcggccc cggcaagttt ctcggtgact gatatgaaag accaaaagga 19860
caagcagacc ggcgacctgc tggccagccc tgacgctgta cgccaagcgc gatatgccga 19920
gcgcatgaag gccaaaggga tgcgtcagcg caagttctgg ctgaccgacg acgaatacga 19980
ggcgctgcgc gagtgcctgg aagaactcag agcggcgcag ggcgggggta gtgaccccgc 20040
cagcgcctaa ccaccaactg cctgcaaagg aggcaatcaa tggctaccca taagcctatc 20100
aatattctgg aggcgttcgc agcagcgccg ccaccgctgg actacgtttt gcccaacatg 20160
gtggccggta cggtcggggc gctggtgtcg cccggtggtg ccggtaaatc catgctggcc 20220
ctgcaactgg ccgcacagat tgcaggcggg ccggatctgc tggaggtggg cgaactgccc 20280
accggcccgg tgatctacct gcccgccgaa gacccgccca ccgccattca tcaccgcctg 20340
cacgcccttg ggcgcacctt cagcgccgag gaacggcaag ccgtggctga cggcctgctg 20400
atccagccgc tgatcggcag cctgcccaac atcatggccc cggagtggtt cgacggcctc 20460
aagcgcgccg ccgagggccg ccgcctgatg gtgctggaca cgctgcgccg gttccacatc 20520
gaggaagaaa acgccagcgg ccccatggcc caggtcatcg gtcgcatgga ggccatcgcc 20580
gccgataccg ggtgctctat cgtgttcctg caccatgcca gcaagggcgc ggccatgatg 20640
ggcgcaggcg accagcagca ggccagccgg ggcagctcgg tactggtcga taacatccgc 20700
tggcagtcct acctgtcgag catgaccagc gccgaggccg aggaatgggg tgtggacgac 20760
gaccagcgcc ggttcttcgt ccgcttcggt gtgagcaagg ccaactatgg cgcaccgttc 20820
gctgatcggt ggttcaggcg gcatgacggc ggggtgctca agcccgccgt gctggagagg 20880
cagcgcaaga gcaagggggt gccccgtggt gaagcctaag aacaagcaca gcctcagcca 20940
cgtccggcac gacccggcgc actgtctggc ccccggcctg ttccgtgccc tcaagcgggg 21000
cgagcgcaag cgcagcaagc tggacgtgac gtatgactac ggcgacggca agcggatcga 21060
gttcagcggc ccggagccgc tgggcgctga tgatctgcgc atcctgcaag ggctggtggc 21120
catggctggg cctaatggcc tagtgcttgg cccggaaccc aagaccgaag gcggacggca 21180
gctccggctg ttcctggaac ccaagtggga ggccgtcacc gctgaatgcc atgtggtcaa 21240
aggtagctat cgggcgctgg caaaggaaat cggggcagag gtcgatagtg gtggggcgct 21300
caagcacata caggactgca tcgagcgcct ttggaaggta tccatcatcg cccagaatgg 21360
ccgcaagcgg caggggtttc ggctgctgtc ggagtacgcc agcgacgagg cggacgggcg 21420
cctgtacgtg gccctgaacc ccttgatcgc gcaggccgtc atgggtggcg gccagcatgt 21480
gcgcatcagc atggacgagg tgcgggcgct ggacagcgaa accgcccgcc tgctgcacca 21540
gcggctgtgt ggctggatcg accccggcaa aaccggcaag gcttccatag ataccttgtg 21600
cggctatgtc tggccgtcag aggccagtgg ttcgaccatg cgcaagcgcc gccagcgggt 21660
gcgcgaggcg ttgccggagc tggtcgcgct gggctggacg gtaaccgagt tcgcggcggg 21720
caagtacgac atcacccggc ccaaggcggc aggctgaccc cccccactct attgtaaaca 21780
agacattttt atcttttata ttcaatggct tattttcctg ctaattggta ataccatgaa 21840
aaataccatg ctcagaaaag gcttaacaat ttcgcgaacg ccagcaagac gtagcccagc 21900
gcgtcggcca gcttgcaatt cgcgctaact tacattaatt gcgttgcgct cactgcccgc 21960
tttccagtcg ggaaacctgt cgtgccagct gcattaatga atcggccaac gcgcggggag 22020
aggcggtttg cgtattgggc gccagggtgg tttttctttt caccagtgag acgggcaaca 22080
gctgattgcc cttcaccgcc tggccctgag agagttgcag caagcggtcc acgctggttt 22140
gccccagcag gcgaaaatcc tgtttgctgg tggttaacgg cgggatataa catgagctgt 22200
cttcggtatc gtcgtatccc actaccgaga tatccgcacc aacgcgcagc ccggactcgg 22260

taatggcgcg cattgcgccc agcgccatct gatcgttggc aaccagcatc gcagtgggaa 22320
cgatgccctc attcagcatt tgcatggttt gttgaaaacc ggacatggca ctccagtcgc 22380
cttcccgttc cgctatcggc tgaatttgat tgcgagtgag atatttatgc cagccagcca 22440
gacgcagacg cgccgagaca gaacttaatg ggcccgctaa cagcgcgatt tgctggtgac 22500
ccaatgcgac cagatgctcc acgcccagtc gcgtaccgtc ttcatgggag aaaataatac 22560
tgttgatggg tgtctggtca gagacatcaa gaaataacgc cggaacatta gtgcaggcag 22620
cttccacagc aatggcatcc tggtcatcca gcggatagtt aatgatcagc ccactgacgc 22680
gttgcgcgag aagattgtgc accgccgctt tacaggcttc gacgccgctt cgttctacca 22740
tcgacaccac cacgctggca cccagttgat cggcgcgaga tttaatcgcc gcgacaattt 22800
gcgacggcgc gtgcagggcc agactggagg tggcaacgcc aatcagcaac gactgtttgc 22860
ccgccagttg ttgtgccacg cggttgggaa tgtaattcag ctccgccatc gccgcttcca 22920
ctttttcccg cgttttcgca gaaacgtggc tggcctggtt caccacgcgg gaaacggtct 22980
gataagagac accggcatac tctgcgacat cgtataacgt tactggtttc acattcacca 23040
ccctgaattg actctcttcc gggcgctatc atgccatacc gcgaaaggtt ttgcaccatt 23100
cgatggtgtc aacgtaaatg ccgcttcgcc ttcgcgcgcg aattgcaagc tgatccgggc 23160
ttatcgactg cacggtgcac caatgcttct ggcgtcaggc agccatcgga agctgtggta 23220
tggctgtgca ggtcgtaaat cactgcataa ttcgtgtcgc tcaaggcgca ctcccgttct 23280
ggataatgtt ttttgcgccg acatcataac ggttctggca aatattctga aatgagctgt 23340
tgacaattaa tcatcggctc gtataatgtg tggaattgtg agcggataac aatttcacac 23400
aggaaaca 23408

<210> 5
<211> 27969
<212> DNA
<213> Artificial

<220>
<223> partial sequence

<400> 5
gaattcgtag gggttactga aaagtgagcg ggaaagaaga gtttcagacc atcaaggagc 60
gggccaagcg caagctggaa cgcgacatgg gtgcggacct gttggccgcg ctcaacgacc 120
cgaaaaccgt tgaagtcatg ctcaacgcgg acggcaaggt gtggcacgaa cgccttggcg 180
agccgatgcg gtacatctgc gacatgcggc ccagccagtc gcaggcgatt atagaaacgg 240
tggccggatt ccacggcaaa gaggtcacgc ggcattcgcc catcctggaa ggcgagttcc 300
ccttggatgg cagccgcttt gccggccaat tgccgccggt cgtggccgcg ccaacctttg 360
cgatccgcaa gcgcgcggtc gccatcttca cgctggaaca gtacgtcgag gcgggcatca 420
tgacccgcga gcaatacgag gtcattaaaa gcgccgtcgc ggcgcatcga aacatcctcg 480
tcattggcgg tactggctcg ggcaagacca cgctcgtcaa cgcgatcatc aatgaaatgg 540
tcgccttcaa cccgtctgag cgcgtcgtca tcatcgagga caccggcgaa atccagtgcg 600
ccgcagagaa cgccgtccaa taccacacca gcatcgacgt ctcgatgacg ctgctgctca 660
agacaacgct gcgtatgcgc cccgaccgca tcctggtcgg tgaggtacgt ggccccgaag 720
cccttgatct gttgatggcc tggaacaccg ggcatgaagg aggtgccgcc accctgcacg 780
caaacaaccc caaagcgggc ctgagccggc tcgccatgct tatcagcatg cacccggatt 840
caccgaaacc cattgagccg ctgattggcg aggcggttca tgtggtcgtc catatcgcca 900
ggacccctag cggccgtcga gtgcaagaaa ttctcgaagt tcttggttac gagaacggcc 960
agtacatcac caaaaccctg taaggagtat ttccaatgac aacggctgtt ccgttccgtc 1020
tgaccatgaa tcgcggcatt ttgttctacc ttgccgtgtt cttcgttctc gctctcgcgt 1080

tatccgcgca tccggcgatg gcctcggaag gcaccggcgg cagcttgcca tatgagagct 1140
ggctgacgaa cctgcgcaac tccgtaaccg gcccggtggc cttcgcgctg tccatcatcg 1200
gcatcgtcgt cgccggcggc gtgctgatct tcggcggcga actcaacgcc ttcttccgaa 1260
ccctgatctt cctggttctg gtgatggcgc tgctggtcgg cgcgcagaac gtgatgagca 1320
ccttcttcgg tcgtggtgcc gaaatcgcgg ccctcggcaa cggggcgctg caccaggtgc 1380
aagtcgcggc ggcggatgcc gtgcgtgcgg tagcggctgg acggctcgcc taatcatggc 1440
tctgcgcacg atccccatcc gtcgcgcagg caaccgagaa aacctgttca tgggtggtga 1500
tcgtgaactg gtgatgttct cgggcctgat ggcgtttgcg ctgattttca gcgcccaaga 1560
gctgcgggcc accgtggtcg gtctgatcct gtggttcggg gcgctctatg cgttccgaat 1620
catggcgaag gccgatccga agatgcggtt cgtgtacctg cgtcaccgcc ggtacaagcc 1680
gtattacccg gcccgctcga ccccgttccg cgagaacacc aatagccaag ggaagcaata 1740
ccgatgatcc aagcaattgc gattgcaatc gcgggcctcg gcgcgcttct gttgttcatc 1800
ctctttgccc gcatccgcgc ggtcgatgcc gaactgaaac tgaaaaagca tcgttccaag 1860
gacgccggcc tggccgatct gctcaactac gccgctgtcg tcgatgacgg cgtaatcgtg 1920
ggcaagaacg gcagctttat ggctgcctgg ctgtacaagg gcgatgacaa cgcaagcagc 1980
accgaccagc agcgcgaagt agtgtccgcc cgcatcaacc aggccctcgc gggcctggga 2040
agtgggtgga tgatccatgt ggacgccgtg cggcgtcctg ctccgaacta cgcggagcgg 2100
ggcctgtcgg cgttccctga ccgtctgacg gcagcgattg aagaagagcg ccggcggcat 2160
ttcgagagcc tgggaacgat gtacgagggc tatttcgtcc tcaccttgac ctggttcccg 2220
ccgctgctcg cccagcgcaa gttcgtcgag ctgatgtttg acgacgacgc gaccgcaccg 2280
gatcgcaagg cgcgcacgcg gggcctcatc gaccaattca agcgtgacgt gcgcagcatc 2340
gagtcgcgcc tgtcgtcggc cgtgtcgctc actcgcttga aggggcacaa gatcgtcaac 2400
gaggacggca cgaccgtcac gcatgacgac ttcctgcgct ggctgcaatt ctgcgtgacg 2460
ggcctgcacc atccggtgca gctccccagc aacccgatgt acctggacgc cctggtcggc 2520
ggacaggaaa tgtggggcgg ggtagtgccc aaggtcggcc gcaagttcgt ccaggtggtc 2580
gctctcgaag gcttcccctt ggagtcctat cccggcatcc tgacggcgct cggcgagctg 2640
ccctgcgagt atcggtggtc gagccggttc atcttcatgg accagcacga agccgtgaag 2700
cacctcgaca agttccgcaa gaagtggcgg cagaagattc gcggcttctt cgaccaggtg 2760
ttcaacacga acaccggccc ggtcgatcag gacgcgcttt cgatggtggc cgatgctgag 2820
gcggccattg ccgaagtcaa cagcggcatc gtggccgtgg gctactacac cagcgtcgtc 2880
gtgctgatgg atgaggaccg cacgcgcctg gaagctgcgg cccgcgatgt tgaaaaggcc 2940
gtcaaccggt tgggctttgc cgcgcgcatc gagtccatca acaccctgga cgccttcctt 3000
ggtagtttgc cgggccacgg cgtggaaaac gtccgccggc cgctcatcaa cacgatgaac 3060
ctggccgacc tgctgccgac cagcaccatc tggaccggca acgcgaacgc gccatgcccg 3120
atgtacccgc cgctgtcgcc ggcgctcatg cactgcgtca cgcaaggatc aacgccgttc 3180
cggctgaacc tgcacgtgcg cgacctcggc cacaccttta tgttcgggcc gaccggcgca 3240
ggtaaatcga cgcacctggc gatcctcgcc gcgcagctcc gtcgctatgc cggcatgtcg 3300
atcttcgcct ttgacaaggg catgtcgatg tacccgctgg ccgccggcat ccgtgcggcc 3360
acgaagggca ccagcggcct gcacttcacc gtggcggccg acgacgaacg cctggcgttc 3420
tgcccgttgc agttcctgag caccaagggc gaccgtgctt gggcgatgga gtggatcgac 3480
accatcctgg cgttgaacgg cgtcgaaacg accccggccc agcgcaacga aatcggcaac 3540
gcgatcatga gcatgcacgc cagcggcgcg cgcacgctct ccgagttcag cgtgacgatt 3600
caggatgagg cgatccgcga ggcgatccgc cagtacaccg tcgatggcgc aatgggccat 3660

ctgctcgacg ccgaagagga cggcttggcg ctgtccgact ttacagtgtt cgagatcgaa 3720
gagctgatga acctcggcga gaaattcgcc ctgcctgtgt tgctctacct gttccgccgt 3780
atcgagcgcg ccctgacggg ccagccggcc gtcatcatcc tggacgaagc ctggttgatg 3840
ctcggccacc cggcattccg cgcgaagatc agggaatggc tcaaggtgct gcgtaaggcc 3900
aactgccttg tgctgatggc aacgcagagc ctgtccgacg ccgccaacag cggcatcctg 3960
gacgtgatcg tggaatcgac cgcgaccaag attttcctgc cgaatattta cgccagggat 4020
gaggacacgg cggccctgta ccgccgcatg ggcctgaacg ctcgccagat cgagattctg 4080
gcccaggccg ttcccaagcg tcagtactac tacgtgtcgg aaaacggccg ccgtctctac 4140
gacctggcac ttggcccgct cgcgctcgcg ttcgtcggcg catccgacaa ggaatccgtc 4200
gccatcatca agaacctgga agccaagttc ggcgaccagt gggtggatga atggctgcgt 4260
ggccggggcc tcgcccttga tgaatacctg gaggcagcat gagttttgca gacacgatca 4320
agggcttgat cttcaagaag aagcccgcaa cggccgcagc agcggcgacg ccggccgcga 4380
ccggcccgca aaccgacaac ccgtacctga cggcgcggcg cacctggaac gaccacgttg 4440
gttccgttgt gtcgcaaaag cagacctggc aggttgtcgg catcctttcg ctgatgatcg 4500
tcctcgcggc ggtcggcggc atcatccaca tcggcagcca gtcgaagttc gtgccctatg 4560
tctacgaggt agacaagctc gggcagacgg ccgccgtggg gccgatgacc agggcgtcga 4620
aagccgatcc gcgtgtcatt cacgcctcgg tggctgagtt cgtcggcgat gctcgcctgg 4680
tgacgccgga cgtagctttg cagcgcaagg ccgtctaccg cctctatgcc aagctcgggc 4740
cgaatgaccc ggccaccgcc aagatgaacg aatggctcaa cggcaccgcc gacgccagcc 4800
cgttcgctcg cgcggccgtc gaaacggtca gcaccgaaat cacttccgta atcccgcaga 4860
cgcccgacac ctggcaggtc gattgggtcg agacgacgcg cgacaggcaa ggcgtggtga 4920
aaggccagcc cgtgcgcatg cgggccttgg tgacggtcta cgtcgtcgag ccgacggcgg 4980
acaccaagga agaacaactg cgaaacaacc cggccgggat ctacgtccgg gacttctcct 5040
ggtcgagact tctgtgaggc actgaattat gaaaaaggaa ctgtttgctt tggtcctggc 5100
cgcgtccgtt agcgtgcctg catttgccgc cgatcccggc gcggacctga ctgacctcta 5160
tttttccggc aagaacccgg agctgaccgc gcaagagcgg gcggccatcg ccatcgccaa 5220
gaagtgggag gcgggtaccg ccggcatgcg gccggtggcc ggccccggtg gttcggtgcg 5280
cttcctgttc ggcgcgcagc agccgagcat cgtatgcgcc gtgctgcaag tgtgcgacgt 5340
ggccctgcaa cccggcgagc aagtcaactc gatcaacctg ggcgacaccg cccgttggac 5400
ggtcgagccg gccattaccg gcagcggcgc gaacgaaacc cagcacctca tcatcaagcc 5460
gatggatgtg ggcctggaaa ccagcctggt cgtgaccacg gaccgccgca gctaccacat 5520
gcgcctgcgc tcgcatcgca cgcagtacat gccgcaggtg tcgttcacct acccggaaga 5580
tgcccttgcg aagtgggacg ccatcaagaa ccgcgaacag cgggatcgcg tcgagaaaac 5640
cattccgcag accggcgagt acctgggcaa cctgagcttc aactactccg tcagcgggtc 5700
cacgtcgtgg aagccggtgc gcgtctacaa cgacggcaag aaaaccatca tccagatgcc 5760
gcactcgatg gaacagaccg aagcgccgac gctcctggtc gttcgcaggg agggcggcct 5820
gttctccgac gatgaaacgg tgatggtcaa ctaccgggtc cagggcgacc gctacatcgt 5880
cgatacgatt ttcgacaagg ccatcctcat cgcgggcgtg ggcagcagcc aggaccgcgt 5940
gaccatttca agggggaact aaaccatgcg taagattctg accgtcatcg cactcgcggc 6000
cacgttggcc ggctgcgcga cctccaagta cggcagcttc gtccaggacg cgccggccgc 6060
ctacaaccag accattgcga ccgacgcggt gaagcagctc gtcaagctct acccgccggc 6120
gcaaaccaag ctggaattgc agcaggctac gcccgatccg ttcggcattg ccctggtcac 6180
tgaccttcgc gcccagggct atgctgtcat ggagtacaag cccgacggca acgcggccgc 6240

agctccggct gctgcgtcct cggccgctgc gaagccggca acgccgcaag cccagggcgg 6300
ctatccgctg cgctacgtgc tggaccaatt cagcgacagc aacctgtatc gcctgaccgt 6360
catggtcggc tctcaatcgc tcacgcgcgc ctacctcgcc caaaacaaca cgatggtccc 6420
ggccggcgca tgggttcgga aggagtaagc caatgagcga agatcaaatg gcaccggacg 6480
catcgccaga tgcggtcaag ccgaaaagcg gggttcgccg cgtcaacaac atgccgatgt 6540
acctcatcgg cggtgtgctc ggcatcttcc tgctggtgat ggccctggtt gctgcggatc 6600
gcgctgcgca gcagaaccag ccgggagctg cgaaggctga gaaggccggc agcaccagca 6660
tgtttgccga cgaaattgcc ggcaaacagc aggacggcat catcaaggcc aagccgctgg 6720
agattccgcc ggaacaaacc gcccagcaac cgacgacgga gctgacgcca gccccggcgc 6780
agggaacgac tatcacggtc gcacggcccg agaacctgga ccagcccccg acgccgccgc 6840
agggtgcgcg caacgaggac ctggaccgca tccgcatggc gaagttgcag atgctggaag 6900
aggcgatcaa ggccaagacg acggtgcgca tcgacgcgcc gcgcagccag ggcagcgccg 6960
gcggcggtgc tccgcagggc cgcgaggaaa cccttgcgcg catccaggag ctgcgtcggc 7020
aggctgagaa cgcccgcgcc accgatccga ccgccgccta tcaggccgcg cttgcgcagg 7080
ctcgcacgat gggcggcgcg gcagggggtg gcggtatggg cggctcgggt gcgccgaccc 7140
tcgtgcagac ctcgaaccgc agtggtggcg gcgctggcta tgggtcgttc gacaaccgca 7200
gcgagggcga ccgttggcgg ctcgactccc agccggaagc acctgcaacg ccctatgtgc 7260
tgcgcgctgg cttcgtcgtt ccggctacgc ttatctcggg catcaactcc gatctgccag 7320
gccaaatcat ggcccaggta tcgcagtcgg tgtacgacac ggcgaccggc aagcacatgc 7380
tcatccccca aggctcgcgc ctggtgggca gctactcgaa cgatgtggcc tacgggcaga 7440
agcgcgttct ggtggcatgg cagcgcatca tcttccccga cggcaaggca atggacattg 7500
gggccatgcc gggcggcgat agcgctgggt atgcaggctt caacgacaag gtcaacaacc 7560
actacttccg caccttcgca tcggcattcc tcatgtcggg cgtcgttgcg ggcatcagct 7620
tgagtcagga ccgtggcaac agcaacagcg gttacggacg acaagacgcg ggttccgcga 7680
tgagtgaagc gttgggtcaa cagctcggcc aagtaacggc gcagatgatc gccaaaaact 7740
tgaatatcgc gccgacgctg gaaatccgtc cgggctatcg cttcaacgtc attgtcacga 7800
aagacatgac gttttctaag ccctaccagg cgtttgacta ttaactccaa ggagtaactt 7860
atgaagaagc tcgctaagaa tgttttagcc gctaaagtag ctctggtgct ggccctctcg 7920
gtcggcacct tggcggtcac gcctgcgcaa gcgggcattc cggtcatcga cggcaccaac 7980
ctgtcacaaa ccactgtcac cgcgattcag caggttgcgc aggtccagaa gcaaatcgag 8040
gaataccgga cgcagttgca gcagtacgaa aacatgctgc aaaacacggt ggccccggcc 8100
gcctacgtgt gggaccaggc gcagtccacc atcaacggcc tgatgagcgc cgttgatacc 8160
ctgaactact acaagaacca ggcgggcagc atcgacgctt acctgggcaa gttcaaggac 8220
gtgtcctact acaaggggtc gccgtgcttc tccctgtcgg gctgctcgga aagcgagcgc 8280
aaggcgatgg aagagaaccg ccgcctggcg tccgaatcgc agaaaaaggc caacgatgcg 8340
ctgttccgtg gcctcgatca gcagcagagc aacctcaagt ccgacgccgc cacgctggag 8400
caattgaagg gcaaggcgac gacggcgcag ggccagttgg aagccctcgg ctacgccaac 8460
cagttcgcca gccagcaggc caaccagctc atgcaaatcc gtggccttct gcttgcgcag 8520
cagaacgcca tcgccacgca gatgcaggcc cagcaggacc ggcaggccca gcaggacgct 8580
gcgggcgcga agctgcgcga gggttcgtac cgcgcaagcc cgtctaagac ctggtgaggg 8640
gaggcgcgat gaagaaatcc aacttcatcg cagttgccgc gctggccgcc gtcatggcgg 8700
ccagcctggc aggctgcgac aacaagcccg acaccgacaa gctgacctgc gccgatctgc 8760
cgaaggtcac ggatgccgct caacgcgcgg agctgttgaa gaagtgcccg cgcggagaac 8820

cgggaggctt caagcccagc gaaaagaaag agtggtgatg acgtatgaaa atccagacta 8880
gagctgccgc gctcgcggtc ctgatgctgg ccttgatgcc ggtagcggca tacgcccaaa 8940
tcgacaattc gggcatcctc gacaacgtat tgcagcgcta ccagaacgcc gcgagcggct 9000
gggccactgt cgtccagaac gccgcaacct ggctgttctg gaccttgacc gtgattagca 9060
tggtctggac cttcggcatg atggcactgc gcaaggccga cattggcgag ttcttcgccg 9120
agttcgtgcg gttcaccatc ttcaccggct tcttctggtg gctgctgacc aacggcccga 9180
atttcgcgtc gtccatctat gcgtccctgc ggcagattgc aggccaggca acggggttgg 9240
ggcaggggct ttcgccgtcc ggcatcgtcg atgttggctt cgagattttc ttcaaggtga 9300
tggacgaaac ctcgtactgg tcgccggtcg atagcttcgt cggtgcctcg ttggcggccg 9360
ccatcctctg catcctggcc ctggtcggcg tgaatatgct tctgctcctg gcgtccggat 9420
ggattcttgc ctacggcggt gtgttcttcc tgggcttcgg cggctcgcgc tggacctcgg 9480
acatggcgat caactactac aagaccgtcc tcggggtcgc cgcgcagctc ttcgcaatgg 9540
tgctgctcgt aggcatcggc aagaccttcc tcgatgacta ctacagccgc atgagcgaag 9600
gcatcaactt caaggaactt ggagtgatgc tgatcgtcgg cctgatcctg ctcgttctgg 9660
tcaacaaggt gccgcagctc atcgccggca tcatcaccgg cgcgagcgtc ggcggtgctg 9720
gtatcggcca gttcggcgct ggcacgctcg tcggtgcggc cgcgacggcc ggcgcggcaa 9780
tcgcaactgg cggcgcatct atcgcggccg gcgctgcggc ggcggccggt ggcgcgcagg 9840
ccatcatggc ggccgcgtcg aaggccagcg ataacgtctc tgccggcact gacattctgt 9900
cgagcatgat gggcggcggc ggtggcggcg gcggtggtag cgccggcacc agcggcggcg 9960
acggcggcgg ctcgggtggc ggcggtggct cgggcggcgg tgaaaccccg atggcctcgg 10020
ccgccggcga caacagcagc ggcgcacgcg gcggcagttc gggcggcggc tcgggtggtg 10080
gccgttcgtc tggcggtatc ggtgccacgg cggccaaggg cggccggatc gcggccgata 10140
ccgtcgccaa cctggcgaaa ggtgccggct cgattgccaa ggccaaggcc ggcgaaatgc 10200
gcgcatcggc ccaggaacgc atcggcgata ccgtaggcgg caagatcgcg caggcaattc 10260
gcggcgcggg tgcggcggcg cagaccgctg caaccgtcgc cgatagcaac agccaggcgc 10320
aggaacaacc tgcaccggca cccgcaccgt cgttcgacga caacagcctt tccgcaagca 10380
acaacaggga agcggccgcc gacgcggatt ccgaagtggc gagcttcgtc aacaagcccg 10440
cccaatcctg aaacgactct taggagctac gaccatgcaa ctgaaaaaag cgttctcgtc 10500
ggccgccctg gtggtggcct tggcctcggc cgcaactggc tcggccagcg cgcaagacgt 10560
gctgacgggc gatacccgcc tggcctgcga ggccattctg tgcctgtcca cgggcagccg 10620
gcccagcgag tgcagcccgt cgctctcgcg gtacttcggc atccacaagc gcaagctgtc 10680
ggacacgctc aaggcgcggc tgaacttcct caacctctgc ccggtatcga accagacgcc 10740
ggaaatgcag acgctcgttt cctcgatttc gcgcggggcc gggcgctgcg atgcgtcctc 10800
gctgaactcc gtgctgcgtg agtggcggag ctgggacgac cagttctaca tcggcaaccg 10860
cctgccggac tactgcgcgg cctacaccgg ccatgcctat accgacttca acacgaccgc 10920
gccgcgctac gtcggcacgc cggaagaggg cggctattgg atcgaggcgg ccgactacga 10980
ccgcgcgctc aaggagtacg aggcgaagct gaaagagcgg cagcagcagt acggtcgcta 11040
tggcagcgac gcctaccgtc ggttcgagcg gtaaggggag gggatagcga tgccgtttgc 11100
caagctgctg gcacggaacg ctctgccggt ggtcgccctg gtggcggcca ctggcttcgg 11160
tgcggcggat gcgaccgccg cacggctctt ccccgatctg tcggaacaga tggaagagcg 11220
cgttgtgtgc tcggtgtctg cggccgctgg atccggcgcg ccgagctcgg tacccgcgct 11280
cgtcgtagtc ctccgggtct agcaaggcca gcatgtgacc gtagcaccag gtcacgcggt 11340
cggagccgca ttcgtaatag ccgtccttgc ggctgccgcc gcccaggccc tcgacgatgg 11400

cttttgccag ctccggtttt tcagcgatta caaggcgttc aaattgcata tatcccccta 11460
ccctcaccag gtcagaaccg gcctgatgac ggtgatgatt tgcgaacgat tgacaggccc 11520
gaagtagcgg ccgtcgaaag acgtgtcgct tacgtcggac ataagcagaa cctcggcggt 11580
ccccagggtg tagctgtcgg actgataacg aggcagcggc cgtcctgatg gatcggcctt 11640
gatgagcgcg ctgtgaggca gcagcccgcc attcacgcgc acgccggcgt cggtgatggc 11700
aacctcgtcg cctttagcgg ctaaaactcg cttcatcatg tagccgtagt cgccggggca 11760
gaaaccgccg gcgatgtagc cccgctcctt ggcgtccgaa aacacgccga cttgcggcgg 11820
gcagaacatg acgtaagccc ccttctccac cggcgcattc gatttccagt acaggccgac 11880
cggaatgctt ttggtggtgt tgaccttcgc gccggcgaga taggccgcgc cggcgagcaa 11940
caaggccgcg ccgcctccga tggcgacgta cttggtgagg cgctggaagc ggctcatatc 12000
gtgatcccct ccccttcctc gacggtggcc gtctggatca gcttgtcgct gaccttcgga 12060
gccggtacgg ccgcgcgggc ctggaatatc gggtctttga agtagagcgg ctgcttgccg 12120
tagatcgcgg gatagccggc gacgtacaca accatgtcgc ccgcctcttc aatgctgccg 12180
tcggcgctct tcttcggccc cggcatgcgc aggcattcat cgggggtcag caatggccgc 12240
tgcacttcct ggaaggtccg cgagacgttg cccaacagcg ccgacgtgcg gcggccgctc 12300
gtcgtgatct gctccttcac gatggtcgtg gtgcctgtca gttttgacag gtgctcggcc 12360
gtctccacgc ggttcggcgg gtaggcgttc tgcacgtggc agttcgacgt gatgctttcg 12420
tcgtggccgt agccggtttc gcggctcttg agctggttaa tgtcctggca gatgaggtag 12480
cacttgatgc cgtagccggc gacgaaggca agggactctt gcaggatttc gagcttgccc 12540
aggctgggga actcgtcgag catcatcagc agacgatgct tgtagtgcgc gacaggacgg 12600
ccgttctcga agtccatctt gtcggccagc agccggacga tcatgttgac catgacgcgc 12660
accagaggcc gcagacgggc cttgtcgttg ggctgcgtca cgatgaacag gcttaccggg 12720
tcgtcgtggt gcatcagttg cttgatgcgg aagtcggact tgctgacgtt gcgggccaca 12780
accgggtcgc ggtacagggc caggtaggac ttggcggtgg acagcacgga accggattct 12840
tcctccgggc ggtccatcat gtcgcgggcc gcagagccga ccgcagggtg gttctgcccg 12900
tcaacgtggc cgtaggtggt catttccatc caaagctcgc ccacgtcgcg gttcgggtcg 12960
gcaagcatgc cgtccaccga cggcagggtg gccggcgtac cctcgttctt agccttgtag 13020
agcgcgtgca ggatgacgcc gacaagcagc gcctggctgg ttttctgcca gtgcgattcc 13080
aggcccttgc cgtccggatc gacgatcagg gtggcaaggt tctgcacgtc gccaacctcg 13140
tactcggtcc ccaagcggat ttcatcgagc gggttccagc acgcgctacc ctgcgcggat 13200
gccggctcaa agcgcacgac cttgttgcgg gcatgcttct tccgccagcc ggcggtcagc 13260
gcccacaact cgcctttcag gtcggtgatg acggcgctgt gcgcccagga aagcagcgtc 13320
ggaacgacca ggccgacgcc cttgccggag cgcgtcggcg cgtaggtcaa gacgtgctcg 13380
gggccgttgt gccgcaggta gtggaacttg ccgtccttgt cctgccagcc gcccacatag 13440
acgccgctgg aagtgggcgg gtgtttgcct gacaccagct cgacgacggt gcgcggccgg 13500
ggcagcaggc cggcggcctg tatgtccttc ttgtcggccc agcgggccga accgtgcaga 13560
tagtcgttcg ccttgccggt gttcgccttg accatctgcg tgacggccgt gcccagcagg 13620
cccacggtcg aaacgaccat acccatgctg gccgcgcgca tgaaatcgtc gggatattgg 13680
ccgtaccact tgccggccca ttgaaggatc gaccagggcg tgtagacgtg gttgatattc 13740
cagccaagtc cggcctgata ctggaaggaa tgggcgaaat attgcgtcgc ggtctgcaag 13800
cctgccccaa gggacaggcc ggcgaggatg ggaacggtct tgctggcctt cggttttttc 13860
gcccgtatct gtggccccac ggcgttgttt cggttcttca tctactccta cctcgggtag 13920
ttttaaggga gcctcgcggg gtcacggtga cgggatcacc gatggcgagg cgcttcatgc 13980

gttgcaccgt ggccttatcg acgggcagca ccagaatctc gtcgttttct ttcctcaaca 14040
gggccagcgc ctggtcctcg acgttccggg tgcctgcata ggacagcgca ccaacataat 14100
cagtatatcg tgcatgcttc ggtatatcga agccgtttag ccgcttttgc tcgcgctcgg 14160
caacatattt ctcggccgcc gcgatctgtt cgggctttag ccctcttcct ggcccagaaa 14220
ctccccgtcg cagtgcgtga gctggttcgg ctccttgctg ctccacgtga ccaggaacat 14280
cacgcggcaa tagcatttca gctccgccgg cgatgcgaac cacaccgagt tgggacagcg 14340
gggatcctct agagtcgacc tgcaggcatg caagcttggc tgttttggcg gatgagagaa 14400
gattttcagc ctgatacaga ttaaatcaga acgcagaagc ggtctgataa aacagaattt 14460
gcctggcggc agtagcgcgg tggtcccacc tgaccccatg ccgaactcag aagtgaaacg 14520
ccgtagcgcc gatggtagtg tggggtctcc ccatgcgaga gtagggaact gccaggcatc 14580
aaataaaacg aaaggctcag tcgaaagact gggcctttcg ttttatctgt tgtttgtcgg 14640
tgaacgctct cctgagtagg acaaatccgc cgggagcgga tttgaacgtt gcgaagcaac 14700
ggcccggagg gtggcgggca ggacgcccgc cataaactgc caggcatcaa attaagcaga 14760
aggccatcct gacggatggc ctttttgcgt ttctacaaac tctttttgtt tatttttcta 14820
aatacattca aatatgtatc cgctcatgtt aattcatgaa ctagtcatgt attcggctgc 14880
aactttgtca tgcttgacac tttatcactg ataaacataa tatgtccacc aacttatcag 14940
tgataaagaa tccgcgcgtt caatcggacc agcggaggct ggtccggagg ccagacgtga 15000
aacccaacat acccctgatc gtaattctga gcactgtcgc gctcgacgct gtcggcatcg 15060
gcctgattat gccggtgctg ccgggcctcc tgcgcgatct ggttcactcg aacgacgtca 15120
ccgcccacta tggcattctg ctggcgctgt atgcgttggt gcaatttgcc tgcgcacctg 15180
tgctgggcgc gctgtcggat cgtttcgggc ggcggccaat cttgctcgtc tcgctggccg 15240
gcgccactgt cgactacgcc atcatggcga cagcgccttt cctttgggtt ctctatatcg 15300
ggcggatcgt ggccggcatc accggggcga ctggggcggt agccggcgct tatattgccg 15360
atatcactga tggcgatgag cgcgcgcggc acttcggctt catgagcgcc tgtttcgggt 15420
tcgggatggt cgcgggacct gtgctcggtg ggctgatggg cggtttctcc ccccacgctc 15480
cgttcttcgc cgcggcagcc ttgaacggcc tcaatttcct gacgggctgt ttccttttgc 15540
cggagtcgca caaaggcgaa cgccggccgt tacgccggga ggctctcaac ccgctcgctt 15600
cgttccggtg ggcccggggc atgaccgtcg tcgccgccct gatggcggtc ttcttcatca 15660
tgcaacttgt cggacaggtg ccggccgcgc tttgggtcat tttcggcgag gatcgctttc 15720
actgggacgc gaccacgatc ggcatttcgc ttgccgcatt tggcattctg cattcactcg 15780
cccaggcaat gatcaccggc cctgtagccg cccggctcgg cgaaaggcgg gcactcatgc 15840
tcggaatgat tgccgacggc acaggctaca tcctgcttgc cttcgcgaca cggggatgga 15900
tggcgttccc gatcatggtc ctgcttgctt cgggtggcat cggaatgccg gcgctgcaag 15960
caatgttgtc caggcaggtg gatgaggaac gccaggggca gctgcaaggc tcactggcgg 16020
cgctcaccag cctgacctcg atcgtcggac ccctcctctt cacggcgatc tatgcggctt 16080
ctataacaac gtggaacggg tgggcatgga ttgcaggcgc tgccctctac ttgctctgcc 16140
tgccggcgct gcgtcgcggg ctttggagcg gcgcagggca acgagccgat cgctgatcgt 16200
ggaaacgata ggcctatgcc cgagcgcgcc ttgattcggg agcggcacgt gaaggcattg 16260
cgctggccaa gcagccgggc gcgtatccgc gccagaacgg cctggccgtg gcattgcgcg 16320
acgtggggcg catcgagcgc accatgttca ttctggactg gctgcaaagc gtggagttgc 16380
gccgccgtgt gaacgctggg ctgaacaaag gcgaagcccg caacgccttg gcccgtgccg 16440
tgttcttcaa ccgcctaggt gaaagccgcg accgcagttt cgagcagcag cgctatcgcc 16500
gcagcggcct caacctggtg acgacggcca tcgtgttatg gaacacggtc tatctggagc 16560

gtgcagcaca tgcgctgcgc ggcagcggtc acggcgtcga tgaccggctg ttgcagtacc 16620
tgtcgccgct gggttggtaa cacatcaacc tgaccggcga ttacctgtgg cgcagcagcg 16680
ccacgatcgg cgcgggcgag ttcaggccgc tacggctgca accggcttag cgtgctttat 16740
tttccgtttt ctgagacgac ccctgcttgt cctggcggtg gcgtttttcg cctcaacggc 16800
acaagcaagc ccaagaccgt cccggcaatg ctcgcagaag ccccgcaaac agccataggc 16860
gttttcttta ccaaccctag ctcaaggcag ccagaacatc caaaaatccg ctgtaggcga 16920
ttctgatggc ttctgttcgg gtatgaggcg atgtgctcca caagcacgac cgacaagcca 16980
gccgaaacgc tcgccaagct ggccgctgcg ctacctgccg gagaggccaa acgctaccgg 17040
gtgatggtgt ccattgtccc ggtggaagaa tgaaggcgag cggggcgggg cggggtgtgt 17100
ctggccgggc ttgtcccggc cgtttctttt ggggtttttg gtaaggactt ttgacgccct 17160
caagggcggc gaaaagtacc gcgcctagtg gatgatcttg gatagatcat ggagggattt 17220
tgcgaatcgc ccgcaaaccc gcgccgttgc taggtttttc agatttcggc ggggaacgtt 17280
acgccggggg ttggggaacg ttacgccggg ggttggggaa cgttacgccg ggggttgggc 17340
ctgctgattt cccacttgcc cttggcgtac tcgttcaccg tccaaccaac ggcggcaagc 17400
tcgacaagcg ccttgcgcgc ggtctggcgg cgctttttca ttgcttcatc gttggctgcg 17460
tctggccata cataaccgca cagcgtgtcg atttcagctt tgccagactt tccggggtca 17520
atccagccac atagccgctg gtgtagcagc cgtgccgggt ctgtttccag cttgcgaact 17580
tctgccatgt tgatgcgggt gtgcgggcgc tctcccataa ccgcatccgc cagccgggga 17640
ttaagcgcaa caaataactt gccttcttgc tcgtcgctcg cgtactcgga cagaatgcgg 17700
aacccttgcc gcttaccacc tctttcgaca atcaccgata ccgcccaaag ccgttcgatg 17760
ctttcccgga tggttttaaa ctgacttcct ccatcttcgg cgtagccaag ttctcgggcc 17820
aactggcgga agctgccctt ggctaccatc gtatctttct ctatggcgtc ccaccgcata 17880
tcaagccata ggcggagttg ctggcctgct tctgattccg tttcgtgccg tagcacgatg 17940
ccgcgcccgt tatctccgga aattgcagcc attgccacca gcccttgcaa tacgcgcaag 18000
tcatcgccgc caagtggttc aggcccccaa aaacggattg agtcatctcc gtagttgtag 18060
gtcacatcga gcttgagcct ctttcgttct ccgcgcttga gactgcggaa aagccccggc 18120
gtgaggcaat gcgcggggtc gtgccgcgca tgggtgaggt catagctcat tgcgctttcc 18180
cccttttttt cctgctccat tgctgattgc ctcctgtagt tcaacgggca acaacacccc 18240
gccgctgtgc cgctgatacc agcggtctag cggggtcgcg tcgtagttct gcttgctcac 18300
gccaaagcgg acaaaaaggc cgcgccgctc gttgccggat ggcgaacgat caaagccccg 18360
gtcactcatg cgctcggctt cctgctccgt cattttggcg acatagccgc accatctggc 18420
gttgtcgatc agggcagacg cgccccgcgc tgcctgctgc tggtcggtct ggccgtcgcg 18480
ggcgctgccc ttgttgacgt ggtgcaggta caggacagcc gccccggtgg tcgccgcgat 18540
gtgttccaac acggaaacaa ggtgggccat gtcgccattg ctgttctcgt cgaggatgtg 18600
aatccggctc agggtgtcca gcacaatcag ccgggcccct gcgctgtagt cgatgacgcg 18660
gcgcaagtgc gcgtcgtcca tcacgtttag ccgcttgccc atgatcggct caagcatcag 18720
gttctcagcg atggcttcgc gggccgactg gttgaggtgc tggccgatgg cgtggacacg 18780
tcgcacaagg gcgggctgtg gatcttcgcc agcgagataa accacgcgcc cggtgtgcgc 18840
cggggttagt cccacaaggt cgccgcctgc cacactgcaa gcgattgaca tggccgcttc 18900
aagagcaaaa aagctcttgc cagttgcgcc aggtgcgaca agtgcgccca cggttccggc 18960
caagaatccg ggccagatga aatcaagttc tggcggctca ttggtgaagg ccgccataat 19020
gtctaaagcc atgcttgtct ccttttcaga cagttgtggt taggcgctgc cggggccgca 19080
atcccctgcc gcgccgctca attttcgccc ctcgatgcca gccacgccga agcctcggcc 19140

aaggcgtcct cataggcgta cgtggccgct tcttctgcgt tctctgctgt ggcgtactcg 19200
gacaacggga acactccgcc gccgacttcc tcgccgcttt ccaacaggcg caaccgccag 19260
ccgccgccaa ggtcggaggg gcgcgggtcg atctggtagc tgtattcgat cattggcggt 19320
aaacctcctt acgattgccg atgcgcataa cgaggatgcg caaagcaccg tcctcgatgc 19380
tggcgatgat tcgccaatcc ccaacgcggt atttccagaa ggctcccagt ttggagcctt 19440
tgagggcttc gccaatgctg cgcgggtcgt cgagctgggc gacacggcca tgcaaaaaag 19500
cgaggatgcg gcgggcggtc tgctggtcga tcttgcctag ctcgcgctcg gcggctgggt 19560
cgagttcaac cttccatgcc atagcgtttc atcacttctt cgagtggcac ggtttgggtt 19620
ttgcctgcgc gaatgtcgat caggcgttgc tctgcaaggt acaaatcttc gaggtcatcc 19680
aagtgctcaa ggatggcttc gcgggcatag aaagtcttgg tccgccctgt ggcctgcgcc 19740
agtgcttcaa ggcgggtttc cacttcggcg ggcagtcgga ttgcaagcat gtttcggctc 19800
ctgttctgaa aacgctatac aagtatatca caaccaccac gcaacgctag agactcattc 19860
ccggcgagtt gtcgcgctcg atcttctgcg cctttgcctg ctcctgctgt cgtacaggct 19920
cgcgcccttc cagcttctcc cactgctgcc ggtacttgcc aagatcggcg gcttgcggct 19980
gtgcggcgct gtaggcatag cgggcggtgc gctgcgcgta gtcgttccaa tcgcggccct 20040
cgtctttctg ccgggtggcc ggtgcgcact ggcggatagc gccctcgatg gccgcttgat 20100
cgtggccggt gacgcgcata cgcacggcaa tcatggaatc cacgcgggac aagtccacct 20160
cgccgccacg ctgccgcttg agcacgtcgc ggtaatgccg ctggtaggcg tcgatcgcgc 20220
tgccgctggc cgctgcaagc tccaaggcgg gcttggcttt cgtgcgctcg ggctgctgcg 20280
ccttcaaggc cgcttgccgc tgatactcgg cgtcgatctg gctggacaag gccagcgcct 20340
tgacgcactc ccggcgctcg gccttgagca agcgcacctc tggatagctg ccgtcctccc 20400
gctggtgctt gggcttgcgg ttctcgtagc cgggtgcgcg gtgcgggtgg atggctcccg 20460
atagcttcgg gtcgccgtat tcacggttca gggcatcgct caggcggttg cccacgtcct 20520
tatcgtgggc cgtccccagc ttcgacacgg tgatgatggc ctgatagttg ccggggctgg 20580
attccagcac ggcggcgggc tggtagccgt ctttgataag ccgctccagc ttctcccggt 20640
tcatgtcgtc gatgaggatg tgatgcttct tgtccgatag cggcgtgtag tagaggtttt 20700
cgccccggcg ctgtaggcgc tgcatctccg gcgtgcgctg ctcgatctcc tgcggtgtga 20760
aaccccgcgt gatgccgtcc ttcttgtcga ggatgaaggt ttgcttcctt ccgtctgcct 20820
gcatcttgat ggacgttacc cggtagcgct ccgcgcctac cgcttccgcg taccgctcga 20880
actgctttag ctgctccgat ttcatcgcct gcgccctctc ctgctgtatc cgctgccgct 20940
cctgctggat gctctcctga agctctgcat ttgtgatctt gaagccgtgc tctgctgcta 21000
acttcccgca catggccttg tattcgtcgt tcccggttac ggtgaagctg ccccacttct 21060
gcgccgatag ctgcaaggcg gcaagcgtgc tgtcccggtt gcgccagtcg tgaatatcga 21120
tgcttttgcc cttgtccacg aaagacacac ccccgccccg ccccccttct tccttccggc 21180
tgtaatgcac ctgctggccc acgatttcag gctgataggc gcgaatgtcg cgcggcgtcg 21240
gcggctcgct gcggtcgccc tcgatgcgct gcggctcgct ggcccggtgt cgccactgct 21300
cggcaagctc cgggcttttc tgcatccgct gccactgctc caggtcggga tagggtctga 21360
actgctgccg gtgctgctcg cgctgcttct ggtgcttctc cttcaaggcg gctttctctg 21420
cggcctgctc ggcggcaatc acgctgcgca tggcgttgag cacttcgccc ttgcctttcc 21480
agttgccgcg catgagttca tcgcggcggg ccttctgctg ctcggcaagt tgcttgcgct 21540
cctgctcctg ccgcttgtcc tgtaccagct tgtccgcgtt cttctccgag tagtgcgcct 21600
tgcgcccggt gatgtagtcc ttccatcccg gcacgtctgg cttgatcggc tccggctcgc 21660
gctgtgctac ctgctgacgc tgcggcggct ggtaagctcc gagtcgcttt tgcaacttgg 21720

ataggctcgc gtcccggtct gcgctgctgg ccttcacgcc aacatcaccg acaaagagcg 21780
tcgccccgct gcctgttttc tcgtagcgca tccccttggc tgctaactcg cggtgcaact 21840
gctcccacgt ctgcgccttt ttgatgatcg gtgcgccgtc ctcaatggcg atgcgttcgg 21900
cggatttctc cccggtgcgg ttttccatgt cgcgcttcgg ctgggctggc tggcgcggct 21960
tgtccttgtc gatgtgctcc cggcctaact caccattctc caacacctga taacgcccgt 22020
tctgctcacg ctgccagcct tgtgcgttct cgattcgtgc tatcgccttg tgcgctgcct 22080
caatgtcgaa gccgttattg atcttcacca ccttgagcgt ttcaggatgc acgcggttga 22140
tggcaaggtg caggtgcaag ttgtccgtgt cggcgtgcaa accatagatg gcttgatggt 22200
ctttaacacc tagctcatcc atgaaaatgc ttaccgcttc ttcgacctgc tccgggctgg 22260
gctgctcgcc ttctcgccaa ctcaggacat agtgattgat cgtatccttg cttcgcactg 22320
cctcttgtga caaggcaatc atttctgccg tctggctctg tggttcatcg gtgatgaatc 22380
cacgcgcccc ggcatggatg cacttctcct gcgagttctc acgctcaggc tcacggatgt 22440
agccagttag gcgggaaacg cgaaccgcct tgcttgctgc cttctgcggg ttcttaacct 22500
tcttgacgat cacggctcag tttcccgata taggctttca agaccacaag cgccccggct 22560
gtgtccttgc tgtacacgcc tccgctctcg ttgtggatgt gcttaagcaa tccaccgatg 22620
cggcgcaact ctttgagcat taccgcgtcg gcgctggcaa tgatcggcct gccaaagtag 22680
cggcggcgga caagctccga catgctcaac ccggcaaggt cggcatcctc tttcaagcgg 22740
gccttctccg ctgcggtcag ccgtacattg ataacggcgt ccagcggttc cggcccctgc 22800
gtttcaaatg gcatcgtgtc tccttctcgc gggggcgggc cggggtgtgt cttggacagt 22860
aagaagcgag agcgccgcac tgtccggggt caaggggata ccccttgcag gataagccag 22920
accgtgagaa gcgaagcgcc gaatgggcag gcgtccgttg gaaccacgtg gcgaagccat 22980
gtgtgttaca acggtcatcc tgtattgctc aaccgctcta ctatcatatc agcagtttag 23040
gcacactaga tgcactttgc aacaagcata aaaggcgcta tgctgtcaac tccgaaacca 23100
gtaagcaaag gagttagcag aatgaaatac acgacagagc agctagaggc tatcgcatcg 23160
aagctgcgtg atatgccaca agtcgaaaaa aagaagcagg agcacagcaa gcaagaggcc 23220
gtgcgcgtgc tatcaaaaga gatcgcggct ttgcaaaagc gcggatacac gcttgaccag 23280
atttcggaaa cgcttcgcgg tgaaggtctg agcatcgcaa ccccgacact caagagctat 23340
ttgcagagag caaagccaac gaaaaaagcc ccggtgcaag cgccgggaga cacacccccg 23400
cccgcccccg ccgtcaagaa gcctgcggat acatcgaagg cgacatttac cccaaaaccc 23460
gacagcgacg acatttaagg agtaagcaac atggcaaaaa gcatttacct gatcggcggc 23520
agcaagggcg gcgttggcaa gtcgcttgtc acgatggcaa cggttgatta cctgcaagaa 23580
cgcggcgaaa gcgtattgct gattgaatcc gacacctcta acccggacgt gtggaaggca 23640
tacaaggaaa gcacggaaac ggagcttatc aatctggatg aggcggacgg atggattcag 23700
ctagtgaact tgtgcgatag caagccggat agcgtcgtgg taattaacgc ggctgcgagg 23760
aataacaagg gagtgagcgc ctacggtgaa acattgaata gcacacttgc ggaactcaag 23820
cgcaagctgg tcacgctgtg ggtgattaac cgccaacggg atagcctcga actgctcaag 23880
gaatacatgg atgcaatccc gaacgcggat gttcatgtgg tgcgcaatgg acacttcgga 23940
gaggaaaaga aattcgagct ttacaacggc tcaaagctgc gcacggcagt agaggaacgc 24000
ggcgggcagt cggtgacatt cccggatatg gccgaccggg tgagcgatga catttacagc 24060
aagcgcatga gcatttcggt agcactcaag gaactgccaa ttggcaaccg tgcggaactt 24120
acccggtggc gcaatgaggc taaaaaggta ttggagggcg taatccatga gtgaccttga 24180
cgacagcttt gcaaagctgc tcggcaggca acccagtgac gcggagcggc aaagccttta 24240
ccgtgtgcgg gatgcactcg ggctgaaaaa caatgatgcg ctctggctgg tgctcatggc 24300

cttgcagcac taccaaggcc agtatgagaa gttcccgcaa gcgatagcac aagcagccaa 24360
agataccttg gtcaatttca aggtaacggc ggatgcaacc gtgaaagcat cagcggaagc 24420
agccaaggcg gatttagcgc aggcggtagc agcagcagcg caagaggtcg cgcacaacac 24480
atctgcaaag cagatgtggc agtgggcggc gggctgcatc gccgtggcct tcctgtgcgt 24540
cggcctgttc ggctggtaca tgcacagcag cggcaaggat tccggctacc aagccggata 24600
cggcgcgggc tacacggaag ccaaggacga gaaagcggcg gcggcatggg caaacacgcc 24660
agaaggtcga acggcctacc gcttcgccca gtccggcgaa ctgcaacgcc ttgcccggtg 24720
cagtggcaag ggctggaagg tcgagaaagg cgcttgctac ccctacccag tagcgaatga 24780
gggcacttac ggatgggcgt tgccatgatc gacgcgctca acatcgccac gatgccgatt 24840
gcagacaagg tgcatcggca catgctggca agctactacg ccttgcaact ggatgccttg 24900
caggccgaag ccaagcggct cggctacttc ttcgcgcaag ccgacacggc ggcaaccatg 24960
ccgccagtgc tggccgattg cttggcgtgg gcggtcgagt accggcgcag gtgctacctg 25020
tacccgaatt gcccggaaag ctgggaacgc cacaccgccg acagcatgag cgacggttac 25080
gcccaagagc attgccgctc gatgctcgcg gcgctggccg cgctcggcat ccgtttgcag 25140
gaaggccagc aagcctacgc cctgctggcg gcggaggaat gccgccaacg cgagaaagcc 25200
agcttgccac ccgagccttc gccactctcg gaagtcgaag tgtccagctt cgtcgatgag 25260
tttttcacca agctggacgc acccaaggag gaagtcccga catgagcaca accgccaaga 25320
tcgactttga aaccggcctg ttcgatgagg gccagtacga ggaagcgcca gacctgccag 25380
ccatccagcc gaaacggcgg acaagtcgcg ccccgttcat cgtggccggt ggcttgctgc 25440
ttggggtcgt ctcagaaaac ggaaattaaa gcacgctaag gcacgacaag cgtgcgcata 25500
tgctgcttca cggactgaag gggctgtttt cctgttcccg gtgcgggcga tccgggcgtt 25560
gcgcgaaacc gcataacccg ctcagttagt acttgacgtt gaagtaaggg ccaaggtcta 25620
tcgtaatgac gcgcggcgct catggcttct ccggccggga gttctcgcgc acctgaccac 25680
acttttttgg agaaatacca tgactaccaa acgcaaagtc gaagttttca gcgcgggatg 25740
cccctcctgc cagaccgcca ttgaactcgt caatcgcctc gcatgcggtt cgtgcgaggt 25800
ctcaatcctc gatatgaacg acatcaatgt ggccaaacgc gcccgtgatc tcggtgttcg 25860
gtcggtgccg gcggtcgcca tcaacggcca gctggcttcg tgctgctccg gcagcggcat 25920
cgaggaacag gcgcttcgct ggcctcggca agcctaggcc tccccatcgg cacgcgcgtg 25980
cctctcgctg gaagctcgcg attaatttct taggcggaac tcttgaccat ggtgttcact 26040
ccaaagttta tattaacact tacaaatgga ggacaccatg cacaccatcg gcaaggtggc 26100
aacgctagcc ggagtcagca ccgatacgct gcgctactat gaaaaagagc acctgatcga 26160
acctgcatct agaaccgctg cgggctaccg gctgtacaac gatgaagcgg tgcggcgcat 26220
ccgttttatc aaaaccgctc agcactgcgg attctcgttg tcggacattc aggagttgct 26280
gacgctgaag cgaactgata gtgcctgttg cgaggatgtg cgcagcgtgg ccatcgagaa 26340
gaggctgcgc atcgcgcgca aactacgggt cttgcaggcc atgtcggccg ccctcaatga 26400
cttgatccag agttgcgaag gcggtgctgc attcgcgaac gccagcaaga cgtagcccag 26460
cgcgtcggcc agcttgcaat tcgcgctaac ttacattaat tgcgttgcgc tcactgcccg 26520
ctttccagtc gggaaacctg tcgtgccagc tgcattaatg aatcggccaa cgcgcgggga 26580
gaggcggttt gcgtattggg cgccagggtg gtttttcttt tcaccagtga gacgggcaac 26640
agctgattgc ccttcaccgc ctggccctga gagagttgca gcaagcggtc cacgctggtt 26700
tgccccagca ggcgaaaatc ctgtttgctg gtggttaacg gcgggatata acatgagctg 26760
tcttcggtat cgtcgtatcc cactaccgag atatccgcac caacgcgcag cccggactcg 26820
gtaatggcgc gcattgcgcc cagcgccatc tgatcgttgg caaccagcat cgcagtggga 26880

acgatgccct cattcagcat ttgcatggtt tgttgaaaac cggacatggc actccagtcg 26940
ccttcccgtt ccgctatcgg ctgaatttga ttgcgagtga gatatttatg ccagccagcc 27000
agacgcagac gcgccgagac agaacttaat gggcccgcta acagcgcgat ttgctggtga 27060
cccaatgcga ccagatgctc cacgcccagt cgcgtaccgt cttcatggga gaaaataata 27120
ctgttgatgg gtgtctggtc agagacatca agaaataacg ccggaacatt agtgcaggca 27180
gcttccacag caatggcatc ctggtcatcc agcggatagt taatgatcag cccactgacg 27240
cgttgcgcga gaagattgtg caccgccgct ttacaggctt cgacgccgct tcgttctacc 27300
atcgacacca ccacgctggc acccagttga tcggcgcgag atttaatcgc cgcgacaatt 27360
tgcgacggcg cgtgcagggc cagactggag gtggcaacgc caatcagcaa cgactgtttg 27420
cccgccagtt gttgtgccac gcggttggga atgtaattca gctccgccat cgccgcttcc 27480
actttttccc gcgttttcgc agaaacgtgg ctggcctggt tcaccacgcg ggaaacggtc 27540
tgataagaga caccggcata ctctgcgaca tcgtataacg ttactggttt cacattcacc 27600
accctgaatt gactctcttc cgggcgctat catgccatac cgcgaaaggt tttgcaccat 27660
tcgatggtgt caacgtaaat gccgcttcgc cttcgcgcgc gaattgcaag ctgatccggg 27720
cttatcgact gcacggtgca ccaatgcttc tggcgtcagg cagccatcgg aagctgtggt 27780
atggctgtgc aggtcgtaaa tcactgcata attcgtgtcg ctcaaggcgc actcccgttc 27840
tggataatgt tttttgcgcc gacatcataa cggttctggc aaatattctg aaatgagctg 27900
ttgacaatta atcatcggct cgtataatgt gtggaattgt gagcggataa caatttcaca 27960
caggaaaca 27969

<210> 6
<211> 3881
<212> DNA
<213> Artificial

<220>
<223> partial sequence

<400> 6
gaattcgagc tcggtacccg gggatcctct agagtcgacc tgcaggcatg caagcttggc 60
tgttttggcg gatgagagaa gattttcagc ctgatacaga ttaaatcaga acgcagaagc 120
ggtctgataa aacagaattt gcctggcggc agtagcgcgg tggtcccacc tgaccccatg 180
ccgaactcag aagtgaaacg ccgtagcgcc gatggtagtg tggggtctcc ccatgcgaga 240
gtagggaact gccaggcatc aaataaaacg aaaggctcag tcgaaagact gggcctttcg 300
ttttatctgt tgtttgtcgg tgaacgctct cctgagtagg acaaatccgc cgggagcgga 360
tttgaacgtt gcgaagcaac ggcccggagg gtggcgggca ggacgcccgc cataaactgc 420
caggcatcaa attaagcaga aggccatcct gacggatggc ctttttgcgt ttctacaaac 480
tctttttgtt tatttttcta aatacattca aatatgtatc cgctcatggg ccaagttttg 540
gcgaaaatga gacgttgatc ggcacgtaag aggttccaac tttcaccata atgaaataag 600
atcactaccg ggcgtatttt ttgagttatc gagattttca ggagctaagg aagctaaaat 660
ggagaaaaaa atcactggat ataccaccgt tgatatatcc caatggcatc gtaaagaaca 720
ttttgaggca tttcagtcag ttgctcaatg tacctataac cagaccgttc agctggatat 780
tacggccttt ttaaagaccg taaagaaaaa taagcacaag ttttatccgg cctttattca 840
cattcttgcc cgcctgatga atgctcatcc ggaattccgt atggcaatga aagacggtga 900
gctggtgata tgggatagtg ttcacccttg ttacaccgtt ttccatgagc aaactgaaac 960
gttttcatcg ctctggagtg aataccacga cgatttccgg cagtttctac acatatattc 1020
gcaagatgtg gcgtgttacg gtgaaaacct ggcctatttc cctaaagggt ttattgagaa 1080
tatgtttttc gtctcagcca atccctgggt gagtttcacc agttttgatt taaacgtggc 1140

caatatggac aacttcttcg cccccgtttt caccatgggc aaatattata cgcaaggcga 1200
caaggtgctg atgccgctgg cgattcaggt tcatcatgcc gtctgtgatg gcttccatgt 1260
cggcagaatg cttaatgaat tacaacagta ctgcgatgag tggcagggcg gggcgtaatt 1320
tttttaaggc agttattggt gcccttaaac gcctggtgct acgcctgaat aagtgataat 1380
aagcggatga atggcagaaa ttcgaaacta atcggggtgg cggttcagtg aaaaatatta 1440
aaggatcttc ttgagatcct ttttttctgc gcgtaatctg ctgcctgtaa acgaaaaaac 1500
ccgccttgtg gcgggtcctt ccgtccgatc agaagtcttc gacctcttct aatcggcggt 1560
ggtaactggc ttggaggagc acagtcacca aaactgtctt ttcagtttag ccttaactgg 1620
ttcataactt caagagcaac tcctctaaat cagttaccaa tggctgctgc cagtggcgtt 1680
aagtcgtgtc ttttccgggt tggccccaag agaacagtta ccggagaagg tgcagcagtc 1740
gggctgaacg gggggttcct gcatacagcc cggcccggag cgaactgcct aagtcgcccc 1800
gagataccag aagacgtgag ctatgagaaa gcgccacgtg tcccgcaagg gagaaaggcg 1860
gaaaaggtat ccggcaatcg atatggcggg aacgtggggg cgcaggaggg agcgattcac 1920
cggaaacggt gatgatcttt aagccacgtc gggtttcgcc accgctggcg gatttcatgg 1980
ttgagtcgga agactcccac agatgcaccg aaaaagtgtc tgttttgtga agtccgtcag 2040
gggggcggag cctatggaaa aagcgccagc ggcgcggcct tttgtcctgg ctttgccttt 2100
ttgctcacat gttctttccg gccttatccc ctgattctgt ggataactgt gttaccgttt 2160
ttgtgtgagt ccgtaccgct cgccgcagcc gaacgaccga gggtagcgag tcagtgagcg 2220
aggaagcgga agagcactgg aagctgcatt ttctccttac gcatctatgc ggcatttcac 2280
acccggcatg gtgcactttt catacaatgc gccctgatgc cgaataagta agccagtatc 2340
ctcgaggcga acgccagcaa gacgtagccc agcgcgtcgg ccagcttgca attcgcgcta 2400
acttacatta attgcgttgc gctcactgcc cgctttccag tcgggaaacc tgtcgtgcca 2460
gctgcattaa tgaatcggcc aacgcgcggg gagaggcggt ttgcgtattg ggcgccaggg 2520
tggtttttct tttcaccagt gagacgggca acagctgatt gcccttcacc gcctggccct 2580
gagagagttg cagcaagcgg tccacgctgg tttgccccag caggcgaaaa tcctgtttgc 2640
tggtggttaa cggcgggata taacatgagc tgtcttcggt atcgtcgtat cccactaccg 2700
agatatccgc accaacgcgc agcccggact cggtaatggc gcgcattgcg cccagcgcca 2760
tctgatcgtt ggcaaccagc atcgcagtgg gaacgatgcc ctcattcagc atttgcatgg 2820
tttgttgaaa accggacatg gcactccagt cgccttcccg ttccgctatc ggctgaattt 2880
gattgcgagt gagatattta tgccagccag ccagacgcag acgcgccgag acagaactta 2940
atgggcccgc taacagcgcg atttgctggt gacccaatgc gaccagatgc tccacgccca 3000
gtcgcgtacc gtcttcatgg gagaaaataa tactgttgat gggtgtctgg tcagagacat 3060
caagaaataa cgccggaaca ttagtgcagg cagcttccac agcaatggca tcctggtcat 3120
ccagcggata gttaatgatc agcccactga cgcgttgcgc gagaagattg tgcaccgccg 3180
ctttacaggc ttcgacgccg cttcgttcta ccatcgacac caccacgctg gcacccagtt 3240
gatcggcgcg agatttaatc gccgcgacaa tttgcgacgg cgcgtgcagg gccagactgg 3300
aggtggcaac gccaatcagc aacgactgtt tgcccgccag ttgttgtgcc acgcggttgg 3360
gaatgtaatt cagctccgcc atcgccgctt ccactttttc ccgcgttttc gcagaaacgt 3420
ggctggcctg gttcaccacg cgggaaacgg tctgataaga gacaccggca tactctgcga 3480
catcgtataa cgttactggt ttcacattca ccaccctgaa ttgactctct tccgggcgct 3540
atcatgccat accgcgaaag gttttgcacc attcgatggt gtcaacgtaa atgccgcttc 3600
gccttcgcgc gcgaattgca agctgatccg ggcttatcga ctgcacggtg caccaatgct 3660
tctggcgtca ggcagccatc ggaagctgtg gtatggctgt gcaggtcgta aatcactgca 3720

taattcgtgt cgctcaaggc gcactcccgt tctggataat gttttttgcg ccgacatcat 3780
aacggttctg gcaaatattc tgaaatgagc tgttgacaat taatcatcgg ctcgtataat 3840
gtgtggaatt gtgagcggat aacaatttca cacaggaaac a 3881

<210> 7
<211> 9150
<212> DNA
<213> Artificial

<220>
<223> partial sequence

<400> 7
gaattcgagc tcggtacccg gggatcctct agagtcgacc tgcaggcatg caagcttggc 60
tgttttggcg gatgagagaa gattttcagc ctgatacaga ttaaatcaga acgcagaagc 120
ggtctgataa aacagaattt gcctggcggc agtagcgcgg tggtcccacc tgaccccatg 180
ccgaactcag aagtgaaacg ccgtagcgcc gatggtagtg tggggtctcc ccatgcgaga 240
gtagggaact gccaggcatc aaataaaacg aaaggctcag tcgaaagact gggcctttcg 300
ttttatctgt tgtttgtcgg tgaacgctct cctgagtagg acaaatccgc cgggagcgga 360
tttgaacgtt gcgaagcaac ggcccggagg gtggcgggca ggacgcccgc cataaactgc 420
caggcatcaa attaagcaga aggccatcct gacggatggc ctttttgcgt ttctacaaac 480
tctttttgtt tatttttcta aatacattca aatatgtatc cgctcatgtt aattcatgaa 540
ctagtcatgt attcggctgc aactttgtca tgcttgacac tttatcactg ataaacataa 600
tatgtccacc aacttatcag tgataaagaa tccgcgcgtt caatcggacc agcggaggct 660
ggtccggagg ccagacgtga aacccaacat acccctgatc gtaattctga gcactgtcgc 720
gctcgacgct gtcggcatcg gcctgattat gccggtgctg ccgggcctcc tgcgcgatct 780
ggttcactcg aacgacgtca ccgcccacta tggcattctg ctggcgctgt atgcgttggt 840
gcaatttgcc tgcgcacctg tgctgggcgc gctgtcggat cgtttcgggc ggcggccaat 900
cttgctcgtc tcgctggccg gcgccactgt cgactacgcc atcatggcga cagcgccttt 960
cctttgggtt ctctatatcg ggcggatcgt ggccggcatc accggggcga ctggggcggt 1020
agccggcgct tatattgccg atatcactga tggcgatgag cgcgcgcggc acttcggctt 1080
catgagcgcc tgtttcgggt tcgggatggt cgcgggacct gtgctcggtg ggctgatggg 1140
cggtttctcc ccccacgctc cgttcttcgc cgcggcagcc ttgaacggcc tcaatttcct 1200
gacgggctgt ttccttttgc cggagtcgca caaaggcgaa cgccggccgt tacgccggga 1260
ggctctcaac ccgctcgctt cgttccggtg ggcccggggc atgaccgtcg tcgccgccct 1320
gatggcggtc ttcttcatca tgcaacttgt cggacaggtg ccggccgcgc tttgggtcat 1380
tttcggcgag gatcgctttc actgggacgc gaccacgatc ggcatttcgc ttgccgcatt 1440
tggcattctg cattcactcg cccaggcaat gatcaccggc cctgtagccg cccggctcgg 1500
cgaaaggcgg gcactcatgc tcggaatgat tgccgacggc acaggctaca tcctgcttgc 1560
cttcgcgaca cggggatgga tggcgttccc gatcatggtc ctgcttgctt cgggtggcat 1620
cggaatgccg gcgctgcaag caatgttgtc caggcaggtg gatgaggaac gccaggggca 1680
gctgcaaggc tcactggcgg cgctcaccag cctgacctcg atcgtcggac ccctcctctt 1740
cacggcgatc tatgcggctt ctataacaac gtggaacggg tgggcatgga ttgcaggcgc 1800
tgccctctac ttgctctgcc tgccggcgct gcgtcgcggg ctttggagcg gcgcagggca 1860
acgagccgat cgctgatcgt ggaaacgata ggcctatgcc tcgagctgaa agcgaccagg 1920
tgctcggcgt ggcaagactc gcagcgaacc cgtagaaagc catgctccag ccgcccgcat 1980
tggagaaatt cttcaaattc ccgttgcaca tagcccggca attcctttcc ctgctctgcc 2040
ataagcgcag cgaatgccgg gtaatactcg tcaacgatct gatagagaag ggtttgctcg 2100

ggtcggtggc tctggtaacg accagtatcc cgatcccggc tggccgtcct ggccgccaca 2160
tgaggcatgt tccgcgtcct tgcaatactg tgtttacata cagtctatcg cttagcggaa 2220
agttctttta ccctcagccg aaatgcctgc cgttgctaga cattgccagc cagtgcccgt 2280
cactcccgta ctaactgtca cgaacccctg caataactgt cacgcccccc tgcaataact 2340
gtcacgaacc cctgcaataa ctgtcacgcc cccaaacctg caaacccagc aggggcgggg 2400
gctggcgggg tgttggaaaa atccatccat gattatctaa gaataatcca ctaggcgcgg 2460
ttatcagcgc ccttgtgggg cgctgctgcc cttgcccaat atgcccggcc agaggccgga 2520
tagctggtct attcgctgcg ctaggctaca caccgcccca ccgctgcgcg gcagggggaa 2580
aggcgggcaa agcccgctaa accccacacc aaaccccgca gaaatacgct ggagcgcttt 2640
tagccgcttt agcggccttt ccccctaccc gaagggtggg ggcgcgtgtg cagccccgca 2700
gggcctgtct cggtcgatca ttcagcccgg ctcatccttc tggcgtggcg gcagaccgaa 2760
caaggcgcgg tcgtggtcgc gttcaaggta cgcatccatt gccgccatga gccgatcctc 2820
cggccactcg ctgctgttca ccttggccaa aatcatggcc cccaccagca ccttgcgcct 2880
tgtttcgttc ttgcgctctt gctgctgttc ccttgcccgc acccgctgaa tttcggcatt 2940
gattcgcgct cgttgttctt cgagcttggc cagccgatcc gccgccttgt tgctcccctt 3000
aaccatcttg acaccccatt gttaatgtgc tgtctcgtag gctatcatgg agccgaaaag 3060
caaaagcaac agcgaggcag catggcgatt tatcacctta cggcgaaaac cggcagcagg 3120
tcgggcggcc aatcggccag ggccaaggcc gactacatcc agcgcgaagg caagtatgcc 3180
cgcgacatgg atgaagtctt gcacgccgaa tccgggcaca tgccggagtt cgtcgagcgg 3240
cccgccgact actgggatgc tgccgacctg tatgaacgcg ccaatgggcg gctgttcaag 3300
gaggtcgaat ttgccctgcc ggtcgagctg accctcgacc agcagaaggc gctggcgtcc 3360
gagttcgccc agcacctgac cggtgccgag cgcctgccgt atacgctggc catccatgcc 3420
ggtggcggcg agaacccgca ctgccacctg atgatctccg agcggatcaa tgacggcatc 3480
gagcggcccg ccgctcagtg gttcaagcgg tacaacggca agaccccgga gaagggcggg 3540
gcacagaaga ccgaagcgct caagcccaag gcatggcttg agcagacccg cgaggcatgg 3600
gccgaccatg ccaaccgggc attagagcgg gctggccacg acgcccgcat tgaccacaga 3660
acacttgagg cgcagggcat cgagcgcctg cccggtgttc acctggggcc gaacgtggtg 3720
gagatggaag gccggggcat ccgcaccgac cgggcagacg tggccctgaa catcgacacc 3780
gccaacgccc agatcatcga cttacaggaa taccgggagg caatagacca tgaacgcaat 3840
cgacagagtg aagaaatcca gaggcatcaa cgagttagcg gagcagatcg aaccgctggc 3900
ccagagcatg gcgacactgg ccgacgaagc ccggcaggtc atgagccaga cccagcaggc 3960
cagcgaggcg caggcggcgg agtggctgaa agcccagcgc cagacagggg cggcatgggt 4020
ggagctggcc aaagagttgc gggaggtagc cgccgaggtg agcagcgccg cgcagagcgc 4080
ccggagcgcg tcgcgggggt ggcactggaa gctatggcta accgtgatgc tggcttccat 4140
gatgcctacg gtggtgctgc tgatcgcatc gttgctcttg ctcgacctga cgccactgac 4200
aaccgaggac ggctcgatct ggctgcgctt ggtggcccga tgaagaacga caggactttg 4260
caggccatag gccgacagct caaggccatg ggctgtgagc gcttcgatat cggcgtcagg 4320
gacgccacca ccggccagat gatgaaccgg gaatggtcag ccgccgaagt gctccagaac 4380
acgccatggc tcaagcggat gaatgcccag ggcaatgacg tgtatatcag gcccgccgag 4440
caggagcggc atggtctggt gctggtggac gacctcagcg agtttgacct ggatgacatg 4500
aaagccgagg gccgggagcc tgccctggta gtggaaacca gcccgaagaa ctatcaggca 4560
tgggtcaagg tggccgacgc cgcaggcggt gaacttcggg ggcagattgc ccggacgctg 4620
gccagcgagt acgacgccga cccggccagc gccgacagcc gccactatgg ccgcttggcg 4680

ggcttcacca accgcaagga caagcacacc acccgcgccg gttatcagcc gtgggtgctg 4740
ctgcgtgaat ccaagggcaa gaccgccacc gctggcccgg cgctggtgca gcaggctggc 4800
cagcagatcg agcaggccca gcggcagcag gagaaggccc gcaggctggc cagcctcgaa 4860
ctgcccgagc ggcagcttag ccgccaccgg cgcacggcgc tggacgagta ccgcagcgag 4920
atggccgggc tggtcaagcg cttcggtgat gacctcagca agtgcgactt tatcgccgcg 4980
cagaagctgg ccagccgggg ccgcagtgcc gaggaaatcg gcaaggccat ggccgaggcc 5040
agcccagcgc tggcagagcg caagcccggc cacgaagcgg attacatcga gcgcaccgtc 5100
agcaaggtca tgggtctgcc cagcgtccag cttgcgcggg ccgagctggc acgggcaccg 5160
gcaccccgcc agcgaggcat ggacaggggc gggccagatt tcagcatgta gtgcttgcgt 5220
tggtactcac gcctgttata ctatgagtac tcacgcacag aagggggttt tatggaatac 5280
gaaaaaagcg cttcagggtc ggtctacctg atcaaaagtg acaagggcta ttggttgccc 5340
ggtggctttg gttatacgtc aaacaaggcc gaggctggcc gcttttcagt cgctgatatg 5400
gccagcctta accttgacgg ctgcaccttg tccttgttcc gcgaagacaa gcctttcggc 5460
cccggcaagt ttctcggtga ctgatatgaa agaccaaaag gacaagcaga ccggcgacct 5520
gctggccagc cctgacgctg tacgccaagc gcgatatgcc gagcgcatga aggccaaagg 5580
gatgcgtcag cgcaagttct ggctgaccga cgacgaatac gaggcgctgc gcgagtgcct 5640
ggaagaactc agagcggcgc agggcggggg tagtgacccc gccagcgcct aaccaccaac 5700
tgcctgcaaa ggaggcaatc aatggctacc cataagccta tcaatattct ggaggcgttc 5760
gcagcagcgc cgccaccgct ggactacgtt ttgcccaaca tggtggccgg tacggtcggg 5820
gcgctggtgt cgcccggtgg tgccggtaaa tccatgctgg ccctgcaact ggccgcacag 5880
attgcaggcg ggccggatct gctggaggtg ggcgaactgc ccaccggccc ggtgatctac 5940
ctgcccgccg aagacccgcc caccgccatt catcaccgcc tgcacgccct tggggcgcac 6000
ctcagcgccg aggaacggca agccgtggct gacggcctgc tgatccagcc gctgatcggc 6060
agcctgccca acatcatggc cccggagtgg ttcgacggcc tcaagcgcgc cgccgagggc 6120
cgccgcctga tggtgctgga cacgctgcgc cggttccaca tcgaggaaga aaacgccagc 6180
ggccccatgg cccaggtcat cggtcgcatg gaggccatcg ccgccgatac cgggtgctct 6240
atcgtgttcc tgcaccatgc cagcaagggc gcggccatga tgggcgcagg cgaccagcag 6300
caggccagcc ggggcagctc ggtactggtc gataacatcc gctggcagtc ctacctgtcg 6360
agcatgacca gcgccgaggc cgaggaatgg ggtgtggacg acgaccagcg ccggttcttc 6420
gtccgcttcg gtgtgagcaa ggccaactat ggcgcaccgt tcgctgatcg gtggttcagg 6480
cggcatgacg gcggggtgct caagcccgcc gtgctggaga ggcagcgcaa gagcaagggg 6540
gtgccccgtg gtgaagccta agaacaagca cagcctcagc cacgtccggc acgacccggc 6600
gcactgtctg gcccccggcc tgttccgtgc cctcaagcgg ggcgagcgca agcgcagcaa 6660
gctggacgtg acgtatgact acggcgacgg caagcggatc gagttcagcg gcccggagcc 6720
gctgggcgct gatgatctgc gcatcctgca agggctggtg gccatggctg ggcctaatgg 6780
cctagtgctt ggcccggaac ccaagaccga aggcggacgg cagctccggc tgttcctgga 6840
acccaagtgg gaggccgtca ccgctgaatg ccatgtggtc aaaggtagct atcgggcgct 6900
ggcaaaggaa atcggggcag aggtcgatag tggtggggcg ctcaagcaca tacaggactg 6960
catcgagcgc ctttggaagg tatccatcat cgcccagaat ggccgcaagc ggcaggggtt 7020
tcggctgctg tcggagtacg ccagcgacga ggcggacggg cgcctgtacg tggccctgaa 7080
ccccttgatc gcgcaggccg tcatgggtgg cggccagcat gtgcgcatca gcatggacga 7140
ggtgcgggcg ctggacagcg aaaccgcccg cctgctgcac cagcggctgt gtggctggat 7200
cgaccccggc aaaaccggca aggcttccat agataccttg tgcggctatg tctggccgtc 7260

agaggccagt ggttcgacca tgcgcaagcg ccgccagcgg gtgcgcgagg cgttgccgga 7320

gctggtcgcg ctgggctgga cggtaaccga gttcgcggcg ggcaagtacg acatcacccg 7380

gcccaaggcg gcaggctgac cccccccact ctattgtaaa caagacattt ttatctttta 7440

tattcaatgg cttattttcc tgctaattgg taataccatg aaaaatacca tgctcagaaa 7500

aggcttaaca atattttgaa aaattgccta ctgagcgctg ccgcacagct ccataggccg 7560

ctttcctggc tttgcttcca gatgtatgct cttctgctcc tgcacccatg cattcgcgaa 7620

cgccagcaag acgtagccca gcgcgtcggc cagcttgcaa ttcgcgctaa cttacattaa 7680

ttgcgttgcg ctcactgccc gctttccagt cgggaaacct gtcgtgccag ctgcattaat 7740

gaatcggcca acgcgcgggg agaggcggtt tgcgtattgg gcgccagggt ggtttttctt 7800

ttcaccagtg agacgggcaa cagctgattg cccttcaccg cctggccctg agagagttgc 7860

agcaagcggt ccacgctggt ttgccccagc aggcgaaaat cctgtttgct ggtggttaac 7920

ggcgggatat aacatgagct gtcttcggta tcgtcgtatc ccactaccga gatatccgca 7980

ccaacgcgca gcccggactc ggtaatggcg cgcattgcgc ccagcgccat ctgatcgttg 8040

gcaaccagca tcgcagtggg aacgatgccc tcattcagca tttgcatggt ttgttgaaaa 8100

ccggacatgg cactccagtc gccttcccgt tccgctatcg gctgaatttg attgcgagtg 8160

agatatttat gccagccagc cagacgcaga cgcgccgaga cagaacttaa tgggcccgct 8220

aacagcgcga tttgctggtg acccaatgcg accagatgct ccacgcccag tcgcgtaccg 8280

tcttcatggg agaaaataat actgttgatg ggtgtctggt cagagacatc aagaaataac 8340

gccggaacat tagtgcaggc agcttccaca gcaatggcat cctggtcatc cagcggatag 8400

ttaatgatca gcccactgac gcgttgcgcg agaagattgt gcaccgccgc tttacaggct 8460

tcgacgccgc ttcgttctac catcgacacc accacgctgg cacccagttg atcggcgcga 8520

gatttaatcg ccgcgacaat ttgcgacggc gcgtgcaggg ccagactgga ggtggcaacg 8580

ccaatcagca acgactgttt gcccgccagt tgttgtgcca cgcggttggg aatgtaattc 8640

agctccgcca tcgccgcttc cactttttcc cgcgttttcg cagaaacgtg gctggcctgg 8700

ttcaccacgc gggaaacggt ctgataagag acaccggcat actctgcgac atcgtataac 8760

gttactggtt tcacattcac caccctgaat tgactctctt ccgggcgcta tcatgccata 8820

ccgcgaaagg ttttgcacca ttcgatggtg tcaacgtaaa tgccgcttcg ccttcgcgcg 8880

cgaattgcaa gctgatccgg gcttatcgac tgcacggtgc accaatgctt ctggcgtcag 8940

gcagccatcg gaagctgtgg tatggctgtg caggtcgtaa atcactgcat aattcgtgtc 9000

gctcaaggcg cactcccgtt ctggataatg ttttttgcgc cgacatcata acggttctgg 9060

caaatattct gaaatgagct gttgacaatt aatcatcggc tcgtataatg tgtggaattg 9120

tgagcggata acaatttcac acaggaaaca 9150

<210> 8
<211> 4496
<212> DNA
<213> Artificial

<220>
<223> partial sequence

<400> 8
gaattcgagc tcggtacccg gggatcctct agagtcgacc tgcaggcatg caagcttggc 60

tgttttggcg gatgagagaa gattttcagc ctgatacaga ttaaatcaga acgcagaagc 120

ggtctgataa aacagaattt gcctggcggc agtagcgcgg tggtcccacc tgaccccatg 180

ccgaactcag aagtgaaacg ccgtagcgcc gatggtagtg tggggtctcc ccatgcgaga 240

gtagggaact gccaggcatc aaataaaacg aaaggctcag tcgaaagact gggcctttcg 300

ttttatctgt tgtttgtcgg tgaacgctct cctgagtagg acaaatccgc cgggagcgga 360

tttgaacgtt gcgaagcaac ggcccggagg gtggcgggca ggacgcccgc cataaactgc 420
caggcatcaa attaagcaga aggccatcct gacggatggc ctttttgcgt ttctacaaac 480
tctttttgtt tatttttcta aatacattca aatatgtatc cgctcatgtt aattcatgaa 540
ctagtcatgt attcggctgc aactttgtca tgcttgacac tttatcactg ataaacataa 600
tatgtccacc aacttatcag tgataaagaa tccgcgcgtt caatcggacc agcggaggct 660
ggtccggagg ccagacgtga aacccaacat acccctgatc gtaattctga gcactgtcgc 720
gctcgacgct gtcggcatcg gcctgattat gccggtgctg ccgggcctcc tgcgcgatct 780
ggttcactcg aacgacgtca ccgcccacta tggcattctg ctggcgctgt atgcgttggt 840
gcaatttgcc tgcgcacctg tgctgggcgc gctgtcggat cgtttcgggc ggcggccaat 900
cttgctcgtc tcgctggccg gcgccactgt cgactacgcc atcatggcga cagcgccttt 960
cctttgggtt ctctatatcg ggcggatcgt ggccggcatc accggggcga ctggggcggt 1020
agccggcgct tatattgccg atatcactga tggcgatgag cgcgcgcggc acttcggctt 1080
catgagcgcc tgtttcgggt tcgggatggt cgcgggacct gtgctcggtg ggctgatggg 1140
cggtttctcc ccccacgctc cgttcttcgc cgcggcagcc ttgaacggcc tcaatttcct 1200
gacgggctgt ttccttttgc cggagtcgca caaaggcgaa cgccggccgt tacgccggga 1260
ggctctcaac ccgctcgctt cgttccggtg ggcccggggc atgaccgtcg tcgccgccct 1320
gatggcggtc ttcttcatca tgcaacttgt cggacaggtg ccggccgcgc tttgggtcat 1380
tttcggcgag gatcgctttc actgggacgc gaccacgatc ggcatttcgc ttgccgcatt 1440
tggcattctg cattcactcg cccaggcaat gatcaccggc cctgtagccg cccggctcgg 1500
cgaaaggcgg gcactcatgc tcggaatgat tgccgacggc acaggctaca tcctgcttgc 1560
cttcgcgaca cggggatgga tggcgttccc gatcatggtc ctgcttgctt cgggtggcat 1620
cggaatgccg gcgctgcaag caatgttgtc caggcaggtg gatgaggaac gccaggggca 1680
gctgcaaggc tcactggcgg cgctcaccag cctgacctcg atcgtcggac ccctcctctt 1740
cacggcgatc tatgcggctt ctataacaac gtggaacggg tgggcatgga ttgcaggcgc 1800
tgccctctac ttgctctgcc tgccggcgct gcgtcgcggg ctttggagcg gcgcagggca 1860
acgagccgat cgctgatcgt ggaaacgata ggcctatgcc tcgaactgcg atgagtggca 1920
gggcggggcg taattttttt aaggcagtta ttggtgccct taaacgcctg gtgctacgcc 1980
tgaataagtg ataataagcg gatgaatggc agaaattcga aactaatcgg ggtggcggtt 2040
cagtgaaaaa tattaaagga tcttcttgag atcctttttt tctgcgcgta atctgctgcc 2100
tgtaaacgaa aaaacccgcc ttgtggcggg tccttccgtc cgatcagaag tcttcgacct 2160
cttctaatcg gcggtggtaa ctggcttgga ggagcacagt caccaaaact gtcttttcag 2220
tttagcctta actggttcat aacttcaaga gcaactcctc taaatcagtt accaatggct 2280
gctgccagtg gcgttaagtc gtgtcttttc cgggttggcc ccaagagaac agttaccgga 2340
gaaggtgcag cagtcgggct gaacgggggg ttcctgcata cagcccggcc cggagcgaac 2400
tgcctaagtc gccccgagat accagaagac gtgagctatg agaaagcgcc acgtgtcccg 2460
caagggagaa aggcggaaaa ggtatccggc aatcgatatg gcgggaacgt gggggcgcag 2520
gagggagcga ttcaccggaa acggtgatga tctttaagcc acgtcgggtt tcgccaccgc 2580
tggcggattt catggttgag tcggaagact cccacagatg caccgaaaaa gtgtctgttt 2640
tgtgaagtcc gtcagggggg cggagcctat ggaaaaagcg ccagcggcgc ggccttttgt 2700
cctggctttg cctttttgct cacatgttct ttccggcctt atcccctgat tctgtggata 2760
actgtgttac cgtttttgtg tgagtccgta ccgctcgccg cagccgaacg accgagggta 2820
gcgagtcagt gagcgaggaa gcggaagagc actggaagct gcatttctcc cttacgcatc 2880
tatgcggcat ttcacacccg gcatggtgca cttttcatac aatgcgccct gatgccgaat 2940

aagtaagcca gtatcctcga ggcgaacgcc agcaagacgt agcccagcgc gtcggccagc 3000

ttgcaattcg cgctaactta cattaattgc gttgcgctca ctgcccgctt tccagtcggg 3060

aaacctgtcg tgccagctgc attaatgaat cggccaacgc gcggggagag gcggtttgcg 3120

tattgggcgc cagggtggtt tttcttttca ccagtgagac gggcaacagc tgattgccct 3180

tcaccgcctg gccctgagag agttgcagca agcggtccac gctggtttgc cccagcaggc 3240

gaaaatcctg tttgctggtg gttaacggcg ggatataaca tgagctgtct tcggtatcgt 3300

cgtatcccac taccgagata tccgcaccaa cgcgcagccc ggactcggta atggcgcgca 3360

ttgcgcccag cgccatctga tcgttggcaa ccagcatcgc agtgggaacg atgccctcat 3420

tcagcatttg catggtttgt tgaaaaccgg acatggcact ccagtcgcct tcccgttccg 3480

ctatcggctg aatttgattg cgagtgagat atttatgcca gccagccaga cgcagacgcg 3540

ccgagacaga acttaatggg cccgctaaca gcgcgatttg ctggtgaccc aatgcgacca 3600

gatgctccac gcccagtcgc gtaccgtctt catgggagaa aataatactg ttgatgggtg 3660

tctggtcaga gacatcaaga aataacgccg gaacattagt gcaggcagct tccacagcaa 3720

tggcatcctg gtcatccagc ggatagttaa tgatcagccc actgacgcgt tgcgcgagaa 3780

gattgtgcac cgccgcttta caggcttcga cgccgcttcg ttctaccatc gacaccacca 3840

cgctggcacc cagttgatcg gcgcgagatt taatcgccgc gacaatttgc gacggcgcgt 3900

gcagggccag actggaggtg gcaacgccaa tcagcaacga ctgtttgccc gccagttgtt 3960

gtgccacgcg gttgggaatg taattcagct ccgccatcgc cgcttccact ttttcccgcg 4020

ttttcgcaga aacgtggctg gcctggttca ccacgcggga aacggtctga taagagacac 4080

cggcatactc tgcgacatcg tataacgtta ctggtttcac attcaccacc ctgaattgac 4140

tctcttccgg gcgctatcat gccataccgc gaaaggtttt gcaccattcg atggtgtcaa 4200

cgtaaatgcc gcttcgcctt cgcgcgcgaa ttgcaagctg atccgggctt atcgactgca 4260

cggtgcacca atgcttctgg cgtcaggcag ccatcggaag ctgtggtatg gctgtgcagg 4320

tcgtaaatca ctgcataatt cgtgtcgctc aaggcgcact cccgttctgg ataatgtttt 4380

ttgcgccgac atcataacgg ttctggcaaa tattctgaaa tgagctgttg acaattaatc 4440

atcggctcgt ataatgtgtg gaattgtgag cggataacaa tttcacacag gaaaca 4496

<210> 9
<211> 5291
<212> DNA
<213> Artificial

<220>
<223> partial sequence

<220>
<221> misc_feature
<222> (1120)..(1120)
<223> n is a, c, g, t or u

<400> 9
gaattcgagc tcggtacccg gggatcctct agagtcgacc tgcaggcatg caagcttggc 60

tgttttggcg gatgagagaa gattttcagc ctgatacaga ttaaatcaga acgcagaagc 120

ggtctgataa aacagaattt gcctggcggc agtagcgcgg tggtcccacc tgaccccatg 180

ccgaactcag aagtgaaacg ccgtagcgcc gatggtagtg tggggtctcc ccatgcgaga 240

gtagggaact gccaggcatc aaataaaacg aaaggctcag tcgaaagact gggcctttcg 300

ttttatctgt tgtttgtcgg tgaacgctct cctgagtagg acaaatccgc cgggagcgga 360

tttgaacgtt gcgaagcaac ggcccggagg gtggcgggca ggacgcccgc cataaactgc 420

caggcatcaa attaagcaga aggccatcct gacggatggc ctttttgcgt ttctacaaac 480

tctttttgtt tatttttcta aatacattca aatatgtatc cgctcatgag acaataaccc 540

tgataaatgc ttcaataata ttgaaaaagg aagagtatga gtattcaaca tttccgtgtc 600
gcccttattc ccttttttgc ggcattttgc cttcctgttt ttgctcaccc agaaacgctg 660
gtgaaagtaa aagatgctga agatcagttg ggtgcacgag tgggttacat cgaactggat 720
ctcaacagcg gtaagatcct tgagagtttt cgccccgaag aacgttttcc aatgatgagc 780
acttttaaag ttctgctatg tggcgcggta ttatcccgtg ttgacgccgg gcaagagcaa 840
ctcggtcgcc gcatacacta ttctcagaat gacttggttg agtactcacc agtcacagaa 900
aagcatctta cggatggcat gacagtaaga gaattatgca gtgctgccat aaccatgagt 960
gataacactg cggccaactt acttctgaca acgatcggag gaccgaagga gctaaccgct 1020
tttttgcaca acatggggga tcatgtaact cgccttgatc gttgggaacc ggagctgaat 1080
gaagccatac caaacgacga gcgtgacacc acgatgcctn cagcaatggc aacaacgttg 1140
cgcaaactat taactggcga actacttact ctagcttccc ggcaacaatt aatagactgg 1200
atggaggcgg ataaagttgc aggaccactt ctgcgctcgg cccttccggc tggctggttt 1260
attgctgata aatctggagc cggtgagcgt gggtctcgcg gtatcattgc agcactgggg 1320
ccagatggta agccctcccg tatcgtagtt atctacacga cggggagtca ggcaactatg 1380
gatgaacgaa atagacagat cgctgagata ggtgcctcac tgattaagca ttggtaactg 1440
tcagaccaag tttactcata tatactttag attgatttaa aacttcattt ttaatttaaa 1500
aggatctagg tgaagatcct ttttgataat ctcatgacca aaatccctta acgtgagttt 1560
tcgttccact gagcgtcaga ccccgtagaa aagatcaaag gatcttcttg agatcctttt 1620
tttctgcgcg taatctgctg cttgcaaaca aaaaaaccac cgctaccagc ggtggtttgt 1680
ttgccggatc aagagctacc aactcttttt ccgaaggtaa ctggcttcag cagagcgcag 1740
ataccaaata ctgtccttct agtgtagccg tagttaggcc accacttcaa gaactctgta 1800
gcaccgccta catacctcgc tctgctaatc ctgttaccag tggctgctgc cagtggcgat 1860
aagtcgtgtc ttaccgggtt ggactcaaga cgatagttac cggataaggc gcagcggtcg 1920
ggctgaacgg ggggttcgtg cacacagccc agcttggagc gaacgaccta caccgaactg 1980
agatacctac agcgtgagca ttgagaaagc gccacgcttc ccgaagggag aaaggcggac 2040
aggtatccgg taagcggcag ggtcggaaca ggagagcgca cgagggagct tccaggggga 2100
aacgcctggt atctttatag tcctgtcggg tttcgccacc tctgacttga gcgtcgattt 2160
ttgtgatgct cgtcaggggg gcggagccta tggaaaaacg ccagcaacgc ggccttttta 2220
cggttcctgg ccttttgctg gccttttgct cacatgttct ttcctgcgtt atcccctgat 2280
tctgtggata accgtattac cgcctttgag tgagctgata ccgctcgccg cagccgaacg 2340
accgagcgca gcgagtcagt gagcgaggaa gcggaagagc gcctgatgcg gtattttctc 2400
cttacgcatc tgtgcggtat ttcacaccgc atatggtgca ctctcagtac aatctgctct 2460
gatgccgcat agttaagcca gtatacactc cgctatcgct acgtgactgg gtcatggctg 2520
cgccccgaca cccgccaaca cccgctgacg cgccctgacg ggcttgtctg ctcccggcat 2580
ccgcttacag acaagctgtg accgtctccg ggagctgcat gtgtcagagg ttttcaccgt 2640
catcaccgaa acgcgcgagg cagctgcggt aaagctcatc agcgtggtcg tgaagcgatt 2700
cacagatgtc tgcctgttca tccgcgtcca gctcgttgag tttctccaga agcgttaatg 2760
tctggcttct gataaagcgg gccatgttaa gggcggtttt ttcctgtttg gtcacttgat 2820
gcctccgtgt aagggggaat ttctgttcat gggggtaatg ataccgatga aacgagagag 2880
gatgctcacg atacgggtta ctgatgatga acatgcccgg ttactggaac gttgtgaggg 2940
taaacaactg gcggtatgga tgcggcggga ccagagaaaa atcactcagg gtcaatgcca 3000
gcgcttcgtt aatacagatg taggtgttcc acagggtagc cagcagcatc ctgcgatgca 3060
gatccggaac ataatggtgc agggcgctga cttccgcgtt tccagacttt acgaaacacg 3120

gaaaccgaag accattcatg ttgttgctca ggtcgcagac gttttgcagc agcagtcgct 3180
tcacgttcgc tcgcgtatcg gtgattcatt ctgctaacca gtaaggcaac cccgccagcc 3240
tagccgggtc ctcaacgaca ggagcacgat catgcgcacc cgtggccagg acccaacgct 3300
gcccgagatg cgccgcgtgc ggctgctgga gatggcggac gcgatggata tgttctgcca 3360
agggttggtt tgcgcattca cagttctccg caagaattga ttggctccaa ttcttggagt 3420
ggtgaatccg ttagcgaggt gccgccggct tccattcagg tcgaggtggc ccggctccat 3480
gcaccgcgac gcaacgcggg gaggcagaca aggtataggg cggcgcctac aatccatgcc 3540
aacccgttcc atgtgctcgc cgaggcggca taaatcgccg tgacgatcag cggtccagtg 3600
atcgaagtta ggctggtaag agccgcgagc gatccttgaa gctgtccctg atggtcgtca 3660
tctacctgcc tggacagcat ggcctgcaac gcgggcatcc cgatgccgcc ggaagcgaga 3720
agaatcataa tggggaaggc catccagcct cgcgtcgcga acgccagcaa gacgtagccc 3780
agcgcgtcgg ccagcttgca attcgcgcta acttacatta attgcgttgc gctcactgcc 3840
cgctttccag tcgggaaacc tgtcgtgcca gctgcattaa tgaatcggcc aacgcgcggg 3900
gagaggcggt ttgcgtattg ggcgccaggg tggtttttct tttcaccagt gagacgggca 3960
acagctgatt gcccttcacc gcctggccct gagagagttg cagcaagcgg tccacgctgg 4020
tttgccccag caggcgaaaa tcctgtttgc tggtggttaa cggcgggata taacatgagc 4080
tgtcttcggt atcgtcgtat cccactaccg agatatccgc accaacgcgc agcccggact 4140
cggtaatggc gcgcattgcg cccagcgcca tctgatcgtt ggcaaccagc atcgcagtgg 4200
gaacgatgcc ctcattcagc atttgcatgg tttgttgaaa accggacatg gcactccagt 4260
cgccttcccg ttccgctatc ggctgaattt gattgcgagt gagatattta tgccagccag 4320
ccagacgcag acgcgccgag acagaactta atgggcccgc taacagcgcg atttgctggt 4380
gacccaatgc gaccagatgc tccacgccca gtcgcgtacc gtcttcatgg gagaaaataa 4440
tactgttgat gggtgtctgg tcagagacat caagaaataa cgccggaaca ttagtgcagg 4500
cagcttccac agcaatggca tcctggtcat ccagcggata gttaatgatc agcccactga 4560
cgcgttgcgc gagaagattg tgcaccgccg ctttacaggc ttcgacgccg cttcgttcta 4620
ccatcgacac caccacgctg gcacccagtt gatcggcgcg agatttaatc gccgcgacaa 4680
tttgcgacgg cgcgtgcagg gccagactgg aggtggcaac gccaatcagc aacgactgtt 4740
tgcccgccag ttgttgtgcc acgcggttgg gaatgtaatt cagctccgcc atcgccgctt 4800
ccactttttc ccgcgttttc gcagaaacgt ggctggcctg gttcaccacg cgggaaacgg 4860
tctgataaga gacaccggca tactctgcga catcgtataa cgttactggt ttcacattca 4920
ccaccctgaa ttgactctct tccgggcgct atcatgccat accgcgaaag gttttgcacc 4980
attcgatggt gtcaacgtaa atgccgcttc gccttcgcgc gcgaattgca agctgatccg 5040
ggcttatcga ctgcacggtg caccaatgct tctggcgtca ggcagccatc ggaagctgtg 5100
gtatggctgt gcaggtcgta aatcactgca taattcgtgt cgctcaaggc gcactcccgt 5160
tctggataat gttttttgcg ccgacatcat aacggttctg gcaaatattc tgaaatgagc 5220
tgttgacaat taatcatcgg ctcgtataat gtgtggaatt gtgagcggat aacaatttca 5280
cacaggaaac a 5291

<210> 10
<211> 8353
<212> DNA
<213> Artificial

<220>
<223> partial sequence

<220>

<221>  misc_feature
<222>  (4182)..(4182)
<223>  n is a, c, g, t or u

<400>  10
   gaattcgagc tcggtacccg ctgtcccaac tcggtgtggt tcgcatcgcc ggcggagctg      60
   aaatgctatt gccgcgtgat gttcctggtc acgtggagca gcaaggagcc gaaccagctc     120
   acgcactgcg acggggagtt tctgggccag gaagagggct aaagcccgaa cagatcgcgg     180
   cggccgagaa atatgttgcc gagcgcgagc aaaagcggct aaacggcttc gatataccga     240
   agcatgcacg atatactgat tatgttggtg cgctgtccta tgcaggcacc cggaacgtcg     300
   aggaccaggc gctggccctg ttgaggaaag aaaacgacga gattctggtg ctgcccgtcg     360
   ataaggccac ggtgcaacgc atgaagcgcc tcgccatcgg tgatcccgtc accgtgaccc     420
   cgcgaggctc ccttaaaact acccgaggta ggagtagatg aagaaccgaa acaacgccgt     480
   ggggccacag atacgggcga aaaaaccgaa ggccagcaag accgttccca tcctcgccgg     540
   cctgtccctt ggggcaggct tgcagaccgc gacgcaatat ttcgcccatt ccttccagta     600
   tcaggccgga cttggctgga atatcaacca cgtctacacg ccctggtcga tccttcaatg     660
   ggccggcaag tggtacggcc aatatcccga cgatttcatg cgcgcggcca gcatgggtat     720
   ggtcgtttcg accgtgggcc tgctgggcac ggccgtcacg cagatggtca aggcgaacac     780
   cggcaaggcg aacgactatc tgcacggttc ggcccgctgg gccgacaaga aggacataca     840
   ggccgccggc ctgctgcccc ggccgcgcac cgtcgtcgag ctggtgtcag gcaaacaccc     900
   gcccacttcc agcggcgtct atgtgggcgg ctggcaggac aaggacggca agttccacta     960
   cctgcggcac aacggccccg agcacgtctt gacctacgcg ccgacgcgct ccggcaaggg    1020
   cgtcggcctg gtcgttccga cgctgctttc ctgggcgcac agcgccgtca tcaccgacct    1080
   gaaaggcgag ttgtgggcgc tgaccgccgg ctggcggaag aagcatgccc gcaacaaggt    1140
   cgtgcgcttt gagccggcat ccgcgcaggg tagcgcgtgc tggaacccgc tcgatgaaat    1200
   ccgcttgggg accgagtacg aggttggcga cgtgcagaac cttgccaccc tgatcgtcga    1260
   tccggacggc aagggcctgg aatcgcactg gcagaaaacc agccaggcgc tgcttgtcgg    1320
   cgtcatcctg cacgcgctct acaaggctaa gaacgagggt acgccggcca ccctgccgtc    1380
   ggtggacggc atgcttgccg acccgaaccg cgacgtgggc gagctttgga tggaaatgac    1440
   cacctacggc cacgttgacg ggcagaacca ccctgcggtc ggctctgcgg cccgcgacat    1500
   gatggaccgc ccggaggaag aatccggttc cgtgctgtcc accgccaagt cctacctggc    1560
   cctgtaccgc gacccggttg tggcccgcaa cgtcagcaag tccgacttcc gcatcaagca    1620
   actgatgcac cacgacgacc cggtaagcct gttcatcgtg acgcagccca acgacaaggc    1680
   ccgtctgcgg cctctggtgc gcgtcatggt caacatgatc gtccggctgc tggccgacaa    1740
   gatggacttc gagaacggcc gtcctgtcgc gcactacaag catcgtctgc tgatgatgct    1800
   cgacgagttc cccagcctgg gcaagctcga aatcctgcaa gagtcccttg ccttcgtcgc    1860
   cggctacggc atcaagtgct acctcatctg ccaggacatt aaccagctca agagccgcga    1920
   aaccggctac ggccacgacg aaagcatcac gtcgaactgc cacgtgcaga acgcctaccc    1980
   gccgaaccgc gtggagacgg ccgagcacct gtcaaaactg acaggcacca cgaccatcgt    2040
   gaaggagcag atcacgacga gcggccgccg cacgtcggcg ctgttgggca acgtctcgcg    2100
   gaccttccag gaagtgcagc ggccattgct gacccccgat gaatgcctgc gcatgccggg    2160
   gccgaagaag agcgccgacg gcagcattga agaggcgggc gacatggttg tgtacgtcgc    2220
   cggctatccc gcgatctacg gcaagcagcc gctctacttc aaagacccga tattccaggc    2280
   ccgcgcggcc gtaccggctc cgaaggtcag cgacaagctg atccagacgg ccaccgtcga    2340
   ggaaggggag gggatcacga tatgagccgc ttccagcgcc tcaccaagta cgtcgccatc    2400
   ggaggcggcg cggccttgtt gctcgccggc gcggcctatc tcgccggcgc gaaggtcaac    2460
   accaccaaaa gcattccggt cggcctgtac tggaaatcga atgcgccggt ggagaagggg 2520
   gcttacgtca tgttctgccc gccgcaagtc ggcgtgtttt cggacgccaa ggagcggggc 2580
   tacatcgccg gcggtttctg ccccggcgac tacggctaca tgatgaagcg agttttagcc 2640
   gctaaaggcg acgaggttgc catcaccgac gccggcgtgc gcgtgaatgg cgggctgctg 2700
   cctcacagcg cgctcatcaa ggccgatcca tcaggacggc cgctgcctcg ttatcagtcc 2760
   gacagctaca ccctggggac cgccgaggtt ctgcttatgt ccgacgtaag cgacacgtct 2820
   ttcgacggcc gctacttcgg gcctgtcaat cgttcgcaaa tcatcaccgt catcaggccg 2880
   gttctgacct ggtgagggta gggggatata tgcaatttga acgccttgta atcgctgaaa 2940
   aaccggagct ggcaaaagcc atcgtcgagg gcctgggcgg cggcagccgc aaggacggct 3000
   attacgaatg cggctccgac cgcgtgacct ggtgctacgg tcacatgctg gccttgctag 3060
   acccggagga ctacgacgag cggggatcct ctagagtcga cctgcaggca tgcaagcttg 3120
   gctgttttgg cggatgagag aagattttca gcctgataca gattaaatca gaacgcagaa 3180
   gcggtctgat aaaacagaat ttgcctggcg gcagtagcgc ggtggtccca cctgacccca 3240
   tgccgaactc agaagtgaaa cgccgtagcg ccgatggtag tgtggggtct ccccatgcga 3300
   gagtagggaa ctgccaggca tcaaataaaa cgaaaggctc agtcgaaaga ctgggccttt 3360
   cgttttatct gttgtttgtc ggtgaacgct ctcctgagta ggacaaatcc gccgggagcg 3420
   gatttgaacg ttgcgaagca acggcccgga gggtggcggg caggacgccc gccataaact 3480
   gccaggcatc aaattaagca gaaggccatc ctgacggatg gcctttttgc gtttctacaa 3540
   actctttttg tttatttttc taaatacatt caaatatgta tccgctcatg agacaataac 3600
   cctgataaat gcttcaataa tattgaaaaa ggaagagtat gagtattcaa catttccgtg 3660
   tcgcccttat tccctttttt gcggcatttt gccttcctgt ttttgctcac ccagaaacgc 3720
   tggtgaaagt aaaagatgct gaagatcagt tgggtgcacg agtgggttac atcgaactgg 3780
   atctcaacag cggtaagatc cttgagagtt ttcgccccga agaacgtttt ccaatgatga 3840
   gcacttttaa agttctgcta tgtggcgcgg tattatcccg tgttgacgcc gggcaagagc 3900
   aactcggtcg ccgcatacac tattctcaga atgacttggt tgagtactca ccagtcacag 3960
   aaaagcatct tacggatggc atgacagtaa gagaattatg cagtgctgcc ataaccatga 4020
   gtgataacac tgcggccaac ttacttctga caacgatcgg aggaccgaag gagctaaccg 4080
   cttttttgca caacatgggg gatcatgtaa ctcgccttga tcgttgggaa ccggagctga 4140
   atgaagccat accaaacgac gagcgtgaca ccacgatgcc tncagcaatg gcaacaacgt 4200
   tgcgcaaact attaactggc gaactactta ctctagcttc ccggcaacaa ttaatagact 4260
   ggatggaggc ggataaagtt gcaggaccac ttctgcgctc ggcccttccg gctggctggt 4320
   ttattgctga taaatctgga gccggtgagc gtgggtctcg cggtatcatt gcagcactgg 4380
   ggccagatgg taagccctcc cgtatcgtag ttatctacac gacggggagt caggcaacta 4440
   tggatgaacg aaatagacag atcgctgaga taggtgcctc actgattaag cattggtaac 4500
   tgtcagacca agtttactca tatatacttt agattgattt aaaacttcat ttttaattta 4560
   aaaggatcta ggtgaagatc ctttttgata atctcatgac caaaatccct taacgtgagt 4620
   tttcgttcca ctgagcgtca gaccccgtag aaaagatcaa aggatcttct tgagatcctt 4680
   tttttctgcg cgtaatctgc tgcttgcaaa caaaaaaacc accgctacca gcggtggttt 4740
   gtttgccgga tcaagagcta ccaactcttt ttccgaaggt aactggcttc agcagagcgc 4800
   agataccaaa tactgtcctt ctagtgtagc cgtagttagg ccaccacttc aagaactctg 4860
   tagcaccgcc tacatacctc gctctgctaa tcctgttacc agtggctgct gccagtggcg 4920
   ataagtcgtg tcttaccggg ttggactcaa gacgatagtt accggataag gcgcagcggt 4980
   cgggctgaac ggggggttcg tgcacacagc ccagcttgga gcgaacgacc tacaccgaac 5040
   tgagatacct acagcgtgag cattgagaaa gcgccacgct tcccgaaggg agaaaggcgg 5100
   acaggtatcc ggtaagcggc agggtcggaa caggagagcg cacgagggag cttccagggg 5160
   gaaacgcctg gtatctttat agtcctgtcg ggtttcgcca cctctgactt gagcgtcgat 5220
   ttttgtgatg ctcgtcaggg gggcggagcc tatggaaaaa cgccagcaac gcggcctttt 5280
   tacggttcct ggccttttgc tggccttttg ctcacatgtt ctttcctgcg ttatcccctg 5340
   attctgtgga taaccgtatt accgcctttg agtgagctga taccgctcgc cgcagccgaa 5400
   cgaccgagcg cagcgagtca gtgagcgagg aagcggaaga gcgcctgatg cggtattttc 5460
   tccttacgca tctgtgcggt atttcacacc gcatatggtg cactctcagt acaatctgct 5520
   ctgatgccgc atagttaagc cagtatacac tccgctatcg ctacgtgact gggtcatggc 5580
   tgcgccccga cacccgccaa cacccgctga cgcgccctga cgggcttgtc tgctcccggc 5640
   atccgcttac agacaagctg tgaccgtctc cgggagctgc atgtgtcaga ggttttcacc 5700
   gtcatcaccg aaacgcgcga ggcagctgcg gtaaagctca tcagcgtggt cgtgaagcga 5760
   ttcacagatg tctgcctgtt catccgcgtc cagctcgttg agtttctcca gaagcgttaa 5820
   tgtctggctt ctgataaagc gggccatgtt aagggcggtt ttttcctgtt tggtcacttg 5880
   atgcctccgt gtaaggggga atttctgttc atgggggtaa tgataccgat gaaacgagag 5940
   aggatgctca cgatacgggt tactgatgat gaacatgccc ggttactgga acgttgtgag 6000
   ggtaaacaac tggcggtatg gatgcggcgg gaccagagaa aaatcactca gggtcaatgc 6060
   cagcgcttcg ttaatacaga tgtaggtgtt ccacagggta gccagcagca tcctgcgatg 6120
   cagatccgga acataatggt gcagggcgct gacttccgcg tttccagact ttacgaaaca 6180
   cggaaaccga agaccattca tgttgttgct caggtcgcag acgttttgca gcagcagtcg 6240
   cttcacgttc gctcgcgtat cggtgattca ttctgctaac cagtaaggca accccgccag 6300
   cctagccggg tcctcaacga caggagcacg atcatgcgca cccgtggcca ggacccaacg 6360
   ctgcccgaga tgcgccgcgt gcggctgctg gagatggcgg acgcgatgga tatgttctgc 6420
   caagggttgg tttgcgcatt cacagttctc cgcaagaatt gattggctcc aattcttgga 6480
   gtggtgaatc cgttagcgag gtgccgccgg cttccattca ggtcgaggtg gcccggctcc 6540
   atgcaccgcg acgcaacgcg gggaggcaga caaggtatag ggcggcgcct acaatccatg 6600
   ccaacccgtt ccatgtgctc gccgaggcgg cataaatcgc cgtgacgatc agcggtccag 6660
   tgatcgaagt taggctggta agagccgcga gcgatccttg aagctgtccc tgatggtcgt 6720
   catctacctg cctggacagc atggcctgca acgcgggcat cccgatgccg ccggaagcga 6780
   gaagaatcat aatggggaag gccatccagc ctcgcgtcgc gaacgccagc aagacgtagc 6840
   ccagcgcgtc ggccagcttg caattcgcgc taacttacat taattgcgtt gcgctcactg 6900
   cccgctttcc agtcgggaaa cctgtcgtgc cagctgcatt aatgaatcgg ccaacgcgcg 6960
   gggagaggcg gtttgcgtat tgggcgccag ggtggttttt cttttcacca gtgagacggg 7020
   caacagctga ttgcccttca ccgcctggcc ctgagagagt tgcagcaagc ggtccacgct 7080
   ggtttgcccc agcaggcgaa aatcctgttt gctggtggtt aacggcggga tataacatga 7140
   gctgtcttcg gtatcgtcgt atcccactac cgagatatcc gcaccaacgc gcagcccgga 7200
   ctcggtaatg gcgcgcattg cgcccagcgc catctgatcg ttggcaacca gcatcgcagt 7260
   gggaacgatg ccctcattca gcatttgcat ggtttgttga aaaccggaca tggcactcca 7320
   gtcgccttcc cgttccgcta tcggctgaat ttgattgcga gtgagatatt tatgccagcc 7380
   agccagacgc agacgcgccg agacagaact taatgggccc gctaacagcg cgatttgctg 7440
   gtgacccaat gcgaccagat gctccacgcc cagtcgcgta ccgtcttcat gggagaaaat 7500
   aatactgttg atgggtgtct ggtcagagac atcaagaaat aacgccggaa cattagtgca 7560
   ggcagcttcc acagcaatgg catcctggtc atccagcgga tagttaatga tcagcccact 7620
   gacgcgttgc gcgagaagat tgtgcaccgc cgctttacag gcttcgacgc cgcttcgttc 7680
   taccatcgac accaccacgc tggcacccag ttgatcggcg cgagatttaa tcgccgcgac 7740
   aatttgcgac ggcgcgtgca gggccagact ggaggtggca acgccaatca gcaacgactg 7800
   tttgcccgcc agttgttgtg ccacgcggtt gggaatgtaa ttcagctccg ccatcgccgc 7860
   ttccactttt tcccgcgttt tcgcagaaac gtggctggcc tggttcacca cgcgggaaac 7920
   ggtctgataa gagacaccgg catactctgc gacatcgtat aacgttactg gtttcacatt 7980
   caccaccctg aattgactct cttccgggcg ctatcatgcc ataccgcgaa aggttttgca 8040
   ccattcgatg gtgtcaacgt aaatgccgct tcgccttcgc gcgcgaattg caagctgatc 8100
   cgggcttatc gactgcacgg tgcaccaatg cttctggcgt caggcagcca tcggaagctg 8160
   tggtatggct gtgcaggtcg taaatcactg cataattcgt gtcgctcaag gcgcactccc 8220
   gttctggata atgttttttg cgccgacatc ataacggttc tggcaaatat tctgaaatga 8280
   gctgttgaca attaatcatc ggctcgtata atgtgtggaa ttgtgagcgg ataacaattt 8340
   cacacaggaa aca 8353

<210> 11
<211> 15073
<212> DNA
<213> Artificial

<220>
<223> partial sequence

<400> 11
gaattcgtag gggttactga aaagtgagcg ggaaagaaga gtttcagacc atcaaggagc 60
gggccaagcg caagctggaa cgcgacatgg gtgcggacct gttggccgcg ctcaacgacc 120
cgaaaaccgt tgaagtcatg ctcaacgcgg acggcaaggt gtggcacgaa cgccttggcg 180
agccgatgcg gtacatctgc gacatgcggc ccagccagtc gcaggcgatt atagaaacgg 240
tggccggatt ccacggcaaa gaggtcacgc ggcattcgcc catcctggaa ggcgagttcc 300
ccttggatgg cagccgcttt gccggccaat tgccgccggt cgtggccgcg ccaacctttg 360
cgatccgcaa gcgcgcggtc gccatcttca cgctggaaca gtacgtcgag gcgggcatca 420
tgacccgcga gcaatacgag gtcattaaaa gcgccgtcgc ggcgcatcga aacatcctcg 480
tcattggcgg tactggctcg ggcaagacca cgctcgtcaa cgcgatcatc aatgaaatgg 540
tcgccttcaa cccgtctgag cgcgtcgtca tcatcgagga caccggcgaa atccagtgcg 600
ccgcagagaa cgccgtccaa taccacacca gcatcgacgt ctcgatgacg ctgctgctca 660
agacaacgct gcgtatgcgc cccgaccgca tcctggtcgg tgaggtacgt ggccccgaag 720
cccttgatct gttgatggcc tggaacaccg ggcatgaagg aggtgccgcc accctgcacg 780
caaacaaccc caaagcgggc ctgagccggc tcgccatgct tatcagcatg cacccggatt 840
caccgaaacc cattgagccg ctgattggcg aggcggttca tgtggtcgtc catatcgcca 900
ggacccctag cggccgtcga gtgcaagaaa ttctcgaagt tcttggttac gagaacggcc 960
agtacatcac caaaaccctg taaggagtat ttccaatgac aacggctgtt ccgttccgtc 1020
tgaccatgaa tcgcggcatt ttgttctacc ttgccgtgtt cttcgttctc gctctcgcgt 1080
tatccgcgca tccggcgatg gcctcggaag gcaccggcgg cagcttgcca tatgagagct 1140
ggctgacgaa cctgcgcaac tccgtaaccg gcccggtggc cttcgcgctg tccatcatcg 1200
gcatcgtcgt cgccggcggc gtgctgatct tcggcggcga actcaacgcc ttcttccgaa 1260
ccctgatctt cctggttctg gtgatggcgc tgctggtcgg cgcgcagaac gtgatgagca 1320
ccttcttcgg tcgtggtgcc gaaatcgcgg ccctcggcaa cggggcgctg caccaggtgc 1380
aagtcgcggc ggcggatgcc gtgcgtgcgg tagcggctgg acggctcgcc taatcatggc 1440
tctgcgcacg atccccatcc gtcgcgcagg caaccgagaa aacctgttca tgggtggtga 1500

tcgtgaactg gtgatgttct cgggcctgat ggcgtttgcg ctgattttca gcgcccaaga 1560
gctgcgggcc accgtggtcg gtctgatcct gtggttcggg gcgctctatg cgttccgaat 1620
catggcgaag gccgatccga agatgcggtt cgtgtacctg cgtcaccgcc ggtacaagcc 1680
gtattacccg gcccgctcga ccccgttccg cgagaacacc aatagccaag ggaagcaata 1740
ccgatgatcc aagcaattgc gattgcaatc gcgggcctcg gcgcgcttct gttgttcatc 1800
ctctttgccc gcatccgcgc ggtcgatgcc gaactgaaac tgaaaaagca tcgttccaag 1860
gacgccggcc tggccgatct gctcaactac gccgctgtcg tcgatgacgg cgtaatcgtg 1920
ggcaagaacg gcagctttat ggctgcctgg ctgtacaagg gcgatgacaa cgcaagcagc 1980
accgaccagc agcgcgaagt agtgtccgcc cgcatcaacc aggccctcgc gggcctggga 2040
agtgggtgga tgatccatgt ggacgccgtg cggcgtcctg ctccgaacta cgcggagcgg 2100
ggcctgtcgg cgttccctga ccgtctgacg gcagcgattg aagaagagcg ccggcggcat 2160
ttcgagagcc tgggaacgat gtacgagggc tatttcgtcc tcaccttgac ctggttcccg 2220
ccgctgctcg cccagcgcaa gttcgtcgag ctgatgtttg acgacgacgc gaccgcaccg 2280
gatcgcaagg cgcgcacgcg gggcctcatc gaccaattca agcgtgacgt gcgcagcatc 2340
gagtcgcgcc tgtcgtcggc cgtgtcgctc actcgcttga aggggcacaa gatcgtcaac 2400
gaggacggca cgaccgtcac gcatgacgac ttcctgcgct ggctgcaatt ctgcgtgacg 2460
ggcctgcacc atccggtgca gctccccagc aacccgatgt acctggacgc cctggtcggc 2520
ggacaggaaa tgtggggcgg ggtagtgccc aaggtcggcc gcaagttcgt ccaggtggtc 2580
gctctcgaag gcttcccctt ggagtcctat cccggcatcc tgacggcgct cggcgagctg 2640
ccctgcgagt atcggtggtc gagccggttc atcttcatgg accagcacga agccgtgaag 2700
cacctcgaca agttccgcaa gaagtggcgg cagaagattc gcggcttctt cgaccaggtg 2760
ttcaacacga acaccggccc ggtcgatcag gacgcgcttt cgatggtggc cgatgctgag 2820
gcggccattg ccgaagtcaa cagcggcatc gtggccgtgg gctactacac cagcgtcgtc 2880
gtgctgatgg atgaggaccg cacgcgcctg gaagctgcgg cccgcgatgt tgaaaaggcc 2940
gtcaaccggt tgggctttgc cgcgcgcatc gagtccatca acaccctgga cgccttcctt 3000
ggtagtttgc cgggccacgg cgtggaaaac gtccgccggc cgctcatcaa cacgatgaac 3060
ctggccgacc tgctgccgac cagcaccatc tggaccggca acgcgaacgc gccatgcccg 3120
atgtacccgc cgctgtcgcc ggcgctcatg cactgcgtca cgcaaggatc aacgccgttc 3180
cggctgaacc tgcacgtgcg cgacctcggc cacaccttta tgttcgggcc gaccggcgca 3240
ggtaaatcga cgcacctggc gatcctcgcc gcgcagctcc gtcgctatgc cggcatgtcg 3300
atcttcgcct ttgacaaggg catgtcgatg tacccgctgg ccgccggcat ccgtgcggcc 3360
acgaagggca ccagcggcct gcacttcacc gtggcggccg acgacgaacg cctggcgttc 3420
tgcccgttgc agttcctgag caccaagggc gaccgtgctt gggcgatgga gtggatcgac 3480
accatcctgg cgttgaacgg cgtcgaaacg accccggccc agcgcaacga aatcggcaac 3540
gcgatcatga gcatgcacgc cagcggcgcg cgcacgctct ccgagttcag cgtgacgatt 3600
caggatgagg cgatccgcga ggcgatccgc cagtacaccg tcgatggcgc aatgggccat 3660
ctgctcgacg ccgaagagga cggcttggcg ctgtccgact ttacagtgtt cgagatcgaa 3720
gagctgatga acctcggcga gaaattcgcc ctgcctgtgt tgctctacct gttccgccgt 3780
atcgagcgcg ccctgacggg ccagccggcc gtcatcatcc tggacgaagc ctggttgatg 3840
ctcggccacc cggcattccg cgcgaagatc agggaatggc tcaaggtgct gcgtaaggcc 3900
aactgccttg tgctgatggc aacgcagagc ctgtccgacg ccgccaacag cggcatcctg 3960
gacgtgatcg tggaatcgac cgcgaccaag attttcctgc cgaatattta cgccagggat 4020
gaggacacgg cggccctgta ccgccgcatg ggcctgaacg ctcgccagat cgagattctg 4080

gcccaggccg ttcccaagcg tcagtactac tacgtgtcgg aaaacggccg ccgtctctac 4140
gacctggcac ttggcccgct cgcgctcgcg ttcgtcggcg catccgacaa ggaatccgtc 4200
gccatcatca agaacctgga agccaagttc ggcgaccagt gggtggatga atggctgcgt 4260
ggccggggcc tcgcccttga tgaatacctg gaggcagcat gagttttgca gacacgatca 4320
agggcttgat cttcaagaag aagcccgcaa cggccgcagc agcggcgacg ccggccgcga 4380
ccggcccgca aaccgacaac ccgtacctga cggcgcggcg cacctggaac gaccacgttg 4440
gttccgttgt gtcgcaaaag cagacctggc aggttgtcgg catcctttcg ctgatgatcg 4500
tcctcgcggc ggtcggcggc atcatccaca tcggcagcca gtcgaagttc gtgccctatg 4560
tctacgaggt agacaagctc gggcagacgg ccgccgtggg gccgatgacc agggcgtcga 4620
aagccgatcc gcgtgtcatt cacgcctcgg tggctgagtt cgtcggcgat gctcgcctgg 4680
tgacgccgga cgtagctttg cagcgcaagg ccgtctaccg cctctatgcc aagctcgggc 4740
cgaatgaccc ggccaccgcc aagatgaacg aatggctcaa cggcaccgcc gacgccagcc 4800
cgttcgctcg cgcggccgtc gaaacggtca gcaccgaaat cacttccgta atcccgcaga 4860
cgcccgacac ctggcaggtc gattgggtcg agacgacgcg cgacaggcaa ggcgtggtga 4920
aaggccagcc cgtgcgcatg cgggccttgg tgacggtcta cgtcgtcgag ccgacggcgg 4980
acaccaagga agaacaactg cgaaacaacc cggccgggat ctacgtccgg gacttctcct 5040
ggtcgagact tctgtgaggc actgaattat gaaaaaggaa ctgtttgctt tggtcctggc 5100
cgcgtccgtt agcgtgcctg catttgccgc cgatcccggc gcggacctga ctgacctcta 5160
tttttccggc aagaacccgg agctgaccgc gcaagagcgg gcggccatcg ccatcgccaa 5220
gaagtgggag gcgggtaccg ccggcatgcg gccggtggcc ggccccggtg gttcggtgcg 5280
cttcctgttc ggcgcgcagc agccgagcat cgtatgcgcc gtgctgcaag tgtgcgacgt 5340
ggccctgcaa cccggcgagc aagtcaactc gatcaacctg ggcgacaccg cccgttggac 5400
ggtcgagccg gccattaccg gcagcggcgc gaacgaaacc cagcacctca tcatcaagcc 5460
gatggatgtg ggcctggaaa ccagcctggt cgtgaccacg gaccgccgca gctaccacat 5520
gcgcctgcgc tcgcatcgca cgcagtacat gccgcaggtg tcgttcacct acccggaaga 5580
tgcccttgcg aagtgggacg ccatcaagaa ccgcgaacag cgggatcgcg tcgagaaaac 5640
cattccgcag accggcgagt acctgggcaa cctgagcttc aactactccg tcagcgggtc 5700
cacgtcgtgg aagccggtgc gcgtctacaa cgacggcaag aaaaccatca tccagatgcc 5760
gcactcgatg gaacagaccg aagcgccgac gctcctggtc gttcgcaggg agggcggcct 5820
gttctccgac gatgaaacgg tgatggtcaa ctaccgggtc cagggcgacc gctacatcgt 5880
cgatacgatt ttcgacaagg ccatcctcat cgcgggcgtg ggcagcagcc aggaccgcgt 5940
gaccatttca agggggaact aaaccatgcg taagattctg accgtcatcg cactcgcggc 6000
cacgttggcc ggctgcgcga cctccaagta cggcagcttc gtccaggacg cgccggccgc 6060
ctacaaccag accattgcga ccgacgcggt gaagcagctc gtcaagctct acccgccggc 6120
gcaaaccaag ctggaattgc agcaggctac gcccgatccg ttcggcattg ccctggtcac 6180
tgaccttcgc gcccagggct atgctgtcat ggagtacaag cccgacggca acgcggccgc 6240
agctccggct gctgcgtcct cggccgctgc gaagccggca acgccgcaag cccagggcgg 6300
ctatccgctg cgctacgtgc tggaccaatt cagcgacagc aacctgtatc gcctgaccgt 6360
catggtcggc tctcaatcgc tcacgcgcgc ctacctcgcc caaaacaaca cgatggtccc 6420
ggccggcgca tgggttcgga aggagtaagc caatgagcga agatcaaatg gcaccggacg 6480
catcgccaga tgcggtcaag ccgaaaagcg gggttcgccg cgtcaacaac atgccgatgt 6540
acctcatcgg cggtgtgctc ggcatcttcc tgctggtgat ggccctggtt gctgcggatc 6600
gcgctgcgca gcagaaccag ccgggagctg cgaaggctga gaaggccggc agcaccagca 6660

tgtttgccga cgaaattgcc ggcaaacagc aggacggcat catcaaggcc aagccgctgg 6720
agattccgcc ggaacaaacc gcccagcaac cgacgacgga gctgacgcca gccccggcgc 6780
agggaacgac tatcacggtc gcacggcccg agaacctgga ccagcccccg acgccgccgc 6840
agggtgcgcg caacgaggac ctggaccgca tccgcatggc gaagttgcag atgctggaag 6900
aggcgatcaa ggccaagacg acggtgcgca tcgacgcgcc gcgcagccag ggcagcgccg 6960
gcggcggtgc tccgcagggc cgcgaggaaa cccttgcgcg catccaggag ctgcgtcggc 7020
aggctgagaa cgcccgcgcc accgatccga ccgccgccta tcaggccgcg cttgcgcagg 7080
ctcgcacgat gggcggcgcg gcagggggtg gcggtatggg cggctcgggt gcgccgaccc 7140
tcgtgcagac ctcgaaccgc agtggtggcg gcgctggcta tgggtcgttc gacaaccgca 7200
gcgagggcga ccgttggcgg ctcgactccc agccggaagc acctgcaacg ccctatgtgc 7260
tgcgcgctgg cttcgtcgtt ccggctacgc ttatctcggg catcaactcc gatctgccag 7320
gccaaatcat ggcccaggta tcgcagtcgg tgtacgacac ggcgaccggc aagcacatgc 7380
tcatccccca aggctcgcgc ctggtgggca gctactcgaa cgatgtggcc tacgggcaga 7440
agcgcgttct ggtggcatgg cagcgcatca tcttccccga cggcaaggca atggacattg 7500
gggccatgcc gggcggcgat agcgctgggt atgcaggctt caacgacaag gtcaacaacc 7560
actacttccg caccttcgca tcggcattcc tcatgtcggg cgtcgttgcg ggcatcagct 7620
tgagtcagga ccgtggcaac agcaacagcg gttacggacg acaagacgcg ggttccgcga 7680
tgagtgaagc gttgggtcaa cagctcggcc aagtaacggc gcagatgatc gccaaaaact 7740
tgaatatcgc gccgacgctg gaaatccgtc cgggctatcg cttcaacgtc attgtcacga 7800
aagacatgac gttttctaag ccctaccagg cgtttgacta ttaactccaa ggagtaactt 7860
atgaagaagc tcgctaagaa tgttttagcc gctaaagtag ctctggtgct ggccctctcg 7920
gtcggcacct tggcggtcac gcctgcgcaa gcgggcattc cggtcatcga cggcaccaac 7980
ctgtcacaaa ccactgtcac cgcgattcag caggttgcgc aggtccagaa gcaaatcgag 8040
gaataccgga cgcagttgca gcagtacgaa aacatgctgc aaaacacggt ggccccggcc 8100
gcctacgtgt gggaccaggc gcagtccacc atcaacggcc tgatgagcgc cgttgatacc 8160
ctgaactact acaagaacca ggcgggcagc atcgacgctt acctgggcaa gttcaaggac 8220
gtgtcctact acaaggggtc gccgtgcttc tccctgtcgg gctgctcgga aagcgagcgc 8280
aaggcgatgg aagagaaccg ccgcctggcg tccgaatcgc agaaaaaggc caacgatgcg 8340
ctgttccgtg gcctcgatca gcagcagagc aacctcaagt ccgacgccgc cacgctggag 8400
caattgaagg gcaaggcgac gacggcgcag ggccagttgg aagccctcgg ctacgccaac 8460
cagttcgcca gccagcaggc caaccagctc atgcaaatcc gtggccttct gcttgcgcag 8520
cagaacgcca tcgccacgca gatgcaggcc cagcaggacc ggcaggccca gcaggacgct 8580
gcgggcgcga agctgcgcga gggttcgtac cgcgcaagcc cgtctaagac ctggtgaggg 8640
gaggcgcgat gaagaaatcc aacttcatcg cagttgccgc gctggccgcc gtcatggcgg 8700
ccagcctggc aggctgcgac aacaagcccg acaccgacaa gctgacctgc gccgatctgc 8760
cgaaggtcac ggatgccgct caacgcgcgg agctgttgaa gaagtgcccg cgcggagaac 8820
cgggaggctt caagcccagc gaaaagaaag agtggtgatg acgtatgaaa atccagacta 8880
gagctgccgc gctcgcggtc ctgatgctgg ccttgatgcc ggtagcggca tacgcccaaa 8940
tcgacaattc gggcatcctc gacaacgtat tgcagcgcta ccagaacgcc gcgagcggct 9000
gggccactgt cgtccagaac gccgcaacct ggctgttctg gaccttgacc gtgattagca 9060
tggtctggac cttcggcatg atggcactgc gcaaggccga cattggcgag ttcttcgccg 9120
agttcgtgcg gttcaccatc ttcaccggct tcttctggtg gctgctgacc aacggcccga 9180
atttcgcgtc gtccatctat gcgtccctgc ggcagattgc aggccaggca acggggttgg 9240

ggcaggggct ttcgccgtcc ggcatcgtcg atgttggctt cgagattttc ttcaaggtga 9300
tggacgaaac ctcgtactgg tcgccggtcg atagcttcgt cggtgcctcg ttggcggccg 9360
ccatcctctg catcctggcc ctggtcggcg tgaatatgct tctgctcctg gcgtccggat 9420
ggattcttgc ctacggcggt gtgttcttcc tgggcttcgg cggctcgcgc tggacctcgg 9480
acatggcgat caactactac aagaccgtcc tcggggtcgc cgcgcagctc ttcgcaatgg 9540
tgctgctcgt aggcatcggc aagaccttcc tcgatgacta ctacagccgc atgagcgaag 9600
gcatcaactt caaggaactt ggagtgatgc tgatcgtcgg cctgatcctg ctcgttctgg 9660
tcaacaaggt gccgcagctc atcgccggca tcatcaccgg cgcgagcgtc ggcggtgctg 9720
gtatcggcca gttcggcgct ggcacgctcg tcggtgcggc cgcgacggcc ggcgcggcaa 9780
tcgcaactgg cggcgcatct atcgcggccg gcgctgcggc ggcggccggt ggcgcgcagg 9840
ccatcatggc ggccgcgtcg aaggccagcg ataacgtctc tgccggcact gacattctgt 9900
cgagcatgat gggcggcggc ggtggcggcg gcggtggtag cgccggcacc agcggcggcg 9960
acggcggcgg ctcgggtggc ggcggtggct cgggcggcgg tgaaaccccg atggcctcgg 10020
ccgccggcga caacagcagc ggcgcacgcg gcggcagttc gggcggcggc tcgggtggtg 10080
gccgttcgtc tggcggtatc ggtgccacgg cggccaaggg cggccggatc gcggccgata 10140
ccgtcgccaa cctggcgaaa ggtgccggct cgattgccaa ggccaaggcc ggcgaaatgc 10200
gcgcatcggc ccaggaacgc atcggcgata ccgtaggcgg caagatcgcg caggcaattc 10260
gcggcgcggg tgcggcggcg cagaccgctg caaccgtcgc cgatagcaac agccaggcgc 10320
aggaacaacc tgcaccggca cccgcaccgt cgttcgacga caacagcctt tccgcaagca 10380
acaacaggga agcggccgcc gacgcggatt ccgaagtggc gagcttcgtc aacaagcccg 10440
cccaatcctg aaacgactct taggagctac gaccatgcaa ctgaaaaaag cgttctcgtc 10500
ggccgccctg gtggtggcct tgggcctcgg cgcaactggc tcggccagcg cgcaagacgt 10560
gctgacgggc gatacccgcc tggcctgcga ggccattctg tgcctgtcca cgggcagccg 10620
gcccagcgag tgcagcccgt cgctctcgcg gtacttcggc atccacaagc gcaagctgtc 10680
ggacacgctc aaggcgcggc tgaacttcct caacctctgc ccggtatcga accagacgcc 10740
ggaaatgcag acgctcgttt cctcgatttc gcgcggggcc gggcgctgcg atgcgtcctc 10800
gctgaactcc gtgctgcgtg agtggcggag ctgggacgac cagttctaca tcggcaaccg 10860
cctgccggac tactgcgcgg cctacaccgg ccatgcctat accgacttca acacgaccgc 10920
gccgcgctac gtcggcacgc cggaagaggg cggctattgg atcgaggcgg ccgactacga 10980
ccgcgcgctc aaggagtacg aggcgaagct gaaagagcgg cagcagcagt acggtcgcta 11040
tggcagcgac gcctaccgtc ggttcgagcg gtaaggggag gggatagcga tgccgtttgc 11100
caagctgctg gcacggaacg ctctgccggt ggtcgccctg gtggcggcca ctggcttcgg 11160
tgcggcggat gcgaccgccg cacggctctt ccccgatctg tcggaacaga tggaagagcg 11220
cgttgtgtgc tcggtgtctg cggccgctgg atcccgctgc aggcatgcaa gcttggctgt 11280
tttggcggat gagagaagat tttcagcctg atacagatta aatcagaacg cagaagcggt 11340
ctgataaaac agaatttgcc tggcggcagt agcgcggtgg tcccacctga ccccatgccg 11400
aactcagaag tgaaacgccg tagcgccgat ggtagtgtgg ggtctcccca tgcgagagta 11460
gggaactgcc aggcatcaaa taaaacgaaa ggctcagtcg aaagactggg cctttcgttt 11520
tatctgttgt ttgtcggtga acgctctcct gagtaggaca aatccgccgg gagcggattt 11580
gaacgttgcg aagcaacggc ccggagggtg gcgggcagga cgcccgccat aaactgccag 11640
gcatcaaatt aagcagaagg ccatcctgac ggatggcctt tttgcgtttc tacaaactct 11700
ttttgtttat ttttctaaat acattcaaat atgtatccgc tcatgggcca agttttggcg 11760
aaaatgagac gttgatcggc acgtaagagg ttccaacttt caccataatg aaataagatc 11820

actaccgggc gtattttttg agttatcgag attttcagga gctaaggaag ctaaaatgga 11880
gaaaaaaatc actggatata ccaccgttga tatatcccaa tggcatcgta aagaacattt 11940
tgaggcattt cagtcagttg ctcaatgtac ctataaccag accgttcagc tggatattac 12000
ggccttttta aagaccgtaa agaaaaataa gcacaagttt tatccggcct ttattcacat 12060
tcttgcccgc ctgatgaatg ctcatccgga attccgtatg gcaatgaaag acggtgagct 12120
ggtgatatgg gatagtgttc acccttgtta caccgttttc catgagcaaa ctgaaacgtt 12180
ttcatcgctc tggagtgaat accacgacga tttccggcag tttctacaca tatattcgca 12240
agatgtggcg tgttacggtg aaaacctggc ctatttccct aaagggttta ttgagaatat 12300
gtttttcgtc tcagccaatc cctgggtgag tttcaccagt tttgatttaa acgtggccaa 12360
tatggacaac ttcttcgccc ccgttttcac catgggcaaa tattatacgc aaggcgacaa 12420
ggtgctgatg ccgctggcga ttcaggttca tcatgccgtc tgtgatggct tccatgtcgg 12480
cagaatgctt aatgaattac aacagtactg cgatgagtgg cagggcgggg cgtaattttt 12540
ttaaggcagt tattggtgcc cttaaacgcc tggtgctacg cctgaataag tgataataag 12600
cggatgaatg gcagaaattc gaaactaatc ggggtggcgg ttcagtgaaa aatattaaag 12660
gatcttcttg agatcctttt ttcctgcgcg taatctgctg gcctgtaaac aaaaaaaccc 12720
gccttgcgcc gggttccgtc cgatcagaag tcttcgactc ttctaatcgg cggtggtaac 12780
tggcttggag gagcacagtc accaaaactg tctttctagt ttagccttaa ctggtcagta 12840
acttcaagag caactcctca aacagttacc aatggctgct gccagtggcg ttaagtcgtg 12900
tctttccggg ttggccccaa gagcagttac caggagaagg tgcagcggtc gggctgaacg 12960
ggggttccgc atacagcccg gcccggagcg aactgcctaa gtctccccga gataccgaag 13020
acgtgagcta tgagaagcgt cacgtgttcc gcagggagag gcggaaaagg taccgcgatc 13080
gatattgcgg cgtgggcgca agagggagcg attcacccgg aaacggtgtg atctttaagc 13140
cacgtcgggt ttcgccaccg ctggcggatt tcatggttga gtcggaagac tcccacagat 13200
gcaccgaaaa agtgtctgtt ttgtgaagtc cgtcaggggg gcggagccta tggaaaaagc 13260
gccagcggcg gcgccttttg tcctgctttg ctttttgctc acatgttctt tccggcctta 13320
tcccctgatt ctgtggataa ctgtgttacc gtttttgtgt gagtccgtac cgctcgccgc 13380
agccgaacga ccgaggtagc gagtcagtga gcgaggaagc ggaagagcac tggaagtgca 13440
ttttctcctt acgcatctat gcggcatttc acacccggca tggtgcactt ttcatacaat 13500
gcgccctgat gccgaataag taagccagta tcctcgaggc gaacgccagc aagacgtagc 13560
ccagcgcgtc ggccagcttg caattcgcgc taacttacat taattgcgtt gcgctcactg 13620
cccgctttcc agtcgggaaa cctgtcgtgc cagctgcatt aatgaatcgg ccaacgcgcg 13680
gggagaggcg gtttgcgtat tgggcgccag ggtggttttt cttttcacca gtgagacggg 13740
caacagctga ttgcccttca ccgcctggcc ctgagagagt tgcagcaagc ggtccacgct 13800
ggtttgcccc agcaggcgaa aatcctgttt gctggtggtt aacggcggga tataacatga 13860
gctgtcttcg gtatcgtcgt atcccactac cgagatatcc gcaccaacgc gcagcccgga 13920
ctcggtaatg gcgcgcattg cgcccagcgc catctgatcg ttggcaacca gcatcgcagt 13980
gggaacgatg ccctcattca gcatttgcat ggtttgttga aaaccggaca tggcactcca 14040
gtcgccttcc cgttccgcta tcggctgaat ttgattgcga gtgagatatt tatgccagcc 14100
agccagacgc agacgcgccg agacagaact taatgggccc gctaacagcg cgatttgctg 14160
gtgacccaat gcgaccagat gctccacgcc cagtcgcgta ccgtcttcat gggagaaaat 14220
aatactgttg atgggtgtct ggtcagagac atcaagaaat aacgccggaa cattagtgca 14280
ggcagcttcc acagcaatgg catcctggtc atccagcgga tagttaatga tcagcccact 14340
gacgcgttgc gcgagaagat tgtgcaccgc cgctttacag gcttcgacgc cgcttcgttc 14400

taccatcgac accaccacgc tggcacccag ttgatcggcg cgagatttaa tcgccgcgac 14460

aatttgcgac ggcgcgtgca gggccagact ggaggtggca acgccaatca gcaacgactg 14520

tttgcccgcc agttgttgtg ccacgcggtt gggaatgtaa ttcagctccg ccatcgccgc 14580

ttccactttt tcccgcgttt tcgcagaaac gtggctggcc tggttcacca cgcgggaaac 14640

ggtctgataa gagacaccgg catactctgc gacatcgtat aacgttactg gtttcacatt 14700

caccaccctg aattgactct cttccgggcg ctatcatgcc ataccgcgaa aggttttgca 14760

ccattcgatg gtgtcaacgt aaatgccgct tcgccttcgc gcgcgaattg caagctgatc 14820

cgggcttatc gactgcacgg tgcaccaatg cttctggcgt caggcagcca tcggaagctg 14880

tggtatggct gtgcaggtcg taaatcactg cataattcgt gtcgctcaag gcgcactccc 14940

gttctggata atgttttttg cgccgacatc ataacggttc tggcaaatat tctgaaatga 15000

gctgttgaca attaatcatc ggctcgtata atgtgtggaa ttgtgagcgg ataacaattt 15060

cacacaggaa aca 15073

<210> 12
<211> 18176
<212> DNA
<213> Artificial

<220>
<223> partial sequence

<400> 12
gaattcgtag ggttactgaa aaagtgagcg ggaaagaaga gtttcagacc atcaaggagc 60

gggccaagcg caagctggaa cgcgacatgg gtgcggacct gttggccgcg ctcaacgacc 120

cgaaaaccgt tgaagtcatg ctcaacgcgg acggcaaggt gtggcacgaa cgccttggcg 180

agccgatgcg gtacatctgc gacatgcggc ccagccagtc gcaggcgatt atagaaacgg 240

tggccggatt ccacggcaaa gaggtcacgc ggcattcgcc catcctggaa ggcgagttcc 300

ccttggatgg cagccgcttt gccggccaat tgccgccggt cgtggccgcg ccaacctttg 360

cgatccgcaa gcgcgcggtc gccatcttca cgctggaaca gtacgtcgag gcgggcatca 420

tgacccgcga gcaatacgag gtcattaaaa gcgccgtcgc ggcgcatcga aacatcctcg 480

tcattggcgg tactggctcg ggcaagacca cgctcgtcaa cgcgatcatc aatgaaatgg 540

tcgccttcaa cccgtctgag cgcgtcgtca tcatcgagga caccggcgaa atccagtgcg 600

ccgcagagaa cgccgtccaa taccacacca gcatcgacgt ctcgatgacg ctgctgctca 660

agacaacgct gcgtatgcgc cccgaccgca tcctggtcgg tgaggtacgt ggccccgaag 720

cccttgatct gttgatggcc tggaacaccg ggcatgaagg aggtgccgcc accctgcacg 780

caaacaaccc caaagcgggc ctgagccggc tcgccatgct tatcagcatg cacccggatt 840

caccgaaacc cattgagccg ctgattggcg aggcggttca tgtggtcgtc catatcgcca 900

ggacccctag cggccgtcga gtgcaagaaa ttctcgaagt tcttggttac gagaacggcc 960

agtacatcac caaaaccctg taaggagtat ttccaatgac aacggctgtt ccgttccgtc 1020

tgaccatgaa tcgcggcatt ttgttctacc ttgccgtgtt cttcgttctc gctctcgcgt 1080

tatccgcgca tccggcgatg gcctcggaag gcaccggcgg cagcttgcca tatgagagct 1140

ggctgacgaa cctgcgcaac tccgtaaccg gcccggtggc cttcgcgctg tccatcatcg 1200

gcatcgtcgt cgccggcggc gtgctgatct tcggcggcga actcaacgcc ttcttccgaa 1260

ccctgatctt cctggttctg gtgatggcgc tgctggtcgg cgcgcagaac gtgatgagca 1320

ccttcttcgg tcgtggtgcc gaaatcgcgg ccctcggcaa cggggcgctg caccaggtgc 1380

aagtcgcggc ggcggatgcc gtgcgtgcgg tagcggctgg acggctcgcc taatcatggc 1440

tctgcgcacg atccccatcc gtcgcgcagg caaccgagaa aacctgttca tgggtggtga 1500

tcgtgaactg gtgatgttct cgggcctgat ggcgtttgcg ctgattttca gcgcccaaga 1560

gctgcgggcc accgtggtcg gtctgatcct gtggttcggg gcgctctatg cgttccgaat 1620
catggcgaag gccgatccga agatgcggtt cgtgtacctg cgtcaccgcc ggtacaagcc 1680
gtattacccg gcccgctcga ccccgttccg cgagaacacc aatagccaag ggaagcaata 1740
ccgatgatcc aagcaattgc gattgcaatc gcgggcctcg gcgcgcttct gttgttcatc 1800
ctctttgccc gcatccgcgc ggtcgatgcc gaactgaaac tgaaaaagca tcgttccaag 1860
gacgccggcc tggccgatct gctcaactac gccgctgtcg tcgatgacgg cgtaatcgtg 1920
ggcaagaacg gcagctttat ggctgcctgg ctgtacaagg gcgatgacaa cgcaagcagc 1980
accgaccagc agcgcgaagt agtgtccgcc cgcatcaacc aggccctcgc gggcctggga 2040
agtgggtgga tgatccatgt ggacgccgtg cggcgtcctg ctccgaacta cgcggagcgg 2100
ggcctgtcgg cgttccctga ccgtctgacg gcagcgattg aagaagagcg ccggcggcat 2160
ttcgagagcc tgggaacgat gtacgagggc tatttcgtcc tcaccttgac ctggttcccg 2220
ccgctgctcg cccagcgcaa gttcgtcgag ctgatgtttg acgacgacgc gaccgcaccg 2280
gatcgcaagg cgcgcacgcg gggcctcatc gaccaattca agcgtgacgt gcgcagcatc 2340
gagtcgcgcc tgtcgtcggc cgtgtcgctc actcgcttga aggggcacaa gatcgtcaac 2400
gaggacggca cgaccgtcac gcatgacgac ttcctgcgct ggctgcaatt ctgcgtgacg 2460
ggcctgcacc atccggtgca gctccccagc aacccgatgt acctggacgc cctggtcggc 2520
ggacaggaaa tgtggggcgg ggtagtgccc aaggtcggcc gcaagttcgt ccaggtggtc 2580
gctctcgaag gcttcccctt ggagtcctat cccggcatcc tgacggcgct cggcgagctg 2640
ccctgcgagt atcggtggtc gagccggttc atcttcatgg accagcacga agccgtgaag 2700
cacctcgaca agttccgcaa gaagtggcgg cagaagattc gcggcttctt cgaccaggtg 2760
ttcaacacga acaccggccc ggtcgatcag gacgcgcttt cgatggtggc cgatgctgag 2820
gcggccattg ccgaagtcaa cagcggcatc gtggccgtgg gctactacac cagcgtcgtc 2880
gtgctgatgg atgaggaccg cacgcgcctg gaagctgcgg cccgcgatgt tgaaaaggcc 2940
gtcaaccggt tgggctttgc cgcgcgcatc gagtccatca acaccctgga cgccttcctt 3000
ggtagtttgc cgggccacgg cgtggaaaac gtccgccggc cgctcatcaa cacgatgaac 3060
ctggccgacc tgctgccgac cagcaccatc tggaccggca acgcgaacgc gccatgcccg 3120
atgtacccgc cgctgtcgcc ggcgctcatg cactgcgtca cgcaaggatc aacgccgttc 3180
cggctgaacc tgcacgtgcg cgacctcggc cacaccttta tgttcgggcc gaccggcgca 3240
ggtaaatcga cgcacctggc gatcctcgcc gcgcagctcc gtcgctatgc cggcatgtcg 3300
atcttcgcct ttgacaaggg catgtcgatg tacccgctgg ccgccggcat ccgtgcggcc 3360
acgaagggca ccagcggcct gcacttcacc gtggcggccg acgacgaacg cctggcgttc 3420
tgcccgttgc agttcctgag caccaagggc gaccgtgctt gggcgatgga gtggatcgac 3480
accatcctgg cgttgaacgg cgtcgaaacg accccggccc agcgcaacga aatcggcaac 3540
gcgatcatga gcatgcacgc cagcggcgcg cgcacgctct ccgagttcag cgtgacgatt 3600
caggatgagg cgatccgcga ggcgatccgc cagtacaccg tcgatggcgc aatgggccat 3660
ctgctcgacg ccgaagagga cggcttggcg ctgtccgact ttacagtgtt cgagatcgaa 3720
gagctgatga acctcggcga gaaattcgcc ctgcctgtgt tgctctacct gttccgccgt 3780
atcgagcgcg ccctgacggg ccagccggcc gtcatcatcc tggacgaagc ctggttgatg 3840
ctcggccacc cggcattccg cgcgaagatc agggaatggc tcaaggtgct gcgtaaggcc 3900
aactgccttg tgctgatggc aacgcagagc ctgtccgacg ccgccaacag cggcatcctg 3960
gacgtgatcg tggaatcgac cgcgaccaag attttcctgc cgaatattta cgccagggat 4020
gaggacacgg cggccctgta ccgccgcatg ggcctgaacg ctcgccagat cgagattctg 4080
gcccaggccg ttcccaagcg tcagtactac tacgtgtcgg aaaacggccg ccgtctctac 4140

gacctggcac ttggcccgct cgcgctcgcg ttcgtcggcg catccgacaa ggaatccgtc 4200
gccatcatca agaacctgga agccaagttc ggcgaccagt gggtggatga atggctgcgt 4260
ggccggggcc tcgcccttga tgaatacctg gaggcagcat gagttttgca gacacgatca 4320
agggcttgat cttcaagaag aagcccgcaa cggccgcagc agcggcgacg ccggccgcga 4380
ccggcccgca aaccgacaac ccgtacctga cggcgcggcg cacctggaac gaccacgttg 4440
gttccgttgt gtcgcaaaag cagacctggc aggttgtcgg catcctttcg ctgatgatcg 4500
tcctcgcggc ggtcggcggc atcatccaca tcggcagcca gtcgaagttc gtgccctatg 4560
tctacgaggt agacaagctc gggcagacgg ccgccgtggg gccgatgacc agggcgtcga 4620
aagccgatcc gcgtgtcatt cacgcctcgg tggctgagtt cgtcggcgat gctcgcctgg 4680
tgacgccgga cgtagctttg cagcgcaagg ccgtctaccg cctctatgcc aagctcgggc 4740
cgaatgaccc ggccaccgcc aagatgaacg aatggctcaa cggcaccgcc gacgccagcc 4800
cgttcgctcg cgcggccgtc gaaacggtca gcaccgaaat cacttccgta atcccgcaga 4860
cgcccgacac ctggcaggtc gattgggtcg agacgacgcg cgacaggcaa ggcgtggtga 4920
aaggccagcc cgtgcgcatg cgggccttgg tgacggtcta cgtcgtcgag ccgacggcgg 4980
acaccaagga agaacaactg cgaaacaacc cggccgggat ctacgtccgg gacttctcct 5040
ggtcgagact tctgtgaggc actgaattat gaaaaaggaa ctgtttgctt tggtcctggc 5100
cgcgtccgtt agcgtgcctg catttgccgc cgatcccggc gcggacctga ctgacctcta 5160
tttttccggc aagaacccgg agctgaccgc gcaagagcgg gcggccatcg ccatcgccaa 5220
gaagtgggag gcgggtaccg ccggcatgcg gccggtggcc ggccccggtg gttcggtgcg 5280
cttcctgttc ggcgcgcagc agccgagcat cgtatgcgcc gtgctgcaag tgtgcgacgt 5340
ggccctgcaa cccggcgagc aagtcaactc gatcaacctg ggcgacaccg cccgttggac 5400
ggtcgagccg gccattaccg gcagcggcgc gaacgaaacc cagcacctca tcatcaagcc 5460
gatggatgtg ggcctggaaa ccagcctggt cgtgaccacg gaccgccgca gctaccacat 5520
gcgcctgcgc tcgcatcgca cgcagtacat gccgcaggtg tcgttcacct acccggaaga 5580
tgcccttgcg aagtgggacg ccatcaagaa ccgcgaacag cgggatcgcg tcgagaaaac 5640
cattccgcag accggcgagt acctgggcaa cctgagcttc aactactccg tcagcgggtc 5700
cacgtcgtgg aagccggtgc gcgtctacaa cgacggcaag aaaaccatca tccagatgcc 5760
gcactcgatg gaacagaccg aagcgccgac gctcctggtc gttcgcaggg agggcggcct 5820
gttctccgac gatgaaacgg tgatggtcaa ctaccgggtc cagggcgacc gctacatcgt 5880
cgatacgatt ttcgacaagg ccatcctcat cgcgggcgtg ggcagcagcc aggaccgcgt 5940
gaccatttca agggggaact aaaccatgcg taagattctg accgtcatcg cactcgcggc 6000
cacgttggcc ggctgcgcga cctccaagta cggcagcttc gtccaggacg cgccggccgc 6060
ctacaaccag accattgcga ccgacgcggt gaagcagctc gtcaagctct acccgccggc 6120
gcaaaccaag ctggaattgc agcaggctac gcccgatccg ttcggcattg ccctggtcac 6180
tgaccttcgc gcccagggct atgctgtcat ggagtacaag cccgacggca acgcggccgc 6240
agctccggct gctgcgtcct cggccgctgc gaagccggca acgccgcaag cccagggcgg 6300
ctatccgctg cgctacgtgc tggaccaatt cagcgacagc aacctgtatc gcctgaccgt 6360
catggtcggc tctcaatcgc tcacgcgcgc ctacctcgcc caaaacaaca cgatggtccc 6420
ggccggcgca tgggttcgga aggagtaagc caatgagcga agatcaaatg gcaccggacg 6480
catcgccaga tgcggtcaag ccgaaaagcg gggttcgccg cgtcaacaac atgccgatgt 6540
acctcatcgg cggtgtgctc ggcatcttcc tgctggtgat ggccctggtt gctgcggatc 6600
gcgctgcgca gcagaaccag ccgggagctg cgaaggctga gaaggccggc agcaccagca 6660
tgtttgccga cgaaattgcc ggcaaacagc aggacggcat catcaaggcc aagccgctgg 6720

agattccgcc ggaacaaacc gcccagcaac cgacgacgga gctgacgcca gccccggcgc 6780
agggaacgac tatcacggtc gcacggcccg agaacctgga ccagcccccg acgccgccgc 6840
agggtgcgcg caacgaggac ctggaccgca tccgcatggc gaagttgcag atgctggaag 6900
aggcgatcaa ggccaagacg acggtgcgca tcgacgcgcc gcgcagccag ggcagcgccg 6960
gcggcggtgc tccgcagggc cgcgaggaaa cccttgcgcg catccaggag ctgcgtcggc 7020
aggctgagaa cgcccgcgcc accgatccga ccgccgccta tcaggccgcg cttgcgcagg 7080
ctcgcacgat gggcggcgcg gcagggggtg gcggtatggg cggctcgggt gcgccgaccc 7140
tcgtgcagac ctcgaaccgc agtggtggcg gcgctggcta tgggtcgttc gacaaccgca 7200
gcgagggcga ccgttggcgg ctcgactccc agccggaagc acctgcaacg ccctatgtgc 7260
tgcgcgctgg cttcgtcgtt ccggctacgc ttatctcggg catcaactcc gatctgccag 7320
gccaaatcat ggcccaggta tcgcagtcgg tgtacgacac ggcgaccggc aagcacatgc 7380
tcatccccca aggctcgcgc ctggtgggca gctactcgaa cgatgtggcc tacggcagaa 7440
agcgcgttct ggtggcatgg cagcgcatca tcttccccga cggcaaggca atggacattg 7500
gggccatgcc gggcggcgat agcgctgggt atgcaggctt caacgacaag gtcaacaacc 7560
actacttccg caccttcgca tcggcattcc tcatgtcggg cgtcgttgcg ggcatcagct 7620
tgagtcagga ccgtggcaac agcaacagcg gttacggacg acaagacgcg ggttccgcga 7680
tgagtgaagc gttgggtcaa cagctcggcc aagtaacggc gcagatgatc gccaaaaact 7740
tgaatatcgc gccgacgctg gaaatccgtc cgggctatcg cttcaacgtc attgtcacga 7800
aagacatgac gttttctaag ccctaccagg cgtttgacta ttaactccaa ggagtaactt 7860
atgaagaagc tcgctaagaa tgttttagcc gctaaagtag ctctggtgct ggccctctcg 7920
gtcggcacct tggcggtcac gcctgcgcaa gcgggcattc cggtcatcga cggcaccaac 7980
ctgtcacaaa ccactgtcac cgcgattcag caggttgcgc aggtccagaa gcaaatcgag 8040
gaataccgga cgcagttgca gcagtacgaa aacatgctgc aaaacacggt ggccccggcc 8100
gcctacgtgt gggaccaggc gcagtccacc atcaacggcc tgatgagcgc cgttgatacc 8160
ctgaactact acaagaacca ggcgggcagc atcgacgctt acctgggcaa gttcaaggac 8220
gtgtcctact acaaggggtc gccgtgcttc tccctgtcgg gctgctcgga aagcgagcgc 8280
aaggcgatgg aagagaaccg ccgcctggcg tccgaatcgc agaaaaaggc caacgatgcg 8340
ctgttccgtg gcctcgatca gcagcagagc aacctcaagt ccgacgccgc cacgctggag 8400
caattgaagg gcaaggcgac gacggcgcag ggccagttgg aagccctcgg ctacgccaac 8460
cagttcgcca gccagcaggc caaccagctc atgcaaatcc gtggccttct gcttgcgcag 8520
cagaacgcca tcgccacgca gatgcaggcc cagcaggacc ggcaggccca gcaggacgct 8580
gcgggcgcga agctgcgcga gggttcgtac cgcgcaagcc cgtctaagac ctggtgaggg 8640
gaggcgcgat gaagaaatcc aacttcatcg cagttgccgc gctggccgcc gtcatggcgg 8700
ccagcctggc aggctgcgac aacaagcccg acaccgacaa gctgacctgc gccgatctgc 8760
cgaaggtcac ggatgccgct caacgcgcgg agctgttgaa gaagtgcccg cgcggagaac 8820
cgggaggctt caagcccagc gaaaagaaag agtggtgatg acgtatgaaa atccagacta 8880
gagctgccgc gctcgcggtc ctgatgctgg ccttgatgcc ggtagcggca tacgcccaaa 8940
tcgacaattc gggcatcctc gacaacgtat tgcagcgcta ccagaacgcc gcgagcggct 9000
gggccactgt cgtccagaac gccgcaacct ggctgttctg gaccttgacc gtgattagca 9060
tggtctggac cttcggcatg atggcactgc gcaaggccga cattggcgag ttcttcgccg 9120
agttcgtgcg gttcaccatc ttcaccggct tcttctggtg gctgctgacc aacggcccga 9180
atttcgcgtc gtccatctat gcgtccctgc ggcagattgc aggccaggca acggggttgg 9240
ggcaggggct ttcgccgtcc ggcatcgtcg atgttggctt cgagattttc ttcaaggtga 9300

tggacgaaac ctcgtactgg tcgccggtcg atagcttcgt cggtgcctcg ttggcggccg 9360
ccatcctctg catcctggcc ctggtcggcg tgaatatgct tctgctcctg gcgtccggat 9420
ggattcttgc ctacggcggt gtgttcttcc tgggcttcgg cggctcgcgc tggacctcgg 9480
acatggcgat caactactac aagaccgtcc tcggggtcgc cgcgcagctc ttcgcaatgg 9540
tgctgctcgt aggcatcggc aagaccttcc tcgatgacta ctacagccgc atgagcgaag 9600
gcatcaactt caaggaactt ggagtgatgc tgatcgtcgg cctgatcctg ctcgttctgg 9660
tcaacaaggt gccgcagctc atcgccggca tcatcaccgg cgcgagcgtc ggcggtgctg 9720
gtatcggcca gttcggcgct ggcacgctcg tcggtgcggc cgcgacggcc ggcgcggcaa 9780
tcgcaactgg cggcgcatct atcgcggccg gcgctgcggc ggcggccggt ggcgcgcagg 9840
ccatcatggc ggccgcgtcg aaggccagcg ataacgtctc tgccggcact gacattctgt 9900
cgagcatgat gggcggcggc ggtggcggcg gcggtggtag cgccggcacc agcggcggcg 9960
acggcggcgg ctcgggtggc ggcggtggct cgggcggcgg tgaaaccccg atggcctcgg 10020
ccgccggcga caacagcagc ggcgcacgcg gcggcagttc gggcggcggc tcgggtggtg 10080
gccgttcgtc tggcggtatc ggtgccacgg cggccaaggg cggccggatc gcggccgata 10140
ccgtcgccaa cctggcgaaa ggtgccggct cgattgccaa ggccaaggcc ggcgaaatgc 10200
gcgcatcggc ccaggaacgc atcggcgata ccgtaggcgg caagatcgcg caggcaattc 10260
gcggcgcggg tgcggcggcg cagaccgctg caaccgtcgc cgatagcaac agccaggcgc 10320
aggaacaacc tgcaccggca cccgcaccgt cgttcgacga caacagcctt tccgcaagca 10380
acaacaggga agcggccgcc gacgcggatt ccgaagtggc gagcttcgtc aacaagcccg 10440
cccaatcctg aaacgactct taggagctac gaccatgcaa ctgaaaaaag cgttctcgtc 10500
ggccgccctg gtggtggcct tgggcctcgg cgcaactggc tcggccagcg cgcaagacgt 10560
gctgacgggc gatacccgcc tggcctgcga ggccattctg tgcctgtcca cgggcagccg 10620
gcccagcgag tgcagcccgt cgctctcgcg gtacttcggc atccacaagc gcaagctgtc 10680
ggacacgctc aaggcgcggc tgaacttcct caacctctgc ccggtatcga accagacgcc 10740
ggaaatgcag acgctcgttt cctcgatttc gcgcggggcc gggcgctgcg atgcgtcctc 10800
gctgaactcc gtgctgcgtg agtggcggag ctgggacgac cagttctaca tcggcaaccg 10860
cctgccggac tactgcgcgg cctacaccgg ccatgcctat accgacttca acacgaccgc 10920
gccgcgctac gtcggcacgc cggaagaggg cggctattgg atcgaggcgg ccgactacga 10980
ccgcgcgctc aaggagtacg aggcgaagct gaaagagcgg cagcagcagt acggtcgcta 11040
tggcagcgac gcctaccgtc ggttcgagcg gtaaggggag gggatagcga tgccgtttgc 11100
caagctgctg gcacggaacg ctctgccggt ggtcgccctg gtggcggcca ctggcttcgg 11160
tgcggcggat gcgaccgccg cacggctctt ccccgatctg tcggaacaga tggaagagcg 11220
cgttgtgtgc tcggtgtctg cggccgctgg atccggcgcg ccgagctcgg tacccgcgct 11280
cgtcgtagtc ctccgggtct agcaaggcca gcatgtgacc gtagcaccag gtcacgcggt 11340
cggagccgca ttcgtaatag ccgtccttgc ggctgccgcc gcccaggccc tcgacgatgg 11400
cttttgccag ctccggtttt tcagcgatta caaggcgttc aaattgcata tatcccccta 11460
ccctcaccag gtcagaaccg gcctgatgac ggtgatgatt tgcgaacgat tgacaggccc 11520
gaagtagcgg ccgtcgaaag acgtgtcgct tacgtcggac ataagcagaa cctcggcggt 11580
ccccagggtg tagctgtcgg actgataacg aggcagcggc cgtcctgatg gatcggcctt 11640
gatgagcgcg ctgtgaggca gcagcccgcc attcacgcgc acgccggcgt cggtgatggc 11700
aacctcgtcg cctttagcgg ctaaaactcg cttcatcatg tagccgtagt cgccggggca 11760
gaaaccgccg gcgatgtagc cccgctcctt ggcgtccgaa aacacgccga cttgcggcgg 11820
gcagaacatg acgtaagccc ccttctccac cggcgcattc gatttccagt acaggccgac 11880

cggaatgctt ttggtggtgt tgaccttcgc gccggcgaga taggccgcgc cggcgagcaa 11940
caaggccgcg ccgcctccga tggcgacgta cttggtgagg cgctggaagc ggctcatatc 12000
gtgatcccct ccccttcctc gacggtggcc gtctggatca gcttgtcgct gaccttcgga 12060
gccggtacgg ccgcgcgggc ctggaatatc gggtctttga agtagagcgg ctgcttgccg 12120
tagatcgcgg gatagccggc gacgtacaca accatgtcgc ccgcctcttc aatgctgccg 12180
tcggcgctct tcttcggccc cggcatgcgc aggcattcat cgggggtcag caatggccgc 12240
tgcacttcct ggaaggtccg cgagacgttg cccaacagcg ccgacgtgcg gcggccgctc 12300
gtcgtgatct gctccttcac gatggtcgtg gtgcctgtca gttttgacag gtgctcggcc 12360
gtctccacgc ggttcggcgg gtaggcgttc tgcacgtggc agttcgacgt gatgctttcg 12420
tcgtggccgt agccggtttc gcggctcttg agctggttaa tgtcctggca gatgaggtag 12480
cacttgatgc cgtagccggc gacgaaggca agggactctt gcaggatttc gagcttgccc 12540
aggctgggga actcgtcgag catcatcagc agacgatgct tgtagtgcgc gacaggacgg 12600
ccgttctcga agtccatctt gtcggccagc agccggacga tcatgttgac catgacgcgc 12660
accagaggcc gcagacgggc cttgtcgttg ggctgcgtca cgatgaacag gcttaccggg 12720
tcgtcgtggt gcatcagttg cttgatgcgg aagtcggact tgctgacgtt gcgggccaca 12780
accgggtcgc ggtacagggc caggtaggac ttggcggtgg acagcacgga accggattct 12840
tcctccgggc ggtccatcat gtcgcgggcc gcagagccga ccgcagggtg gttctgcccg 12900
tcaacgtggc cgtaggtggt catttccatc caaagctcgc ccacgtcgcg gttcgggtcg 12960
gcaagcatgc cgtccaccga cggcagggtg gccggcgtac cctcgttctt agccttgtag 13020
agcgcgtgca ggatgacgcc gacaagcagc gcctggctgg ttttctgcca gtgcgattcc 13080
aggcccttgc cgtccggatc gacgatcagg gtggcaaggt tctgcacgtc gccaacctcg 13140
tactcggtcc ccaagcggat ttcatcgagc gggttccagc acgcgctacc ctgcgcggat 13200
gccggctcaa agcgcacgac cttgttgcgg gcatgcttct tccgccagcc ggcggtcagc 13260
gcccacaact cgcctttcag gtcggtgatg acggcgctgt gcgcccagga aagcagcgtc 13320
ggaacgacca ggccgacgcc cttgccggag cgcgtcggcg cgtaggtcaa gacgtgctcg 13380
gggccgttgt gccgcaggta gtggaacttg ccgtccttgt cctgccagcc gcccacatag 13440
acgccgctgg aagtgggcgg gtgtttgcct gacaccagct cgacgacggt gcgcggccgg 13500
ggcagcaggc cggcggcctg tatgtccttc ttgtcggccc agcgggccga accgtgcaga 13560
tagtcgttcg ccttgccggt gttcgccttg accatctgcg tgacggccgt gcccagcagg 13620
cccacggtcg aaacgaccat acccatgctg gccgcgcgca tgaaatcgtc gggatattgg 13680
ccgtaccact tgccggccca ttgaaggatc gaccagggcg tgtagacgtg gttgatattc 13740
cagccaagtc cggcctgata ctggaaggaa tgggcgaaat attgcgtcgc ggtctgcaag 13800
cctgccccaa gggacaggcc ggcgaggatg ggaacggtct tgctggcctt cggttttttc 13860
gcccgtatct gtggccccac ggcgttgttt cggttcttca tctactccta cctcgggtag 13920
ttttaaggga gcctcgcggg gtcacggtga cgggatcacc gatggcgagg cgcttcatgc 13980
gttgcaccgt ggccttatcg acgggcagca ccagaatctc gtcgttttct ttcctcaaca 14040
gggccagcgc ctggtcctcg acgttccggg tgcctgcata ggacagcgca ccaacataat 14100
cagtatatcg tgcatgcttc ggtatatcga agccgtttag ccgcttttgc tcgcgctcgg 14160
caacatattt ctcggccgcc gcgatctgtt cgggctttag ccctcttcct ggcccagaaa 14220
ctccccgtcg cagtgcgtga gctggttcgg ctccttgctg ctccacgtga ccaggaacat 14280
cacgcggcaa tagcatttca gctccgccgg cgatgcgaac cacaccgagt tgggacagcg 14340
gggatcctct agagtcgacc tgcaggcatg caagcttggc tgttttggcg gatgagagaa 14400
gattttcagc ctgatacaga ttaaatcaga acgcagaagc ggtctgataa aacagaattt 14460

gcctggcggc agtagcgcgg tggtcccacc tgaccccatg ccgaactcag aagtgaaacg 14520
ccgtagcgcc gatggtagtg tggggtctcc ccatgcgaga gtagggaact gccaggcatc 14580
aaataaaacg aaaggctcag tcgaaagact gggcctttcg ttttatctgt tgtttgtcgg 14640
tgaacgctct cctgagtagg acaaatccgc cgggagcgga tttgaacgtt gcgaagcaac 14700
ggcccggagg gtggcgggca ggacgcccgc cataaactgc caggcatcaa attaagcaga 14760
aggccatcct gacggatggc ctttttgcgt ttctacaaac tctttttgtt tatttttcta 14820
aatacattca aatatgtatc cgctcatggg ccaagttttg gcgaaaatga gacgttgatc 14880
ggcacgtaag aggttccaac tttcaccata atgaaataag atcactaccg ggcgtatttt 14940
ttgagttatc gagattttca ggagctaagg aagctaaaat ggagaaaaaa atcactggat 15000
ataccaccgt tgatatatcc caatggcatc gtaaagaaca ttttgaggca tttcagtcag 15060
ttgctcaatg tacctataac cagaccgttc agctggatat tacggccttt ttaaagaccg 15120
taaagaaaaa taagcacaag ttttatccgg cctttattca cattcttgcc cgcctgatga 15180
atgctcatcc ggaattccgt atggcaatga aagacggtga gctggtgata tgggatagtg 15240
ttcacccttg ttacaccgtt ttccatgagc aaactgaaac gttttcatcg ctctggagtg 15300
aataccacga cgatttccgg cagtttctac acatatattc gcaagatgtg gcgtgttacg 15360
gtgaaaacct ggcctatttc cctaaagggt ttattgagaa tatgtttttc gtctcagcca 15420
atccctgggt gagtttcacc agttttgatt taaacgtggc caatatggac aacttcttcg 15480
cccccgtttt caccatgggc aaatattata cgcaaggcga caaggtgctg atgccgctgg 15540
cgattcaggt tcatcatgcc gtctgtgatg gcttccatgt cggcagaatg cttaatgaat 15600
tacaacagta ctgcgatgag tggcagggcg gggcgtaatt tttttaaggc agttattggt 15660
gcccttaaac gcctggtgct acgcctgaat aagtgataat aagcggatga atggcagaaa 15720
ttcgaaacta atcggggtgg cggttcagtg aaaaatatta aaggatcttc ttgagatcct 15780
ttttttctgc gcgtaatctg ctggcctgta aacaaaaaaa cccgccttgc gccgggttcc 15840
gtccgatcag aagtcttcga ctcttctaat cggcggtggt aactggcttg gaggagcaca 15900
gtcaccaaaa ctgtctttct agtttagcct taactggtca gtaacttcaa gagcaactcc 15960
tcaaacagtt accaatggct gctgccagtg gcgttaagtc gtgtctttcc gggttggccc 16020
caagagcagt taccaggaga aggtgcagcg gtcgggctga acgggggttc cgcatacagc 16080
ccggcccgga gcgaactgcc taagtctccc cgagataccg aagacgtgag ctatgagaag 16140
cgtcacgtgt tccgcaggga gaggcggaaa aggtaccgcg atcgatattg cggcgtgggc 16200
gcaagaggga gcgattcacc cggaaacggt gtgatcttta agccacgtcg ggtttcgcca 16260
ccgctggcgg atttcatggt tgagtcggaa gactcccaca gatgcaccga aaaagtgtct 16320
gttttgtgaa gtccgtcagg ggggcggagc ctatggaaaa agcgccagcg gcggcgcctt 16380
ttgtcctgct ttgctttttg ctcacatgtt ctttccggcc ttatcccctg attctgtgga 16440
taactgtgtt accgtttttg tgtgagtccg taccgctcgc cgcagccgaa cgaccgaggt 16500
agcgagtcag tgagcgagga agcggaagag cactggaagt gcattttctc cttacgcatc 16560
tatgcggcat ttcacacccg gcatggtgca cttttcatac aatgcgccct gatgccgaat 16620
aagtaagcca gtatcctcga ggcgaacgcc agcaagacgt agcccagcgc gtcggccagc 16680
ttgcaattcg cgctaactta cattaattgc gttgcgctca ctgcccgctt tccagtcggg 16740
aaacctgtcg tgccagctgc attaatgaat cggccaacgc gcggggagag gcggtttgcg 16800
tattgggcgc cagggtggtt tttcttttca ccagtgagac gggcaacagc tgattgccct 16860
tcaccgcctg gccctgagag agttgcagca agcggtccac gctggtttgc cccagcaggc 16920
gaaaatcctg tttgctggtg gttaacggcg ggatataaca tgagctgtct tcggtatcgt 16980
cgtatcccac taccgagata tccgcaccaa cgcgcagccc ggactcggta atggcgcgca 17040

ttgcgcccag cgccatctga tcgttggcaa ccagcatcgc agtgggaacg atgccctcat 17100
tcagcatttg catggtttgt tgaaaaccgg acatggcact ccagtcgcct tcccgttccg 17160
ctatcggctg aatttgattg cgagtgagat atttatgcca gccagccaga cgcagacgcg 17220
ccgagacaga acttaatggg cccgctaaca gcgcgatttg ctggtgaccc aatgcgacca 17280
gatgctccac gcccagtcgc gtaccgtctt catgggagaa aataatactg ttgatgggtg 17340
tctggtcaga gacatcaaga aataacgccg gaacattagt gcaggcagct tccacagcaa 17400
tggcatcctg gtcatccagc ggatagttaa tgatcagccc actgacgcgt tgcgcgagaa 17460
gattgtgcac cgccgcttta caggcttcga cgccgcttcg ttctaccatc gacaccacca 17520
cgctggcacc cagttgatcg gcgcgagatt taatcgccgc gacaatttgc gacggcgcgt 17580
gcagggccag actggaggtg gcaacgccaa tcagcaacga ctgtttgccc gccagttgtt 17640
gtgccacgcg gttgggaatg taattcagct ccgccatcgc cgcttccact ttttcccgcg 17700
ttttcgcaga aacgtggctg gcctggttca ccacgcggga aacggtctga taagagacac 17760
cggcatactc tgcgacatcg tataacgtta ctggtttcac attcaccacc ctgaattgac 17820
tctcttccgg gcgctatcat gccataccgc gaaaggtttt gcaccattcg atggtgtcaa 17880
cgtaaatgcc gcttcgcctt cgcgcgcgaa ttgcaagctg atccgggctt atcgactgca 17940
cggtgcacca atgcttctgg cgtcaggcag ccatcggaag ctgtggtatg gctgtgcagg 18000
tcgtaaatca ctgcataatt cgtgtcgctc aaggcgcact cccgttctgg ataatgtttt 18060
ttgcgccgac atcataacgg ttctggcaaa tattctgaaa tgagctgttg acaattaatc 18120
atcggctcgt ataatgtgtg gaattgtgag cggataacaa tttcacacag gaaaca 18176

<210> 13
<211> 35998
<212> DNA
<213> Artificial

<220>
<223> partial sequence

<400> 13
gaattcgagc tcggtacccg gggatcctct agatgacctg attgtgcgta ttgcgaccgg 60
aaccctccgg gcgattctcg ccgtgggcac tggccttgtt tacgttcaat tcgtagggct 120
ggcctttgtg ctggcgatgt ttgggaccgt gacttttgcg gtgattgtgg cggccgtcaa 180
gaccgcgatg tttggagctt aatgcacatg gcaaccaaga caaagaaggc acagcacacg 240
gccgaactgg tgaagctctc cgctgacctg ctgccgttaa ttgaagccct ggaagtcgaa 300
atagagacgg tttcccagcg catggcggcg ctgaaaaagg ccggcatcat ttacgcatcc 360
gaacattggc gcaaggaaaa gggcgagccg aaatacttct atctgctgta tccgcagcag 420
cccggcgagc cgcgcaagcg cgaatacatc gggtgcgatc ctgagcgcat cgcagaagct 480
cgccagggca tcgcccgttc caaggagtac gacgagcttg ccaccatcct agcgggcctc 540
accagtcgcg tgcatcacgt tacccaggcg atgcacgaag ctcgccgcta ccttaccggc 600
aagcgctggt aagccccttt cgcccttgcg gagactgcgc acatggactt gtcgcacggc 660
tttatccccc tagcgctgaa tgcgtcactg tccgcgctcg atgccctcgc cgctggcgac 720
gggcttcgct ccgatgacct gatcgcaggc gcaatcgcgc tcgatctgct agctagccaa 780
ggtcattact cacatacgcg cttcaacggt ttgatgacgg ccgtgcttgg cttgcgcatc 840
ctagcgacgc ggcaaagcat ctctgaaagc caggaaggac gccgagcggc gacgcacttc 900
gcggccatcg tgcgcgaaat gcaaaaccat cttttcaact gaccaccacc aacagcagga 960
gaacaaacca tgaaccaaga cgatttgcgc acgcttacgt ccaccccgga gccgatgacg 1020
gccgcagaac gggccgcgct gcaacggttg ctcgctatcg ccaaaagtga caccgggcag 1080
tcccggcgca ccgctgattt tctgctggca tggtggaacg ccggtagttg cggcagcttc 1140

gaccttaccg agctatgggg cctcgacacc gccattgctc aagacatggt gacggtgttc 1200
gcgcttgtcg cgcgggtcca cgtctacccc gacaccctcg gcttcaaggc cgactttgaa 1260
gccatcgtgc gtgagtggcg gccggagctg gtgaaatgaa tacgcagacc accccgaaga 1320
agcgcagcaa ggtcaagctg gccgttttga tcctcctgag cctgggcggc tgggccgccg 1380
ccattggcac agggatgacc ctcggcgcgg cgaaaaatca agccgttgag gatcaacggc 1440
aagtgcagtt gtggcgggcc aaggcggcca aggcttacac cgaattgctc aaagccaagt 1500
ccggtacacc tgggcaaatc acggtcccgg ccggctcact ggtggactgc gcagcaaatg 1560
gcaccggaca gctagagtgc agcggcttga tattgccgcc cgccgattac tgactggcga 1620
cagcaaacgg acctggccag ctcggccgcg tcaccaaaca agcgaccgtt aagcaggtaa 1680
aaccgcatga aaatcatgtt cgcaatcgca cttctgacgc tcaccgcaaa cgccatggca 1740
gcggagccag ccgtgaacat agcccccggc acgtcggtta agtgcgctcc attcccgact 1800
atcaaaacgc tcaatgcgag cctttttgag ggcgggaagc tggtcggcgc ggggcaagtg 1860
gcgtgctacg tcatagacaa ccagccgcta ccaaagggga ccaaactagt cgcggggcaa 1920
gtggacggcc ccatagccaa ttcgtacacc ctggcgtggt cagctctaca gctacaggat 1980
ggctatgtag tgccattgga taacaacttg attgtgactc ggccgggggg gccggatcat 2040
atcgttctca catttcggcg cggcctcgat gcgggtgtga gtgcccagga agcggcggaa 2100
aagtagtcca tccccacgga aagcaaagcc cggccaagag ccgggttttc tttttggcga 2160
cggttgagca gatccagggc gggcggtcag tcaccaaagg ccggaagcgg tgtcaagggc 2220
gggaccggag gccgcagccg tgagcgcagc gaggggaccg ggcgaggatg ccggcgaagc 2280
gaacccttga tgccggtgga gtgcccccca cccttcgcgc agggatgcag ggagctttac 2340
cccctgcaac cctgctgccc ggcgagggac ggagagtcca gaggggcagc gcccctttgg 2400
gtaagcccgc cagggcgcgc aggaaggcca cagcgcggcg cagcaggtct tgccggtgcg 2460
aacccacgcg ggcgcaggct ggagggccac cacgggccgt taaagccaca aagcgggcct 2520
gtggataacc ctgttatttc agcgacttat ccacgctgaa agtgtcttgc gcgcagagcg 2580
ttgatttacc taggcaaaaa gcacttctcc acagaatcgc acgctgaaag tgtcttgcac 2640
cctcgcgctg aaactgtctt acgcaccgcg aaccaaaacg gccgcaaagc cccgccaatg 2700
ctggctattc tggccgtcgc cgatggctcc ccctgtagtt ttctttaaag atatacctat 2760
aaaaaatcct gtaggttatg gcgcggcgtt gtgaacaacc gccgaaaaca aaagcgcctg 2820
cccttcgtgc ttcgcactcg ccccgccgcc tacggcgacg gcctgtcggt gggcaagccc 2880
acctcaaaaa tgtgtgccct ttgggcacga ctctttacca aaagccggcg actcgactat 2940
tgcgtagtgg atggtctaag cggcccggcc gacaagtcgg ccgccctcac ggcaagccgt 3000
gacccctgcg gggcgctaag acccggacaa atcacgcccg accggctacg ccggtcgccc 3060
gctcagccgg gttcgtgtgc gacccatcca tgaaccgtcc gcgctggtgg agaggtcgaa 3120
aaggttttgc agggaaaagg aaggggtcgg ttgaactcgg agtgcccata ggaccgccag 3180
agagggccgc cagagccgtt ttcagcctga tttgatacct cgggcacccc ctgccctgaa 3240
agcgcgctgt agggcttcct atcggcccgc gtgattctga gtgaactaga cggcgatggc 3300
cgcttacgca cattccctct aaccctcggg tttacggcca tatcaggcgc ttctatttgt 3360
tttcccgttg ttgtcttttg ttttcctgtt gttgtctttt ttgttttccc ggtgttatac 3420
tggctgtgcg gtcaatgtct ggccgtgcca cctggagaac gtcatgcaga tcaacgtgtt 3480
tgaaggtcat cttgcgaaaa ccccaacaat cagcgggaca ggcgaccgcg ctgtaacccg 3540
tttcaccctg atttccaacg aatacgccgg caaggatcgc gaaacaggcg aggcccgcga 3600
gcggtcggtt tcgatccagt tcacggcctt tcgtcagaag gccgaaacca tcgcaaagaa 3660
cgcgatgaag ggcgaccagc tcatcattca gtaccgaatc gagaacaacg actacacgaa 3720

agacggcgaa accgtctacg ggtacaactt cgttgtcgag gacttcaagt acggtgcgcc 3780
cggcaaggaa aaacgagagc aattcaaggc gagtgtgtga gcaatggcgt cccctacccc 3840
aaggatggac gccgcgcagt tcgaccgcgt agcgtccaaa tgcagatggt ctgagcggtc 3900
cttgggcgtt gtgcgcgctg tattggttga cgggctgtcg ctgccggatg cggtggcgac 3960
ggcaataccg gcaatgacaa ccaagcaagc gcgagttctt cgggacaggt tcaccgcgaa 4020
agcggagagc ctgcgccttg aggaattcat gcgacgcgaa acaccaaagc tcgctaccac 4080
agcactagag ccgtatgcca gcgaagtaag cacccttcgg gacaagggtt acacgattga 4140
gcaaatcgtc gcgttcttca aggcaaacgg tgtgaagaca tcaccgacga ccgttcggaa 4200
ctttttaagg agcattcggg catgaaaacc actgtacttg cgaaccagaa aggcggcgtc 4260
ggtaagtccg caatcgcggt tcaattcgca gactacttgg cgcttttgcg caacaagcgc 4320
gttctcgtta tcgacttcga ccatcagcgc aacacgtcga aagcgatccg cacaggtggc 4380
cttgccactg tatcggccgt gaccagcagc atgattttga agaacaaaat tacgagcatc 4440
gagcacgctg attttgtctt gatcgctggt gatgctgact tgatcaaaat ggaaaaacag 4500
gcggccgagc acaacgcttt cgcgacgaac ctttacacgt tcctggcatc cgtctccgat 4560
cagttcgatt tctgcatcat cgacaccaac ccgaaccccg acattcggca actcgcttcg 4620
cttgtcgtga gtgacttcgt gctgtcgcca ttgcagttga accaggaggc aatcgacggc 4680
atcggcgacc tgttgaatca cgagaacatc ggtattcgca agatacaggc gaccttgaac 4740
aagaaactgc aactcattgg cattctgccg aacatggtcg aaccgacacc gttccagcgt 4800
gacaacttcc gcgacttgtc caaggctttc gcaaagctgc tgatccccct tgataacggc 4860
tttgccgcca tcaagaaaac caccgcgatt ccagaggcgc aggcccaggg cctgcccgtt 4920
tggaaagtag gcaaaaccag tgcgcgcgaa gcctggacgc agatgcgccc gatcttcgac 4980
aagatcgctc agaccatggg agttgaacaa gatgcttgac ctttccgcct tggaagaaaa 5040
gccaaccgcg cccggcgcgg agccgaccgg caaagccatg cgcgccaagc tgaccgatgt 5100
cgagccggac cccggccagc cgcgcaccga gaagaagcag gaggagattg aagaactcgg 5160
cacaaacatc aaatcgcgcg gcgtaaagca gcctatctcc gtcaagaagc acccaaccaa 5220
gccgggcaag tggcttatca atgacggcga gctgcgttac ctcagttcgt tgtgggccgg 5280
cgtcgaagac attccgattg tcgttgacga ggattttgac gacttcgacc aagtgaacgc 5340
caatgaaaag cggttcgcat tacgaccgat ggaactcgcc gtattcatca agcgtaagct 5400
tgatgaaggg gtgaagaaag ctgaaatcgc tcgccgactg gggaagccgg gcagtgctat 5460
cactgaattg ctggcattgg tggatgcgcc gcaatgcatc caagtcgtct acacctctgg 5520
tcgctgcacg tcaccaaaga cgctctacga gttgcgggct ctacacgaca agttcggcga 5580
gaaagtcgag gcatggtgcg aggcggccga ggaaattact cgcaagggtg tttccgaact 5640
cggcgacatt ttgaaaggca agaagaagcc agcaccgggc agtgacaaca tcgctggtgc 5700
ggaccaaacc agcggcggcg gatcagaaag tcttagtcat gacgaagact ttccagggcg 5760
agacggttcg caaaacagcg acagcggcaa tggtggggga cgtgagacgg aagacgatga 5820
ccaggatacg ggcggcaaaa agtcgagcgc tagcggccag ggcaagggcg agggtgacga 5880
caatatcgac acgggcgagc tgacgagttg gccgcgcggg aaggcggtat ctgatcccga 5940
tagtatgaaa aagccgctgc tgatcgttga gcacgacggc cgcgcggcgg cggttctgct 6000
taaccgtcgt ccttccaccg ctggactgat ccgcatacgt tacgaggatg gcggcggcga 6060
cgccgaagtc gatgccggtt cactgaaaat caacctgctg accgaggccg aaaaatgaac 6120
gccaggaagg cggcccgtaa cggtgtcgcc ggcctgatcg tggccgggct agtgctcgcg 6180
tttgttgtcg gtaggcagca gacgcgggcc gcaacaccga ccgaattttg tacgctgcat 6240
ttcagcggcg gcgtgatgct tgaaggcgta ccggtagcga agacgccaga gcaagaggcc 6300

aggggcctca tgcaccgcga cgatgccggc cccggcatgc tgttctcctg ggacaaagcc 6360
gaaccgcgcg ttttctggat gcggaatacc cgcatgccac tatcaatcgg cttcatgtcc 6420
gcagacggaa ccttgttcgc catcgaggac atgcagccaa acagcgacga gtaccatttt 6480
tcgcaagacc ccgcccagga tgccctagaa ctcgcgcaag gcgatttctc acgcaggggc 6540
ctgggtgtgg gttcccgcct gctacgtcga gagtgccagg accggaacac gtattaggct 6600
ggggccggtg tcaagggtga aaccggaggc cgcagcccgt agcgaagcgc agggaccggg 6660
cgaggatttc agcgaagcga acccttgatt ccggtgtaag ccgaaaaccc ttcgggcggg 6720
gttgcaggag agtttttccc tcctgcaacc ctgctaccgg cgagggcgga gagtccagag 6780
gggcagcgcc cctttgggca agcccgaagg gcgcgcagtc tgacccatcc cattttttgc 6840
ttcacaactc actcgacatt tctcgttcgt gttctgcaaa accacgtttc ttttcccaag 6900
ctcggcattc cttcgtcatg actaaagatt agaggcttgt gaggtctatt gcaggatagg 6960
ctaacgccgg taaagtgtat tacactacgt tcatacgtat gtccccttaa ttggattgga 7020
ggcgcttgtg ccgcgaaatt ctgagattgg agccacccga ggtagctatg tctatacctt 7080
gcggctgccc gacgatctgc gtgatcagtg gacggcctac tgttcacgca acaagaaaaa 7140
agcggctgcc acgatgcggg cgcttatgcg ttatttgatc caagacgata tgccgcccga 7200
ggttcgtcgt tggatcgctg cgcaaacaga gggcgagcca gacgacggtc caaaggaacg 7260
tctcgaagtt cgcctaacac cgacagagtt ccagtcggta aaagttcgtt ccgaggccga 7320
aggatgttcc ccccagcgct gggtcataaa cagcgttcgt gcaagcctca cccatgaacc 7380
gcaattcacg atggaaacga cgaaagcgtt atgggagtcc tcttaccagc tccgcgcaat 7440
cggtcggaac ctaaaccaga tcgccaagcg cttgaatgag gggaagtcgg ccaaagtcac 7500
gaccgaacag atggaaaagc tagcgagcta catctaccag cacacggaca aggtggcgtc 7560
cgtccaggac gcgagcctta gccgttgggg cgtacaaatt caggggatcg ccgatggcaa 7620
agcgagagat tgacggtgtt ctgaaggatt ggggcgagcg tgtcgattac ggccgagtcc 7680
agggccgtaa aggcaagaac attgccggag gtcgctattc caagccggcc ccggaaaaga 7740
aaccatcggg ccgcgagaag cttgaagcca cagtgcgtaa agcacccgaa gtcatggtga 7800
aaatctccgg gggcggcaag gatatgcgca gtatcaaagc tcacatggac tacatatcgc 7860
gcaatggtgc tgtcgagctg gaggacgagc agggcagggt tcaccagggc aaggaggacg 7920
ttcgagccgt gcgcgatgac tggcgaggcg gcggcatccc gtacgaggat ggcaccaagc 7980
gcgaagcgtt taacatcgtt ttgtcgatgc ctcctggcac gaacaggcag gcagtcaaag 8040
atgcagcgcg ggctttcgcc gccgagctgt tcggtaatca tcaatacgtt tttgcggcgc 8100
acgatgacga gaaacacccg catgttcact tggcggtcaa ggccgttgac ctagatgggg 8160
tgcgcctcaa cccgcgcaaa gcagatttgc agaattggcg cgagacgttc gccgaaaagc 8220
tccgggcgca agggatcgac gcgaacgcga cgcctcgcaa ggcgcggggc attgtcagaa 8280
aagcggaaaa acaagccgtc gtccacatcg agcagcgggg taaaactccg cgcgtcaagg 8340
cgagtcggcg cgcagatgcg gaacgcgagg caagcggcgt ggcagtaaag cccaatccag 8400
cgcaagacag aatcagcgtg actcgaaaag acgttcagcg cgcatacggc acggccgccc 8460
gtgcgctggc gaagggcgag gccaaggaca agcagttggc cgtcgagatt gttcacttcg 8520
tccagcagat gccgccgccg atcacgcagc acgaagccat ggtgcggcag cttcgcgaga 8580
agaaacggga tgatgcgcgg actatcggtc ccgctaagac caaagagact caacaggaca 8640
aggaccaggg cggccccgac cagtcgcgct aatcttggtg tatatcagcc aaagtgatat 8700
attttggttt catcgtatag ggtattaagg gggctgtatg tcacagcgca acttggaaag 8760
ggcacaggtt ttcgcggagc tagagcaaca tctcaccgag gcggaacgcg cgaagttcga 8820
agaatggaag caagagaacg ccccaggtga aggctcgacc tcgccggagg attggcctgg 8880

atggtgcgac gttgttatta gacacgccag tcgtgaacgt tcggtgagct tggagacagt 8940
ttcaaacgtg ttctattacg ctgcctgcgg cgttgccatg ttatgtatgg cattcgtttt 9000
tgtgtcaggg cttctaaccg tcctgggcgt gatcgatgat gggaacgaaa gcactgccgt 9060
tatcaatgcg aacctgaatg gcggcgcgca ggtcgttttt cttcttgtca tcgcgcacgt 9120
ccttatgaaa ctggtgggtc gaagtttcaa gcgccggacg aagttgctgc catgacccta 9180
ggcggatgac aaaaaaagcg cggcatatgc cgcgcttttg ctttcccatg gggttgacgc 9240
ttagcggtcg aattgccgcc tttgattctg atactggccg ccgtttgcgt cttcgtccgc 9300
gatccgcttc tggtactttg ccagagcggc gtcgtagtta gccgggtcta cccaaaaacc 9360
gccgcgatcc ggcgtgccaa cgtaccgggc cgcaacgcca ttggtttgat cgacactatc 9420
gttactcgcg taagccgagc aatgcgaagg catcgtgttg cggatagtcc ggtggccgtc 9480
atcgcgatag ctccagacca tttgttgcga gttcaaagca gcagcatcgc aacggcccgc 9540
accgttggcg attgcccctt tcaaatcgct catttcacct tgttcatcgg ggcacatgtc 9600
gaggaagttg cggcgggcct tgatggtgtc gcgcatcttc ttcttcgtga tgttgaagta 9660
tcgcgatagt gagggttggc attcgccggg ccgctgcccc gtggaaaggc acagaatcgc 9720
ttcgcaggcc agccgtgtgt cacctgagaa catatcttcg gcctgggcgg gtagagcgcc 9780
gaacatcgtc gcagcgatgg acgacgctat gagtagacgt ttcatagatt ccctatctcc 9840
tagcggttga acacttcgat atcaacgcgg cggttctgcg cacgtccggc cgcagaaacg 9900
ttgtccgcga cgtaatcgcc ggccgaaacg tagttcactg agattttcag cggcgataca 9960
ccttgcgcga cgagccagtt cttggctgcc agggcgcgat tcagggcgac acgttcgtcg 10020
gccgcacttg gcctgtcgcc gtcagtgcgg ccacgaaccc aaatttgccg aacgttaccg 10080
cccaggagcg gaagcagcgt agcgacctgg gtttgcgtcg gtcggaatgc gctgctgttg 10140
aatgggaaat agacgcttac cgtttgcgag gctggcaatg ccggcttcac aggcgctgcc 10200
agcaccacgg gctgcgtaga ctgattgcgc agcttgtctt gcgcgtctgc aagctcagca 10260
cgaagcgaaa gcacctcggc cgtgcgtgcg tcattgaccg ggtgccgatt cgacccgcta 10320
acggtcggag gtttcggcgg ggatgagcac gcggccaata agaccgcgca cgacaaaata 10380
gccaatttgt gcatgtgtga accccgtaat aggaaacatt ggtgagccaa tattatatat 10440
cttggcaagc ggaccgcaat caaagtcgcg ctgaaattgt cttggatggt gacgactgcc 10500
caacctggcc atcctttcgg cgtcaccagt tgcaacgagg atcggccttg ttggcgacaa 10560
ccaggcgcaa ccaggtccgg acttttgtcg ccaaaaaaaa gcgctccacg tcggaagcgc 10620
tgggagtggt gacagatccc gccggcgccg gcggggaata tgtcaccaaa cgagaggggg 10680
agggggaaag gatggtgaca tgccggcttg atcgagcggg ccggcttgtc accagaggcc 10740
gaatcatgga atcacgtaag tccttgtccc cgtgggcgaa tcagaaattc caagacaaat 10800
tcagcgtagt caagactgcg tgaatttacc gttcaagcgc gcgttcctgg ctagcgactt 10860
gaccttgccc gcgcgtgtac tcgaccttca ccattgcacc gacagccggc agtttgtcct 10920
tgaataagcc ggcatcgtgc ttgaccagct cgggcttgcc cttcgcattg gtctggagct 10980
gatacaccga cttcttgtcg atgtgcagca cattgccttc gtaactcttc accggggccg 11040
tcttgtccgg tttcgttgct ccggtcaggt catagacgcc cttgggcaac gagttgcggg 11100
agccgacgtg cctcgacacc cattcgccgt tctcctgggt gtcaacaagc cgtgactcgt 11160
tggaaatgac tagcagttgg tggccctctg ctttcaacgc ccgttgcccc aggaaaccct 11220
ctttattgac atcacgcata actaactctc cttaacgact gcgttcggaa ttacgttcaa 11280
caaccgggcg ggtccgctcg gcatcatgcg acttcgacgg cgctgccttg tcgtagatcg 11340
cgacggccgg caccttgccg gccttggcgc gctcttgaag ccgcgtatcc accgcttggc 11400
gcaacgcttc acgttgtgcc gggtctttca ccttcgcgtc gatcagagcg gccgcgacgg 11460

cctcgacaac cttgtgcttg tcggatttct ggacttccca ggtgttgcgg ttcgtctcga 11520
taggctcgct accgatgacc ttgcctttgt catcgcgctt caaagcgttc accacgacca 11580
tctgactgcc cttgtattca agtcgaacgt cgtcgcccgg cttcgccttg ctttcaccca 11640
tggccctttc aagatcgatg ccccaaacgg ttttctcgcc gtccttcgtg gcgagcttga 11700
cgtagtagtt cggcttttcg tctgggtcgt ggttgtagtt cgccgcgccg tgttcaagca 11760
ctcgacccgc atagacccgc agcgcggccg tagcggcctt ttctgccccg tcgccacctt 11820
tgccctggct gcgtgtgttc gtgctctcct gggcgcgttc tgtcgctttc gcggcttgtt 11880
ccggcgcgga aggggagcgg ccacggccag tagtccgttc ggccttttgc tggtcgcggg 11940
cagcatcatg ttcaacggtg ttgcgcatcg accgttcgcg gcgttcttcc agggcgcgca 12000
aatcctgttc ttgaggcttg tagccgcgca cctcgatacc gcgcaagctc gcttccatcc 12060
agacttcgcg cttgaagtcg ggatgaccat tgacggtgat cgtcttccag cctttcgcat 12120
cggccatcgt cgccatggac tttgctacac gctcatcgtt ggtggccgtc accatgcgct 12180
cgcctttgtc cttgaaggcg atcttgccgg gctggtcttt gaagtggaac ttcgcgccgg 12240
ccactcggta ctgctggtga agctggtcca tcagttccgc ttgacggttc ttttcgcctt 12300
cggtcacgac ttggctgtcg taatcagtgt caccgccagt tcgtgcggca aggtcgttag 12360
cgttgtcctg ttcctcggct ttctcggaca ggttctcaac gtcgatacgc ttaccctcgg 12420
cctggatgcc gaggcgttct tggtcagcgg ccaacagctc tcggtcacgc tggcgagcgc 12480
gcgcggcacg ctccagggct tcggctacat caacttgttc aagttgcggt tcggtcgcgt 12540
gctcaaccgt gttcgactcg cccggttcat ccggttcaat ctcaagcgac acttcggcag 12600
ccttacgggc ttcctttgca gccgttcggc gcgccgattc ttcggggctg attccgtact 12660
cgttttgcag gtcttcgcct ttgagcgacc ccttaacctc ggggttctcc aagatcgcct 12720
tggcgttctt gtcgcctact gcgtccgcca gggcgtccgc atcgaggcct ttcagtcgag 12780
cgacttgttt gttgcccata tgaaacgaca cggtgtaagc ggcttcgccg gctttttcgg 12840
tgcgctcgaa acgaagcgac gtggccggtt tgtcgtcaat catggtgatg ccgtaggccg 12900
gaccggacac ttgcttgtct tcgacgccgg gcactgcccg ttctctagct ggggccgcta 12960
cgccgagcga tttatcgcgt tcggctatcg ccatgttgtt ttgttcatag gtctgctgcg 13020
catcctcggg gccgaggaat tgcgacgcta tgtattcatc gaaccgttct ttgggcatca 13080
gcttcaatgc acgttctggc tcacctgcga tgtctcgatt gacgtaattc tcatagtggg 13140
ccttatcgac ttcctgaggc tcccccatgt agtcccaccc gttttcgggg tagtacagag 13200
acgacaagac ctgtttatgc gcgtcttggc cgatagcctc gatggccgta tctctgagct 13260
tcgcggttaa ttcggtttgc tggttgttct gttccattgc cgtagcccct tatcgttcca 13320
ttccgtgatc ggcttgcttc gcttgcgaag gggccggctt tgcctgttcc ttcgcgccct 13380
gttcttggcg ctgctggggc tggaacgctt gctgcgtcac gttgcccagg tgcttcaaga 13440
aagcctcgcg ctgcttgacg ttcttgatgt tctccatggc gtatttctcg gcctcgctca 13500
tgacctttgt tgcggtggaa acgttctgat ccgccgcctt ttccgtggac cgggccgcgc 13560
gttgctgctg ttcttttgca gcatcccacg gataggcttt ggcctgatcg ccccggtagt 13620
gaacctgaat gttccggttc tccaggtcgt tgttggcatc gcgacctttc agggacgcgc 13680
ccatcatttc cacgtcagtg cgactatgga cgacggccgt ttgttggttc ttgccgactg 13740
cctgcacgac aaaatctttg tcggcatgaa tgaccggacc tttgtaacta ccgttctcct 13800
ttgcggggaa cacttcggcc tcgacaccga gcttgccggc gacgctgttg cgtatttcgg 13860
gagggatatt gccggcatcc atgcgatacg cgcctacggt cggcgctttg gatttgtcgg 13920
ctttttgggg agtgttggca gagtcgccaa tggtgcgttc ttgagacatc gaaggttctc 13980
ctaagtgatg ggcattgccc gcattgcgac ggggccgaca tggccccgtg cgcaaaatat 14040

gtaaccttgg ttagccaaga ttatctgttt tggctcactg gcttgtcaag aacgctcaaa 14100
tccagtatgg gcgcgtcgtc ttcaagctcg ggcggcaggt ctggaacgtc ttccatttcg 14160
tccagggcgg cgagcatgtc cgcttcgtcg ggcaagtgac cgtcgtcagg aaaaacgtca 14220
tccatcatgg ccgccagttc gtcatcggtc ggctcatcgg cgacaagctc accttcgtca 14280
tgacgaagat tttcatcagc ctgcacggcg acttcctcag caactgcggc cacggcgacg 14340
acgggcgcaa cggcaggcgc tgccttcggt tttcgtttcg ccttgggctt tggttccggt 14400
tttggcgcgg ccggcgcagc ctggggcact tcctcggcgg ccgtgttcgc gttggtttcc 14460
gcgagcgctt ccgcttccgc ctcggcatcc agggcggcaa gttcgtcgtc acttggcagc 14520
gcgcccgagc cgtcatcatc gaccggagca tcgtcgtcgt attcgttcgg atcggcagca 14580
tcgagcgcgt cgaagaagtc gttcacgaaa ccttcaattt cgtcaggttc caggccatcc 14640
gatccggcga ctacggcaat tttcgaggta tcgacagcaa gcgcccggag gtcgatgcct 14700
ttgtctacat cggcaaccgt catttcacgc acacgcgatt gcacgacggc ttcgtgcagg 14760
tccaggttca aatcaggtac gggcgcggcc agctcacccg aaccccatgt gtcctcaagc 14820
tgctttttgg acggcagctt tttgcccagg gcggccaaag tcggcgaaac cgacttgagc 14880
cgatccatga aagcatggtc cgagaaatag gaaatcttgt cgcatttgat gggtttcatg 14940
ttctcaaggc tgatgatttg cttgcgctgg ctcatttcct tgagttcctg cggcatcatc 15000
agcgctcgtt tttgccctgc gccttcgcct tcggcctcgg agacgctgga cccactcttg 15060
ccgtggctac ggttgcgcga atgcaccgtc atgtagccga gcatttctga atactcgttg 15120
gcgtcgcgtt gttcctttgg cgtgtagagg atttgcagcg catggttcgt tacgaacgtg 15180
cgggcactct cccggccgta gcctttcggc gactccgctt cgagctggcc cggcgactgg 15240
atgatggtca gcaggcgcag accgtaaccg gccatgtagg agttcgcctt gtcgatgatg 15300
ccgatccggc caggggccgt gaattcgtca tccagcatca ggcaggtgaa tttcagctcc 15360
ggtgtcgagt gcagcagttg gtttgtgttg aggttgacca actgcgtgta gaacatgttc 15420
agcagaagct tggcctcaga gagcttgttc gccgggatgc ccaggtagat cgacatgcgc 15480
tttttgcgca cgtcgcgcag gtcgaagtcg ttcgcgctcg ttgctgcatc gacaattggg 15540
cttgcccaaa tggtcaacgg cacgtgtaac gtcgccatga tgctcgacag cgtgttgtcc 15600
gaggtggacg tgaagcggtt cagagcgtcc acgcattcca tggacagcat gggcaagcct 15660
tcgccgtccc acaccgtcaa tggaactagg cggcggtcgg attcaaattc cgcgccggct 15720
ccactcagca gcaattcgaa ttggtcgagt tcggtgttcc tgacgttctc cacgactgcc 15780
acgggcacgt catcgcacag ggcttcggcc tcggcttcgt cgcagcctcg aattgccatg 15840
acggcctgca cgacggcttc gcggtcgtcg cccaggtcgt tcaacatcag gtcgccgtac 15900
tcgcggtagt ttcgttcggt gatgaagcct tgaatgtgct cttttaccgg ccggcccttg 15960
ccggacgatt gccgcagcaa ttcgcccatg gtgcgcggca aggtcggggt ttcgcacagg 16020
tacagcgtca ggcccaggaa caggttgcga gctgcatcat cgaaaaacgc atcgcggcct 16080
tcgcctggat acagcgaata gccaatcgcc aggatgtccg ttacccgcaa gcggggatcg 16140
tcgctgacat agccaagggg gttgtagcga tgcgtcttgc cgtgtagcgg gttgccgtcc 16200
gcgtcctcgc tgatcgagaa cgggttgaac aggaagacct cttggcccca cttggcgcga 16260
tacttcgacg tgatgaggaa gttttctaac ttcacatcga gcacgacgac ggattccgcc 16320
caattgagca ggttcggaat cacgatcccg acacccttac cgcttcgcgt tggggcggcc 16380
agcaacacga attgcatgct ggcgtacatc aggtatcgac ggccccactt gccgacgatg 16440
atgccggttt tggcgaacag gcccgctttc gcaatctcgc cggcagtcgc gaaccgcgca 16500
tcaccatgga gcgaacgaac ctcccgcata gccgcgatga ttgcgacgag ggcacccccg 16560
taggcaacga cggccgcgac gatcaaggcg accttgaggc gtttcgcgac gaccggatcg 16620

gccgcgtaaa actgccagta ggtccaccac gtccagaagt ccgtcttccc catggggttc 16680
gtcttgctga aagcgaagaa caggaagccg gacagccata ttgccccggc cgtcgcggcg 16740
atgaacccga caatgccgag taaccatttg agccacttcg ggatttccat gcttaacgct 16800
ccttggtgac gaccgccgct aaccaggtgc ggctggctgt cgccacgtct atttgttgaa 16860
actggtgaca ccgcggcttg ctgcagcaag tccaggcgtc accaaatatg gcgacagatt 16920
cgagcgatcg agctgccttg tttgtcacca ccatggtctt accgtgatgt cgatcacgtc 16980
gaagacaacg ccaagccaca gggcggcaac agtcgcaaga ccgccgaagc cgagcatcat 17040
gaagaagaac tgagcggggc cgagactctt gcggttttgc tgggcatcca gctcgaaccc 17100
cgaaagcgca tcgggttcgg gttcggattc ggtgtaaggc ttgctaaccg cgaattccgc 17160
gtaggcttgt ttcacatctt tccagcgttc aaccccgcac aggacctcgc cgcgcgtgtc 17220
gtccagtccg cagtacgcga ggatggcggc gggcagttgc gagcgcgtct tgggagacat 17280
ttcgctgtaa tgcgtgcaat gaagtgctcg gaacgccgga tagatttctc gctccatcgg 17340
cgttttatct ttatcaggcc aaagcacagg gcggtcttga gggcctgtaa gagcgctgac 17400
cgctgcgtag gcgctatcga ggtcacagat ggaaaagaac gtcccggcca agagcttttc 17460
aaacgtggcc gctaaaacct cagcgcgaaa atccagcagt tcgtcgcttg gcatcgacgg 17520
atcgatgtgg gcaaacacag catccaaata ggcaatcgat gcgtcatcag gttgatgttt 17580
gaaatcgtcc ataaaggctt cctcacgcgc ccgctttggc gcgtttcatc gttggttcgt 17640
accaaagttc ggtaatgtga cgaccgagtt cagggtgata cacgtcgttg tggacgtgaa 17700
tcacgatgtc cacgacctga tagagcagcc gcttgatgac gttgtagggc agggtacgac 17760
cctgccggtt ctgcaacatc atcagagcca tgcgctcgaa agccatgtca cacgacccag 17820
catgcagact ggtgatgctt cccccgtgac cggaggcgca gacgttgacg aaatcgaacg 17880
tctcgccgcc gcgcagctca gccaggagaa tgcgcgtcgg tttcatccgc aggcagcttt 17940
tcagcaacga tgcagccgtt accggtgcgt tttcttcttc cttggcttcg ctcgggtaga 18000
acaggtgaac gtggttcggg tgatccggca agaacaactc gggcacgtct tcaatcgtga 18060
tgatgcgctg tgaggtcgga atcgcctgca tcaacgcctt catgagcgtg gtcttgcccg 18120
agccagtttc gccggccacg acaatgactt tttcgagctg gaccgcccga cgcaggaagc 18180
cgacgtaatc gccggcttgc ttgagggcta gcagttcatg ttcgtccggc gacaactcgc 18240
cattcattgg ccgaatgtgc ttgaagaagc cttgctcttc atagtcggtc agcgtgcgac 18300
gggagaagga cggctttcgg atcgtgaccg aaatcgtccc gtgttctgcc gctggtggca 18360
tcacgatttg cacacgctca ccagcaggca gcacggccga aaggatcggc cgggaatcgg 18420
tgatgtcttg attggcgaac ttcgcggtcg cggttgccag ggattggcag tgatcgaacg 18480
tcaaagcgtc aaccttgtgg gtttcccaca cgctttgccg ttcgcaccag atttcacccg 18540
gccggttcac gcacacttcg gtgatagccg gatcggccat ccatatgttg accacttcca 18600
gatggaagtt aaccgaagtc gctcgctgta ccgcaccgtt gccagcgaga tattcgttcg 18660
aattattgag ctgcgacatc gtacacaccc gaaaagtcca gatcgcgggc gacgtagata 18720
ccgacttgct cgccctgatt cttgtagagg gttggcggaa tgttgagagt gttgcgcaac 18780
acttccgccg ccatgttctg cgaagcttcg ccggtcgagt tgaaattgaa ctggttgcca 18840
tcgccgccgc tgctgctttg actggtaacg cctcgcgcca cgtcatcgac cagggacagc 18900
atgagcgcgc cgccgaaacg ctcccaaaag tgcgtatcga cgtagccagg aaggccagcg 18960
ccgcccaggg gatcggtgcc cggcgagtcg agattgacca cgacgccgtt cggcgtcttg 19020
atgcggttcc acagcacaaa gatgcgggcc atgccctggc gcatgttcga tttgtattcg 19080
cccgtgacgg tcgaacccct ttcgaccagc agcaccttgc cgttgtcgct gtagatgttc 19140
cgcgtcacga cgcacgcggt catgcctgga acagtcgtgt ccaacttcgt ttgcaggccg 19200

caatcaatca tggtgccctt ggtcagcagg aaattccggt tgcccaggtt ggtcgccttg 19260
cgggttgctg tttgggtcga actcagaagc gccgacatac cgccttcgcc gtcctggccg 19320
gagttgcctt gctgtctcgc gccgccatca gcgccggccg gcgcggtttt tgccgagctg 19380
ctgccaacca tcagcgacga cccggattta tccaagacag gtgccgattg atgttgagca 19440
gcttcgccgg gaagcgccgg ggcagtagcc gtttgttgct ggcccgtcac agccggaacg 19500
acagttgtcg ttggggtctg ttcgggcaat ggcggtgcgg ccttcacagc cggctccgtg 19560
aaggtgcggg gcggtacggc ggttgtcagt ttcagctttt tttccgcttc ggccgccgcg 19620
ctggcgtcgg cccggtgcgt ccacttccaa taaccgacgc cgcccgccac ggccagaacg 19680
gccaggatcg ccagcagagc taaggcccgc tttttcgtgg aagggccgtc ctgattcacg 19740
ctcggcatgc cgcgctcgcc atcgagcgct tccagttccg agggcgcgtt gttggcttga 19800
ttgctcattt ctctacctcg cccgatttga tgacacgttg aacgcctgga acggtcgttc 19860
ccttgttcgg cggcaagccg tcagggtcga aactctcgtt gtagacaccg acgatggagt 19920
tacccaggcg cagggcgaat tcacgaacga cccgatgaat caccagaacg tccgggttgt 19980
tcgggtcgat atggctgttc ggaatgctct ctgtaccgtc tgctgctacg aggaaagccg 20040
ttgggaaatc ccggttgttc gggaagcgca ggtaggtaaa gcgcccatcg tcgtaagcca 20100
tggttggcgc aatcccttcg gagttgtcgc cgatttccat ggagtaatgc cagttcatag 20160
gagctggctg cacatcgagc ttggcttgcg cggcccgctt gcgggcttcc tcgcgttcct 20220
tcgcccgagc ctcgaccgga tacaggtact ggacccggta cgcgacacgc tgattgatgg 20280
ccggcttgcc gtcgttgccg ccacccggca tgagcttcaa gtcgatgtcg tacatgtact 20340
tgttggtcgt caccatcaag ttcgtgtccc aatcgccagc cttgggcggc atgacgacat 20400
cgacaccctc ggcattctgc accttgatgg atttgggctt catgtagacg atgttgcgcc 20460
ggtcaaggaa ttcccatccc tggctaaagc cggatgcgat atccaaaacg tgttcgtcct 20520
cgccgaagac aacgcgggca cccaggccgg gcagtgcccg gaccaaaacc acgtcgccgg 20580
ggttgtagtt cacgtactgg atgcgattgt cataacgcga cgaggtaggc acatcgaccg 20640
cgtaggcggc cgagctggca aagcccagga ccagcgacag agcgatgacg gcgtgtttca 20700
ttgtccaccc ccgaccattt cagggtcttg gcgatagctc agcacctgga acccgaacgg 20760
gttcaccaga cgctgcgatt ccgagataag cgacgggttg cggtattcgt atgcaacggt 20820
cgcaacccac ttcgtgacgg tgcctggacc gtcatcatcg gtgcgtttgg tggtcttgat 20880
gaatcgcacg gtgccaacgc cgcgaccatt gggaacgacg ctcacaatct caaccgtggc 20940
ccgtaccgat ttgccaaact tcttgtcgag ggcttcgggg ccttcgaaca gagcggcgta 21000
gtccttgcca acgtttggcg aggccagcag ccccacggtg ttgtagtcct tctggagcgt 21060
gtaccagtcg tatgtttcac gggcgcggac aaattgggcc agccaatact tgtccatcac 21120
ttcctcatag ccgacgccct tgctgttgag ggcggtcacg atgtcaggaa cgcccgtggt 21180
gttatccacg cgaatgacgt agggaatggt ttccttgagc ggcatcatca agacgatagc 21240
gagccaagac agcaccgcta cccctacggc tccataggcc acgtaccacg cccgttgctc 21300
tgacttttcg atagcttggg tgcgcgaggc ttcccaattg ttttcttgct cgaacgggtc 21360
tttggcctgg acggcattcg gcggggtcac ttcgtcaccc gccgcctttt tgcgtctgaa 21420
catacgttac tcccctttga aatcaatgac gggtgaaggc gtcgccgctt tgatcggcgt 21480
cggagcttgc ttgttgattg gcacgcgcgg tttcccttcc agttggggaa gcacggacgg 21540
ccgattggtc gcgcatccag cgagagcggt cgcgagcatg agaaggacta aagcgcgttt 21600
catagtgttg gtcctacagt tagtggtgga cgtgccagat tatacacgcg ccacgaccaa 21660
attaccctat tttggtaatc gtgagcgcaa aaaaagccgc cctgtccccc gaaggaaaca 21720
gagcggcgag agagttttat cgcttgactt ggccgccttg cgcgctgccc cagttcttgc 21780

ccatgttctg catgacgtgt tgccggtagg ccgggttcca catcgtgtta cctgcaacca 21840
tatggttcga ccggcgcgcc gtggtcatca tgccggactg catgtcgcgg cgggtggaca 21900
tcgggtcaac ggcattgcgt gcggcgctaa gccccttcat cgcgcctgtg accggcgaaa 21960
tcaggtgccg aatgcccatg gcggccatcg acaccccgcc ggccagcccg gaggctatgc 22020
cgcccgcctg gaggatgatc caaatcagca cccccgtaag agcgccgatc tgcaaggccg 22080
caaacatcgg gttatcgtca ccgctacccg agaagtccgc gccctggatg aaggtatcgt 22140
atgccttcat ggcaaacgtc atgaccacgg ccatgacgac gatggttagc gtgtagttca 22200
tcacctgggc gaaccagcta tcgaagaact tcgcagtcat cgggaacatc aggcagagga 22260
tgaacagcgg ccccatggcg aagacaatcg caagcgagaa tttcgccacg atgaccacag 22320
cgccgccgag catcgagacg agcacggtgc ccacggcaac aaccaagcca gcaatgaccc 22380
agccgagcgc cgcgccgatg ctccaaccag cctcatcggc tttctggaag cacgtcccca 22440
caagctccat gcccttgccc agcgactggt caagaacctg atagatcgag gacgcctgag 22500
tgccgccaac gttgagcgcg tccgcaaggc cggtttcgag tccgtttaat gatcccatca 22560
ccgcgtccgt gtacccgtcg gctgtcagag caaaggcggc aatgatgatg atcttcaagc 22620
actgcttgag gaacgtgtaa aacggagcct caaccgcgcc cgtgctgatc gcgtagccgg 22680
tcagggtgat gtacagcgtc acccccgtca gggcgagcat ttgaagccca aacatcagct 22740
tcgaggcggc gggcgacaca aacgctgtgg tcgcgttttg caccgtctcg ccaatgaact 22800
gaaagaccat cgggtccata cgtcaccgtc ctatttcttt ttgagttcgt cggctgtgag 22860
cggcgcaaca ggcttgatgc caccgccacg cgcgccccag gttgtggcgc tgtcggcacg 22920
ggcggcattg acgcagttcg gcgtcgccat gcgctcgccg gggttctcgt tgcacttggc 22980
gagcatggcc ttacgctcgg ccgcgtgttc cttgtaccaa tccgtggtct gtacgggtgc 23040
gtcgtcttcc ttgcagccgg acaggacaac cagaacagca gcagcaaggc ccatcgcgag 23100
ggtgcgtttc ataagtgaat ctcctatcag ttggtgacgc ggccgggttg gccaggcgca 23160
gcggctgtca ccatgccggt aacgaatacg tcaccttggg ccggtgtgtt gaagcgctcg 23220
cgcaatctgt ccgccacggc ggccgcaaag tggtttttga aaccgaaacg ggagccgaca 23280
atgatcatca gccccacgaa aataatggcc gccgtagcaa tcgtggtcat caacaaccgc 23340
acgaacgctt gcacgtcggc cgtgctggcc gtttgcagtg cgtgcagcgt ctcggtgcag 23400
ctctccggcg tcacaattgc agcaccaaca aacagccaga acaggaacag aggcaccgcg 23460
accagaagct tgccggcaaa agtcagggcg acatgcgcag tattcgccag ttgccccagg 23520
aacggccggg catcttgaaa aacttcaagc tgcacagcgg cgtattccga atcgctcaag 23580
gtgccaatag gtacgttgtt gagccacact ttccaggtcg tgggcttatt ctcagtaatg 23640
cgacgcttgt acgtctcagc gtgccgggcg gccaggagct gcacccccag gacaaacagc 23700
ccgctcaacg cggccgacag aacaagactc agcaccatgg aaagcgcgcc gcgttcgctg 23760
tagtcggttg tcagaaggtt gaacgtcacc gaggccacgg ccgcgatgcc aaagggtaca 23820
gcgctctttt tcagtgttgc tttcatcgcc ggttccccgg tcgaatggtg acaagcgacg 23880
ccggcaggat ctctgccggc gtcaccagtt gcgttactgc ccgtcgaaag tgaccgggcg 23940
tacctcgata ccgccgcgcg cggaccatgt gcgggcctga atctcacgct gacgttgagc 24000
ctgcaccttg tcttcggcct gggcgaccat cgaataaagt tgcagcttcg tcgcttcgtt 24060
ctggattttg gcctgctcga tggcgatccg gccttgcagc tccgcgatgg ccttcgggtc 24120
ttgcgtcgtg ttgatgcgag ccatcaggcc gtcaatctgc gagagccggc ttttcgccgc 24180
gtcgtaggca tccagggcaa ccgccttatc ctgggaaggc ttcacggccc gcgcttcgca 24240
tgcacgctgc gcatcacggg tcttgatgta ggtgcagcta tcgaacacct tgttgtcggc 24300
ataaacctgc ttgccgcgac cggtaaggcc ggcgtaaccg ccgctcttca cggagtcgta 24360

gacctgctgc cagtcttgcg gcaggtaatc ccgcagagcc gggttgtcca tgatgttacc 24420
cataccacgc gatccggtga tcgactggta ttgctgcttc atctggtcaa tttgcgaagt 24480
catttgctcg acttgctgtg cccaacgcgc cgtattggcc gcgaactcgg ccatggtcat 24540
gggcgagtcg ctggcctggg ttgtcaccgt tacgggaatt tgcgcattgg cggcccccac 24600
aaatgccgct gcaactaacg cgccgacgag aatccttttc atagtcacct cacgcttgct 24660
tggaagacgc cacgcgcgcc ttacggcgtt cgtggaacac agggagccac tgccgggggt 24720
cgtctccaac ctcggcaatg acagcatcaa gcagttcgtt attgtccgaa ctgccggata 24780
ggatagccag ttcatcatcg aaaccggcca gatcgagccg cgcaatcatg gattggtggc 24840
cctgtttcac caaaaacatc cggctttctt cgctcatgct cttgatgatt tcgaactccg 24900
cgtcggtaac tttaaagccg tccgtgtatt cgtggtaatc ggctttcggg ttcggcaggt 24960
agatttcagt cgccacctgt tgcaccaggg acgccgcgat tttcgatttc aacaggctgc 25020
taggcatttg cgtcgcgaag acgcccaagc cattttgctt acggatggtc aattgcttgt 25080
tgccggcgaa ctcggcaaag gcttcgtcat ccacccactt ccacgcctca tccatccagt 25140
agatgaagcg ccggccgtca atgaccgatt ccatgcgatg gagcaggtac atcgaaatcg 25200
gcgtgcgcac gtcgctgttg tcgaggaagt cggttccatc gaacccgtag ttgttgtggg 25260
tcgtgaagtc gatttgatct tccgggcaat ccagcaccca ccagaatgcg ccgcgtttgc 25320
cgttgccgtc gtcgtagcac caacgcgaca gccgcttgga cacgctgttt tcgcggtcct 25380
cgcggtcggt gccctcggtg atgttctgaa tgaccgtaga gagccggcgc agagggcgcg 25440
gcatgcgcat caccgtccgg acggcgtggc taatgcgcag ttcgtcggtg gttgtgacgc 25500
gctggtcttc gcccgagacg agcacacgca ccaaccgctc aaggaacaga atattgtcct 25560
cggtcggctc catctggaac gggttgaagc cggtcggctt accatttttg atcgaaaggt 25620
acttgccgcc aataacaaga atggtcgctt tcgcgccttc gtccttgtcc atatagacgt 25680
tgcagaaccc ggacggggaa ttgtgtttgt acttctgcga ttggcaaacc gcgaagttca 25740
tgaacacagt tttacccgca ccggactgcc cgatgatccg ggtgttgccg agcagcttct 25800
tgtcgtaatt gtcttcgtcg cccttcgagt agtggaaatt caggtagagc ggctggcccg 25860
atggggtttt gagcagtgtc acggcctgcc cccaagggtt gccgtcccgc ttgcctgccc 25920
ggaaattgtg gaagctcgac aggcccgcga aattcttgct ggtcaggccg gccacacggg 25980
gccgatagga ccagttgcac ggcaactgag cgtaaaacgc cgcatcggtc gcagtcgcca 26040
ccattgccgc caggaagccg cgatcctgaa tgatcgtcat cgccgaagtg gtgttgcggc 26100
gcagtttttc cacagattcg ccgaagatca tcagcgaata gtggtactcg cccatggcga 26160
attcgccctg gatgagctgg tcgatggcga gccgcatttc ttcaatctgc gtggcgctac 26220
catcttccgc gttcgacagt tgcttcattt ggcgctccaa gaactctttg ccttggcgct 26280
tggacatgaa gctataggac tgcgtaatga cgtactcgta atcctcgtac atcaagccgt 26340
tgagaatgcc cggttcggtg tgcgcggtgt agtccttgaa gtcgatgcac tgcgcaaagc 26400
gggtcgtctt cggcgagcga atttcaatcg tctcggtccc tacgaacacc caggcggtgc 26460
ccaggtattc attgattggc ccggacggga cgcgcacccg ttgccactcg ccgctgacga 26520
ggaagttcag gaactccagt tgctgggagt acatcacccc ttcgtcatcg gtgtacgtcg 26580
tcagttcttc aaggccgcgc ctttcatcga cgccatagcg ccgcatgctg gactcgattt 26640
gataggcgat ttcatccagc ttgcggatgg cggccttctg gtcttgcttg atttcagcca 26700
acgaccgacg cgacgacttg accagggcac ggtcgatgcg cgacgggttc ggccggtaca 26760
cgaccgtcaa atacagctca ttcgccatca ttcgataacc ggcgaaggag tcgtagtatt 26820
tcttgtccag gtcccggcaa aaatcgttgt cgtacaccga tttgagccgg tcactggtct 26880
tgcgccgcac gttgtgcgtc cagagggcga cgtggttcgt gccgatggac cgaaacagcg 26940

tattcagttg ctctttgcgc aacagaattt cgtccgcgtc ggtcgtctcg aaggcgatcc 27000
ccgccacctt ccagatgcgc aggtaatcgc cgtccttcgt gacgatggtg ttcggcgcta 27060
catggctgct gtagggcaga aagggcgcta ccgtcctttc tcgattcagt tgttggaaat 27120
acttcggttg agcgttcatg cgcgcctcac ttccgtttct tgaaagggat cgggctgtaa 27180
gccgaagcct tccagaaccg tccgttgtgg ttgtagttca ccccccggaa cagcaatttc 27240
aggcccaaga gcctgaattg ctgatcgtcc gactttgcga taagccgcat cgtcaggacg 27300
attgggaaca gcgccagggc aaggaaaacg gtcgtccaga ccgctatcag gatgaccacg 27360
ccagaaacga cggcgagcgg caccaaaggc acgccgaaca ccatcgccgg ccgcgtgcac 27420
cccttaaaga gcgggtcgcg cgggtcttgc atgtccattg gtgccgacat cgctgctacc 27480
ccttatccca ggaggaagcg ggcaagctcg gccgcgccac cgatgagcaa gccaccggcc 27540
aggattttgg cgcactcggc gatgtccgca tgcttgaaca ggaacttgta accggcccac 27600
atgatggcga tggtcacaac ggtgatagac acgccgcgca ggatcgacag cacgttgtcc 27660
atgaaggtgt tcaccttgtc caagccacct tgcgcgaacg caggttcagc ggcgaacaga 27720
gcgacgaaag ccagcagagc cagcagcgga acggcgctct ttgcgccctt gttgatggtc 27780
agtgattcca taggtagttc gactccaaag tagatagaaa acgcaagtgc gggttgatcg 27840
gcccgcgccg tgaaaacgat tagtggtcag ttcatccgat gctctcccct gccgctttct 27900
aaccggatta tagcgcagtg aacaccaaaa tatatcattt tggtgttctt cttttacatc 27960
ccatggtaat gcgctatcgc ttcccggtgg acttcccggc cggctttttg gccgccttcg 28020
gaacgggttt cccgttcttg tcatccatca cgtttttgca gccggtcgtg tacccgctgc 28080
acccccacca gtagcccgtg ccttgcgtgc tcttgcgccg atacatcgct ttgccgcagg 28140
acgggcaagg tgctgtcttg atgacgacgg gcttgccctt ttcgtcatca aggaaggtct 28200
tgcattcgtc attggtgcag aaccagccga agccaccctt gggcttgccg gtttccttgt 28260
ccttcttctg gtagcgccgc aggtcggatt tgcacttggg gcagttatga atcgtcgtga 28320
gtggcttgcc gtccacgtcg tcacagaagg tcttgcattc atcgctcggg caggcccaca 28380
ggtagccgcc gctctttcgc ttgaaacggc gcaaggatga atcgcactta gggcatggat 28440
acgatttccg ctccacgggc ctgcccttcg catcgtccat gaaatgcttg cactcatcgt 28500
tcttgcaaca ccagaacggc ccacgagtgg cgtggttgaa gcgacgcaag ccggatgcgc 28560
atttcgggca ggtgaaccga ggtttgaact cgccgcccag ggcaacgtct accagctcct 28620
tgttcagttg ctcgtatgca ggagccacaa catcaaagaa cgtcgttttg ccctctgcaa 28680
tctggtcgag ctggccttca aggtcgcggg taaaatccag ttccataaag gcgaaaccgc 28740
ctttgacaag gttgtctacg atcagctcgc caagttcagt tggctcaagg aagcgtttca 28800
cctcggccag atacccgcgc gtcatgatat tgctcatgat cgccgggtac gtcgctggcc 28860
ggccaatccc ttcggcttcc agcttcgcaa tgagcgatgc tttcgtgtag cgcttaggcg 28920
gcatggtctg cttcttgagc agcttgccag tgtcagcgcg cttatcagat ccgacctcaa 28980
gcatcggcac cttgccggcg tccttgcctt cggcttcgtc cttgtcgtct aaatcggcgt 29040
cttcgactgc atcttttgca gtcagcacac gccacccggc atcaatgagc acgcggcctt 29100
tggccttgaa ctcgaaggct tgcgggccat cggtcgattc cagggtgaca gcgttgacct 29160
tgtatcgcgc gtcagcgagt tgtgacgcta ccgagcgttg ccatatcagc cgatacagcg 29220
cccgctgttt gtcgtcttcg ccggcttctt cgacctcgat atgggtcggc cgaatggctt 29280
cgtgcgcctc ctgggcaccg cccttcgact tgaaccggcg cggtgcgtcc ggcagcttcc 29340
agcccttgcc ttcggcaaag gcacggattt cgctgatagc gtcgtcgccg aaattcacgc 29400
tgtcggtgcg gatgtacgtg atcgcgcctt gctcaaagag cttctgcgcg agtttggccg 29460
taacctcggg atcgagcttc aacgcgacac ttgccgcttg cagcagcaaa gacgtggaga 29520

acggcgacgg cggcgcttcg cgggcggtgt ccgtagtgga gtcgatgacg cggaactgac 29580
gcgagttagc ggcacggttc gccagttctt cgtccatcac gtaaggcact tgttccgacg 29640
tgaaaggctt cgtgttccac tcggccttcc aagcgtcgcc gtcgaaaaac acgaccgcgc 29700
cgaagtggtt cgtcttcgag aaccgcttaa tgcgacgctc aaggtctacc acgacacgca 29760
ccgcgacact ttggacgcgg cccgctgaaa gattcatgcc gagcatgtca gacagcagcg 29820
gcgacaccat gtagccaacc atgcgatcca gggcgcggcg cgcttcctgg gcctgcacaa 29880
gatcgtcatc gattttgcgc ggcttcgcca gggcagcccg gataaccttg ccggtcactt 29940
cgtcgaaggt gacacgcaga taatcgtctt cacccaaaga gagcgattct tttaggtgcc 30000
atgcgatggc ttccccttcg cgatccgggt cggtcgcgag gtagaccatc tctgcgtttc 30060
gaacttcttt gaggatgcga gtgactcggg cctcaccgcc tggaaacgtt cggtccccga 30120
ccttggtctc aggcagaaat tcgtaaacca tcgtgaagtt tggcttctcg atacctaaac 30180
cagttttggg aaggtcgcgg atgtggccga cacttgccat taccttccag ccttccccaa 30240
gctccgcctc gatctttttg atcttgttcg ggctttccac caccatcaat ttcataaatc 30300
gtcctcagaa gacgacgacg gagctttttg cttcgccgtc agtcccggcc ttttcgctgg 30360
tcggggccgg ttcgttttgt ttggtgccgg actgatcccc cgcaggggtt tcggtcggga 30420
ccacccgttg taggcgcacc gcttcgcgtt ccgacgaggt tcgcagcacg accggcgaat 30480
ggtcggccgg ctcggccgct acaggtgccg gggtcttcgg cacggatgcg ctatccgcag 30540
ccttgacggc tttcggacgc accgcgccgc cctcccctgg cttgattgaa ggaacgacag 30600
ggatcgcctt cggctggacg ttcgcttgcg cgagcacctt ttgcacatag ctcggttcgc 30660
cggccttgtc aggccggaaa cctcgggtga aattgccgga gtagtagcag gagatagcgg 30720
cctgcaaagc cgcttgttcg ttggcggtgc gcggggtcgc acgcacgaaa caatcttcaa 30780
gaatcttcga gcctacgcgg aggttcgcgc aggcgtcgaa ggcttgttcg taggtgagct 30840
tgtacttggg gaggttgtag cggttgactt gcgcaacccc tacagagaag ttccaaccgt 30900
ctgcctcaag cgcctttgcg gtcgctatgg cttcgtcacg gttggatggt tgccgggcta 30960
gccggccacc aacgacgccg atagcgtagg ggttatagct tgattcgaca tgaacaaccg 31020
ctgccatcgt ctgcggtgcg accgtgggcg cgcattgctc cgcaagggcc aaaaattcca 31080
gcatggttct gtctccgatt catcagtgga cagataccaa tataccatac tttggtcatt 31140
gaacattgcc ttttttggtt gcggatgttc gggtctgagc ctttggcttg gggtgtttgg 31200
ggcgggcttt cgtcttgggt gtaccgggca cgtcttggcc tcttattagt tcgaaacggt 31260
cagtcggata ggcttccttc aagtggtcgt tgaggcgctt taagtccttg aaaagcttcg 31320
gcactgtccg gtcgcgtcgt gttgtcaagt accactcttt gccgggttca gagagcattt 31380
gagcgatgat gtagaaccat tccggtagac ccgtgccctt gtgtttctca agaacgtggg 31440
atttgtccga aaattcggcg gtaacgtaga agcctatggg cgtctccatg actctgagct 31500
tctcaattat ttgatgcttc gccgcaagct ctagagtcga cctgcaggca tgcaagcttg 31560
gctgttttgg cggatgagag aagattttca gcctgataca gattaaatca gaacgcagaa 31620
gcggtctgat aaaacagaat ttgcctggcg gcagtagcgc ggtggtccca cctgacccca 31680
tgccgaactc agaagtgaaa cgccgtagcg ccgatggtag tgtggggtct ccccatgcga 31740
gagtagggaa ctgccaggca tcaaataaaa cgaaaggctc agtcgaaaga ctgggccttt 31800
cgttttatct gttgtttgtc ggtgaacgct ctcctgagta ggacaaatcc gccgggagcg 31860
gatttgaacg ttgcgaagca acggcccgga gggtggcggg caggacgccc gccataaact 31920
gccaggcatc aaattaagca gaaggccatc ctgacggatg gcctttttgc gtttctacaa 31980
actctttttg tttatttttc taaatacatt caaatatgta tccgctcatg ttaattcatg 32040
aactagtcat gtattcggct gcaactttgt catgcttgac actttatcac tgataaacat 32100

aatatgtcca ccaacttatc agtgataaag aatccgcgcg ttcaatcgga ccagcggagg 32160
ctggtccgga ggccagacgt gaaacccaac atacccctga tcgtaattct gagcactgtc 32220
gcgctcgacg ctgtcggcat cggcctgatt atgccggtgc tgccgggcct cctgcgcgat 32280
ctggttcact cgaacgacgt caccgcccac tatggcattc tgctggcgct gtatgcgttg 32340
gtgcaatttg cctgcgcacc tgtgctgggc gcgctgtcgg atcgtttcgg gcggcggcca 32400
atcttgctcg tctcgctggc cggcgccact gtcgactacg ccatcatggc gacagcgcct 32460
ttcctttggg ttctctatat cgggcggatc gtggccggca tcaccggggc gactggggcg 32520
gtagccggcg cttatattgc cgatatcact gatggcgatg agcgcgcgcg gcacttcggc 32580
ttcatgagcg cctgtttcgg gttcgggatg gtcgcgggac ctgtgctcgg tgggctgatg 32640
ggcggtttct ccccccacgc tccgttcttc gccgcggcag ccttgaacgg cctcaatttc 32700
ctgacgggct gtttcctttt gccggagtcg cacaaaggcg aacgccggcc gttacgccgg 32760
gaggctctca acccgctcgc ttcgttccgg tgggcccggg gcatgaccgt cgtcgccgcc 32820
ctgatggcgg tcttcttcat catgcaactt gtcggacagg tgccggccgc gctttgggtc 32880
attttcggcg aggatcgctt tcactgggac gcgaccacga tcggcatttc gcttgccgca 32940
tttggcattc tgcattcact cgcccaggca atgatcaccg gccctgtagc cgcccggctc 33000
ggcgaaaggc gggcactcat gctcggaatg attgccgacg gcacaggcta catcctgctt 33060
gccttcgcga cacggggatg gatggcgttc ccgatcatgg tcctgcttgc ttcgggtggc 33120
atcggaatgc cggcgctgca agcaatgttg tccaggcagg tggatgagga acgccagggg 33180
cagctgcaag gctcactggc ggcgctcacc agcctgacct cgatcgtcgg acccctcctc 33240
ttcacggcga tctatgcggc ttctataaca acgtggaacg ggtgggcatg gattgcaggc 33300
gctgccctct acttgctctg cctgccggcg ctgcgtcgcg ggctttggag cggcgcaggg 33360
caacgagccg atcgctgatc gtggaaacga taggcctatg cctcgaactg cgatgagtgg 33420
cagggcgggg cgtaattttt ttaaggcagt tattggtgcc cttaaacgcc tggtgctacg 33480
cctgaataag tgataataag cggatgaatg gcagaaattc gaaactaatc ggggtggcgg 33540
ttcagtgaaa aatattaaag gatcttcttg agatcctttt tttctgcgcg taatctgctg 33600
cctgtaaacg aaaaaacccg ccttgtggcg ggtccttccg tccgatcaga agtcttcgac 33660
ctcttctaat cggcggtggt aactggcttg gaggagcaca gtcaccaaaa ctgtcttttc 33720
agtttagcct taactggttc ataacttcaa gagcaactcc tctaaatcag ttaccaatgg 33780
ctgctgccag tggcgttaag tcgtgtcttt tccgggttgg ccccaagaga acagttaccg 33840
gagaaggtgc agcagtcggg ctgaacgggg ggttcctgca tacagcccgg cccggagcga 33900
actgcctaag tcgccccgag ataccagaag acgtgagcta tgagaaagcg ccacgtgtcc 33960
cgcaagggag aaaggcggaa aaggtatccg gcaatcgata tggcgggaac gtgggggcgc 34020
aggagggagc gattcaccgg aaacggtgat gatctttaag ccacgtcggg tttcgccacc 34080
gctggcggat ttcatggttg agtcggaaga ctcccacaga tgcaccgaaa aagtgtctgt 34140
tttgtgaagt ccgtcagggg ggcggagcct atggaaaaag cgccagcggc gcggcctttt 34200
gtcctggctt tgcctttttg ctcacatgtt ctttccggcc ttatcccctg attctgtgga 34260
taactgtgtt accgtttttg tgtgagtccg taccgctcgc cgcagccgaa cgaccgaggg 34320
tagcgagtca gtgagcgagg aagcggaaga gcactggaag ctgcattttc tccttacgca 34380
tctatgcggc atttcacacc cggcatggtg cacttttcat acaatgcgcc ctgatgccga 34440
ataagtaagc cagtatcctc gaggcgaacg ccagcaagac gtagcccagc gcgtcggcca 34500
gcttgcaatt cgcgctaact tacattaatt gcgttgcgct cactgcccgc tttccagtcg 34560
ggaaacctgt cgtgccagct gcattaatga atcggccaac gcgcggggag aggcggtttg 34620
cgtattgggc gccagggtgg tttttctttt caccagtgag acgggcaaca gctgattgcc 34680

cttcaccgcc tggccctgag agagttgcag caagcggtcc acgctggttt gccccagcag 34740

gcgaaaatcc tgtttgctgg tggttaacgg cgggatataa catgagctgt cttcggtatc 34800

gtcgtatccc actaccgaga tatccgcacc aacgcgcagc ccggactcgg taatggcgcg 34860

cattgcgccc agcgccatct gatcgttggc aaccagcatc gcagtgggaa cgatgccctc 34920

attcagcatt tgcatggttt gttgaaaacc ggacatggca ctccagtcgc cttcccgttc 34980

cgctatcggc tgaatttgat tgcgagtgag atatttatgc cagccagcca gacgcagacg 35040

cgccgagaca gaacttaatg ggcccgctaa cagcgcgatt tgctggtgac ccaatgcgac 35100

cagatgctcc acgcccagtc gcgtaccgtc ttcatgggag aaaataatac tgttgatggg 35160

tgtctggtca gagacatcaa gaaataacgc cggaacatta gtgcaggcag cttccacagc 35220

aatggcatcc tggtcatcca gcggatagtt aatgatcagc ccactgacgc gttgcgcgag 35280

aagattgtgc accgccgctt tacaggcttc gacgccgctt cgttctacca tcgacaccac 35340

cacgctggca cccagttgat cggcgcgaga tttaatcgcc gcgacaattt gcgacggcgc 35400

gtgcagggcc agactggagg tggcaacgcc aatcagcaac gactgtttgc ccgccagttg 35460

ttgtgccacg cggttgggaa tgtaattcag ctccgccatc gccgcttcca ctttttcccg 35520

cgttttcgca gaaacgtggc tggcctggtt caccacgcgg gaaacggtct gataagagac 35580

accggcatac tctgcgacat cgtataacgt tactggtttc acattcacca ccctgaattg 35640

actctcttcc gggcgctatc atgccatacc gcgaaaggtt ttgcaccatt cgatggtgtc 35700

aacgtaaatg ccgcttcgcc ttcgcgcgcg aattgcaagc tgatccgggc ttatcgactg 35760

cacggtgcac caatgcttct ggcgtcaggc agccatcgga agctgtggta tggctgtgca 35820

ggtcgtaaat cactgcataa ttcgtgtcgc tcaaggcgca ctcccgttct ggataatgtt 35880

ttttgcgccg acatcataac ggttctggca aatattctga aatgagctgt tgacaattaa 35940

tcatcggctc gtataatgtg tggaattgtg agcggataac aatttcacac aggaaaca 35998

<210> 14
<211> 4913
<212> DNA
<213> Artificial

<220>
<223> partial sequence

<400> 14
atctacgtct gtcgagaagt ttctgatcga aaagttcgac agcgtctccg acctgatgca 60

gctctcgcag ggcgaagaat ctcgtgcttt cagcttcgat gtaggagggc gtggatatgt 120

cctgcgggta aatagctgcg ccgatggttt ctacaaagat cgttatgttg atcggcactt 180

tgcatcggcc gcgctcccga ttccggaagt gcttgacatt ggggaattta tgcggtgtga 240

aataccgcac agatgcgtaa ggagaaaata ccgcatcagg cgccattcgc cattcaggct 300

gcgcaactgt tgggaagggc gatcggtgcg ggcctcttcg ctattacgcc agctggcgaa 360

agggggatgt gctgcaaggc gattaagttg ggtaacgcca gggttttccc agtcacgacg 420

ttgtaaaacg acggccagtg ccaagcttgg gctgcaggtc gactctagag gatccgcggc 480

cgcgcgcgat atcgaattcg taatcatgtc atagctgttt cctgtgtgaa attgttatcc 540

gctcacaatt ccacacaaca tacgagccgg aagcataaag tgtaaagcct ggggtgccta 600

atgagtgagc taactcacat taattgcgtt gcgctcactg cccgctttcc agtcgggaaa 660

cctgtcgtgc cagctgcatt aatgaatcgg ccaacgcgcg gggagaggcg gtttgcgtat 720

tgggcgctct tccgcttcct cgctcactga ctcgctgcgc tcggtcgttc ggctgcggcg 780

agcggtatca gctcactcaa aggcggtaat acggttatcc acagaatcag gggataacgc 840

aggaaagaac atgtgagcaa aaggccagca aaaggccagg aaccgtaaaa aggccgcgtt 900

gctggcgttt ttccataggc tccgcccccc tgacgagcat cacaaaaatc gacgctcaag 960

tcagaggtgg cgaaacccga caggactata aagataccag gcgtttcccc ctggaagctc 1020
cctcgtgcgc tctcctgttc cgaccctgcc gcttaccgga tacctgtccg cctttctccc 1080
ttcgggaagc gtggcgcttt ctcatagctc acgctgtagg tatctcagtt cggtgtaggt 1140
cgttcgctcc aagctgggct gtgtgcacga accccccgtt cagcccgacc gctgcgcctt 1200
atccggtaac tatcgtcttg agtccaaccc ggtaagacac gacttatcgc cactggcagc 1260
agccactggt aacaggatta gcagagcgag gtatgtaggc ggtgctacag agttcttgaa 1320
gtggtggcct aactacggct acactagaag aacagtattt ggtatctgcg ctctgctgaa 1380
gccagttacc ttcggaaaaa gagttggtag ctcttgatcc ggcaaacaaa ccaccgctgg 1440
tagcggtggt ttttttgttt gcaagcagca gattacgcgc agaaaaaaag gatctcaaga 1500
agatcctttg atcttttcta cggggtctga cgctcagtgg aacgaaaact cacgttaagg 1560
gattttggtc atgagattat caaaaaggat cttcacctag atccttttgg ttcatgtgca 1620
gctccatcag caaaagggga tgataagttt atcaccaccg actatttgca acagtgccgt 1680
tgatcgtgct atgatcgact gatgtcatca gcggtggagt gcaatgtcgt gcaatacgaa 1740
tggcgaaaag ccgagctcat cggtcagctt ctcaaccttg gggttacccc cggcggtgtg 1800
ctgctggtcc acagctcctt ccgtagcgtc cggcccctcg aagatgggcc acttggactg 1860
atcgaggccc tgcgtgctgc gctgggtccg ggagggacgc tcgtcatgcc ctcgtggtca 1920
ggtctggacg acgagccgtt cgatcctgcc acgtcgcccg ttacaccgga ccttggagtt 1980
gtctctgaca cattctggcg cctgccaaat gtaaagcgca gcgcccatcc atttgccttt 2040
gcggcagcgg ggccacaggc agagcagatc atctctgatc cattgcccct gccacctcac 2100
tcgcctgcaa gcccggtcgc ccgtgtccat gaactcgatg ggcaggtact tctcctcggc 2160
gtgggacacg atgccaacac gacgctgcat cttgccgagt tgatggcaaa ggttccctat 2220
ggggtgccga gacactgcac cattcttcag gatggcaagt tggtacgcgt cgattatctc 2280
gagaatgacc actgctgtga gcgctttgcc ttggcggaca ggtggctcaa ggagaagagc 2340
cttcagaagg aaggtccagt cggtcatgcc tttgctcggt tgatccgctc ccgcgacatt 2400
gtggcgacag ccctgggtca actgggccga gatccgttga tcttcctgca tccgccagag 2460
gcgggatgcg aagaatgcga tgccgctcgc cagtcgattg gctgagctca tgagcggaga 2520
acgagatgac gttggagggg caaggtcgcg ctgattgctg gggcaacacg tggagcggat 2580
cggggattgt ctttcttcag ctcgctgatg atatgctgac gctcaatgcc gtttggcctc 2640
cgactaacga aaatcccgca tttggacggc tgatccgatt ggcacggcgg acggcgaatg 2700
gcggagcaga cgctcgtccg ggggcaatga gatatgaaaa agcctgaact caccgcgacg 2760
tatcgggccc tggccagcta gctagagtcg acctgcaggc atgcaagctc tagcgattcc 2820
agacgtcccg aaggcgtggc gcggcttccc cgtgccggag caatcgccct gggtgggtta 2880
cacgacgccc ctctatggcc cgtactgacg gacacaccga agccccggcg gcaaccctca 2940
gcggatgccc cggggcttca cgttttccca ggtcagaagc ggttttcggg agtagtgccc 3000
caactggggt aacctttgag ttctctcagt tgggggcgta gggtcgccga catgacacaa 3060
ggggttgtga ccggggtgga cacgtacgcg ggtgcttacg accgtcagtc gcgcgagcgc 3120
gagaattcga gcgcagcaag cccagcgaca cagcgtagcg ccaacgaaga caaggcggcc 3180
gaccttcagc gcgaagtcga gcgcgacggg ggccggttca ggttcgtcgg gcatttcagc 3240
gaagcgccgg gcacgtcggc gttcgggacg gcggagcgcc cggagttcga acgcatcctg 3300
aacgaatgcc gcgccgggcg gctcaacatg atcattgtct atgacgtgtc gcgcttctcg 3360
cgcctgaagg tcatggacgc gattccgatt gtctcggaat tgctcgccct gggcgtgacg 3420
attgtttcca ctcaggaagg cgtcttccgg cagggaaacg tcatggacct gattcacctg 3480
attatgcggc tcgacgcgtc gcacaaagaa tcttcgctga agtcggcgaa gattctcgac 3540

acgaagaacc ttcagcgcga attgggcggg tacgtcggcg ggaaggcgcc ttacggcttc 3600
gagcttgttt cggagacgaa ggagatcacg cgcaacggcc gaatggtcaa tgtcgtcatc 3660
aacaagcttg cgcactcgac cactcccctt accggaccct tcgagttcga gcccgacgta 3720
atccggtggt ggtggcgtga gatcaagacg cacaaacacc ttcccttcaa gccgggcagt 3780
caagccgcca ttcacccggg cagcatcacg gggctttgta agcgcatgga cgctgacgcc 3840
gtgccgaccc ggggcgagac gattgggaag aagaccgctt caagcgcctg ggacccggca 3900
accgttatgc gaatccttcg ggacccgcgt attgcgggct tcgccgctga ggtgatctac 3960
aagaagaagc cggacggcac gccgaccacg aagattgagg gttaccgcat tcagcgcgac 4020
ccgatcacgc tccggccggt cgagcttgat tgcggaccga tcatcgagcc cgctgagtgg 4080
tatgagcttc aggcgtggtt ggacggcagg gggcgcggca aggggctttc ccgggggcaa 4140
gccattctgt ccgccatgga caagctgtac tgcgagtgtg gcgccgtcat gacttcgaag 4200
cgcggggaag aatcgatcaa ggactcttac cgctgccgtc gccggaaggt ggtcgacccg 4260
tccgcacctg gcagcacgaa aggcacgtgc aacgtcagca tggcggcact cgacaagttc 4320
gttgcggaac gcatcttcaa caagatcagg cacgccgaag gcgacgaaga gacgttggcg 4380
cttctgtggg aagccgcccg acgcttcggc aagctcactg aggcgcctga gaagagcggc 4440
gaacgggcga accttgttgc ggagcgcgcc gacgccctga acgcccttga agagctgtac 4500
gaagaccgcg cggcaggcgc gtacgacgga cccgttggca ggaagcactt ccggaagcaa 4560
caggcagcgc tgacgctccg gcagcaaggg gcggaagagc ggcttgccga acttgaagcc 4620
gccgaagccc cgaagcttcc ccttgaccaa tggttccccg aagacgccga cgctgacccg 4680
accggcccta agtcgtggtg ggggcgcgcg tcagtagacg acaagcgcgt gttcgtcggg 4740
ctcttcgtag acaagatcgt tgtcacgaag tcgactacgg gcagggggca gggaacgccc 4800
atcgagaagc gcgcttcgat cacgtgggcg aagccgccga ccgacgacga cgaagacgac 4860
gcccaggacg gcacggaaga cgtagcggcg tagcgagaca cccgggaagc ctg 4913

<210> 15
<211> 53381
<212> DNA
<213> Artificial

<220>
<223> partial sequence

<400> 15
ttcacccccg aacacgagca cggcacccgc gaccactatg ccaagaatgc ccaaggtaaa 60
aattgccggc cccgccatga agtccgtgaa tgccccgacg gccgaagtga agggcaggcc 120
gccacccagg ccgccgccct cactgcccgg cacctggtcg ctgaatgtcg atgccagcac 180
ctgcggcacg tcaatgcttc cgggcgtcgc gctcgggctg atcgcccatc ccgttactgc 240
cccgatcccg gcaatggcaa ggactgccag cgccgcgatg aggaagcggg tgccccgctt 300
cttcatcttc gcgcctcggg cctcgaggcc gcctacctgg gcgaaaacat cggtgtttgt 360
ggcattcata cggactcctg ttgggccagc tcgcgcacgg gctggcgggt cagcttggct 420
tgaagatcgc cacgcattgc ggcgatctgc ttctcggcat ccttgcgctt ctgcacgcct 480
tcctgctgga tgcgaataac gtcctcgacg gtcttgatga gcgtcgtctg aacctgcttg 540
agcgtgtcca cgtcgatcac caggcgttgg ttctccttcg ccgtctcgac ggacgtgcga 600
tgcagcaggg ccgcattgcg cttcatcagg tcgttggtgg tgtcgtcgat ggccgtggcc 660
agttcgacgg cgttcttctg ctcgttgagg ctcaaggcca gcatgaattg ccgcttccac 720
gccggcacgg tgatttcgcg gatggtgtgg aatttatcga ccagcatctg gttgttggcc 780
tggatcatgc ggatggtcgg caggctctgc atggccgaat gttgcaaggc gatcaggtcg 840
ccgatgcgct tgtccaggtt ggcaaccatc gcatcgaggt cggccagctc ctgcacgcgg 900

cccgggtcgt tcccgacatt gccgcgcaga ccctcggcct gctcgcgcag ctcggcaagg 960
cggaccttgc cggccgcgat gtggacgcca agaaggcggt gttcctcgcg cacggctgcg 1020
aacatttcgt cgagcgaggc attgcgctgc gcgatgcctt gctgggtggt ctgcacttcg 1080
ctgaccaggt gttcgatctg ctcgcgggtc gtgtcgaagc gcgccatgaa gcccgtcgaa 1140
cggacgcgga agcggtcgat cagcgggcca atcaggggca ggcgggaacg gttgtcggac 1200
aaagggccga cgttcaggga acgggccttg gcgacaacct gggtcagttt ctcgcctgct 1260
tcgtccaggt cgctgttgcg cacctggtcc agcaggctat cggcgtagcg ggacgtgtgc 1320
tcggccacgt cgcggccgaa ctcggcaacg gtctgcggac tgccgacctc gatccgctgc 1380
gcgaccgcat ggacttccgg cacgtcgctt tcctgcaagc ccagctcgcg cagggttgcc 1440
ggggtcatgt cgaaggcgac gataggggcc ttggcgtcgt gcgtcgtttt cagtgcgttc 1500
atagggttct cccgccgtgt tattggttga tgccttccag gctctgcgaa aggctccgca 1560
tgagcgcctg gtgagctttg gccgcctcgg cgaccattgc cggattcatg ttcttggtgg 1620
tgatgagcgc gagggtgtgc tgacgccaga cgggcaccag gacggatgcc gtttcagaga 1680
agcggtccag catgtccacg gcctgcgccc gcgtgagctt catctgagtg acgctcattt 1740
catgggacgc catgagggtt gccaggttgg cgagcttgcg cgcgaagcgt tcgcgcggct 1800
tgtcgaactc gatcacgccg gccttggccg cgccggcctc ggggttctcg tccaggaact 1860
cgcgcccggc ttgaatgtag gctctgagcc ggtctacctc ggcctcatgc gtattgagca 1920
tgtcatccaa ggcgcgcaac gtgtcccgca cgcgctgcgc tacgccctcg gcttcgtcca 1980
gcaactggtc gagcgtcttg cgggcgacct gatacctcac ctggcgttca acctcacggc 2040
caagcatctt ctcgaaccag gtaggctttt ccgcgatctt gcgggggtcc gcgtcggcca 2100
gcttcgccac gatctggctg attttgtcgg ccagcgcggc aactgcgccg tgctccatca 2160
gattcgacag ctcgttgagg gaatccgccc cgtcgatgcc ggccccgtac tcgccaatcg 2220
tcgccggcga cgcgaagagg gcgggcaaaa cctccccctt caatcgcgcc atgttcacgc 2280
tttgttcttc cattcgatac accctcgcgg tgggttaatt gcttttcgat ggaagaagtt 2340
tagctaaact ttctatccct cgtcaacacc tttagccgct aaaatttggg gacaggtcat 2400
ttacagaaag ccagctcact cctggcgttg ccccttgagc gccgctaggc gcgcagcatc 2460
cttcgcgctg agaaagaacg tcatcagcgg cccgaccgtc ttgcttgaac cgtcggcaaa 2520
gcaaacatcc atcgaacagc cttgcgtgtg ggggtccacg ccttcgacca gtttccaagg 2580
gtccatgccc cagccctcgg cctccggatt gaaccagtac gccgatgcgt cgccgtttag 2640
gtcgctgtcg gcgtagtcct tgaccagcac ggccacccgc ccggtcgtcg ggcacacgta 2700
gcccggctgc ttaggttcct gtcttggcat tgctcaaagc tccttgaagg ggccgctcta 2760
cagccccttg ggcttgtaga gcgacacgaa ataggtgagt gcggtcagta ccgcgaaatg 2820
caccaggaac gtccagccgg catgaacgcc aagggtgttc cagtggtaca gcatccgcag 2880
gaactgaaag aaaacgtcga tagagatgat ccacttcgcc accggccaca ccaggacagt 2940
aacgacccat acaaagcgga ccagggcctg gacacccttg gcaaaagtga accggggcgg 3000
ggccttgctc ggggcctcaa cgcgcggggc aggggcctcg gcctccactt ccacgcctgg 3060
gaacttgata atcttcgaca ttgcttgacc ctccacggcg atgcgtgttc aattcgtcca 3120
gcgctcgcgc gcctagaccg tgatgtgaca gcatcgaggt caagcgcccc ggagaaatcc 3180
ggggcgtcat ccctatgccc cgtccaactc gggaaccggc ttttccctgg tgctcaacct 3240
ggccggctcg acccacttgg tgacttgctg ccactcgtta ccactgcgaa cggctacccg 3300
aatctgcaca cgtttagccg cgatcttcgt cactatgcca gcaacacaaa cggaataccc 3360
gtacccgccg cgcggggtgt gctgccagtt caccctatct cctacttgca tcatcatccc 3420
ctggcgtcag tgaccggccc ggaatttcgc caagtcgatt tcgttgaagg tccagcgctg 3480

tttcgcccgt tcctccaacc tcgacgactc ccgcatgacc tcgatgcgca ggcgctcgac 3540
ctggtgcatc agctcatcag cgcgccgctg cttctcggca atagcattcc gctgcgcgac 3600
cagctcctgg tctacgttcg gcagctcgtc gatccacggc atgaacttat cggtcatcgg 3660
attggcctcc ggtaattgac ctgggaatct acccggcctc aaaacaagaa tagggcataa 3720
tgccctaact tgtcaagcaa ttttagctaa acaattgagg ggattcagcg aggcgtcatg 3780
cttgaaaaca cctttcccct ggcgtgcaat cagcttgtcc cggcggcagc gcactgccgc 3840
agcgcggcca gcaaggtgcc ttgctcgatc cgctgtcgtt cctccggcgt aatgcaatcg 3900
ccgcagatac cgcccagctc ttcgcgcagg gcgtccgccc cgatcaggct gcggctaagg 3960
gtcgaaatgg acttaccgca gcggcggcaa ttggtggtga caatctcgat cttgcggttg 4020
ggcatggccc tatctccttg agagaggccc gaccgtagcc gggcctcgtt ccgttaccag 4080
cctgcgcgag ccttcgcgcg ttcgacttcc tgggtggacc agtggccctt tgcttcaccc 4140
tccagggtgc aaccttgcag gtacgccttg tgccgcagct cttcggcctg gcgggtaagc 4200
tctgccgcct gctccatcag cgccgcgatc tggtcacgac gggccagaaa atccgtggcg 4260
gccgcgccgg gcagctcatc gagccaagac atgatcttgc ttttcatcgg ggttgatcct 4320
ccggttgctg acctgggcga agtgcccggc cttggattgc tatattaggg cattatgccc 4380
tagatagtca aggaaattta gctaaacaat ttgcggcggg cgcacgaaaa aacccggctt 4440
gcgggccggg ctgctggcag catatcgcaa cgatcagggt tggcggtttt agccgctaaa 4500
gtcctctccc ttggcgtaaa gtcctgcggg cgtcagccct ggcctttcca gatcgcccca 4560
atccccgcta gatcgcaaag gatcgcccag gcggcatagg ggatcggcga atcctcgcca 4620
acccaacgcc gcaccgtgcg gtcgcccttg gcacccaagc ccaagatgcg cgcagcctgt 4680
ccgccggtga ggccggccaa gtgcaagact tcccggattt ctgcgccggt cggctgcacc 4740
cagcgttccg ccgggcgcag gcactcaagc cggatattca cgtcgctcat gctgcttttc 4800
tcctaatcgt tatcaaatgg cggcccgcaa ttggtcgtag ccgtagcacg actcgatgca 4860
acgcgggtca tattcacaaa cctttctacc gttgcgattg atgcgatagc gcggcggctt 4920
gtcgtcgtgc gcccacacct gtcgcgtttc gggactgatc ttcgtaatga tgacgtgctt 4980
cccgatttcc ttggcaaaaa ccgggtgatg cacgctcaca atctcgcacc gcaggccagg 5040
aaagaacgtc gccggccacg ccggcgcgtc ctcgtccttg gccggctccg gctccggctt 5100
ggccggtgta accggctccc tgcgctgccc ggccggctcc tgagctttcg cacgcgcggc 5160
cgcgagcttc gccttggcgc tggccgccca tttgcgggcg atgaatgcct ggtgcgccgg 5220
cagcacggca tcaacccagg cgaagcccgg cggcagttcg tccggcttcc gccagtcctc 5280
gaccacggcc cgcacgcggc gcggcggtgg cgtgataccc gcgagccacg ccggcagtgg 5340
ctcgtcggca aggcggtcct ggtcgcgggc gtccagatac cggcgatgcg agcgcaacag 5400
ggcgcgcaaa accccgtccg ctacctggtc caccgtcatg ccgccgcgcg catggtcgta 5460
atgggagacg taattgagca tttccaggcg cttgcgcctg gtcaacgaaa gagtcagcgc 5520
cgtaggcgct gccatttttg gggtgaggcc gttcgcggcc gaggggcgca gcccctgggg 5580
ggatgggagg cccgcgttag cgggccggga gggttcgaga agggggggca ccccccttcg 5640
gcgtgcgcgg tcacgcgcca gggcgcagcc ctggttaaaa acaaggttta taaatattgg 5700
tttaaaagca ggttaaaaga caggttagcg gtggccgaaa aacgggcgga aacccttgca 5760
aatgctggat tttctgcctg tggacagccc ctcaaatgtc aataggtgcg cccctcatct 5820
gtcatcactc tgcccctcaa gtgtcaagga tcgcgcccct catctgtcag tagtcgcgcc 5880
cctcaagtgt caataccgca gggcacttat ccccaggctt gtccacatca tctgtgggaa 5940
actcgcgtaa aatcaggcgt tttcgccgat ttgcgaggct ggccagctcc acgtcgccgg 6000
ccgaaatcga gcctgcccct catctgtcaa cgccgcgccg ggtgagtcgg cccctcaagt 6060

gtcaacgtcc gcccctcatc tgtcagtgag ggccaagttt tccgcgtggt atccacaacg 6120
ccggcggccg cggtgtctcg cacacggctt cgacggcgtt tctggcgcgt ttgcagggcc 6180
atagacggcc gccagcccag cggcgagggc aaccagcccg gtgagcgtcg gaaaggcgct 6240
ggaagccccg tagcgacgcg gagaggggcg agacaagcca agggcgcagg ctcgatgcgc 6300
agcacgacat agccggttct cgcaaggacg agaatttccc tgcggtgccc ctcaagtgtc 6360
aatgaaagtt tccaacgcga gccattcgcg agagccttga gtccacgcta gatctatctc 6420
atctgcgcaa ggcagaacgt gaagacggcc gccctggacc tcgcccgcga gcgccaggcg 6480
cacgaggccg gcgcgcggac ccgcgccacg gcccacgagc ggacgccgca gcaggagcgc 6540
cagaaggccg ccagagaggc cgagcgcggc cgtgaggctt ggacgctagg gcagggcatg 6600
aaaaagcccg tagcgggctg ctacgggcgt ctgacgcggt ggaaaggggg aggggatgtt 6660
gtctacatgg ctctgctgta gtgagtgggt tgcgctccgg cagcggtcct gatcaatcgt 6720
caccctttct cggtccttca acgttcctga caacgagcct ccttttcgcc aatccatcga 6780
caatcaccgc gagtccctgc tcgaacgctg cgtccggacc ggcttcgtcg aaggcgtcta 6840
tcgcggcccg caacagcggc gagagcggag cctgttcaac ggtgccgccg cgctcgccgg 6900
catcgctgtc gccggcctgc tcctcaagca cggccccaac agtgaagtag ctgattgtca 6960
tcagcgcatt gacggcgtcc ccggccgaaa aacccgcctc gcagaggaag cgaagctgcg 7020
cgtcggccgt ttccatctgc ggtgcgcccg gtcgcgtgcc ggcatggatg cgcgcgccat 7080
cgcggtaggc gagcagcgcc tgcctgaagc tgcgggcatt cccgatcaga aatgagcgcc 7140
agtcgtcgtc ggctctcggc accgaatgcg tatgattctc cgccagcatg gcttcggcca 7200
gtgcgtcgag cagcgcccgc ttgttcctga agtgccagta aagcgccggc tgctgaaccc 7260
ccaaccgttc cgccagtttg cgtgtcgtca gaccgtctac gccgacctcg ttcaacaggt 7320
ccagggcggc acggatcact gtattcggct gcaactttgt catgcttgac actttatcac 7380
tgataaacat aatatgtcca ccaacttatc agtgataaag aatccgcgcg ttcaatcgga 7440
ccagcggagg ctggtccgga ggccagacgt gaaacccaac atacccctga tcgtaattct 7500
gagcactgtc gcgctcgacg ctgtcggcat cggcctgatt atgccggtgc tgccgggcct 7560
cctgcgcgat ctggttcact cgaacgacgt caccgcccac tatggcattc tgctggcgct 7620
gtatgcgttg gtgcaatttg cctgcgcacc tgtgctgggc gcgctgtcgg atcgtttcgg 7680
gcggcggcca atcttgctcg tctcgctggc cggcgccact gtcgactacg ccatcatggc 7740
gacagcgcct ttcctttggg ttctctatat cgggcggatc gtggccggca tcaccggggc 7800
gactggggcg gtagccggcg cttatattgc cgatatcact gatggcgatg agcgcgcgcg 7860
gcacttcggc ttcatgagcg cctgtttcgg gttcgggatg gtcgcgggac ctgtgctcgg 7920
tgggctgatg ggcggtttct ccccccacgc tccgttcttc gccgcggcag ccttgaacgg 7980
cctcaatttc ctgacgggct gtttcctttt gccggagtcg cacaaaggcg aacgccggcc 8040
gttacgccgg gaggctctca acccgctcgc ttcgttccgg tgggcccggg gcatgaccgt 8100
cgtcgccgcc ctgatggcgg tcttcttcat catgcaactt gtcggacagg tgccggccgc 8160
gctttgggtc attttcggcg aggatcgctt tcactgggac gcgaccacga tcggcatttc 8220
gcttgccgca tttggcattc tgcattcact cgcccaggca atgatcaccg gccctgtagc 8280
cgcccggctc ggcgaaaggc gggcactcat gctcggaatg attgccgacg gcacaggcta 8340
catcctgctt gccttcgcga cacggggatg gatggcgttc ccgatcatgg tcctgcttgc 8400
ttcgggtggc atcggaatgc cggcgctgca agcaatgttg tccaggcagg tggatgagga 8460
acgtcagggg cagctgcaag gctcactggc ggcgctcacc agcctgacct cgatcgtcgg 8520
acccctcctc ttcacggcga tctatgcggc ttctataaca acgtggaacg ggtgggcatg 8580
gattgcaggc gctgccctct acttgctctg cctgccggcg ctgcgtcgcg ggctttggag 8640

cggcgcaggg caacgagccg atcgctgatc gtggaaacga taggcctatg ccatgcgggt 8700
caaggcgact tccggcaagc tatacgcgcc ctaggagtgc ggttggaacg ttggcccagc 8760
cagatactcc cgatcacgag caggacgccg atgatttgaa gcgcactcag cgtctgatcc 8820
aagaacaacc atcctagcaa cacggcggtc cccgggctga gaaagcccag taaggaaaca 8880
actgtaggtt cgagtcgcga gatcccccgg aaccaaagga agtaggttaa acccgctccg 8940
atcaggccga gccacgccag gccgagaaca ttggttcctt gtaggcatcg ggattggcgg 9000
atcaaacact aaagctactg gaacgagcag aagtcctccg gccgccagtt gccaggccgt 9060
aaaggtgagc agaggcacgg gaggttgcca cttgcgggtc agcacggttc cgaacgccat 9120
ggaaaccgcc cccgccaggc ccgctgcgac gccgacagga tctagcgctg cgtttggtgt 9180
caacaccaac agcgccacgc ccgcagttcc gcaaatagcc cccaggaccg ccatcaatcg 9240
tatcgggcta cctagcagag cggcagagat gaacacgacc atcagcggct gcacagcgcc 9300
taccgtcgcc gcgacccgcc cggcaggcgg tagaccgaaa taaacaacaa gctccagaat 9360
agcgaaatat taagtgcgcc gaggatgaag atgcgcatcc accagattcc cgttggaatc 9420
tgtcggacga tcatcacgag caataaaccc gccggcaacg cccgcagcag cataccggcg 9480
acccctcggc ctcgctgttc gggctccacg aaaacgccgg acagatgcgc cttgtgagcg 9540
tccttggggc cgtcctcctg tttgaagacc gacagcccaa tgatctcgcc gtcgatgtag 9600
gcgccgaatg ccacggcatc tcgcaaccgt tcagcgaacg cctccatggg ctttttctcc 9660
tcgtgctcgt aaacggaccc gaacatctct ggagctttct tcagggccga caatcggatc 9720
tcgcggaaat cctgcacgtc ggccgctcca agccgtcgaa tctgagcctt aatcacaatt 9780
gtcaatttta atcctctgtt tatcggcagt tcgtagagcg cgccgtgcgc ccgagcgata 9840
ctgagcgaag caagtgcgtc gagcagtgcc cgcttgttcc tgaaatgcca gtaaagcgct 9900
ggctgctgaa cccccagccg gaactgaccc cacaaggccc tagcgtttgc aatgcaccag 9960
gtcatcattg acccaggcgt gttccaccag gccgctgcct cgcaactctt cgcaggcttc 10020
gccgacctgc tcgcgccact tcttcacgcg ggtggaatcc gatccgcaca tgaggcggaa 10080
ggtttccagc ttgagcgggt acggctcccg gtgcgagctg aaatagtcga acatccgtcg 10140
ggccgtcggc gacagcttgc ggtacttctc ccatatgaat ttcgtgtagt ggtcgccagc 10200
aaacagcacg acgatttcct cgtcgatcag gacctggcaa cgggacgttt tcttgccacg 10260
gtccaggacg cggaagcggt gcagcagcga caccgattcc aggtgcccaa cgcggtcgga 10320
cgtgaagccc atcgccgtcg cctgtaggcg cgacaggcat tcctcggcct tcgtgtaata 10380
ccggccattg atcgaccagc ccaggtcctg gcaaagctcg tagaacgtga aggtgatcgg 10440
ctcgccgata ggggtgcgct tcgcgtactc caacacctgc tgccacacca gttcgtcatc 10500
gtcggcccgc agctcgacgc cggtgtaggt gatcttcacg tccttgttga cgtggaaaat 10560
gaccttgttt tgcagcgcct cgcgcgggat tttcttgttg cgcgtggtga acagggcaga 10620
gcgggccgtg tcgtttggca tcgctcgcat cgtgtccggc cacggcgcaa tatcgaacaa 10680
ggaaagctgc atttccttga tctgctgctt cgtgtgtttc agcaacgcgg cctgcttggc 10740
ctcgctgacc tgttttgcca ggtcctcgcc ggcggttttt cgcttcttgg tcgtcatagt 10800
tcctcgcgtg tcgatggtca tcgacttcgc caaacctgcc gcctcctgtt cgagacgacg 10860
cgaacgctcc acggcggccg atggcgcggg cagggcaggg ggagccagtt gcacgctgtc 10920
gcgctcgatc ttggccgtag cttgctggac catcgagccg acggactgga aggtttcgcg 10980
gggcgcacgc atgacggtgc ggcttgcgat ggtttcggca tcctcggcgg aaaaccccgc 11040
gtcgatcagt tcttgcctgt atgccttccg gtcaaacgtc cgattcattc accctccttg 11100
cgggattgcc ccgactcacg ccggggcaat gtgcccttat tcctgatttg acccgcctgg 11160
tgccttggtg tccagataat ccaccttatc ggcaatgaag tcggtcccgt agaccgtctg 11220

gccgtccttc tcgtacttgg tattccgaat cttgccctgc acgaatacca gcgacccctt 11280
gcccaaatac ttgccgtggg cctcggcctg agagccaaaa cacttgatgc ggaagaagtc 11340
ggtgcgctcc tgcttgtcgc cggcatcgtt gcgccactct tcattaaccg ctatatcgaa 11400
aattgcttgc ggcttgttag aattgccatg acgtacctcg gtgtcacggg taagattacc 11460
gataaactgg aactgattat ggctcatatc gaaagtctcc ttgagaaagg agactctagt 11520
ttagctaaac attggttccg ctgtcaagaa ctttagcggc taaaattttg cgggccgcga 11580
ccaaaggtgc gaggggcggc ttccgctgtg tacaaccaga tatttttcac caacatcctt 11640
cgtctgctcg atgagcgggg catgacgaaa catgagctgt cggagagggc aggggtttca 11700
atttcgtttt tatcagactt aaccaacggt aaggccaacc cctcgttgaa ggtgatggag 11760
gccattgccg acgccctgga aactccccta cctcttctcc tggagtccac cgaccttgac 11820
cgcgaggcac tcgcggagat tgcgggtcat cctttcaaga gcagcgtgcc gcccggatac 11880
gaacgcatca gtgtggtttt gccgtcacat aaggcgttta tcgtaaagaa atggggcgac 11940
gacacccgaa aaaagctgcg tggaaggctc tgacgccaag ggttagggct tgcacttcct 12000
tctttagccg ctaaaacggc cccttctctg cgggccgtcg gctcgcgcat catatcgaca 12060
tcctcaacgg aagccgtgcc gcgaatggca tcgggcgggt gcgctttgac agttgttttc 12120
tatcagaacc cctacgtcgt gcggttcgat tagctgtttg tcttgcaggc taaacacttt 12180
cggtatatcg tttgcctgtg cgataatgtt gctaatgatt tgttgcgtag gggttactga 12240
aaagtgagcg ggaaagaaga gtttcagacc atcaaggagc gggccaagcg caagctggaa 12300
cgcgacatgg gtgcggacct gttggccgcg ctcaacgacc cgaaaaccgt tgaagtcatg 12360
ctcaacgcgg acggcaaggt gtggcacgaa cgccttggcg agccgatgcg gtacatctgc 12420
gacatgcggc ccagccagtc gcaggcgatt atagaaacgg tggccggatt ccacggcaaa 12480
gaggtcacgc ggcattcgcc catcctggaa ggcgagttcc ccttggatgg cagccgcttt 12540
gccggccaat tgccgccggt cgtggccgcg ccaacctttg cgatccgcaa gcgcgcggtc 12600
gccatcttca cgctggaaca gtacgtcgag gcgggcatca tgacccgcga gcaatacgag 12660
gtcattaaaa gcgccgtcgc ggcgcatcga aacatcctcg tcattggcgg tactggctcg 12720
ggcaagacca cgctcgtcaa cgcgatcatc aatgaaatgg tcgccttcaa cccgtctgag 12780
cgcgtcgtca tcatcgagga caccggcgaa atccagtgcg ccgcagagaa cgccgtccaa 12840
taccacacca gcatcgacgt ctcgatgacg ctgctgctca agacaacgct gcgtatgcgc 12900
cccgaccgca tcctggtcgg tgaggtacgt ggccccgaag cccttgatct gttgatggcc 12960
tggaacaccg ggcatgaagg aggtgccgcc accctgcacg caaacaaccc caaagcgggc 13020
ctgagccggc tcgccatgct tatcagcatg cacccggatt caccgaaacc cattgagccg 13080
ctgattggcg aggcggttca tgtggtcgtc catatcgcca ggacccctag cggccgtcga 13140
gtgcaagaaa ttctcgaagt tcttggttac gagaacggcc agtacatcac caaaaccctg 13200
taaggagtat ttccaatgac aacggctgtt ccgttccgtc tgaccatgaa tcgcggcatt 13260
ttgttctacc ttgccgtgtt cttcgttctc gctctcgcgt tatccgcgca tccggcgatg 13320
gcctcggaag gcaccggcgg cagcttgcca tatgagagct ggctgacgaa cctgcgcaac 13380
tccgtaaccg gcccggtggc cttcgcgctg tccatcatcg gcatcgtcgt cgccggcggc 13440
gtgctgatct tcggcggcga actcaacgcc ttcttccgaa ccctgatctt cctggttctg 13500
gtgatggcgc tgctggtcgg cgcgcagaac gtgatgagca ccttcttcgg tcgtggtgcc 13560
gaaatcgcgg ccctcggcaa cggggcgctg caccaggtgc aagtcgcggc ggcggatgcc 13620
gtgcgtgcgg tagcggctgg acggctcgcc taatcatggc tctgcgcacg atccccatcc 13680
gtcgcgcagg caaccgagaa aacctgttca tgggtggtga tcgtgaactg gtgatgttct 13740
cgggcctgat ggcgtttgcg ctgattttca gcgcccaaga gctgcgggcc accgtggtcg 13800

gtctgatcct gtggttcggg gcgctctatg cgttccgaat catggcgaag gccgatccga 13860
agatgcggtt cgtgtacctg cgtcaccgcc ggtacaagcc gtattacccg gcccgctcga 13920
ccccgttccg cgagaacacc aatagccaag ggaagcaata ccgatgatcc aagcaattgc 13980
gattgcaatc gcgggcctcg gcgcgcttct gttgttcatc ctctttgccc gcatccgcgc 14040
ggtcgatgcc gaactgaaac tgaaaaagca tcgttccaag gacgccggcc tggccgatct 14100
gctcaactac gccgctgtcg tcgatgacgg cgtaatcgtg ggcaagaacg gcagctttat 14160
ggctgcctgg ctgtacaagg gcgatgacaa cgcaagcagc accgaccagc agcgcgaagt 14220
agtgtccgcc cgcatcaacc aggccctcgc gggcctggga agtgggtgga tgatccatgt 14280
ggacgccgtg cggcgtcctg ctccgaacta cgcggagcgg ggcctgtcgg cgttccctga 14340
ccgtctgacg gcagcgattg aagaagagcg ccggcggcat ttcgagagcc tgggaacgat 14400
gtacgagggc tatttcgtcc tcaccttgac ctggttcccg ccgctgctcg cccagcgcaa 14460
gttcgtcgag ctgatgtttg acgacgacgc gaccgcaccg gatcgcaagg cgcgcacgcg 14520
gggcctcatc gaccaattca agcgtgacgt gcgcagcatc gagtcgcgcc tgtcgtcggc 14580
cgtgtcgctc actcgcttga aggggcacaa gatcgtcaac gaggacggca cgaccgtcac 14640
gcatgacgac ttcctgcgct ggctgcaatt ctgcgtgacg ggcctgcacc atccggtgca 14700
gctccccagc aacccgatgt acctggacgc cctggtcggc ggacaggaaa tgtggggcgg 14760
ggtagtgccc aaggtcggcc gcaagttcgt ccaggtggtc gctctcgaag gcttcccctt 14820
ggagtcctat cccggcatcc tgacggcgct cggcgagctg ccctgcgagt atcggtggtc 14880
gagccggttc atcttcatgg accagcacga agccgtgaag cacctcgaca agttccgcaa 14940
gaagtggcgg cagaagattc gcggcttctt cgaccaggtg ttcaacacga acaccggccc 15000
ggtcgatcag gacgcgcttt cgatggtggc cgatgctgag gcggccattg ccgaagtcaa 15060
cagcggcatc gtggccgtgg gctactacac cagcgtcgtc gtgctgatgg atgaggaccg 15120
cacgcgcctg gaagctgcgg cccgcgatgt tgaaaaggcc gtcaaccggt tgggctttgc 15180
cgcgcgcatc gagtccatca acaccctgga cgccttcctt ggtagtttgc cgggccacgg 15240
cgtggaaaac gtccgccggc cgctcatcaa cacgatgaac ctggccgacc tgctgccgac 15300
cagcaccatc tggaccggca acgcgaacgc gccatgcccg atgtacccgc cgctgtcgcc 15360
ggcgctcatg cactgcgtca cgcaaggatc aacgccgttc cggctgaacc tgcacgtgcg 15420
cgacctcggc cacaccttta tgttcgggcc gaccggcgca ggtaaatcga cgcacctggc 15480
gatcctcgcc gcgcagctcc gtcgctatgc cggcatgtcg atcttcgcct ttgacaaggg 15540
catgtcgatg tacccgctgg ccgccggcat ccgtgcggcc acgaagggca ccagcggcct 15600
gcacttcacc gtggcggccg acgacgaacg cctggcgttc tgcccgttgc agttcctgag 15660
caccaagggc gaccgtgctt gggcgatgga gtggatcgac accatcctgg cgttgaacgg 15720
cgtcgaaacg accccggccc agcgcaacga aatcggcaac gcgatcatga gcatgcacgc 15780
cagcggcgcg cgcacgctct ccgagttcag cgtgacgatt caggatgagg cgatccgcga 15840
ggcgatccgc cagtacaccg tcgatggcgc aatgggccat ctgctcgacg ccgaagagga 15900
cggcttggcg ctgtccgact ttacagtgtt cgagatcgaa gagctgatga acctcggcga 15960
gaaattcgcc ctgcctgtgt tgctctacct gttccgccgt atcgagcgcg ccctgacggg 16020
ccagccggcc gtcatcatcc tggacgaagc ctggttgatg ctcggccacc cggcattccg 16080
cgcgaagatc agggaatggc tcaaggtgct gcgtaaggcc aactgccttg tgctgatggc 16140
aacgcagagc ctgtccgacg ccgccaacag cggcatcctg gacgtgatcg tggaatcgac 16200
cgcgaccaag attttcctgc cgaatattta cgccagggat gaggacacgg cggccctgta 16260
ccgccgcatg ggcctgaacg ctcgccagat cgagattctg gcccaggccg ttcccaagcg 16320
tcagtactac tacgtgtcgg aaaacggccg ccgtctctac gacctggcac ttggcccgct 16380

cgcgctcgcg ttcgtcggcg catccgacaa ggaatccgtc gccatcatca agaacctgga 16440
agccaagttc ggcgaccagt gggtggatga atggctgcgt ggccggggcc tcgcccttga 16500
tgaatacctg gaggcagcat gagttttgca gacacgatca agggcttgat cttcaagaag 16560
aagcccgcaa cggccgcagc agcggcgacg ccggccgcga ccggcccgca aaccgacaac 16620
ccgtacctga cggcgcggcg cacctggaac gaccacgttg gttccgttgt gtcgcaaaag 16680
cagacctggc aggttgtcgg catcctttcg ctgatgatcg tcctcgcggc ggtcggcggc 16740
atcatccaca tcggcagcca gtcgaagttc gtgccctatg tctacgaggt agacaagctc 16800
gggcagacgg ccgccgtggg gccgatgacc agggcgtcga aagccgatcc gcgtgtcatt 16860
cacgcctcgg tggctgagtt cgtcggcgat gctcgcctgg tgacgccgga cgtagctttg 16920
cagcgcaagg ccgtctaccg cctctatgcc aagctcgggc cgaatgaccc ggccaccgcc 16980
aagatgaacg aatggctcaa cggcaccgcc gacgccagcc cgttcgctcg cgcggccgtc 17040
gaaacggtca gcaccgaaat cacttccgta atcccgcaga cgcccgacac ctggcaggtc 17100
gattgggtcg agacgacgcg cgacaggcaa ggcgtggtga aggccagccc cgtgcgcatg 17160
cgggccttgg tgacggtcta cgtcgtcgag ccgacggcgg acaccaagga agaacaactg 17220
cgaaacaacc cggccgggat ctacgtccgg gacttctcct ggtcgagact tctgtgaggc 17280
actgaattat gaaaaaggaa ctgtttgctt tggtcctggc cgcgtccgtt agcgtgcctg 17340
catttgccgc cgatcccggc gcggacctga ctgacctcta tttttccggc aagaacccgg 17400
agctgaccgc gcaagagcgg gcggccatcg ccatcgccaa gaagtgggag gcgggtaccg 17460
ccggcatgcg gccggtggcc ggccccggtg gttcggtgcg cttcctgttc ggcgcgcagc 17520
agccgagcat cgtatgcgcc gtgctgcaag tgtgcgacgt ggccctgcaa cccggcgagc 17580
aagtcaactc gatcaacctg ggcgacaccg cccgttggac ggtcgagccg gccattaccg 17640
gcagcggcgc gaacgaaacc cagcacctca tcatcaagcc gatggatgtg ggcctggaaa 17700
ccagcctggt cgtgaccacg gaccgccgca gctaccacat gcgcctgcgc tcgcatcgca 17760
cgcagtacat gccgcaggtg tcgttcacct acccggaaga tgcccttgcg aagtgggacg 17820
ccatcaagaa ccgcgaacag cgggatcgcg tcgagaaaac cattccgcag accggcgagt 17880
acctgggcaa cctgagcttc aactactccg tcagcgggtc cacgtcgtgg aagccggtgc 17940
gcgtctacaa cgacggcaag aaaaccatca tccagatgcc gcactcgatg gaacagaccg 18000
aagcgccgac gctcctggtc gttcgcaggg agggcggcct gttctccgac gatgaaacgg 18060
tgatggtcaa ctaccgggtc cagggcgacc gctacatcgt cgatacgatt ttcgacaagg 18120
ccatcctcat cgcgggcgtg ggcagcagcc aggaccgcgt gaccatttca agggggaact 18180
aaaccatgcg taagattctg accgtcatcg cactcgcggc cacgttggcc ggctgcgcga 18240
cctccaagta cggcagcttc gtccaggacg cgccggccgc ctacaaccag accattgcga 18300
ccgacgcggt gaagcagctc gtcaagctct acccgccggc gcaaaccaag ctggaattgc 18360
agcaggctac gcccgatccg ttcggcattg ccctggtcac tgaccttcgc gcccagggct 18420
atgctgtcat ggagtacaag cccgacggca acgcggccgc agctccggct gctgcgtcct 18480
cggccgctgc gaagccggca acgccgcaag cccagggcgg ctatccgctg cgctacgtgc 18540
tggaccaatt cagcgacagc aacctgtatc gcctgaccgt catggtcggc tctcaatcgc 18600
tcacgcgcgc ctacctcgcc caaaacaaca cgatggtccc ggccggcgca tgggttcgga 18660
aggagtaagc caatgagcga agatcaaatg gcaccggacg catcgccaga tgcggtcaag 18720
ccgaaaagcg gggttcgccg cgtcaacaac atgccgatgt acctcatcgg cggtgtgctc 18780
ggcatcttcc tgctggtgat ggccctggtt gctgcggatc gcgctgcgca gcagaaccag 18840
ccgggagctg cgaaggctga gaaggccggc agcaccagca tgtttgccga cgaaattgcc 18900
ggcaaacagc aggacggcat catcaaggcc aagccgctgg agattccgcc ggaacaaacc 18960

gcccagcaac cgacgacgga gctgacgcca gccccggcgc agggaacgac tatcacggtc 19020
gcacggcccg agaacctgga ccagcccccg acgccgccgc agggtgcgcg caacgaggac 19080
ctggaccgca tccgcatggc gaagttgcag atgctggaag aggcgatcaa ggccaagacg 19140
acggtgcgca tcgacgcgcc gcgcagccag ggcagcgccg gcggcggtgc tccgcagggc 19200
cgcgaggaaa cccttgcgcg catccaggag ctgcgtcggc aggctgagaa cgcccgcgcc 19260
accgatccga ccgccgccta tcaggccgcg cttgcgcagg ctcgcacgat gggcggcgcg 19320
gcagggggtg gcggtatggg cggctcgggt gcgccgaccc tcgtgcagac ctcgaaccgc 19380
agtggtggcg gcgctggcta tgggtcgttc gacaaccgca gcgagggcga ccgttggcgg 19440
ctcgactccc agccggaagc acctgcaacg ccctatgtgc tgcgcgctgg cttcgtcgtt 19500
ccggctacgc ttatctcggg catcaactcc gatctgccag gccaaatcat ggcccaggta 19560
tcgcagtcgg tgtacgacac ggcgaccggc aagcacatgc tcatccccca aggctcgcgc 19620
ctggtgggca gctactcgaa cgatgtggcc tacgggcaga agcgcgttct ggtggcatgg 19680
cagcgcatca tcttccccga cggcaaggca atggacattg gggccatgcc gggcggcgat 19740
agcgctgggt atgcaggctt caacgacaag gtcaacaacc actacttccg caccttcgca 19800
tcggcattcc tcatgtcggg cgtcgttgcg ggcatcagct tgagtcagga ccgtggcaac 19860
agcaacagcg gttacggacg acaagacgcg ggttccgcga tgagtgaagc gttgggtcaa 19920
cagctcggcc aagtaacggc gcagatgatc gccaaaaact tgaatatcgc gccgacgctg 19980
gaaatccgtc cgggctatcg cttcaacgtc attgtcacga aagacatgac gttttctaag 20040
ccctaccagg cgtttgacta ttaactccaa ggagtaactt atgaagaagc tcgctaagaa 20100
tgttttagcc gctaaagtag ctctggtgct ggccctctcg gtcggcacct tggcggtcac 20160
gcctgcgcaa gcgggcattc cggtcatcga cggcaccaac ctgtcacaaa ccactgtcac 20220
cgcgattcag caggttgcgc aggtccagaa gcaaatcgag gaataccgga cgcagttgca 20280
gcagtacgaa aacatgctgc aaaacacggt ggccccggcc gcctacgtgt gggaccaggc 20340
gcagtccacc atcaacggcc tgatgagcgc cgttgatacc ctgaactact acaagaacca 20400
ggcgggcagc atcgacgctt acctgggcaa gttcaaggac gtgtcctact acaaggggtc 20460
gccgtgcttc tccctgtcgg gctgctcgga aagcgagcgc aaggcgatgg aagagaaccg 20520
ccgcctggcg tccgaatcgc agaaaaaggc caacgatgcg ctgttccgtg gcctcgatca 20580
gcagcagagc aacctcaagt ccgacgccgc cacgctggag caattgaagg gcaaggcgac 20640
gacggcgcag ggccagttgg aagccctcgg ctacgccaac cagttcgcca gccagcaggc 20700
caaccagctc atgcaaatcc gtggccttct gcttgcgcag cagaacgcca tcgccacgca 20760
gatgcaggcc cagcaggacc ggcaggccca gcaggacgct gcgggcgcga agctgcgcga 20820
gggttcgtac cgcgcaagcc cgtctaagac ctggtgaggg gaggcgcgat gaagaaatcc 20880
aacttcatcg cagttgccgc gctggccgcc gtcatggcgg ccagcctggc aggctgcgac 20940
aacaagcccg acaccgacaa gctgacctgc gccgatctgc cgaaggtcac ggatgccgct 21000
caacgcgcgg agctgttgaa gaagtgcccg cgcggagaac cgggaggctt caagcccagc 21060
gaaaagaaag agtggtgatg acgtatgaaa atccagacta gagctgccgc gctcgcggtc 21120
ctgatgctgg ccttgatgcc ggtagcggca tacgcccaaa tcgacaattc gggcatcctc 21180
gacaacgtat tgcagcgcta ccagaacgcc gcgagcggct gggccactgt cgtccagaac 21240
gccgcaacct ggctgttctg gaccttgacc gtgattagca tggtctggac cttcggcatg 21300
atggcactgc gcaaggccga cattggcgag ttcttcgccg agttcgtgcg gttcaccatc 21360
ttcaccggct tcttctggtg gctgctgacc aacggcccga atttcgcgtc gtccatctat 21420
gcgtccctgc ggcagattgc aggccaggca acggggttgg ggcaggggct ttcgccgtcc 21480
ggcatcgtcg atgttggctt cgagattttc ttcaaggtga tggacgaaac ctcgtactgg 21540

tcgccggtcg atagcttcgt cggtgcctcg ttggcggccg ccatcctctg catcctggcc 21600
ctggtcggcg tgaatatgct tctgctcctg gcgtccggat ggattcttgc ctacggcggt 21660
gtgttcttcc tgggcttcgg cggctcgcgc tggacctcgg acatggcgat caactactac 21720
aagaccgtcc tcggggtcgc cgcgcagctc ttcgcaatgg tgctgctcgt aggcatcggc 21780
aagaccttcc tcgatgacta ctacagccgc atgagcgaag gcatcaactt caaggaactt 21840
ggagtgatgc tgatcgtcgg cctgatcctg ctcgttctgg tcaacaaggt gccgcagctc 21900
atcgccggca tcatcaccgg cgcgagcgtc ggcggtgctg gtatcggcca gttcggcgct 21960
ggcacgctcg tcggtgcggc cgcgacggcc ggcgcggcaa tcgcaactgg cggcgcatct 22020
atcgcggccg gcgctgcggc ggcggccggt ggcgcgcagg ccatcatggc ggccgcgtcg 22080
aaggccagcg ataacgtctc tgccggcact gacattctgt cgagcatgat gggcggcggc 22140
ggtggcggcg gcggtggtag cgccggcacc agcggcggcg acggcggcgg ctcgggtggc 22200
ggcggtggct cgggcggcgg tgaaaccccg atggcctcgg ccgccggcga caacagcagc 22260
ggcgcacgcg gcggcagttc gggcggcggc tcgggtggtg gccgttcgtc tggcggtatc 22320
ggtgccacgg cggccaaggg cggccggatc gcggccgata ccgtcgccaa cctggcgaaa 22380
ggtgccggct cgattgccaa ggccaaggcc ggcgaaatgc gcgcatcggc ccaggaacgc 22440
atcggcgata ccgtaggcgg caagatcgcg caggcaattc gcggcgcggg tgcggcggcg 22500
cagaccgctg caaccgtcgc cgatagcaac agccaggcgc aggaacaacc tgcaccggca 22560
cccgcaccgt cgttcgacga caacagcctt tccgcaagca acaacaggga agcggccgcc 22620
gacgcggatt ccgaagtggc gagcttcgtc aacaagcccg cccaatcctg aaacgactct 22680
taggagctac gaccatgcaa ctgaaaaaag cgttctcgtc ggccgccctg gtggtggcct 22740
tgggcctcgg cgcaactggc tcggccagcg cgcaagacgt gctgacgggc gatacccgcc 22800
tggcctgcga ggccattctg tgcctgtcca cgggcagccg gcccagcgag tgcagcccgt 22860
cgctctcgcg gtacttcggc atccacaagc gcaagctgtc ggacacgctc aaggcgcggc 22920
tgaacttcct caacctctgc ccggtatcga accagacgcc ggaaatgcag acgctcgttt 22980
cctcgatttc gcgcggggcc gggcgctgcg atgcgtcctc gctgaactcc gtgctgcgtg 23040
agtggcggag ctgggacgac cagttctaca tcggcaaccg cctgccggac tactgcgcgg 23100
cctacaccgg ccatgcctat accgacttca acacgaccgc gccgcgctac gtcggcacgc 23160
cggaagaggg cggctattgg atcgaggcgg ccgactacga ccgcgcgctc aaggagtacg 23220
aggcgaagct gaaagagcgg cagcagcagt acggtcgcta tggcagcgac gcctaccgtc 23280
ggttcgagcg gtaaggggag gggatagcga tgccgtttgc caagctgctg gcacggaacg 23340
ctctgccggt ggtcgccctg gtggcggcca ctggcttcgg tgcggcggat gcgaccgccg 23400
cacggctctt ccccgatctg tcggaacaga tggaagagcg cgttgtgtgc tcggtgtctg 23460
cggccgcgaa gtacgagatt ccggccaaca ttcttctcgc cattcgggaa aaggagggcg 23520
gcaagccggg ccagtgggtc aagaacacca atggcaccta tgacgtgggc gagctgcaat 23580
tcaacaccgc ctacctgggc gacctggcga agtatgggat cacggcccag gacgttgctg 23640
cggcaggctg ctatccctat gacctggcgg cctggcggtt gcgcgggcac attcgcaacg 23700
acaggggcga tctgtggaca cgcgccgcta actatcactc gcgcacgccg tcgaagaacg 23760
cgatctatcg cgccgatctg atggtgaagg ccgacaagtg ggcgaagtgg ctggatgcgc 23820
gtttcgtcac cgtcaactat ggccccagct cgccggcgca gccggcaggg aaggggacca 23880
cacttgcggc cgctgatacg tcggcagcag cgccggccga agcgcagccg atgaagcaag 23940
gccggatcac ccgcaccagc ctccgcagct cgggttacgt accccggcag ctcatcatca 24000
acaacacgcc ataaggagga acggccgttt agcggctaaa gcctatgggc attcgcaacc 24060
tgacgcagcg atacatgaac ggggccaggg cctacgcggc ctgggcggca tcgcaggcga 24120

aagcgccgtt tgatcttctg gtactgggca tcgggcctgt catcgtcttt ggcctggtcg 24180
cgcatacgct gctcgcgttc ctgcccacat gggccatgta cgccgccggc gctctgctgg 24240
tcctcgcggc cctgcctttg gcgctgcacg tcctccggga atacgcgctg cgctatgggc 24300
gcaaatagcg ccctgcaggg cgttcttact ccaaggggga gggcatgaat acacgcgcca 24360
tgaacgacgc cagcggccgg gcctcgctgc ctgccatggt gatcgccgac ggcaccattg 24420
aagccttgaa gtggctcgcc ttgcttccca tgaccgggga tcacgtcaac aagtacctgt 24480
tcaacggtac gctgccatat ctgttcgagg cggggcgctt ggccctgcct cttttcgttt 24540
tcgtcctggc gtacaacctc gcccgcccgg gcgcgctcga gcgcggtttg tacgggcgag 24600
cgatgaaacg cctgttggcc ttcggcctgg tcgcctcggt cccgttcatt gcgttgggtg 24660
gagtggtggg cggatggtgg ccgctgaacg tcatgttcac gctgttggcc gcaaccgcga 24720
tgctctacct ggtcgagcgc ggccgctcgg tcgctcctat agcgctgttc gtcgtggccg 24780
gcggcctggt cgagttctgt tggccggcgc tgctgctggc cgcgtctgtc tggttgtacc 24840
tcaagcgccc gacgtgggcg gccgcgttga tggcgctgct gtcttgcgcg tccctgtggt 24900
acatcaatgg caacctttgg gcgcttgctg ttgtgcccct ggtgatcgtc gccgccggcg 24960
tcgatcttcg tgtcccgcgc ctgcgctggg ccttttacac gtactacccg ctgcatcttg 25020
ccgctctttg gctgatccgc attccgatgc gcgaggcggg ctacttgttt ttcacctgac 25080
ctttgagatt ccaatatgca attgctcaag aaatgcacca tcgcggccct gccgctgctc 25140
gccctgtccg gctgcgcact gctgaacatc cccatgccga cgccgcccgg ttcgaccccg 25200
ccggaaatgc tgaccgtgcc agtggcgcaa atctgccgcg acgctgacaa gaaccctgtt 25260
cgggcaacgg agctgtacgg caagaaaggg ttgtcggcca ccggcaaggt gcaggtgatt 25320
tccgaaggct tcaagcctcg ctatcgggtg ctgctgccgc ctggcagcgc ctcggtccat 25380
gctgggaccg ataaccagct cgccatcaag tcggtttcca ccggccagac cacgcgcgtc 25440
actggcaccg tgaaggacgt gtcctacgac cataacggct gctcgatctc gcttgacgat 25500
gcgaagttct actgagggga gggcggcgga tgctgacacg gttgaagggc ttccttgctc 25560
gtcgccgcga gttgaaggaa ctggatgtgt ccgtggtgag ccggccccgg ccggctccgg 25620
cggaattggt ccaggttgat gcacgcgagg ccgtttggcg cgtgccggtg cccggccagg 25680
ccgaccgctt catgtcggcc aagcctggcg cgatcaacga tgaaatgttc gtggttcggg 25740
tggacaccga agcgttctat cgggcttggc tgcgcagcag ctcgacgggc cgcgaaacgc 25800
ggtcggacaa ctgcccgctg cgctcggaaa tgccgcagga ctacaagttc aagcacgccg 25860
tccagggctt cgcgcacggc agggaaaatc ctgtgccgct ggccttcgcc ggcgcgcacc 25920
aggagcgcca ccgggtggac attggtttca gcaacggggt cacgcgctcg ttctggctga 25980
ttgccaacaa ggctccgtcg ttcccgatcc aggtccacgg ccgggagtcg gccgagctgc 26040
tgaacaaggt ttgcggcctc gatcctgcgc cgctgtcgtt cacggaactg ttcgcgcagg 26100
cccaacgcca ggctccgcag gtcgccacac cggcccggcc tgcgccggca gcggccaccc 26160
ggccagctcc caaggtgcag ccacgccccg gccgaagcgg cccgcgcaaa ggccgcggac 26220
tctgactaca accgtgcgca aggcgcatta gggaggatgt atgtatgtaa tcgcctgcgg 26280
catcgttgcc ggcttggcgg ctgcggtggc cctgttgggc ttcacgccga tgatggaggc 26340
gcttgccgcc ggcgaacgcc gcaaggcact cgcgcaatgg acgcggacga tgttcctggt 26400
gctgctgcct gtcgtgctga tgtgcgcgcc catcgggtcc agcatttacg acgccgtgca 26460
agcggacgct ggcaagccca tcgctttcca caacggccgg atcacggtcg tcatggccct 26520
ggtcggcagc ttggccgttg tcctggtcgc ggctgcgcgt gcggtggtca accgcaagca 26580
tgccagcttc tggttcgtcg gctgggtgat ggcgtcggtt ttggccggcg gcgtcggcgc 26640
gatcgccagc gcgaagcaac tggcgttcct cggcgaacat agcggcatgg tggccttcgg 26700

cttcttccgc gaccaggtga aggacatgca ctgcgatgcg gacgtgatcc tggcccggtg 26760
ggatgaaaag gcgaactcgc cggtggtcta ccgctgcccg aaggcgtacc tgctcaacag 26820
gttcgcatcc gcgcccttcg tgccctggcc ggactacacc gagggggaaa gcgaggatct 26880
aggtagggcg ctcgcagcgg ccctgcggga cgcgaaaagg tgagaaaagc cgggcactgc 26940
ccggctttat ttttgctgct gcgcgttcca ggccgcccac actcgtttga cctggctcgg 27000
gctgcatccg accagcttgg ccgtcttggc aatgctcgat ccgccggagc gaagcgtgat 27060
gatgcggtcg tgcatgccgg cgtcacgttt gcggccggtg tagcggccgg cggccttcgc 27120
caactggaca ccctgacgtt gacgctcgcg ccgatcctcg tagtcgtcgc gggccatctg 27180
caaggcgagc ttcaaaagca tgtcctggac ggattccaga acgattttcg ccactccgtt 27240
cgcctcggcg gccagctccg acaggtccac cacgccaggc acggccagct tggccccttt 27300
ggcccggatc gacgcaacca ggcgctcggc ctcggccaac ggcaagcggc tgatgcggtc 27360
gatcttctcc gcaacgacga cttcaccagg ttgcaggtcc gcgatcatgc gcagcagctc 27420
gggccggtcg gcgcgtgcgc cggacgcctt ctcgcggtag atgccggcga cgtagtaccc 27480
ggcggcccgc gtggccgcta caaggctctc ctggcgttca agattctgct cgtccgtact 27540
ggcgcgcagg tagatgcggg cgaccttcaa ccttcgtccc tccggttgtt gctctcgcgt 27600
cgccatttcc acggctcgac ggcgtgcgga tcggaccaga ggccgacgcg cttgcctcgc 27660
gcctcctgtt cgagccgcag catttcaggg tcggccgcgc ggccgtggaa gcgataggcc 27720
cacgccatgc cctggtgaac catcgcggcg ttgacgttgc gcggctgcgg cggccggctg 27780
gccagctcca tgttgaccca cacggtgccc agcgtgcggc cgtaacggtc ggtgtccttc 27840
tcgtcgacca ggacgtgccg gcggaacacc atgccggcca gcgcctggcg cgcacgttcg 27900
ccgaaggctt gccgcttttc cggcgcgtca atgtccacca ggcgcacgcg caccggctgc 27960
ttgtctacca gcacgtcgat ggtgtcgccg tcgatgatgc gcacgacctc gccgcgcagc 28020
tcggcccatg ccggcgaggc aacgaccagg acggccagcg cggcagcggc gcgcagcatg 28080
gcgtagcttc ggcgcttcat gcgtggcccc attgctgatg atcggggtac gccaggtgca 28140
gcactgcatc gaaattggcc ttgcagtagc cgtccagcgc cacccgcgag ccgaacgccg 28200
gcgaaaggta ctcgaccagg ccgggccggt cgcggacctc gcgccccagg acgtggatgc 28260
gccggccgcg tgtgccgtcg ggtccaggca cgaaggccag cgcctcgatg ttgaagtcga 28320
tggatagaag ttgtcggtag tgcttggccg ccctcatcgc gtcccccttg gtcaaattgg 28380
gtatacccat ttgggcctag tctagccggc atggcgcatt acagcaatac gcaatttaaa 28440
tgcgcctagc gcattttccc gaccttaatg cgcctcgcgc tgtagcctca cgcccacata 28500
tgtgctaatg tggttacgtg tattttatgg aggttatcca atgagccgcc tgacaatcga 28560
catgacggac cagcagcacc agagcctgaa agccctggcc gccttgcagg gcaagaccat 28620
taagcaatac gccctcgaac gtctgttccc cggtgacgct gatgccgatc aggcatggca 28680
ggaactgaaa accatgctgg ggaaccgcat caacgatggg cttgccggca aggtgtccac 28740
caagagcgtc ggcgaaattc ttgatgaaga actcagcggg gatcgcgctt gacggcctac 28800
atcctcacgg ctgaggccga agccgatcta cgcggcatca tccgctacac gcgccgggag 28860
tggggcgcgg cgcaggtgcg ccgctatatc gctaagctgg aacagggcat agccaggctt 28920
gccgccggcg aaggcccgtt taaggacatg agcgaactct ttcccgcgct gcggatggcc 28980
cgctgcgaac accactacgt tttttgcctg ccgcgtgcgg gcgaacccgc gttggtcgtg 29040
gcgatcctgc atgagcgcat ggacctcatg acgcgacttg ccgacaggct caagggctga 29100
tttcagccgc taaaaatcgc gccactcaca acgtcctgat ggcgtactta cccaaagaac 29160
agctaggaga atcatttatg ctcagcacac ttccacaagc tcatgcaact ttcttgaacc 29220
gcatccgcga tgcggtcgct tccgatgttc gcttccgcgc tcttctgatc ggcggctctt 29280

acgttcacgg aggactcgat gagcactccg atttggattt cgacatcgtt gttgaggaca 29340
actgctacgc agatgtcttg tctacacgca aggattttgc cgaggcactg cccggcttcc 29400
tcaacgcgtt caccggcgaa catgtaggag aaccgcgcct tctgatctgc ctatatggtc 29460
cgccactgct acacatcgat ttgaagtttt ctcttgcttc cgatctcgac cagcaaatcg 29520
agcggcgggc ggttctgttt gctcgtgatc cggcagagat cgagaagcgc attgaggcgg 29580
cagcggtggc atggccaaac cgtccctccg agtggttcga agcacgttgt cagcgccagt 29640
gatataagac ggtaattcac catttggatt gtccgctcca cccaacatgt tgtttcctta 29700
aggttctcac accagaaagg acatcaacat gctgagcaga gaggactttt acatgataaa 29760
gcaaatgcgc cagcagggcg cgtacattgt cgatattgcg actcagattg gttgctctga 29820
acggacggtc agacgctacc tcaaataccc tgaaccgcca gccagaaaga cccgccacaa 29880
aatggttaag ctgaaaccgt ttatggatta catcgacatg cgcctggcag agaatgtctg 29940
gaatagtgag gttatctttg cggagattaa ggcaatgggt tatacgggcg gacgttccat 30000
gctgcgttac tacatccagc ccaaacgtaa aatgcgtccg tcaaaaagaa cagttcgctt 30060
cgaaactcag cctagatacc agctccagca cgactggggc gaagttgagg tggaggttgc 30120
cgggcaacgg tgcaaagtta actttgcggt taatacgctg ggttctcccc gccgcttcca 30180
tgtcttcgcc gcaccaaaac aggatgctga gcatacctac gaatcactgg ttcgcgcctt 30240
ccgctacttc ggtggttgtg tgaaaacggt gctggttgat aaccagaagg ctgcggtgct 30300
gaagaataac aacgggaaag tcgtgttcaa ctccggattc ctgttgctgg ccgaccacta 30360
taacttcctg ccacgggcat gccgtccacg cagggccaga acaaaaggta aggttgagcg 30420
gatggtgaaa tacctcaagg agaacttctt cgttcggtac cgcaggttcg acagcttcac 30480
tcatgttaat caacaactgg agcaatggat agccgatgtg gctgacaaac gggaacttcg 30540
ccagttcaaa gaaacgccgg aacagcgctt cgcgctggag caggaacatc tgcagccgtt 30600
accggatacg gacttcgata ccagttactt cgacatccgc catgtgtcct gggacagcta 30660
tatcgaggtt ggtggtaatc gttacagcgt tcccgaagcg ctgtgtggtc agccggtatc 30720
gatacgaata tcgctggatg acgagttgcg gatctacagt aatgagaaac tggtggcctc 30780
acatcgcctc tgttcggcat cgtctggctg gcagacagtg ccggagcatc acgccccgct 30840
ctggcagcag gtcagtcagg tggaacatcg accactgagt gcctatgagg agctgttgtg 30900
atgcatgagc tggaagtcct gctgagtcgc ctgaaaatgg agcatctgag ttatcacgtt 30960
gaaagcctgc tggaacaggc agctaaaaaa gagctgaact accgggagtt cctgtgcatg 31020
gcgctacagc aggaatggaa cggcaggcat cagcgcggta tggagtccag gctgaagcag 31080
gctcgtctgc cgtgggtcaa aacgctggag cagttcgact ttaccttcca gccgggcatc 31140
gaccgtaagg ttgtccggga actggctggt ctggcgttcg tggagcgcag cgaaaacgtg 31200
atcctgctgg gacctcctgg tgtcggaaaa actcatctgg ccatagctct tggcgtgaaa 31260
gcggtggatg cgggacatcg ggtactgttt atgccactgg acagactgat cgcgacactg 31320
atgaaagcga aacaggaaaa ccggctggag cgtcagctgc agcaactgag ttatgcccgg 31380
gtgttgatcc tggatgaaat aggctatctg ccgatgaaca gagaggaagc cagcctgttc 31440
ttccggctac tgaaccgtcg atatgaaaaa gcgagcatca tactgacgtc aaacaaaggg 31500
ttcgcagact ggggagaaat gttcggagat cacgtgctgg caacagcgat actggatcgg 31560
ttgctacatc actcaaccac gctgaatatc aaaggagaga gttaccggtt aaaagagaaa 31620
cgtaaagctg gagtgctgac caaaaacaca acgccaatca gtgatgatga aatggtgaaa 31680
agcggacagc atcagtaacg aaagtatctt agcgggcatg aaaatggcaa ataacggtca 31740
aacatcgtgg cgttgacaac gtgcctggat ctggctacac tatgcggcca ccaagctcgc 31800
ccgtggagtc tctacgaagc gatcggcatg ctcggtttcc ttccgtgagc aagtgttagg 31860

acctttgctc taccgtcgcg ctggaaagga ccagcgcgga gtgaggcgat tggaaaccct 31920
tcgactggga tgaagagcgc agacctagcc accaccattg cgctgcacga tgcgttgtct 31980
gtcagggatg ccatcaaagc atctgcctcc atctatctcg acctccgagc cgccgatccg 32040
tcgttggaac cgacaacgca tatgccaggt cttctgtacg acttaataga acgtgcggta 32100
ccaggcacgc ctaaccgtca gtgagattgg atgagtgaac gatattgatc gagaagagcc 32160
ctgcgcagcc gctgccgtgc ccgagagcat ggcggctcac gtgatgggat acaaatgggc 32220
gcgtgataag gttggtcagt ccggctgcgc ggtctatcgg ctgcatagca agtcaggcat 32280
ggctcctgac aacagcaata catggaaaaa cggcatatca agtgctgaaa tcggatttcg 32340
gagcccgtct cgttgttgtt gacgcattgg cggcgttcat gcgccgactg catgcgatcc 32400
cagtgagcga atgctccgtt caacagtgga ccacgcatgc aggcttgccc gagcgcggga 32460
gtatcgaggc gggggttgtt gatgtcgatg acttcgataa ggagcgcgaa gggtggacgg 32520
ccgaacaggt ttgggaggcg atgcatcgcc tcctaccgct cgcgccggac ccagtcgtga 32580
cgcacggcga tttttcactc gataatctac ttatcgtcga aggtaaggta gtcggctgca 32640
tcgacgttgg gcgggctggt attgctgatc gataccaaga ccttgccgtg ttatggaact 32700
gtcttgagga gttcgaacct tcgcttcagg agaggcttgt tgcgcaatat ggcattgccg 32760
atccggatag gcgcaagctg caatttcatc tcctgctgga cgaacttttc taaggcgatg 32820
ccccctcgac ctcgatcagg gaggcgttca ggacgactca caaagaaagc cgggcaatgc 32880
ccggcttttt ctgctgctac ctccgtagtc gtaaggtcgt tgcaggtgct cgggtgcggt 32940
acaactcgcc ggtcgccagc tcaagcgcga tcacgtcgtt gccgtcgtag ttgacgatga 33000
tgctgttggg ccgactgtcc tcacgcttcg cagggagagg ccagccttca atcgaagccg 33060
gcgcaagctc gtagtgcttc ccggtttcga cgctgcgcag cgtccaggtc ctgcaaccgg 33120
ccacgccggt cgcagaaacc acggcgagcg agccgcgaaa atcgtgcggg tacgcctcga 33180
tgttcatacg cctcctagat cgagcgcgag cgtttctgct cggccttggc cgcctgttcc 33240
tgggacacct cgccgatgac cttgccctgg ccccggctgt aggcgatttc gtagttcttg 33300
ccgacaaccg gcggcttctc aaagatgccc cggctgtgtt tcacgatccc gccttcgctg 33360
aactggtaga cgttgcgccc atcgtcgtgc agcacctggc cgacgtgctt gtgcgggtgg 33420
acgtttttgc ttgcgtcctt cgcatcgctc aactggtgaa tgcctttcgg tagccccgcc 33480
tcgggcaaca ccttcatggt cagccattcg ccgttcacta cctggtccac ctggcggctg 33540
ccgttcatga cggcgatctt gacgctgccc tcgggtttca tgatgacgcc agggcttgcc 33600
gatgtgcgtg gtgccccgat ctgtactttg ttcatacgct ctagttctcc ttagtaggtt 33660
ctcgcgcggc gttgccgctg ttcttgctgc tcgatgtctt gctgcttgag ctgctgcacc 33720
ttctgccgct ggccctcgtc gagaagcacc ttgccgacag cacttctcac ctggcgttca 33780
accccgtcct tgcccaggct gctgcgctcg gacaggtcgt tgaaatcggt gtgcttcttc 33840
atgttcgaca gggcggcgag ctggccatcg ttcaacagcg attccttgag cttggccgtg 33900
tcggcctcgc tcaactggac cttgccggcc gccgcgtccg cgaggcgctt ttcagcgtgc 33960
aaatggttgc ggtagttctc cggggtgatc ggcggcagct ccttcgggta ggcgttctcg 34020
cccggcgcga agattgggaa gatggccttg ccgccgaccg ccttggcggc ctcctgtgcc 34080
ttcgtcctgc cgggattcac gccctgggtg atctgcacct ggcggtcgtc gtcgccggcg 34140
atcacaacgg gcttgtccgg gaatttcgcg tgcagggcct cggcaacagc ctgtaggttg 34200
ccggaatcga acgcggcgac agtcgcgtgc cccagcgctt cggccactgt ggcggcggtg 34260
gcatagcctt cgccgatcac cagcgccggc gcggccgcga gcgcatccat gccaccgacg 34320
acatggaagc atccttcctt gcggctgtcc ttggcgaagc gcttggtgcc gtcctcctgg 34380
atgtactgca tggtccattg cttgccgtcg gcgtcgtagg ccgggatgta ggttttctgg 34440

ccctcctggt cggtaaggac gccggcgtgc acctgtagac ccttgtcgcg caggtacggc 34500
gtcggttccg tgatgggaac caggctttgc gcctggcggc cgatgcgctg cgccgtggct 34560
tcgtgctggc gttcttgttc ctcggcacgc gcggccagct tggccgccgc ctcggcctgc 34620
atcttggcct tctcggcggg gtccagggcg tagcccttgg ccttccactt catttcgacg 34680
ccggtgcggt tgtttttgat gtaaccggcc gggtggccgt cgaggtggcc gacgtagaag 34740
cccgacttct cgcccttctt gtcgccctcg gtctcgatgc ggtgcttctt gccgtccatg 34800
atggggtgct cgccgcctgg ggtgacgacg cagcccatgc ttttcagggc ctccgcgaac 34860
tcatcttcgg gggtgacggc cggggattgc tgggtgggca cgttgtccgg cagccagcgt 34920
tgcagcttgc ccatgtcggc gttcggtccg gcgtaccagg acttggccac cttgtcccac 34980
tgcgcgccgg ccgccttggc aacctggcgc tcgccgtagg gcacggccag gtagacgcgc 35040
tcctgggccg cgttggggcg ctcggccgtg ggttgggtag gctgggcctc ggcgcgggcc 35100
tctacggccg ctgtagcgcc ctcgcgcgcc catttggcga acggggcagg gtcaacccct 35160
gccggaacgt accaggcgcg ttcctggcgg tcccagcgcg ctccaagggc cttcacctcg 35220
tctttctcct tgaacggcac gttcaagtag gcgcgctcgg gcttggcggg ggcttgagcg 35280
gccgccggct gctcggcggc gttcatggcc tgggccattt cctgctgctc gcgctcgtag 35340
tcggcgatcc ggcgctgtag gtcctcgtcg tgcagcatgg ccgtgccctc ggcggccttg 35400
cgcgcctcct tggcggcaac gcggtcctcg tcggtgctgt tgggatcgcg gcgaactcgc 35460
tcttcatgga tgcgggcgaa cttcgcggcc tgctcgtact cgttggccgt cgcgtaagcg 35520
tcgatcacgg ccaggcggtc ggcgagcgct tcggcattgg tctgcgcgtc ggggccggcg 35580
aagtcggcaa gccattggtg gccgccccac gcatggttcg catagacgcc ccaaaactcc 35640
ggctctcggt cgccggccgg caccacggac cgttcgccgt cgtgctcgac ctcgacgttg 35700
gcctggacct ggacgcggcc ggtccaatcg gcaggcagct caaagcccag cgtggtttcg 35760
gtcagcgcgg ccagcgattg gttgccttcc gccggctccg cgccggcgcg gtacatgcgc 35820
agggtctgcg cgatcagctc gtcggccggc gcaatggccg gtcgtgccac ctggtcttgc 35880
tgttgctcca tagttgcccc ctgcgcaggc tcgatggcct gctgggtcgt ttgttcttga 35940
atttgcttct gctcgaacgc caggacgaaa tcctggatct tctccgcgtc ggcggccgcg 36000
cggaaaatct ctagcgggtc ctcttgtagc gccttgatcc acgatccgac ataggccgcg 36060
tgctggccgg ggtcgtggcc gatgcccagc tcgtcgccca ggatcatgct ggcaatctcg 36120
gcccgcagct cttccttggc gtacccctcg ctcccgaagg gatgcgccag gtcgcggtcc 36180
agccgcgacg ggtggccggt ccagtgcccc agctcatgga gcgcggttgc gtagtagttg 36240
tcggcgctcg ggaactggcc tttgtcgggc agatggatgc tgtccgtgga cggccgataa 36300
aacgcgcggt cgtgctcgcc gtggcggatg gtggcacctg acgccgcaag gatgtgctcg 36360
gcccgctcga cggcgctcca agtctgttcc ttgcgttcca acggcggcag gccgtcgatc 36420
tgctccgcat tgaacacggt ggcgaagaac acgcgcgggc gttcgagctg caccgtcacc 36480
ttgaccggat cgccgttggc atcgaggacc ggcttgccgg tctgctcgtc ggtcttggtc 36540
tgctcttcgc tgaacttcca atactggatc ggcgtgcctt tctcgccgcg acgcacctgt 36600
gcgccggcgg cagcggcctg cttgtaggtc atccagcgcg ggtccgcatg gccctgggcc 36660
atgagctgaa tcgcgttgat gcccttgtaa cgcttcccgg tagtcgggtt gagcgggatg 36720
aaggagccgg gcatgcccgg ttcccacggt ttttgccacg gcgcagtgcc ggctttcagt 36780
tgctcaatga ggcgttcggc aacctgctcg tggaacggct ttttgacctc tgccatagcc 36840
aattacctcc cgtcattggc ggccgcggtc gtcgtgtcct cgggcacggt cgcgtccacc 36900
aggtcaatgt cgctctcggc ggcgtcctgc tcgctcaagg cgtcctcggg gaaggccccg 36960
gccttctccg cttcttccgg gtcgaactcg acctggaagc cgggcgtcat cgcatcggcg 37020

agcttttcac gcagggcggc ggcggcgttc gcggtatcgt cgaacggcgc aaagtcgtcc 37080
tgctgcacgg tcttgggatc attcatcgct ttcactcctg gttggtgccg ttacggcctt 37140
tgctgtagtc cggcctgcct ttcaggtcgg ggtatgtctg cttgcacgtc gggaagttgc 37200
tgcaccccca ccagaacatg ccgcgcttct tgccaggccg acgggaaagg ccgtggccgc 37260
aggccatgca cttgtgcagc tcggagactt tcggggcctc gcgcgggacg ggcttgccgc 37320
ccttgtcgtc gcacgcgaac ttgcagccgt cggcaaagcc ggtgcagccc caaaagtatt 37380
cgttcttgtc cttcttcttg aggcgtcgca gcggcttgcc gcaggacggg caagggtgcg 37440
tgtcgatctt catgttgagg ccgttgtctt tgatgttggc gacctcggcg ccgatgtatt 37500
ccatcagctc gttgacgaac gacagcgtgt cgcgctcgcc ggcctggatg gccttctgct 37560
gctcatgcca gagcgcggtc atgtcgggga atctggccgt gtcgggcagt gcgtcgtaca 37620
gctcttcgcc ggtcggcgtg gacacgatgt gcttgccctt ctccaccagg tagccgcgct 37680
cgaaaagcgt ggcgatgatg gagtctcgcg ttgccggcgt gccgatcccg ccgtgctcgc 37740
cttgcttgcc cttgtccttt tcgatcaaga ttttccgcag gcggtcatcg cggatgtatt 37800
tcgcaacgcg ggtaaggtcc gacagcagag attccatcgt gtacagcggc tgcggtttcg 37860
tctcctgctg ctcggccttc gcatcggtgc aggtgccggc ctggccgtca cgcagcttgc 37920
gcaggtcctg ttcaatgtcg tcggcattgc cttccaggtc ctcgttgccg gcgtcgttct 37980
tgtagagaat cttccagccc ggcgacgtgg tgacgttcga gcgcacgccg aaacgatgat 38040
cgccgacctg ggcaagcacg tcggtctggt catacagatg cttcggccag aactgcgcga 38100
cgtaggcgcg cgcgatcagc aggtaaatct tctgctcggc atcggtgagc ttcgacaggt 38160
cggccgtgct ttcggtcggg atgatcgcgt ggtgcgcgga aaccttggac gagttgaagg 38220
cgcggctctt gatcgtcgga ttggcgcgct gcgcagcagc ggccagcatg ggggccgtct 38280
gtgcgatggc cgccagcacg cccggcgcat cgccgtgctg ttcctcgctc aagtattcgc 38340
agtcggaacg gttgtaggtg atgagcttgt gcttctcgcg cagggcctgc gtaatgtcct 38400
tcacctggtc cggcttgaag ccgaacttgc gcgaggcgtc catttgcagt ttcagcaggt 38460
tgtagggcag cggcgcagcc gcttccttcg ccttggtggt cacggacacg atgcgggcgg 38520
gttggccgct cacggcggcc gcgatgccct cggcgtgctc cttgttgctg aggcggcctt 38580
tctcgtccac cggatcgccg tcggcgacct ggtaacgggc cgggaactga atgccctcga 38640
cctcgaactg gccgttcacc aggtagtagt aggttttctg gtgggccgcg ttctcgcggc 38700
aacggcgcac gacaaggccc aggatcggag tctgcacgcg ccccacgctc aacagcccct 38760
gatagccctt cgcgcgtgcc gcaagcgtgt acaggcgcgt gatgttgaag ccgtatagct 38820
ggtcgccgac gctgcgggcc tcggccgcag cggacaggcc ggcgaactcg cggttgtcgc 38880
gcatcgcggc gagctgccgg cgcacgatct tcacgttgtt gtcgttgata agcagccgct 38940
gcaccggcag acggcagttg gcgtattcca ggatttcatc gaccagaagc tggccttcgt 39000
cgtccgggtc gccggcgtga accacgcttt tcgcctgctt caacaggctg aggatggtct 39060
tgaactgagc tttcgcaccg gcatcgccgg acggtttctt gcgccaggga atatggacga 39120
tgggcaggtc ggccatgttc cagttggcgt agcgctcgtc gtagtcctcc gggtctagca 39180
aggccagcat gtgaccgtag caccaggtca cgcggtcgga gccgcattcg taatagccgt 39240
ccttgcggct gccgccgccc aggccctcga cgatggcttt tgccagctcc ggtttttcag 39300
cgattacaag gcgttcaaat tgcatatatc cccctaccct caccaggtca gaaccggcct 39360
gatgacggtg atgatttgcg aacgattgac aggcccgaag tagcggccgt cgaaagacgt 39420
gtcgcttacg tcggacataa gcagaacctc ggcggtcccc agggtgtagc tgtcggactg 39480
ataacgaggc agcggccgtc ctgatggatc ggccttgatg agcgcgctgt gaggcagcag 39540
cccgccattc acgcgcacgc cggcgtcggt gatggcaacc tcgtcgcctt tagcggctaa 39600

aactcgcttc atcatgtagc cgtagtcgcc ggggcagaaa ccgccggcga tgtagccccg 39660
ctccttggcg tccgaaaaca cgccgacttg cggcgggcag aacatgacgt aagccccctt 39720
ctccaccggc gcattcgatt tccagtacag gccgaccgga atgcttttgg tggtgttgac 39780
cttcgcgccg gcgagatagg ccgcgccggc gagcaacaag gccgcgccgc ctccgatggc 39840
gacgtacttg gtgaggcgct ggaagcggct catatcgtga tcccctcccc ttcctcgacg 39900
gtggccgtct ggatcagctt gtcgctgacc ttcggagccg gtacggccgc gcgggcctgg 39960
aatatcgggt ctttgaagta gagcggctgc ttgccgtaga tcgcgggata gccggcgacg 40020
tacacaacca tgtcgcccgc ctcttcaatg ctgccgtcgg cgctcttctt cggccccggc 40080
atgcgcaggc attcatcggg ggtcagcaat ggccgctgca cttcctggaa ggtccgcgag 40140
acgttgccca acagcgccga cgtgcggcgg ccgctcgtcg tgatctgctc cttcacgatg 40200
gtcgtggtgc ctgtcagttt tgacaggtgc tcggccgtct ccacgcggtt cggcgggtag 40260
gcgttctgca cgtggcagtt cgacgtgatg ctttcgtcgt ggccgtagcc ggtttcgcgg 40320
ctcttgagct ggttaatgtc ctggcagatg aggtagcact tgatgccgta gccggcgacg 40380
aaggcaaggg actcttgcag gatttcgagc ttgcccaggc tggggaactc gtcgagcatc 40440
atcagcagac gatgcttgta gtgcgcgaca ggacggccgt tctcgaagtc catcttgtcg 40500
gccagcagcc ggacgatcat gttgaccatg acgcgcacca gaggccgcag acgggccttg 40560
tcgttgggct gcgtcacgat gaacaggctt accgggtcgt cgtggtgcat cagttgcttg 40620
atgcggaagt cggacttgct gacgttgcgg gccacaaccg ggtcgcggta cagggccagg 40680
taggacttgg cggtggacag cacggaaccg gattcttcct ccgggcggtc catcatgtcg 40740
cgggccgcag agccgaccgc agggtggttc tgcccgtcaa cgtggccgta ggtggtcatt 40800
tccatccaaa gctcgcccac gtcgcggttc gggtcggcaa gcatgccgtc caccgacggc 40860
agggtggccg gcgtaccctc gttcttagcc ttgtagagcg cgtgcaggat gacgccgaca 40920
agcagcgcct ggctggtttt ctgccagtgc gattccaggc ccttgccgtc cggatcgacg 40980
atcagggtgg caaggttctg cacgtcgcca acctcgtact cggtccccaa gcggatttca 41040
tcgagcgggt tccagcacgc gctaccctgc gcggatgccg gctcaaagcg cacgaccttg 41100
ttgcgggcat gcttcttccg ccagccggcg gtcagcgccc acaactcgcc tttcaggtcg 41160
gtgatgacgg cgctgtgcgc ccaggaaagc agcgtcggaa cgaccaggcc gacgcccttg 41220
ccggagcgcg tcggcgcgta ggtcaagacg tgctcggggc cgttgtgccg caggtagtgg 41280
aacttgccgt ccttgtcctg ccagccgccc acatagacgc cgctggaagt gggcgggtgt 41340
ttgcctgaca ccagctcgac gacggtgcgc ggccggggca gcaggccggc ggcctgtatg 41400
tccttcttgt cggcccagcg ggccgaaccg tgcagatagt cgttcgcctt gccggtgttc 41460
gccttgacca tctgcgtgac ggccgtgccc agcaggccca cggtcgaaac gaccataccc 41520
atgctggccg cgcgcatgaa atcgtcggga tattggccgt accacttgcc ggcccattga 41580
aggatcgacc agggcgtgta gacgtggttg atattccagc caagtccggc ctgatactgg 41640
aaggaatggg cgaaatattg cgtcgcggtc tgcaagcctg ccccaaggga caggccggcg 41700
aggatgggaa cggtcttgct ggccttcggt tttttcgccc gtatctgtgg ccccacggcg 41760
ttgtttcggt tcttcatcta ctcctacctc gggtagtttt aagggagcct cgcggggtca 41820
cggtgacggg atcaccgatg gcgaggcgct tcatgcgttg caccgtggcc ttatcgacgg 41880
gcagcaccag aatctcgtcg ttttctttcc tcaacagggc cagcgcctgg tcctcgacgt 41940
tccgggtgcc tgcataggac agcgcaccaa cataatcagt atatcgtgca tgcttcggta 42000
tatcgaagcc gtttagccgc ttttgctcgc gctcggcaac atatttctcg gccgccgcga 42060
tctgttcggg ctttagccct cttcctggcc cagaaactcc ccgtcgcagt gcgtgagctg 42120
gttcggctcc ttgctgctcc acgtgaccag gaacatcacg cggcaatagc atttcagctc 42180

cgccggcgat gcgaaccaca ccgagttggg acagcgctcg caaacggttt tggctttggg 42240
gcggcggctt tcgtccaatg cgtccaacgt tgggcttgcg gagtgcgacg gttccgccgg 42300
cgctgacggc gcgagcgtcc cgtcggtcgc cgtcgccgcc tgtggcgttg agggtggttc 42360
tggctgcggc aggtcgaatg cctccatcgc cgccgcgatc tcttcgtccg tcatttcgtt 42420
cgggttgctc atgtgcttgc tccttcgtca gtagttcttg acggcggcgc tcaagggcgg 42480
cgtcgtcaaa ggtgattgcc agacggccag cggcggccgc ctgcgcgatc cgctccttga 42540
actctgctgt gccgttgacg gtgatccggt cgccgaagcg ctccattgcc aggcgcaggg 42600
cggcgtccag gccgtccgtg gtggcctcgc gcgagacttg caggcggtcg ccgtcgtcgc 42660
ggacggcgct gctgccgacg cgatagatga tggttccctt cttcgtgatg ttgtccgtca 42720
cggccgcatg gcccggcttg gcctcgccgc tgccctggat ggtgttgccc ttgaggtcgc 42780
tgcggccctc gcgtgcgcgc agcgcggcca gggccttgtc gtcgcccttc atcgcctcgg 42840
ccttgagcca gtcggcccac gcgcggcgct gcgtgcgctc ctggaccgcc tgacggccct 42900
gccggtactc gcggttgatc ttgtccaggt cggcgcgcag agccttgtgc gcctgcgcgt 42960
acatcagtcg ctttgcaatg cgcccctcgc ccagcagctt gatagcggcg cggcgcagcc 43020
ggttgctgcg catcgcggct tcaatcaggc ggtcacgacg ccggcgcagc gtgtccagct 43080
cgcccttgcg cacggccccc atttcctggc gttcagactg ataccgggcg tatagctcgg 43140
tggtgtcgat gcgggtcttg agcggcttcg ctcgatactc ccgccgccgg ggggcttcgc 43200
cgccctcggc tggcgtgaat gccccgaatc gggcttcgag cttcggcttg gacaggtcgc 43260
gcgaaacggt gctggccttg accgtcgtgc cgtcgccggc ctcgaagatg aagccgtttc 43320
cgcgctcgcg cagcttaagc ccgttttccc gcaggacgcg gtgcaggtcc tcccaggatt 43380
gcgccgcttg cagctccggc aggcattcgc gcttgatcca gccgaccagg ctttccacgc 43440
ccgcgtgccg ctccatgtcg ttcgcgcggt tctcggaaac gcgctgccgc gtttcgtgat 43500
tgtcacgctc aagcccgtag tcccgttcga gcgtcgcgca gaggtcagcg agggcgcggt 43560
aggcccgata cggctcatgg atggtgtttc gggtcgggtg aatcttgttg atggcgatat 43620
ggatgtgcag gttgtcggtg tcgtgatgca cggcactgac gcgctgatgc tcggcgaagc 43680
caagcccagc gcagatgcgg tcctcaatcg cgcgcaacgt ctccgcgtcg ggcttctctc 43740
ccgcgcggaa gctaaccagc aggtgatagg tcttgtcggc ctcggaacgg gtgttgccgt 43800
gctgggtcgc catcacctcg gccatgacag cgggcagggt gtttgcctcg cagttcgtga 43860
cgcgcacgtg acccaggcgc tcggtcttgc cttgctcgtc ggtgatgtac ttcaccagct 43920
ccgcgaagtc gctcttcttg atggagcgca tggggacgtg cttggcaatc acgcgcaccc 43980
cccggccgtt ttagcggcta aaaaagtcat ggctctgccc tcgggcggac cacgcccatc 44040
atgaccttgc caagctcgtc ctgcttctct tcgatcttcg ccagcagggc gaggatcgtg 44100
gcatcaccga accgcgccgt gcgcgggtcg tcggtgagcc agagtttcag caggccgccc 44160
aggcggccca ggtcgccatt gatgcgggcc agctcgcgga cgtgctcata gtccacgacg 44220
cccgtgattt tgtagccctg gccgacggcc agcaggtagg ccgacaggct catgccggcc 44280
gccgccgcct tttcctcaat cgctcttcgt tcgtctggaa ggcagtacac cttgataggt 44340
gggctgccct tcctggttgg cttggtttca tcagccatcc gcttgccctc atctgttacg 44400
ccggcggtag ccggccagcc tcgcagagca ggattcccgt tgagcaccgc caggtgcgaa 44460
taagggacag tgaagaagga acacccgctc gcgggtgggc ctacttcacc tatcctgccc 44520
ggctgacgcc gttggataca ccaaggaaag tctacacgaa ccctttggca aaatcctgta 44580
tatcgtgcga aaaaggatgg atataccgaa aaaatcgcta taatgacccc gaagcagggt 44640
tatgcagcgg aaaagcgctg cttccctgct gttttgtgga atatctaccg actggaaaca 44700
ggcaaatgca ggaaattact gaactgaggg gacaggcgag agacgatgcc aaagagctac 44760

accgacgagc tggccgagtg ggttgaatcc cgcgcggcca agaagcgccg gcgtgatgag 44820
gctgcggttg cgttcctggc ggtgagggcg gatgtcgagg cggcgttagc gtccggctat 44880
gcgctcgtca ccatttggga gcacatgcgg gaaacgggga aggtcaagtt ctcctacgag 44940
acgttccgct cgcacgccag gcggcacatc aaggccaagc ccgccgatgt gcccgcaccg 45000
caggccaagg ctgcggaacc cgcgccggca cccaagacgc cggagccacg gcggccgaag 45060
caggggggca aggctgaaaa gccggccccc gctgcggccc cgaccggctt caccttcaac 45120
ccaacaccgg acaaaaagga tctactgtaa tggcgaaaat tcacatggtt ttgcagggca 45180
agggcggggt cggcaagtcg gccatcgccg cgatcattgc gcagtacaag atggacaagg 45240
ggcagacacc cttgtgcatc gacaccgacc cggtgaacgc gacgttcgag ggctacaagg 45300
ccctgaacgt ccgccggctg aacatcatgg ccggcgacga aattaactcg cgcaacttcg 45360
acaccctggt cgagctgatt gcgccgacca aggatgacgt ggtgatcgac aacggtgcca 45420
gctcgttcgt gcctctgtcg cattacctca tcagcaacca ggtgccggct ctgctgcaag 45480
aaatggggca tgagctggtc atccataccg tcgtcaccgg cggccaggct ctcctggaca 45540
cggtgagcgg cttcgcccag ctcgccagcc agttcccggc cgaagcgctt ttcgtggtct 45600
ggctgaaccc gtattggggg cctatcgagc atgagggcaa gagctttgag cagatgaagg 45660
cgtacacggc caacaaggcc cgcgtgtcgt ccatcatcca gattccggcc ctcaaggaag 45720
aaacctacgg ccgcgatttc agcgacatgc tgcaagagcg gctgacgttc gaccaggcgc 45780
tggccgatga atcgctcacg atcatgacgc ggcaacgcct caagatcgtg cggcgcggcc 45840
tgtttgaaca gctcgacgcg gcggccgtgc tatgagcgac cagattgaag agctgatccg 45900
ggagattgcg gccaagcacg gcatcgccgt cggccgcgac gacccggtgc tgatcctgca 45960
taccatcaac gcccggctca tggccgacag tgcggccaag caagaggaaa tccttgccgc 46020
gttcaaggaa gagctggaag ggatcgccca tcgttggggc gaggacgcca aggccaaagc 46080
ggagcggatg ctgaacgcgg ccctggcggc cagcaaggac gcaatggcga aggtaatgaa 46140
ggacagcgcc gcgcaggcgg ccgaagcgat ccgcagggaa atcgacgacg gccttggccg 46200
ccagctcgcg gccaaggtcg cggacgcgcg gcgcgtggcg atgatgaaca tgatcgccgg 46260
cggcatggtg ttgttcgcgg ccgccctggt ggtgtgggcc tcgttatgaa tcgcagaggc 46320
gcagatgaaa aagcccggcg ttgccgggct tgtttttgcg ttagctgggc ttgtttgaca 46380
ggcccaagct ctgactgcgc ccgcgctcgc gctcctgggc ctgtttcttc tcctgctcct 46440
gcttgcgcat cagggcctgg tgccgtcggg ctgcttcacg catcgaatcc cagtcgccgg 46500
ccagctcggg atcgtccgcg cgcatcttgc gcgtcgccag ttcctcgatc ttgggcgcgt 46560
gaatgcccat gccttccttg atttcgcgca ccatgtccag ccgcgtgtgc agggtctgca 46620
agcgggcttg ctgttgggcc tgctgctgct gccaggcggc ctttgtacgc ggcagggaca 46680
gcaagccggg ggcattggac tgtagctgct gcaaacgcgc ctgctgacgg tctacgagct 46740
gttctaggcg gtcctcgatg cgctccacct ggtcatgctt tgcctgcacg tagagcgcaa 46800
gggtctgctg gtaggtctgc tcgatgggcg cggattctaa gagggcctgc tgttccgtct 46860
cggcctcctg ggccgcctgt agcaaatcct cgccgctgtt gccgctggac tgctttactg 46920
ccggggactg ctgttgccct gctcgcgccg tcgtcgcagt tcggcttgcc cccactcgat 46980
tgactgcttc atttcgagcc gcagcgatgc gatctcggat tgcgtcaacg gacggggcag 47040
cgcggaggtg tccggcttct ccttgggtga gtcggtcgat gccatagcca aaggtttcct 47100
tccaaaatgc gtccattgct ggaccgtgtt tctcattgat gcccgcaagc atcttcggct 47160
tgaccgccag gtcaagcgcg ccttcatggg cggtcatgac ggacgccgcc atgaccttgc 47220
cgccgttgtt ctcgatgtag ccgcgtaatg aggcaatggt gccgcccatc gtcagcgtgt 47280
catcgacaac gatgtacttc tggccgggga tcacctcccc ctcgaaagtc gggttgaacg 47340

ccaggcgatg atctgaaccg gctccggttc gggcgacctt ctcccgctgc acaatgtccg 47400
tttcgacctc aaggccaagg cggtcggcca gaacgaccgc catcatggcc ggaatcttgt 47460
tgttccccgc cgcctcgacg gcgaggactg gaacgatgcg gggcttgtcg tcgccgatca 47520
gcgtcttgag ctgggcaaca gtgtcgtccg aaatcaggcg ctcgaccaaa ttaagcgccg 47580
cttccgcgtc gccctgcttc gcagcctggt attcaggctc gttggtcaaa gaaccaaggt 47640
cgccgttgcg aaccaccttc gggaagtctc cccacggtgc gcgctcggct ctgctgtagc 47700
tgctcaagac gcctcccttt ttagccgcta aaactctaac gagtgcgccc gcgactcaac 47760
ttgacgcttt cggcacttac ctgtgccttg ccacttgcgt cataggtgat gcttttcgca 47820
ctcccgattt caggtacttt atcgaaatct gaccgggcgt gcattacaaa gttcttcccc 47880
acctgttggt aaatgctgcc gctatctgcg tggacgatgc tgccgtcgtg gcgctgcgac 47940
ttatcggcct tttgggccat atagatgttg taaatgccag gtttcagggc cccggcttta 48000
tctaccttcg tggttcgtcc atgcgccttg gttctcggtc tcggacaatt ctttcgccca 48060
ttcatgacca ggaggcggtg tttcattggg tgactcctga cggttgcctc tggtgttcaa 48120
acgtgtcctg gtcgcttgcc ggctaaaaaa aagccgacct cggcagttcg aggccggctt 48180
tccctagagc cgggcgcgtc aaggttgttc catctatttt agtgaactgc gttcgattta 48240
tcagttactt tcctcccgct ttgtgtttcc tcccactcgt ttccgcgtct agccgacccc 48300
tcaacatagc ggcctcttct tgggctgcct ttgcctcttg ccgcgcttcg tcacgctcgg 48360
cttgcaccgt cgtaaagcgc tcggcctgcc tggccgcctc ttgcgccgcc aacttccttt 48420
gctcctggtg ggcctcggcg tcggcctgcg ccttcgcttt caccgctgcc aactccgtgc 48480
gcaaactctc cgcttcgcgc ctggtcgcgt cgcgctcgcc gcgaagcgcc tgcatttcct 48540
ggttggccgc gtccagggtc ttgcggctct cttctttgaa tgcgcgggcg tcctggtgag 48600
cgtagtccag ctcggcgcgc agctcctgcg ctcgacgctc cacctcgtcg gcccgctgcg 48660
tcgccagcgc ggcccgctgc tcggctcctg ccagggcggt gcgtgcttcg gccagggctt 48720
gccgctggcg tgcggccagc tcggccgcct cggcggcctg ctgctctagc aatgtaacgc 48780
gcgcctgggc ttcttccagc tcgcgggcct gcgcctcgaa ggcgtcggcc agctccccgc 48840
gcacggcttc caactcgttg cgctcacgat cccagccggc ttgcgctgcc tgcaacgatt 48900
cattggcaag ggcctgggcg gcttgccaga gggcggccac ggcctggttg ccggcctgct 48960
gcaccgcgtc cggcacctgg actgccagcg gggcggcctg cgccgtgcgc tggcgtcgcc 49020
attcgcgcat gccggcgctg gcgtcgttca tgttgacgcg ggcggcctta cgcactgcat 49080
ccacggtcgg gaagttctcc cggtcgcctt gctcgaacag ctcgtccgca gccgcaaaaa 49140
tgcggtcgcg cgtctctttg ttcagttcca tgttggctcc ggtaattggt aagaataata 49200
atactcttac ctaccttatc agcgcaagag tttagctgaa cagttctcga cttaacggca 49260
ggttttttag cggctgaagg gcaggcaaaa aaagccccgc acggtcggcg ggggcaaagg 49320
gtcagcggga aggggattag cgggcgtcgg gcttcttcat gcgtcggggc cgcgcttctt 49380
gggatggagc acgacgaagc gcgcacgcgc atcgtcctcg gccctatcgg cccgcgtcgc 49440
ggtcaggaac ttgtcgcgcg ctaggtcctc cctggtgggc accaggggca tgaactcggc 49500
ctgctcgatg taggtccact ccatgaccgc atcgcagtcg aggccgcgtt ccttcaccgt 49560
ctcttgcagg tcgcggtacg cccgctcgtt gagcggctgg taacgggcca attggtcgta 49620
aatggctgtc ggccatgagc ggcctttcct gttgagccag cagccgacga cgaagccggc 49680
aatgcaggcc cctggcacaa ccaggccgac gccgggggca ggggatggca gcagctcgcc 49740
aaccaggaac cccgccgcga tgatgccgat gccggtcaac cagcccttga aactatccgg 49800
ccccgaaaca cccctgcgca ttgcctggat gctgcgccgg atagcttgca acatcaggag 49860
ccgtttcttt tgttcgtcag tcatggtccg ccctcaccag ttgttcgtat cggtgtcgga 49920

cgaactgaaa tcgcaagagc tgccggtatc ggtccagccg ctgtccgtgt cgctgctgcc 49980
gaagcacggc gaggggtccg cgaacgccgc agacggcgta tccggccgca gcgcatcgcc 50040
cagcatggcc ccggtcagcg agccgccggc caggtagccc agcatggtgc tgttggtcgc 50100
cgccggccac cagggccgac gtgacgaaat cgccgtcatt ccctctggat tgttcgctgc 50160
tcggcggggc agtgcgccgc gccggcggcg tcgtggatgg ctcgggttgg ctggcctgcg 50220
acggccggcg aaaggtgcgc agcagctcgt tatcgaccgg ctgcggcgtc ggggccgccg 50280
ccttgcgctg cggtcggtgt tccttcttcg gctcgcgcag cttgaacagc atgatcgcgg 50340
aaaccagcag caacgccgcg cctacgcctc ccgcgatgta gaacagcatc ggattcattc 50400
ttcggtcctc cttgtagcgg aaccgttgtc tgtgcggcgc gggtggcccg cgccgctgtc 50460
tttggggatc agccctcgat gagcgcgacc agtttcacgt cggcaaggtt cgcctcgaac 50520
tcctggccgt cgtcctcgta cttcaaccag gcatagcctt ccgccggcgg ccgacggttg 50580
aggataaggc gggcagggcg ctcgtcgtgc tcgacctgga cgatggcctt tttcagcttg 50640
tccgggtccg gctccttcgc gcccttttcc ttggcgtcct taccgtcctg gtcgccgtcc 50700
tcgccgtcct ggccgtcgcc ggcctccgcg tcacgctcgg catcagtctg gccgttgaag 50760
gcatcgacgg tgttgggatc gcggcccttc tcgtccagga actcgcgcag cagcttgacc 50820
gtgccgcgcg tgatttcctg ggtgtcgtcg tcaagccacg cctcgacttc ctccgggcgc 50880
ttcttgaagg ccgtcaccag ctcgttcacc acggtcacgt cgcgcacgcg gccggtgttg 50940
aacgcatcgg cgatcttctc cggcaggtcc agcagcgtga cgtgctgggt gatgaacgcc 51000
ggcgacttgc cgatttcctt ggcgatatcg cctttcttct tgcccttcgc cagctcgcgg 51060
ccaatgaagt cggcaatttc gcgcggggtc agctcgttgc gttgcaggtt ctcgataacc 51120
tggtcggctt cgttgtagtc gttgtcgatg aacgccggga tggacttctt gccggcccac 51180
ttcgagccac ggtagcggcg ggcgccgtga ttgatgatat agcggcccgg ctgctcctgg 51240
ttctcgcgca ccgaaatggg tgacttcacc ccgcgctctt tgatcgtggc accgatttcc 51300
gcgatgctct ccggggaaaa gccggggttg tcggccgtcc gcggctgatg cggatcttcg 51360
tcgatcaggt ccaggtccag ctcgataggg ccggaaccgc cctgagacgc cgcaggagcg 51420
tccaggaggc tcgacaggtc gccgatgcta tccaacccca ggccggacgg ctgcgccgcg 51480
cctgcggctt cctgagcggc cgcagcggtg tttttcttgg tggtcttggc ttgagccgca 51540
gtcattggga aatctccatc ttcgtgaaca cgtaatcagc cagggcgcga acctctttcg 51600
atgccttgcg cgcggccgtt ttcttgatct tccagaccgg cacaccggat gcgagggcat 51660
cggcgatgct gctgcgcagg ccaacggtgg ccggaatcat catcttgggg tacgcggcca 51720
gcagctcggc ttggtggcgc gcgtggcgcg gattccgcgc atcgaccttg ctgggcacca 51780
tgccaaggaa ttgcagcttg gcgttcttct ggcgcacgtt cgcaatggtc gtgaccatct 51840
tcttgatgcc ctggatgctg tacgcctcaa gctcgatggg ggacagcaca tagtcggccg 51900
cgaagagggc ggccgccagg ccgacgccaa gggtcggggc cgtgtcgatc aggcacacgt 51960
cgaagccttg gttcgccagg gccttgatgt tcgccccgaa cagctcgcgg gcgtcgtcca 52020
gcgacagccg ttcggcgttc gccagtaccg ggttggactc gatgagggcg aggcgcgcgg 52080
cctggccgtc gccggctgcg ggtgcggttt cggtccagcc gccggcaggg acagcgccga 52140
acagcttgct tgcatgcagg ccggtagcaa agtccttgag cgtgtaggac gcattgccct 52200
gggggtccag gtcgatcacg gcaacccgca agccgcgctc gaaaaagtcg aaggcaagat 52260
gcacaagggt cgaagtcttg ccgacgccgc ctttctggtt ggccgtgacc aaagttttca 52320
tcgtttggtt tcctgttttt tcttggcgtc cgcttcccac ttccggacga tgtacgcctg 52380
atgttccggc agaaccgccg ttacccgcgc gtacccctcg ggcaagttct tgtcctcgaa 52440
cgcggcccac acgcgatgca ccgcttgcga cactgcgccc ctggtcagtc ccagcgacgt 52500

tgcgaacgtc gcctgtggct tcccatcgac taagacgccc cgcgctatct cgatggtctg 52560
ctgccccact tccagcccct ggatcgcctc ctggaactgg ctttcggtaa gccgtttctt 52620
catggataac acccataatt tgctccgcgc cttggttgaa catagcggtg acagccgcca 52680
gcacatgaga gaagtttagc taaacatttc tcgcacgtca acacctttag ccgctaaaac 52740
tcgtccttgg cgtaacaaaa caaaagcccg gaaaccgggc tttcgtctct tgccgcttat 52800
ggctctgcac ccggctccat caccaacagg tcgcgcacgc gcttcactcg gttgcggatc 52860
gacactgcca gcccaacaaa gccggttgcc gccgccgcca ggatcgcgcc gatgatgccg 52920
gccacaccgg ccatcgccca ccaggtcgcc gccttccggt tccattcctg ctggtactgc 52980
ttcgcaatgc tggacctcgg ctcaccatag gctgaccgct cgatggcgta tgccgcttct 53040
ccccttggcg taaaacccag cgccgcaggc ggcattgcca tgctgcccgc cgctttcccg 53100
accacgacgc gcgcaccagg cttgcggtcc agaccttcgg ccacggcgag ctgcgcaagg 53160
acataatcag ccgccgactt ggctccacgc gcctcgatca gctcttgcac tcgcgcgaaa 53220
tccttggcct ccacggccgc catgaatcgc gcacgcggcg aaggctccgc agggccggcg 53280
tcgtgatcgc cgccgagaat gcccttcacc aagttcgacg acacgaaaat catgctgacg 53340
gctatcacca tcatgcagac ggatcgcacg aacccgctga a 53381

<210> 16
<211> 5442
<212> DNA
<213> Artificial

<220>
<223> partial sequence

<400> 16
ggtcgactct agaggatccg cggccgcgcg cgatatcgaa ttcgtaatca tgtcatagct 60
gtttcctgtg tgaaattgtt atccgctcac aattccacac aacatacgag ccggaagcat 120
aaagtgtaaa gcctggggtg cctaatgagt gagctaactc acattaattg cgttgcgctc 180
actgcccgct ttccagtcgg gaaacctgtc gtgccagctg cattaatgaa tcggccaacg 240
cgcggggaga ggcggtttgc gtattgggcg ctcttccgct tcctcgctca ctgactcgct 300
gcgctcggtc gttcggctgc ggcgagcggt atcagctcac tcaaaggcgg taatacggtt 360
atccacagaa tcaggggata acgcaggaaa gaacatgtga gcaaaaggcc agcaaaaggc 420
caggaaccgt aaaaaggccg cgttgctggc gtttttccat aggctccgcc cccctgacga 480
gcatcacaaa aatcgacgct caagtcagag gtggcgaaac ccgacaggac tataaagata 540
ccaggcgttt ccccctggaa gctccctcgt gcgctctcct gttccgaccc tgccgcttac 600
cggatacctg tccgcctttc tcccttcggg aagcgtggcg ctttctcata gctcacgctg 660
taggtatctc agttcggtgt aggtcgttcg ctccaagctg ggctgtgtgc acgaaccccc 720
cgttcagccc gaccgctgcg ccttatccgg taactatcgt cttgagtcca acccggtaag 780
acacgactta tcgccactgg cagcagccac tggtaacagg attagcagag cgaggtatgt 840
aggcggtgct acagagttct tgaagtggtg gcctaactac ggctacacta gaagaacagt 900
atttggtatc tgcgctctgc tgaagccagt taccttcgga aaaagagttg gtagctcttg 960
atccggcaaa caaaccaccg ctggtagcgg tggttttttt gtttgcaagc agcagattac 1020
gcgcagaaaa aaaggatctc aagaagatcc tttgatcttt tctacggggt ctgacgctca 1080
gtggaacgaa aactcacgtt aagggatttt ggtcatgaga ttatcaaaaa ggatcttcac 1140
ctagatcctt ttggttcatg tgcagctcca tcagcaaaag gggatgataa gtttatcacc 1200
accgactatt tgcaacagtg ccgttgatcg tgctatgatc gactgatgtc atcagcggtg 1260
gagtgcaatg tcgtgcaata cgaatggcga aaagccgagc tcatcggtca gcttctcaac 1320
cttggggtta cccccggcgg tgtgctgctg gtccacagct ccttccgtag cgtccggccc 1380

ctcgaagatg ggccacttgg actgatcgag gccctgcgtg ctgcgctggg tccgggaggg 1440
acgctcgtca tgccctcgtg gtcaggtctg gacgacgagc cgttcgatcc tgccacgtcg 1500
cccgttacac cggaccttgg agttgtctct gacacattct ggcgcctgcc aaatgtaaag 1560
cgcagcgccc atccatttgc ctttgcggca gcggggccac aggcagagca gatcatctct 1620
gatccattgc ccctgccacc tcactcgcct gcaagcccgg tcgcccgtgt ccatgaactc 1680
gatgggcagg tacttctcct cggcgtggga cacgatgcca acacgacgct gcatcttgcc 1740
gagttgatgg caaaggttcc ctatggggtg ccgagacact gcaccattct tcaggatggc 1800
aagttggtac gcgtcgatta tctcgagaat gaccactgct gtgagcgctt tgccttggcg 1860
gacaggtggc tcaaggagaa gagccttcag aaggaaggtc cagtcggtca tgcctttgct 1920
cggttgatcc gctcccgcga cattgtggcg acagccctgg gtcaactggg ccgagatccg 1980
ttgatcttcc tgcatccgcc agaggcggga tgcgaagaat gcgatgccgc tcgccagtcg 2040
attggctgag ctcatgagcg gagaacgaga tgacgttgga ggggcaaggt cgcgctgatt 2100
gctggggcaa cacgtggagc ggatcgggga ttgtctttct tcagctcgct gatgatatgc 2160
tgacgctcaa tgccgtttgg cctccgacta acgaaaatcc cgcatttgga cggctgatcc 2220
gattggcacg gcggacggcg aatggcggag cagacgctcg tccgggggca atgagatatg 2280
aaaaagcctg aactcaccgc gacgtatcgg gccctggcca gctagctaga gtcgacctgc 2340
aggcatgcgc ctgactgcgt tagcaattta actgtgataa actaccgcat taaagcttca 2400
agcgcctcca atccaattaa ggggacatac gtatgaacgt agtgtaatac actttaccgg 2460
cgttagccta tcctgcaata gacctcacaa gcctctaatc tttagtcatg acgaaggaat 2520
gccgagcttg ggaaaagaaa cgtggttttg cagaacacga acgagaaatg tcgagtgagt 2580
tgtgaagcaa aaaatgggat gggtcagact gcgcgccctt cgggcttgcc caaaggggcg 2640
ctgcccctct ggactctccg ccctcgccgg tagcagggtt gcaggaggga aaaactctcc 2700
tgcaaccccg cccgaagggt tttcggctta caccggaatc aagggttcgc ttcgctgaaa 2760
tcctcgcccg gtccctgcgc ttcgctacgg gctgcggcct ccggtttcac ccttgacacc 2820
ggccccagca agcttatcga tgataagctg tcaaacatca ggagctaagg agcatgcaag 2880
ctctagcgat tccagacgtc ccgaaggcgt ggcgcggctt ccccgtgccg gagcaatcgc 2940
cctgggtggg ttacacgacg cccctctatg gcccgtactg acggacacac cgaagccccg 3000
gcggcaaccc tcagcggatg ccccggggct tcacgttttc ccaggtcaga agcggttttc 3060
gggagtagtg ccccaactgg ggtaaccttt gagttctctc agttgggggc gtagggtcgc 3120
cgacatgaca caaggggttg tgaccggggt ggacacgtac gcgggtgctt acgaccgtca 3180
gtcgcgcgag cgcgagaatt cgagcgcagc aagcccagcg acacagcgta gcgccaacga 3240
agacaaggcg gccgaccttc agcgcgaagt cgagcgcgac gggggccggt tcaggttcgt 3300
cgggcatttc agcgaagcgc cgggcacgtc ggcgttcggg acggcggagc gcccggagtt 3360
cgaacgcatc ctgaacgaat gccgcgccgg gcggctcaac atgatcattg tctatgacgt 3420
gtcgcgcttc tcgcgcctga aggtcatgga cgcgattccg attgtctcgg aattgctcgc 3480
cctgggcgtg acgattgttt ccactcagga aggcgtcttc cggcagggaa acgtcatgga 3540
cctgattcac ctgattatgc ggctcgacgc gtcgcacaaa gaatcttcgc tgaagtcggc 3600
gaagattctc gacacgaaga accttcagcg cgaattgggc gggtacgtcg gcgggaaggc 3660
gccttacggc ttcgagcttg tttcggagac gaaggagatc acgcgcaacg gccgaatggt 3720
caatgtcgtc atcaacaagc ttgcgcactc gaccactccc cttaccggac ccttcgagtt 3780
cgagcccgac gtaatccggt ggtggtggcg tgagatcaag acgcacaaac accttccctt 3840
caagccgggc agtcaagccg ccattcaccc gggcagcatc acggggcttt gtaagcgcat 3900
ggacgctgac gccgtgccga cccggggcga gacgattggg aagaagaccg cttcaagcgc 3960

ctgggacccg gcaaccgtta tgcgaatcct tcgggacccg cgtattgcgg gcttcgccgc 4020
tgaggtgatc tacaagaaga agccggacgg cacgccgacc acgaagattg agggttaccg 4080
cattcagcgc gacccgatca cgctccggcc ggtcgagctt gattgcggac cgatcatcga 4140
gcccgctgag tggtatgagc ttcaggcgtg gttggacggc agggggcgcg gcaaggggct 4200
ttcccggggg caagccattc tgtccgccat ggacaagctg tactgcgagt gtggcgccgt 4260
catgacttcg aagcgcgggg aagaatcgat caaggactct taccgctgcc gtcgccggaa 4320
ggtggtcgac ccgtccgcac ctgggcagca cgaaggcacg tgcaacgtca gcatggcggc 4380
actcgacaag ttcgttgcgg aacgcatctt caacaagatc aggcacgccg aaggcgacga 4440
agagacgttg gcgcttctgt gggaagccgc ccgacgcttc ggcaagctca ctgaggcgcc 4500
tgagaagagc ggcgaacggg cgaaccttgt tgcggagcgc gccgacgccc tgaacgccct 4560
tgaagagctg tacgaagacc gcgcggcagg cgcgtacgac ggacccgttg gcaggaagca 4620
cttccggaag caacaggcag cgctgacgct ccggcagcaa ggggcggaag agcggcttgc 4680
cgaacttgaa gccgccgaag ccccgaagct tccccttgac caatggttcc ccgaagacgc 4740
cgacgctgac ccgaccggcc ctaagtcgtg gtgggggcgc gcgtcagtag acgacaagcg 4800
cgtgttcgtc gggctcttcg tagacaagat cgttgtcacg aagtcgacta cgggcagggg 4860
gcagggaacg cccatcgaga agcgcgcttc gatcacgtgg gcgaagccgc cgaccgacga 4920
cgacgaagac gacgcccagg acggcacgga agacgtagcg gcgtagcgag acacccggga 4980
agcctgatct acgtctgtcg agaagtttct gatcgaaaag ttcgacagcg tctccgacct 5040
gatgcagctc tcgcagggcg aagaatctcg tgctttcagc ttcgatgtag gagggcgtgg 5100
atatgtcctg cgggtaaata gctgcgccga tggtttctac aaagatcgtt atgttgatcg 5160
gcactttgca tcggccgcgc tcccgattcc ggaagtgctt gacattgggg aatttatgcg 5220
gtgtgaaata ccgcacagat gcgtaaggag aaaataccgc atcaggcgcc attcgccatt 5280
caggctgcgc aactgttggg aagggcgatc ggtgcgggcc tcttcgctat tacgccagct 5340
ggcgaaaggg ggatgtgctg caaggcgatt aagttgggta acgccagggt tttcccagtc 5400
acgacgttgt aaaacgacgg ccagtgccaa gcttgggctg ca 5442

<210> 17
<211> 4957
<212> DNA
<213> Artificial

<220>
<223> partial sequence

<400> 17
atctacgtct gtcgagaagt ttctgatcga aaagttcgac agcgtctccg acctgatgca 60
gctctcgcag ggcgaagaat ctcgtgcttt cagcttcgat gtaggagggc gtggatatgt 120
cctgcgggta aatagctgcg ccgatggttt ctacaaagat cgttatgttg atcggcactt 180
tgcatcggcc gcgctcccga ttccggaagt gcttgacatt ggggaattta tgcggtgtga 240
aataccgcac agatgcgtaa ggagaaaata ccgcatcagg cgccattcgc cattcaggct 300
gcgcaactgt tgggaagggc gatcggtgcg ggcctcttcg ctattacgcc agctggcgaa 360
agggggatgt gctgcaaggc gattaagttg ggtaacgcca gggttttccc agtcacgacg 420
ttgtaaaacg acggccagtg ccaagcttgg gctgcaggtc gactctagag gatccgcggc 480
cgcgcgcgat atcgaattcg taatcatgtc atagctgttt cctgtgtgaa attgttatcc 540
gctcacaatt ccacacaaca tacgagccgg aagcataaag tgtaaagcct ggggtgccta 600
atgagtgagc taactcacat taattgcgtt gcgctcactg cccgctttcc agtcgggaaa 660
cctgtcgtgc cagctgcatt aatgaatcgg ccaacgcgcg gggagaggcg gtttgcgtat 720
tgggcgctct tccgcttcct cgctcactga ctcgctgcgc tcggtcgttc ggctgcggcg 780

agcggtatca gctcactcaa aggcggtaat acggttatcc acagaatcag gggataacgc 840
aggaaagaac atgtgagcaa aaggccagca aaaggccagg aaccgtaaaa aggccgcgtt 900
gctggcgttt ttccataggc tccgcccccc tgacgagcat cacaaaaatc gacgctcaag 960
tcagaggtgg cgaaacccga caggactata aagataccag gcgtttcccc ctggaagctc 1020
cctcgtgcgc tctcctgttc cgaccctgcc gcttaccgga tacctgtccg cctttctccc 1080
ttcgggaagc gtggcgcttt ctcatagctc acgctgtagg tatctcagtt cggtgtaggt 1140
cgttcgctcc aagctgggct gtgtgcacga accccccgtt cagcccgacc gctgcgcctt 1200
atccggtaac tatcgtcttg agtccaaccc ggtaagacac gacttatcgc cactggcagc 1260
agccactggt aacaggatta gcagagcgag gtatgtaggc ggtgctacag agttcttgaa 1320
gtggtggcct aactacggct acactagaag aacagtattt ggtatctgcg ctctgctgaa 1380
gccagttacc ttcggaaaaa gagttggtag ctcttgatcc ggcaaacaaa ccaccgctgg 1440
tagcggtggt ttttttgttt gcaagcagca gattacgcgc agaaaaaaag gatctcaaga 1500
agatcctttg atcttttcta cggggtctga cgctcagtgg aacgaaaact cacgttaagg 1560
gattttggtc atgagattat caaaaaggat cttcacctag atccttttgg ttcatgtgca 1620
gctccatcag caaaagggga tgataagttt atcaccaccg actatttgca acagtgccgt 1680
tgatcgtgct atgatcgact gatgtcatca gcggtggagt gcaatgtcgt gcaatacgaa 1740
tggcgaaaag ccgagctcat cggtcagctt ctcaaccttg gggttacccc cggcggtgtg 1800
ctgctggtcc acagctcctt ccgtagcgtc cggcccctcg aagatgggcc acttggactg 1860
atcgaggccc tgcgtgctgc gctgggtccg ggagggacgc tcgtcatgcc ctcgtggtca 1920
ggtctggacg acgagccgtt cgatcctgcc acgtcgcccg ttacaccgga ccttggagtt 1980
gtctctgaca cattctggcg cctgccaaat gtaaagcgca gcgcccatcc atttgccttt 2040
gcggcagcgg ggccacaggc agagcagatc atctctgatc cattgcccct gccacctcac 2100
tcgcctgcaa gcccggtcgc ccgtgtccat gaactcgatg ggcaggtact tctcctcggc 2160
gtgggacacg atgccaacac gacgctgcat cttgccgagt tgatggcaaa ggttccctat 2220
ggggtgccga gacactgcac cattcttcag gatggcaagt tggtacgcgt cgattatctc 2280
gagaatgacc actgctgtga gcgctttgcc ttggcggaca ggtggctcaa ggagaagagc 2340
cttcagaagg aaggtccagt cggtcatgcc tttgctcggt tgatccgctc ccgcgacatt 2400
gtggcgacag ccctgggtca actgggccga gatccgttga tcttcctgca tccgccagag 2460
gcgggatgcg aagaatgcga tgccgctcgc cagtcgattg gctgagctca tgagcggaga 2520
acgagatgac gttggagggg caaggtcgcg ctgattgctg gggcaacacg tggagcggat 2580
cggggattgt ctttcttcag ctcgctgatg atatgctgac gctcaatgcc gtttggcctc 2640
cgactaacga aaatcccgca tttggacggc tgatccgatt ggcacggcgg acggcgaatg 2700
gcggagcaga cgctcgtccg ggggcaatga gatatgaaaa agcctgaact caccgcgacg 2760
tatcgggccc tggccagcta gctagagtcg acctgcaggc atgcccagtt tctcgaagag 2820
aaaccggtaa gtgcgccctc ccgcatgcaa gctctagcga ttccagacgt cccgaaggcg 2880
tggcgcggct tccccgtgcc ggagcaatcg ccctgggtgg gttacacgac gcccctctat 2940
ggcccgtact gacggacaca ccgaagcccc ggcggcaacc ctcagcggat gccccggggc 3000
ttcacgtttt cccaggtcag aagcggtttt cgggagtagt gccccaactg gggtaacctt 3060
tgagttctct cagttggggg cgtagggtcg ccgacatgac acaaggggtt gtgaccgggg 3120
tggacacgta cgcgggtgct tacgaccgtc agtcgcgcga gcgcgaaaat tcgagcgcag 3180
caagcccagc gacacagcgt agcgccaacg aagacaaggc ggccgacctt cagcgcgaag 3240
tcgagcgcga cgggggccgg ttcaggttcg tcgggcattt cagcgaagcg ccgggcacgt 3300
cggcgttcgg gacggcggag cgcccggagt tcgaacgcat cctgaacgaa tgccgcgccg 3360

ggcggctcaa catgatcatt gtctatgacg tgtcgcgctt ctcgcgcctg aaggtcatgg 3420
acgcgattcc gattgtctcg gaattgctcg ccctgggcgt gacgattgtt tccactcagg 3480
aaggcgtctt ccggcaggga aacgtcatgg acctgattca cctgattatg cggctcgacg 3540
cgtcgcacaa agaatcttcg ctgaagtcgg cgaagattct cgacacgaag aaccttcagc 3600
gcgaattggg cgggtacgtc ggcgggaagg cgccttacgg cttcgagctt gtttcggaga 3660
cgaaggagat cacgcgcaac ggccgaatgg tcaatgtcgt catcaacaag cttgcgcact 3720
cgaccactcc ccttaccgga cccttcgagt tcgagcccga cgtaatccgg tggtggtggc 3780
gtgagatcaa gacgcacaaa caccttccct tcaagccggg cagtcaagcc gccattcacc 3840
cgggcagcat cacggggctt tgtaagcgca tggacgctga cgccgtgccg acccggggcg 3900
agacgattgg gaagaagacc gcttcaagcg cctgggaccc ggcaaccgtt atgcgaatcc 3960
ttcgggaccc gcgtattgcg ggcttcgccg ctgaggtgat ctacaagaag aagccggacg 4020
gcacgccgac cacgaagatt gagggttacc gcattcagcg cgacccgatc acgctccggc 4080
cggtcgagct tgattgcgga ccgatcatcg agcccgctga gtggtatgag cttcaggcgt 4140
ggttggacgg cagggggcgc ggcaaggggc tttcccgggg gcaagccatt ctgtccgcca 4200
tggacaagct gtactgcgag tgtggcgccg tcatgacttc gaagcgcggg gaagaatcga 4260
tcaaggactc ttaccgctgc cgtcgccgga aggtggtcga cccgtccgca cctgggcagc 4320
acgaaggcac gtgcaacgtc agcatggcgg cactcgacaa gttcgttgcg gaacgcatct 4380
tcaacaagat caggcacgcc gaaggcgacg aagagacgtt ggcgcttctg tgggaagccg 4440
cccgacgctt cggcaagctc actgaggcgc ctgagaagag cggcgaacgg gcgaaccttg 4500
ttgcggagcg cgccgacgcc ctgaacgccc ttgaagagct gtacgaagac cgcgcggcag 4560
gcgcgtacga cggacccgtt ggcaggaagc acttccggaa gcaacaggca gcgctgacgc 4620
tccggcagca aggggcggaa gagcggcttg ccgaacttga agccgccgaa gccccgaagc 4680
ttccccttga ccaatggttc cccgaagacg ccgacgctga cccgaccggc cctaagtcgt 4740
ggtgggggcg cgcgtcagta gacgacaagc gcgtgttcgt cgggctcttc gtagacaaga 4800
tcgttgtcac gaagtcgact acgggcaggg ggcagggaac gcccatcgag aagcgcgctt 4860
cgatcacgtg ggcgaagccg ccgaccgacg acgacgaaga cgacgcccag gacggcacgg 4920
aagacgtagc ggcgtagcga gacacccggg aagcctg 4957

<210> 18
<211> 4957
<212> DNA
<213> Artificial

<220>
<223> partial sequence

<400> 18
atctacgtct gtcgagaagt ttctgatcga aaagttcgac agcgtctccg acctgatgca 60
gctctcgcag ggcgaagaat ctcgtgcttt cagcttcgat gtaggagggc gtggatatgt 120
cctgcgggta aatagctgcg ccgatggttt ctacaaagat cgttatgttg atcggcactt 180
tgcatcggcc gcgctcccga ttccggaagt gcttgacatt ggggaattta tgcggtgtga 240
aataccgcac agatgcgtaa ggagaaaata ccgcatcagg cgccattcgc cattcaggct 300
gcgcaactgt tgggaagggc gatcggtgcg ggcctcttcg ctattacgcc agctggcgaa 360
agggggatgt gctgcaaggc gattaagttg ggtaacgcca gggttttccc agtcacgacg 420
ttgtaaaacg acggccagtg ccaagcttgg gctgcaggtc gactctagag gatccgcggc 480
cgcgcgcgat atcgaattcg taatcatgtc atagctgttt cctgtgtgaa attgttatcc 540
gctcacaatt ccacacaaca tacgagccgg aagcataaag tgtaaagcct ggggtgccta 600
atgagtgagc taactcacat taattgcgtt gcgctcactg cccgctttcc agtcgggaaa 660

cctgtcgtgc cagctgcatt aatgaatcgg ccaacgcgcg gggagaggcg gtttgcgtat 720
tgggcgctct tccgcttcct cgctcactga ctcgctgcgc tcggtcgttc ggctgcggcg 780
agcggtatca gctcactcaa aggcggtaat acggttatcc acagaatcag gggataacgc 840
aggaaagaac atgtgagcaa aaggccagca aaaggccagg aaccgtaaaa aggccgcgtt 900
gctggcgttt ttccataggc tccgcccccc tgacgagcat cacaaaaatc gacgctcaag 960
tcagaggtgg cgaaacccga caggactata aagataccag gcgtttcccc ctggaagctc 1020
cctcgtgcgc tctcctgttc cgaccctgcc gcttaccgga tacctgtccg cctttctccc 1080
ttcgggaagc gtggcgcttt ctcatagctc acgctgtagg tatctcagtt cggtgtaggt 1140
cgttcgctcc aagctgggct gtgtgcacga accccccgtt cagcccgacc gctgcgcctt 1200
atccggtaac tatcgtcttg agtccaaccc ggtaagacac gacttatcgc cactggcagc 1260
agccactggt aacaggatta gcagagcgag gtatgtaggc ggtgctacag agttcttgaa 1320
gtggtggcct aactacggct acactagaag aacagtattt ggtatctgcg ctctgctgaa 1380
gccagttacc ttcggaaaaa gagttggtag ctcttgatcc ggcaaacaaa ccaccgctgg 1440
tagcggtggt ttttttgttt gcaagcagca gattacgcgc agaaaaaaag gatctcaaga 1500
agatcctttg atcttttcta cggggtctga cgctcagtgg aacgaaaact cacgttaagg 1560
gattttggtc atgagattat caaaaaggat cttcacctag atccttttgg ttcatgtgca 1620
gctccatcag caaaagggga tgataagttt atcaccaccg actatttgca acagtgccgt 1680
tgatcgtgct atgatcgact gatgtcatca gcggtggagt gcaatgtcgt gcaatacgaa 1740
tggcgaaaag ccgagctcat cggtcagctt ctcaaccttg gggttacccc cggcggtgtg 1800
ctgctggtcc acagctcctt ccgtagcgtc cggcccctcg aagatgggcc acttggactg 1860
atcgaggccc tgcgtgctgc gctgggtccg ggagggacgc tcgtcatgcc ctcgtggtca 1920
ggtctggacg acgagccgtt cgatcctgcc acgtcgcccg ttacaccgga ccttggagtt 1980
gtctctgaca cattctggcg cctgccaaat gtaaagcgca gcgcccatcc atttgccttt 2040
gcggcagcgg ggccacaggc agagcagatc atctctgatc cattgcccct gccacctcac 2100
tcgcctgcaa gcccggtcgc ccgtgtccat gaactcgatg ggcaggtact tctcctcggc 2160
gtgggacacg atgccaacac gacgctgcat cttgccgagt tgatggcaaa ggttccctat 2220
ggggtgccga gacactgcac cattcttcag gatggcaagt tggtacgcgt cgattatctc 2280
gagaatgacc actgctgtga gcgctttgcc ttggcggaca ggtggctcaa ggagaagagc 2340
cttcagaagg aaggtccagt cggtcatgcc tttgctcggt tgatccgctc ccgcgacatt 2400
gtggcgacag ccctgggtca actgggccga gatccgttga tcttcctgca tccgccagag 2460
gcgggatgcg aagaatgcga tgccgctcgc cagtcgattg gctgagctca tgagcggaga 2520
acgagatgac gttggagggg caaggtcgcg ctgattgctg gggcaacacg tggagcggat 2580
cggggattgt ctttcttcag ctcgctgatg atatgctgac gctcaatgcc gtttggcctc 2640
cgactaacga aaatcccgca tttggacggc tgatccgatt ggcacggcgg acggcgaatg 2700
gcggagcaga cgctcgtccg ggggcaatga gatatgaaaa agcctgaact caccgcgacg 2760
tatcgggccc tggccagcta gctagagtcg acctgcaggc atgcgggagg gcgcacttac 2820
cggtttctct tcgagaaact gggcatgcaa gctctagcga ttccagacgt cccgaaggcg 2880
tggcgcggct tccccgtgcc ggagcaatcg ccctgggtgg gttacacgac gcccctctat 2940
ggcccgtact gacggacaca ccgaagcccc ggcggcaacc ctcagcggat gccccggggc 3000
ttcacgtttt cccaggtcag aagcggtttt cgggagtagt gccccaactg gggtaacctt 3060
tgagttctct cagttggggg cgtagggtcg ccgacatgac acaaggggtt gtgaccgggg 3120
tggacacgta cgcgggtgct tacgaccgtc agtcgcgcga gcgcgaaaat tcgagcgcag 3180
caagcccagc gacacagcgt agcgccaacg aagacaaggc ggccgacctt cagcgcgaag 3240

tcgagcgcga cgggggccgg ttcaggttcg tcgggcattt cagcgaagcg ccgggcacgt 3300
cggcgttcgg gacggcggag cgcccggagt tcgaacgcat cctgaacgaa tgccgcgccg 3360
ggcggctcaa catgatcatt gtctatgacg tgtcgcgctt ctcgcgcctg aaggtcatgg 3420
acgcgattcc gattgtctcg gaattgctcg ccctgggcgt gacgattgtt tccactcagg 3480
aaggcgtctt ccggcaggga aacgtcatgg acctgattca cctgattatg cggctcgacg 3540
cgtcgcacaa agaatcttcg ctgaagtcgg cgaagattct cgacacgaag aaccttcagc 3600
gcgaattggg cgggtacgtc ggcgggaagg cgccttacgg cttcgagctt gtttcggaga 3660
cgaaggagat cacgcgcaac ggccgaatgg tcaatgtcgt catcaacaag cttgcgcact 3720
cgaccactcc ccttaccgga cccttcgagt tcgagcccga cgtaatccgg tggtggtggc 3780
gtgagatcaa gacgcacaaa caccttccct tcaagccggg cagtcaagcc gccattcacc 3840
cgggcagcat cacggggctt tgtaagcgca tggacgctga cgccgtgccg acccggggcg 3900
agacgattgg gaagaagacc gcttcaagcg cctgggaccc ggcaaccgtt atgcgaatcc 3960
ttcgggaccc gcgtattgcg ggcttcgccg ctgaggtgat ctacaagaag aagccggacg 4020
gcacgccgac cacgaagatt gagggttacc gcattcagcg cgacccgatc acgctccggc 4080
cggtcgagct tgattgcgga ccgatcatcg agcccgctga gtggtatgag cttcaggcgt 4140
ggttggacgg cagggggcgc ggcaaggggc tttcccgggg gcaagccatt ctgtccgcca 4200
tggacaagct gtactgcgag tgtggcgccg tcatgacttc gaagcgcggg gaagaatcga 4260
tcaaggactc ttaccgctgc cgtcgccgga aggtggtcga cccgtccgca cctgggcagc 4320
acgaaggcac gtgcaacgtc agcatggcgg cactcgacaa gttcgttgcg gaacgcatct 4380
tcaacaagat caggcacgcc gaaggcgacg aagagacgtt ggcgcttctg tgggaagccg 4440
cccgacgctt cggcaagctc actgaggcgc ctgagaagag cggcgaacgg gcgaaccttg 4500
ttgcggagcg cgccgacgcc ctgaacgccc ttgaagagct gtacgaagac cgcgcggcag 4560
gcgcgtacga cggacccgtt ggcaggaagc acttccggaa gcaacaggca gcgctgacgc 4620
tccggcagca aggggcggaa gagcggcttg ccgaacttga agccgccgaa gccccgaagc 4680
ttccccttga ccaatggttc cccgaagacg ccgacgctga cccgaccggc cctaagtcgt 4740
ggtgggggcg cgcgtcagta gacgacaagc gcgtgttcgt cgggctcttc gtagacaaga 4800
tcgttgtcac gaagtcgact acgggcaggg ggcagggaac gcccatcgag aagcgcgctt 4860
cgatcacgtg ggcgaagccg ccgaccgacg acgacgaaga cgacgcccag gacggcacgg 4920
aagacgtagc ggcgtagcga gacacccggg aagcctg 4957

<210> 19
<211> 5062
<212> DNA
<213> Artificial

<220>
<223> partial sequence

<400> 19
atctacgtct gtcgagaagt ttctgatcga aaagttcgac agcgtctccg acctgatgca 60
gctctcgcag ggcgaagaat ctcgtgcttt cagcttcgat gtaggagggc gtggatatgt 120
cctgcgggta aatagctgcg ccgatggttt ctacaaagat cgttatgttg atcggcactt 180
tgcatcggcc gcgctcccga ttccggaagt gcttgacatt ggggaattta tgcggtgtga 240
aataccgcac agatgcgtaa ggagaaaata ccgcatcagg cgccattcgc cattcaggct 300
gcgcaactgt tgggaagggc gatcggtgcg ggcctcttcg ctattacgcc agctggcgaa 360
agggggatgt gctgcaaggc gattaagttg ggtaacgcca gggttttccc agtcacgacg 420
ttgtaaaacg acggccagtg ccaagcttgg gctgcaggtc gactctagag gatccgcggc 480
cgcgcgcgat atcgaattcg taatcatgtc atagctgttt cctgtgtgaa attgttatcc 540

gctcacaatt ccacacaaca tacgagccgg aagcataaag tgtaaagcct ggggtgccta 600
atgagtgagc taactcacat taattgcgtt gcgctcactg cccgctttcc agtcgggaaa 660
cctgtcgtgc cagctgcatt aatgaatcgg ccaacgcgcg gggagaggcg gtttgcgtat 720
tgggcgctct tccgcttcct cgctcactga ctcgctgcgc tcggtcgttc ggctgcggcg 780
agcggtatca gctcactcaa aggcggtaat acggttatcc acagaatcag gggataacgc 840
aggaaagaac atgtgagcaa aaggccagca aaaggccagg aaccgtaaaa aggccgcgtt 900
gctggcgttt ttccataggc tccgcccccc tgacgagcat cacaaaaatc gacgctcaag 960
tcagaggtgg cgaaacccga caggactata aagataccag gcgtttcccc ctggaagctc 1020
cctcgtgcgc tctcctgttc cgaccctgcc gcttaccgga tacctgtccg cctttctccc 1080
ttcgggaagc gtggcgcttt ctcatagctc acgctgtagg tatctcagtt cggtgtaggt 1140
cgttcgctcc aagctgggct gtgtgcacga accccccgtt cagcccgacc gctgcgcctt 1200
atccggtaac tatcgtcttg agtccaaccc ggtaagacac gacttatcgc cactggcagc 1260
agccactggt aacaggatta gcagagcgag gtatgtaggc ggtgctacag agttcttgaa 1320
gtggtggcct aactacggct acactagaag aacagtattt ggtatctgcg ctctgctgaa 1380
gccagttacc ttcggaaaaa gagttggtag ctcttgatcc ggcaaacaaa ccaccgctgg 1440
tagcggtggt ttttttgttt gcaagcagca gattacgcgc agaaaaaaag gatctcaaga 1500
agatcctttg atcttttcta cggggtctga cgctcagtgg aacgaaaact cacgttaagg 1560
gattttggtc atgagattat caaaaaggat cttcacctag atccttttgg ttcatgtgca 1620
gctccatcag caaaagggga tgataagttt atcaccaccg actatttgca acagtgccgt 1680
tgatcgtgct atgatcgact gatgtcatca gcggtggagt gcaatgtcgt gcaatacgaa 1740
tggcgaaaag ccgagctcat cggtcagctt ctcaaccttg gggttacccc cggcggtgtg 1800
ctgctggtcc acagctcctt ccgtagcgtc cggcccctcg aagatgggcc acttggactg 1860
atcgaggccc tgcgtgctgc gctgggtccg ggagggacgc tcgtcatgcc ctcgtggtca 1920
ggtctggacg acgagccgtt cgatcctgcc acgtcgcccg ttacaccgga ccttggagtt 1980
gtctctgaca cattctggcg cctgccaaat gtaaagcgca gcgcccatcc atttgccttt 2040
gcggcagcgg ggccacaggc agagcagatc atctctgatc cattgcccct gccacctcac 2100
tcgcctgcaa gcccggtcgc ccgtgtccat gaactcgatg ggcaggtact tctcctcggc 2160
gtgggacacg atgccaacac gacgctgcat cttgccgagt tgatggcaaa ggttccctat 2220
ggggtgccga gacactgcac cattcttcag gatggcaagt tggtacgcgt cgattatctc 2280
gagaatgacc actgctgtga gcgctttgcc ttggcggaca ggtggctcaa ggagaagagc 2340
cttcagaagg aaggtccagt cggtcatgcc tttgctcggt tgatccgctc ccgcgacatt 2400
gtggcgacag ccctgggtca actgggccga gatccgttga tcttcctgca tccgccagag 2460
gcgggatgcg aagaatgcga tgccgctcgc cagtcgattg gctgagctca tgagcggaga 2520
acgagatgac gttggagggg caaggtcgcg ctgattgctg gggcaacacg tggagcggat 2580
cggggattgt ctttcttcag ctcgctgatg atatgctgac gctcaatgcc gtttggcctc 2640
cgactaacga aaatcccgca tttggacggc tgatccgatt ggcacggcgg acggcgaatg 2700
gcggagcaga cgctcgtccg ggggcaatga gatatgaaaa agcctgaact caccgcgacg 2760
tatcgggccc tggccagcta gctagagtcg acctgcaggc atgcgcgcct tttatgcttg 2820
ttgcaaagtg catctagtgt gcctaaactg ctgatatgat agtagagcgg ttgagcaata 2880
caggatgacc gttgtaacac acatggcttc gccacgtggt tccaacggac gcctgcccat 2940
tcggcgcgca tgcaagctct agcgattcca gacgtcccga aggcgtggcg cggcttcccc 3000
gtgccggagc aatcgccctg ggtgggttac acgacgcccc tctatggccc gtactgacgg 3060
acacaccgaa gccccggcgg caaccctcag cggatgcccc ggggcttcac gttttcccag 3120

gtcagaagcg gttttcggga gtagtgcccc aactggggta acctttgagt tctctcagtt 3180
gggggcgtag ggtcgccgac atgacacaag gggttgtgac cggggtggac acgtacgcgg 3240
gtgcttacga ccgtcagtcg cgcgagcgcg aaaattcgag cgcagcaagc ccagcgacac 3300
agcgtagcgc caacgaagac aaggcggccg accttcagcg cgaagtcgag cgcgacgggg 3360
gccggttcag gttcgtcggg catttcagcg aagcgccggg cacgtcggcg ttcgggacgg 3420
cggagcgccc ggagttcgaa cgcatcctga acgaatgccg cgccgggcgg ctcaacatga 3480
tcattgtcta tgacgtgtcg cgcttctcgc gcctgaaggt catggacgcg attccgattg 3540
tctcggaatt gctcgccctg ggcgtgacga ttgtttccac tcaggaaggc gtcttccggc 3600
agggaaacgt catggacctg attcacctga ttatgcggct cgacgcgtcg cacaaagaat 3660
cttcgctgaa gtcggcgaag attctcgaca cgaagaacct tcagcgcgaa ttgggcgggt 3720
acgtcggcgg gaaggcgcct tacggcttcg agcttgtttc ggagacgaag gagatcacgc 3780
gcaacggccg aatggtcaat gtcgtcatca acaagcttgc gcactcgacc actcccctta 3840
ccggaccctt cgagttcgag cccgacgtaa tccggtggtg gtggcgtgag atcaagacgc 3900
acaaacacct tcccttcaag ccgggcagtc aagccgccat tcacccgggc agcatcacgg 3960
ggctttgtaa gcgcatggac gctgacgccg tgccgacccg gggcgagacg attgggaaga 4020
agaccgcttc aagcgcctgg gacccggcaa ccgttatgcg aatccttcgg gacccgcgta 4080
ttgcgggctt cgccgctgag gtgatctaca agaagaagcc ggacggcacg ccgaccacga 4140
agattgaggg ttaccgcatt cagcgcgacc cgatcacgct ccggccggtc gagcttgatt 4200
gcggaccgat catcgagccc gctgagtggt atgagcttca ggcgtggttg gacggcaggg 4260
ggcgcggcaa ggggctttcc cgggggcaag ccattctgtc cgccatggac aagctgtact 4320
gcgagtgtgg cgccgtcatg acttcgaagc gcggggaaga atcgatcaag gactcttacc 4380
gctgccgtcg ccggaaggtg gtcgacccgt ccgcacctgg gcagcacgaa ggcacgtgca 4440
acgtcagcat ggcggcactc gacaagttcg ttgcggaacg catcttcaac aagatcaggc 4500
acgccgaagg cgacgaagag acgttggcgc ttctgtggga agccgcccga cgcttcggca 4560
agctcactga ggcgcctgag aagagcggcg aacgggcgaa ccttgttgcg gagcgcgccg 4620
acgccctgaa cgcccttgaa gagctgtacg aagaccgcgc ggcaggcgcg tacgacggac 4680
ccgttggcag gaagcacttc cggaagcaac aggcagcgct gacgctccgg cagcaagggg 4740
cggaagagcg gcttgccgaa cttgaagccg ccgaagcccc gaagcttccc cttgaccaat 4800
ggttccccga agacgccgac gctgacccga ccggccctaa gtcgtggtgg gggcgcgcgt 4860
cagtagacga caagcgcgtg ttcgtcgggc tcttcgtaga caagatcgtt gtcacgaagt 4920
cgactacggg cagggggcag ggaacgccca tcgagaagcg cgcttcgatc acgtgggcga 4980
agccgccgac cgacgacgac gaagacgacg cccaggacgg cacggaagac gtagcggcgt 5040
agcgagacac ccgggaagcc tg 5062

<210> 20
<211> 44
<212> DNA
<213> artificial

<220>
<223> Primer

<400> 20
cgggagggcg cacttaccgg tttctcttcg agaaactggg catg 44

<210> 21
<211> 44
<212> DNA
<213> artificial

<220>

<223>  Primer

<400>  21
cccagtttct cgaagagaaa ccggtaagtg cgccctcccg catg                    44


<210>  22
<211>  31
<212>  DNA
<213>  artificial

<220>
<223>  Primer

<400>  22
ttactgatgc atgcgcgccg aatgggcagg c                                  31


<210>  23
<211>  40
<212>  DNA
<213>  artificial

<220>
<223>  Primer

<400>  23
ttactgatgc atgcgcgcct tttatgcttg ttgcaaagtg                         40


<210>  24
<211>  33
<212>  DNA
<213>  artificial

<220>
<223>  Primer

<400>  24
ttatgcataa gcttgctggg gccggtgtca agg                                33


<210>  25
<211>  42
<212>  DNA
<213>  artificial

<220>
<223>  Primer

<400>  25
ttactgataa gcttcaagcg cctccaatcc aattaagggg ac                      42


<210>  26
<211>  31
<212>  DNA
<213>  artificial

<220>
<223>  Primer

<400>  26
ctccattgca tgctccttag ctcctgatgt t                                  31


<210>  27
<211>  30
<212>  DNA
<213>  artificial

<220>
<223>  Primer

<400>  27
ttcatagcat gcgcctgact gcgttagcaa                                    30


<210>  28
<211>  21
<212>  DNA
<213>  artificial

<220>
<223>  Primer

<400>  28
tgacgctcaa tgccgtttgg c                                                21

<210>  29
<211>  19
<212>  DNA
<213>  artificial

<220>
<223>  Primer

<400>  29
cagttggggc actactccc                                                   19

<210>  30
<211>  18
<212>  DNA
<213>  artificial

<220>
<223>  Primer

<400>  30
gtgcgtcacg actgggtc                                                    18

<210>  31
<211>  42
<212>  DNA
<213>  artificial

<220>
<223>  Primer

<400>  31
ttaattcatg aactagtcat gtattcggct gcaactttgt ca                         42

<210>  32
<211>  28
<212>  DNA
<213>  artificial

<220>
<223>  Primer

<400>  32
ctcgaggcat aggcctatcg tttccacg                                         28

<210>  33
<211>  13
<212>  DNA
<213>  artificial

<220>
<223>  Primer

<400>  33
ccccatgcat tcg                                                         13

<210>  34
<211>  17
<212>  DNA
<213>  artificial

<220>
<223>  Primer

<400>  34
cgaatgcatg gggtgca                                                     17

<210>  35
<211>  18
<212>  DNA
<213>  artificial

<220>
<223>  Primer

<400>  35
gatccggcgc gccgagct                                                    18

<210> 36
<211> 10
<212> DNA
<213> artificial

<220>
<223> Primer

<400> 36
cggcgcgccg 10

<210> 37
<211> 27
<212> DNA
<213> artificial

<220>
<223> Primer

<400> 37
ctccgctcat atgcgagggc ttctctg 27

<210> 38
<211> 26
<212> DNA
<213> artificial

<220>
<223> Primer

<400> 38
ggcatgcata tgtcacgctg ccgcaa 26